# EUROPEAN PATENT APPLICATION

(11) **EP 4 844 060 A1**
(43) Date of publication of application: **25.11.2026**
(21) Application number: 25178355.1
(22) Date of filing: 22.05.2025
(51) Int. Cl.: C12N 9/24, C12N 9/22

(54) **CELL PENETRATING PEPTIDES FOR TRANSPORTING CARGO**

(71) Applicant: Ntrans Technologies BV, 2333CH Leiden (NL)
(72) Inventor: The designation of the inventor has not yet been filed
(74) Representative: Carpmaels & Ransford LLP

(57) **Abstract**

Provided herein are cell penetrating peptides (CPPs) derived from fungal glycoside hydrolase enzymes for transporting cargo, such as cargo for gene editing applications, across a cell membrane. Also provided herein are methods, pharmaceutical compositions, and kits comprising said CPPs. Such CPPs, methods, pharmaceutical compositions, and kits are useful in therapy, both *in vivo* and *ex vivo,* and diagnosis. Also provided herein are methods for preparing said CPPs using host cells, vectors, and encoding nucleic acids.

## Description

### TECHNICAL FIELD

This invention relates to cell penetrating peptides (CPPs) derived from fungal glycoside hydrolases, and to pharmaceutical compositions and kits comprising the same. The invention also relates to methods for transporting cargo across a cell membrane using the CPPs of the invention, including cargo for gene editing applications, and to methods for preparing the CPPs of the invention, using host cells, vectors, and nucleic acids encoding the CPPs. The invention also relates to uses of the CPPs in therapy, both *in vivo* and *ex vivo,* and diagnosis.

### BACKGROUND

Many biological macromolecules, such as proteins, peptides, and nucleic acids, exhibit potent and specific biological activities, making them highly attractive as therapeutic agents and research tools. However, the clinical and experimental utility of these macromolecules is often limited by their poor membrane permeability and inability to access intracellular targets.

CPPs have emerged as a promising solution for intracellular delivery of diverse cargo types, including both macromolecules such as proteins, peptides, and nucleic acids, and also other types of cargo such as small molecules, nanoparticles, and viral vectors. CPPs are short peptides, typically comprising 8 to 30 amino acids, that facilitate the transport of their associated cargo across a cell membrane (Váňová et al., Int. J. Pharm., 2020, 576, 119008; Ruseska et al., Beilstein J Nanotechnol., 2020, 11, 101-123). The discovery of CPPs dates back to the late 1980s, when the trans-activator of transcription (TAT) protein from HIV was found to cross biological membranes (Frankel et al., Cell, 1988, 55(6), 1189-1193). Subsequent studies identified a key internal sequence, the TAT protein transduction domain (PTD; residues 48-57), as responsible for this activity (Vives et al., J. Biol. Chem., 1997, 272(25), 16010-7). Additional naturally occurring CPPs were later discovered, including the Antennapedia-derived peptide from Drosophila (Derossi et al., J. Biol. Chem., 1994, 269, 10444-10450), suggesting that multiple peptide motifs can confer membrane-translocating properties.

Despite the early promise of CPPs, one of the major obstacles to their effective use has been endosomal entrapment, wherein the internalised CPP-cargo complex becomes sequestered within endosomal compartments, limiting cytosolic delivery. A notable advance came with the identification of the HA2 subunit of the influenza virus hemagglutinin (HA) protein, which facilitates pH-dependent membrane fusion. Wadia et al. demonstrated that fusion of the TAT sequence with the HA2 domain yielded a bifunctional peptide (TAT-HA2) combining membrane penetration and endosomal escape, significantly enhancing delivery efficiency (Wadia et al., Nat. Med., 2004, 10(3), 310-315).

Since the development of TAT-HA2, over 1700 CPPs have been identified and characterised (https://webs.iiitd.edu.in/raghava/cppsite/). These CPPs have been used to deliver a wide array of cargoes into cells, including peptides, proteins, antisense oligonucleotides, nanoparticles, and liposomes (Schwarze et al., Science., 1999, 285(5433), 1569-1572; Qian et al., Biochemistry., 2016, 55(18), 2601-2612; Patel et al., Sci. Rep., 2019, 9(1), 6298).

Building on this foundational work, recent efforts have focused on enhancing CPP performance, particularly by improving cellular uptake and endosomal escape. For example, cyclisation of CPPs, such as cyclic TAT, has been shown to improve membrane permeability and facilitate direct cytosolic delivery (Patel et al., Sci. Rep., 2019, 9(1), 6298; Qian et al., ACS. Chem. Biol., 2012, 8(2), 423-431). Cyclic CPPs interact with plasma membrane phospholipids, undergo endocytosis, and efficiently escape endosomes, potentially by inducing vesicle budding. These cyclic peptides are also more resistant to proteolysis and exhibit improved oral bioavailability, making them attractive for therapeutic applications.

More recently, CPPs have been employed for CRISPR/Cas9 gene editing system delivery (Foss et al., Nat. Biomed. Eng., 2023, 7(5), 647-660; Chen et al., Nat. Commun., 2024, 15(1727); Zhang et al., Nat. Biotechnol., 2024, 42(2), 305-315). The CRISPR/Cas9 system, consisting of a Cas9 nuclease and a guide RNA (gRNA), enables site-specific genome modification and has transformed biomedical research and gene therapy. Traditional delivery methods, including viral vectors and plasmids, can lead to prolonged Cas9 expression, increasing the risk of off-target effects. Direct delivery of pre-formed ribonucleoprotein (RNP) complexes is more transient and precise, but faces challenges with intracellular delivery. CPPs offer a non-viral alternative, with benefits including low toxicity, ease of synthesis, and the ability to form non-covalent or covalent complexes with RNPs for direct application to cells (Lino et al., Drug. Deliv., 2018, 25(1), 1234-1257).

Despite these advances, transduction efficiency of CPPs remains highly variable, influenced by the CPP sequence, cargo type, and target cell characteristics. Many CPPs perform inconsistently across different cell types or exhibit suboptimal intracellular release, limiting their broader application.

Accordingly, there remains a need for improved CPPs that exhibit enhanced transduction efficiency, robust intracellular release, and low cytotoxicity across a range of cell types. Such CPPs would enable more reliable delivery of therapeutic and research cargoes, including components for gene editing, for in vivo and ex vivo applications.

### SUMMARY OF THE INVENTION

The invention provides CPPs for transporting cargo across a cell membrane, wherein the amino acid sequence of the CPP comprises a 14 to 142 contiguous amino acid portion having at least 80% sequence identity to a same-length portion within a protein loop domain of a fungal glycoside hydrolase, wherein the protein loop domain of the fungal glycoside hydrolase is found between an N-terminal flagging sequence and a C-terminal flagging sequence, wherein the N-terminal flagging sequence is XWX₁XX₂X₃X₄X₅XXXXX₆X₇GX₈PX₉YX₁₀YXX₁₁XX₁₂XXXXLXXX₁₃ (SEQ ID NO: 7),
wherein:
X₁ is T, S, V or A;
X₂ is A or S;
X₃ is G or H;
X₄ is F or V;
X₅ is P, A, D or S;
X₆ is V, M, I or A;
X₇ is L, I or M;
X₈ is V, L or I;
X₉ is S or A;
X₁₀ is G or A;
X₁₁ is S, A, N, Q or V;
X₁₂ is S or T; and
X₁₃ is R or K,
wherein the C-terminal flagging sequence is XXXXX₁XX₂X₃X₄X(g)XXX₅X₆X₇QFX₈XX₉X₁₀XZX₁₂, wherein:
X₁ is N, S, G, D or E;
X₂ is D, -, G, A or E;
X₃ is G, -, S or A;
X₄ is G, -, T, S, P, K or H;
(g) is a gap of up to 6 amino acids;
X₅ is G, - or N;
X₆ is Q, T or G;
X₇ is V or I;
X₈ is R, N or S;
X₉ is L, I or M;
X₁₀ is V, I, L, A or F;
X₁₂ is G, A or K, and
wherein the CPP is capable of providing gene editing in a cell when covalently or non-covalently coupled to cargo for CRISPR/Cas9 gene editing, and wherein "-" represents a gap of one amino acid.

The invention also provides a CPP for transporting cargo across a cell membrane, wherein the amino acid sequence of the CPP comprises a contiguous amino acid portion having at least 80% sequence identity to the amino acid sequence of any one of SEQ ID NOs: 558-636, and wherein the CPP is capable of providing gene editing in a cell when covalently or non-covalently coupled to cargo for CRISPR/Cas9 gene editing.

The invention also provides a CPP for transporting cargo across a cell membrane, wherein the amino acid sequence of the CPP comprises a P2-P3 region of 27 contiguous amino acids that is at least 60% identical to the amino acid sequence of SEQ ID NO: 583, and wherein the CPP is capable of providing gene editing in a cell when covalently or non-covalently coupled to cargo for CRISPR/Cas9 gene editing.

The invention also provides a pharmaceutical composition comprising a CPP of the invention and one or more pharmaceutically acceptable carriers, diluents, and/or excipients.

The invention also provides a method for transporting cargo across a cell membrane into a cell using a CPP of the invention or a pharmaceutical composition of the invention, wherein the method comprises the step of contacting the cell with the CPP and the cargo, and wherein the CPP mediates the transport of the cargo across the cell membrane.

The invention also provides a CPP of the invention or a pharmaceutical composition of the invention for use in therapy, wherein the CPP transports cargo across a cell membrane into the cells of a subject, wherein the cargo has a therapeutic effect on the cells thereby treating, preventing, or ameliorating a disease or disorder in the subject.

The invention also provides a CPP of the invention or a pharmaceutical composition of the invention for use in a method of diagnosis, wherein the CPP transports diagnostic cargo across a cell membrane into the cells of a subject, wherein the diagnostic cargo is for the purpose of imaging, cell labelling, and/or cell tracking.

The invention also provides a nucleic acid encoding a CPP of the invention.

The invention also provides a vector comprising a nucleic acid of the invention.

The invention also provides a host cell comprising a vector of the invention.

The invention also provides a method of preparing a CPP of the invention comprising cultivating a host cell comprising a vector comprising a nucleic acid encoding the CPP in a culture medium and separating the CPP from the culture medium or from the host cell lysate after host cell lysis.

The invention also provides a kit comprising i) a CPP of the invention and ii) cargo, wherein the CPP and the cargo are for simultaneous, sequential or separate administration, optionally packaged in one or more containers.

### BRIEF DESCRIPTION OF THE FIGURES

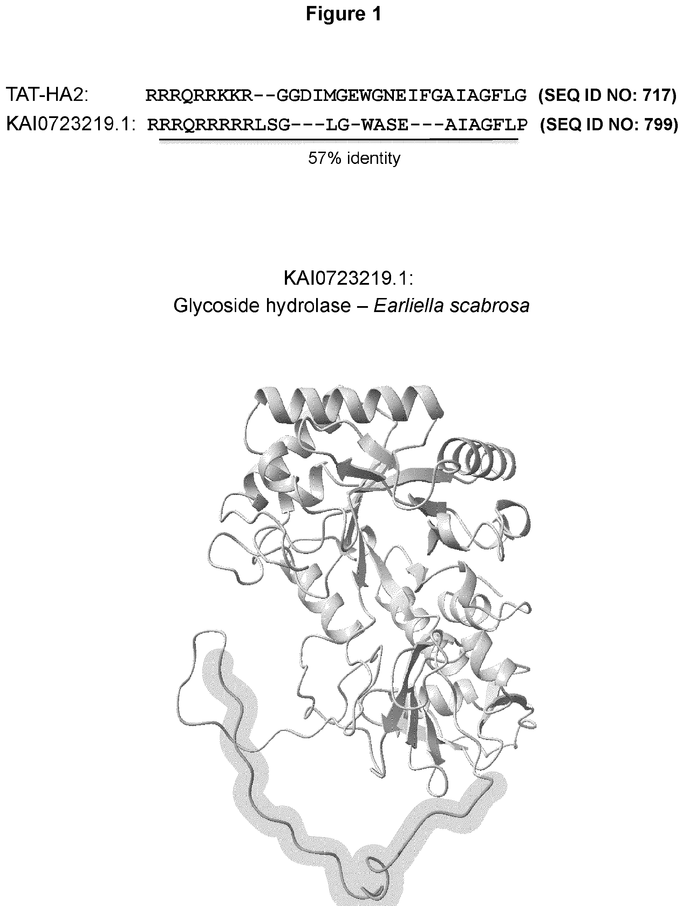

**Figure 1****:** Alignment of the TAT-HA2 sequence and part of a protein loop domain sequence from an exemplary glycoside hydrolase from *Earliella scabrosa* (GHES). The same protein loop domain is shown within the predicted 3D structure of the full GHES protein, highlighted with grey shading. 3D protein structure prediction using RosettaFold2.

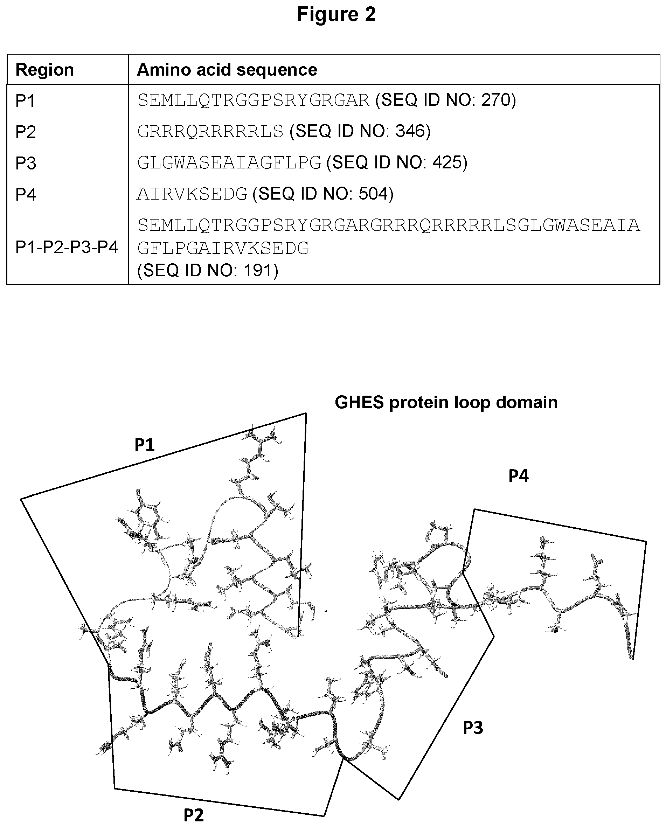

**Figure 2****:** Sequences of the P1, P2, P3, and P4 regions in the GHES protein loop domain. Identification of the P1, P2, P3, and P4 regions within the predicted structure of the GHES protein loop domain. 3D GHES protein loop domain predicted using RosettaFold2.

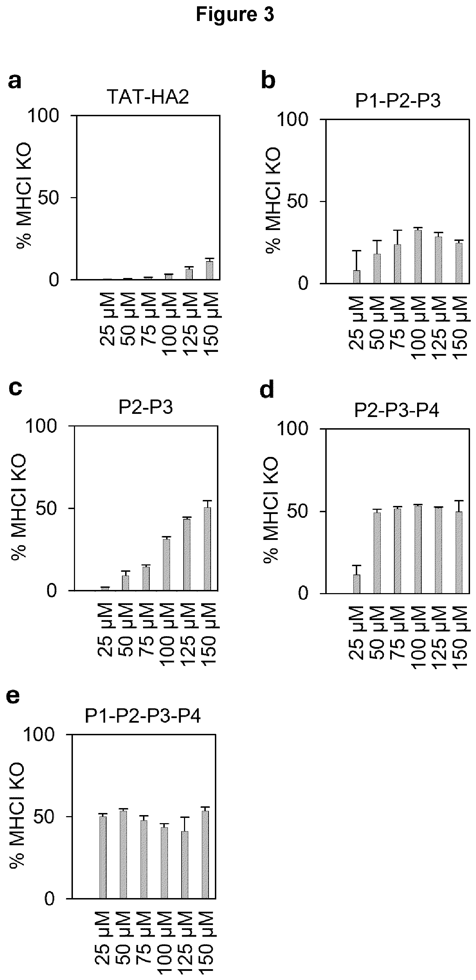

**Figure 3****:** Identification of functional CPP regions in the GHES protein loop domain sequence as assessed by MHCI knock-out percentage efficiency with increasing concentration of CPPs in Jurkat cells. **(a)** control TAT-HA2; **(b-e)** regions in the GHES protein loop domain. Error bars correspond to the standard deviation of the average of n=3 independent biological replicates.

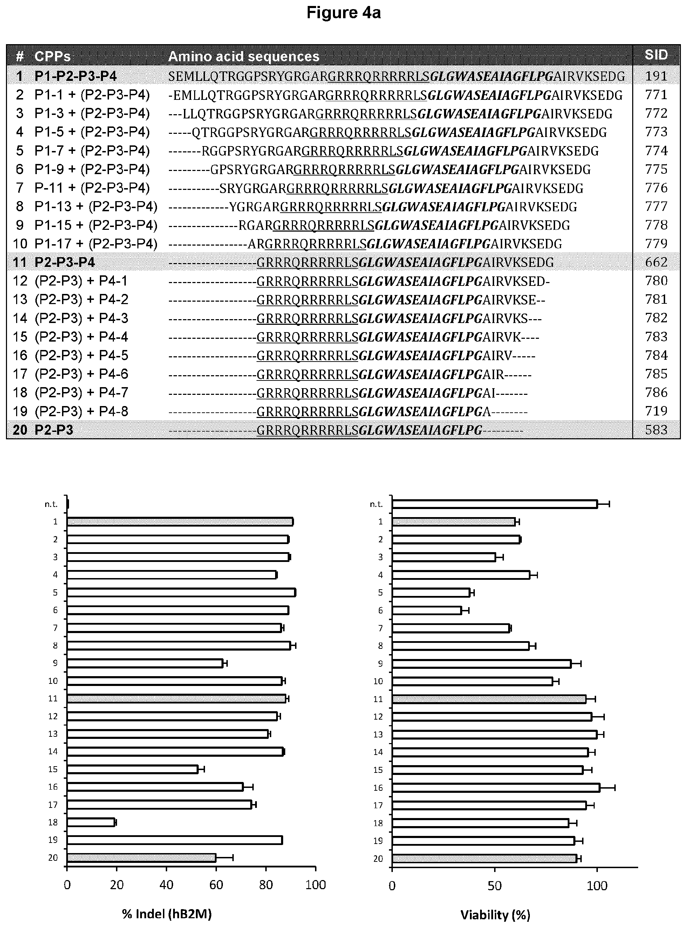

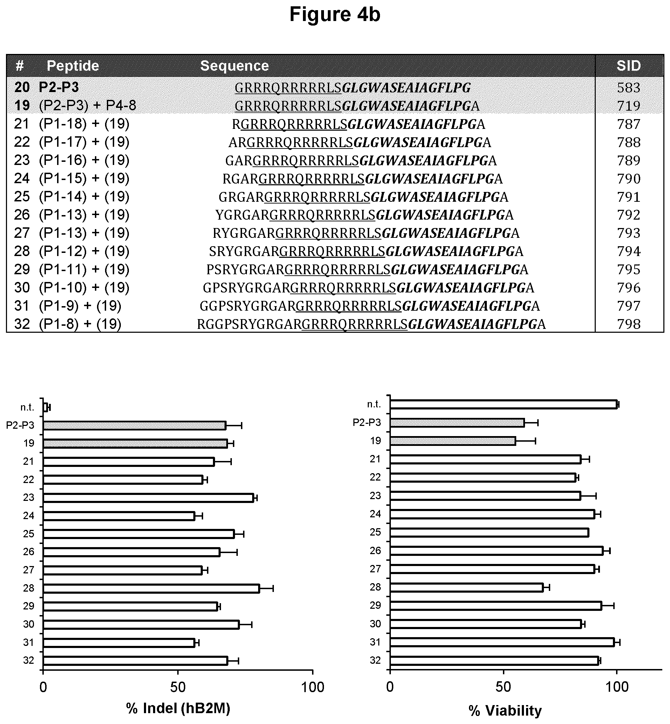

**Figures 4a** **and** **4b****:** Indel frequency in the human Beta-2-Microglobulin (B2M) gene and viability of CD8+ T-cells when using CPP variants derived from the GHES protein loop domain sequence. In the Tables, P2 regions are underlined, P3 regions are in bold italics, any part of the P1 region is positioned N-terminally to the P2 region, and any part of the P4 region is positioned C-terminally to the P3 region. Error bars correspond to the standard deviation of the average of n=3 independent biological replicates. n.t. = non-treated.

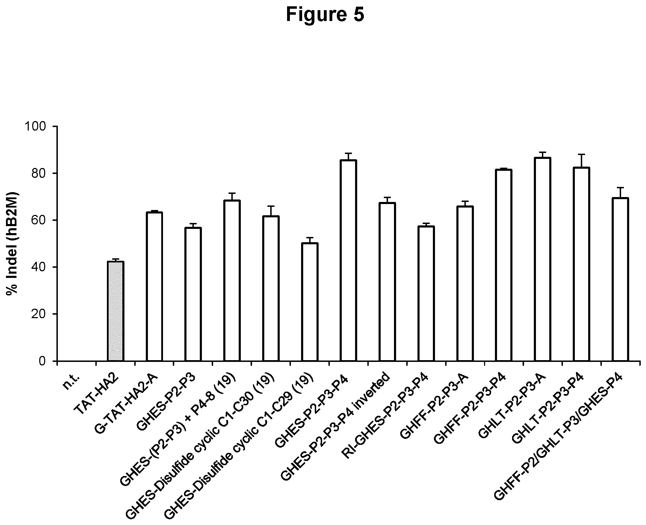

**Figure 5****:** Indel frequency in the human B2M gene in CD8+ T-cells when using the following peptides: TAT-HA2, GHES CPP variants, cyclisation of GHES peptides, chimeric peptide, inverted sequence, retro-inverso forms and addition of P4 in GHFF (glycoside hydrolase from *Fomes fomentarius*) and GHLT (glycoside hydrolase from *Lentinus tigrinus*), see Table 7 for sequences. Error bars correspond to the standard deviation of the average of n=3 independent biological replicates. n.t.= non-treated.

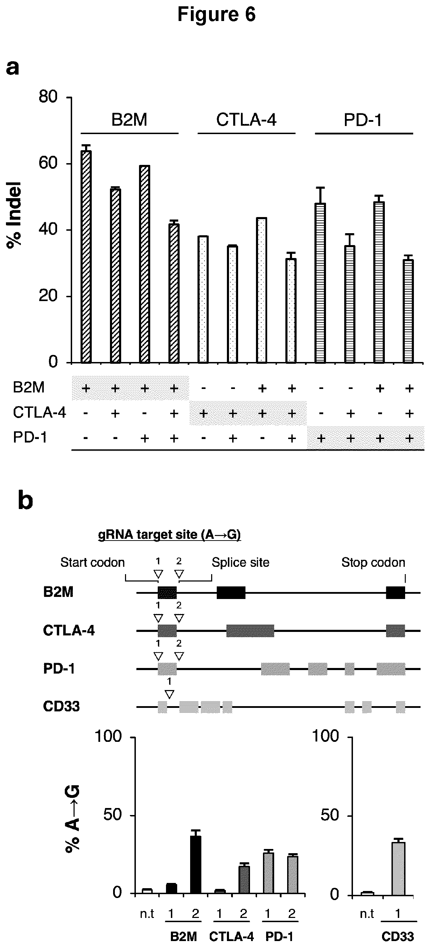

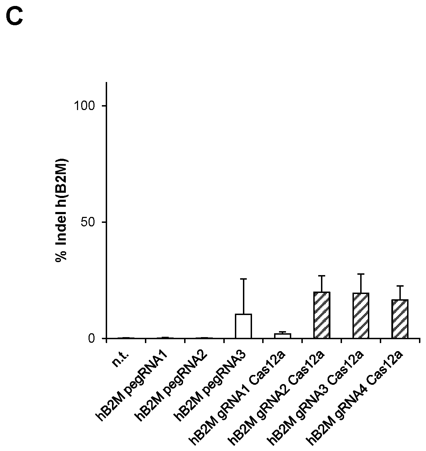

**Figures 6a, 6b** **and** **6c****: (a)** Editing efficiency of peptide GHES-P2-P3 + P4-8 (19) in CD8+ T-cells on the following endogenous genes: B2M, CTLA-4, and PD-1. Single, dual and triple guide RNAs combinations were tested for each target gene. **(b)** Base editing efficiency in CD8+ T-cells in endogenous genes. gRNA target sites in B2M, CTLA-4, PD-1, and CD33 genes are displayed in a schematic with exons represented by rectangular boxes. Triangles numbered 1 or 2 delineate the target region of each guide in which an A to G change is expected (start codon and splice site). **(c)** Indel frequency in the human B2M gene when using the peptide GHES-P2-P3-P4 CPP in combination with either a Prime editor or Cas12a. Error bars correspond to the standard deviation of the average of n=3 independent biological replicates. n.t. = non-treated.

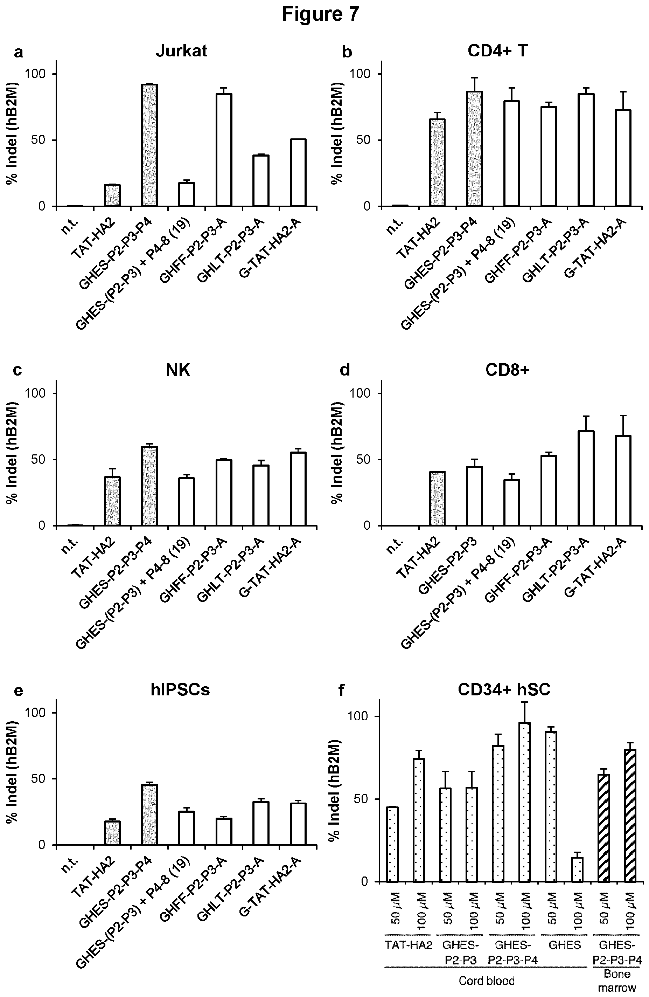

**Figure 7****:** Indel frequency in the human B2M gene when using GHES CPP variants in the following cell types: Jurkat cells, CD4+T-cells, NK-cells, CD8+T-cells, human induced pluripotent stem cells (hIPSCs), and CD34+ hematopoietic stem cells (HSCs) from cord blood and bone marrow. Error bars correspond to the standard deviation of the average of n=3 independent biological replicates. n.t. = non-treated.

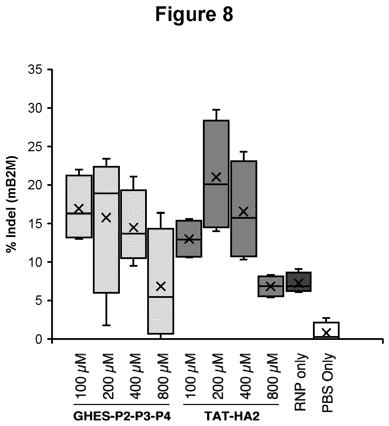

**Figure 8****:** Indel frequency in the mouse B2M gene. Editing efficiency was analysed from tissue tibialis anterior muscle injected with increasing concentrations of GHES P2-P3-P4, TAT-HA2, RNP and PBS only.

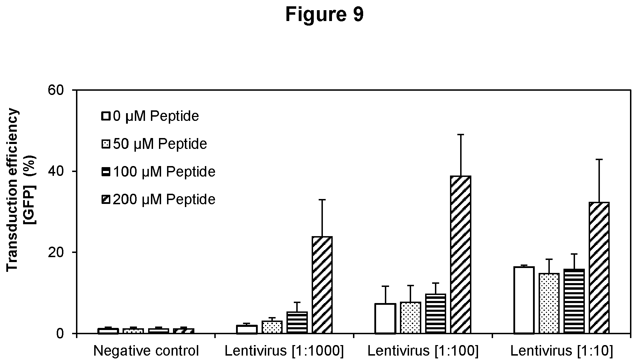

**Figure 9****:** Use of GHES-P2-P3-P4 peptide as enhancer for lentivirus delivery in HEK293T cells. Transduction efficiency measured as percentage of green fluorescent cells from the total cell population. Lentivirus to cell culture ratios of 1:10, 1:100 and 1:1000 (v:v) were used, with increasing concentrations of GHES-P2-P3-P4 peptide.

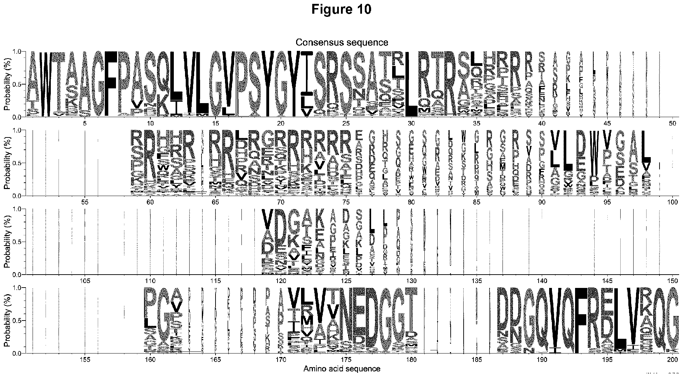

**Figure 10****:** Protein loop domain analogues from other exemplary fungal glycoside hydrolases were identified. Figure 10 shows derivation of the consensus sequences for N-terminal flagging sequence in amino acid sequence positions 1 to 33, C-terminal flagging sequence in positions 71 to 200 and internal protein loop domain in positions 26 to 178. The height of the stack indicates the amino acid conservation at that position, the height of symbols within the stack indicates the relative frequency of each amino at that position and the width of the stack is proportional to the fraction of valid amino acids in that position.

### DEFINITIONS

Generally, nomenclature used in connection with cell and tissue culture, molecular biology, immunology, microbiology, genetics, protein and nucleic acid chemistry and hybridisation, analytical chemistry, synthetic organic chemistry, and medicinal and pharmaceutical chemistry described herein is well-known and commonly used in the art. The methods and techniques provided herein are generally performed according to conventional methods known to those skilled in the art and as described in various general and more specific references that are cited and discussed throughout the present specification unless otherwise indicated. Enzymatic reactions and purification techniques are performed according to manufacturer's specifications, by standard practices known to those skilled in the art, or as described herein. Standard techniques are used for chemical syntheses, chemical analyses, pharmaceutical preparation, formulation, and delivery, and treatment of patients.

The term "a" or "an", unless specifically stated otherwise, means "one or more". For example, it can mean "only one" or "more than one", for example "two, three, four, five or more".

The term "comprising" encompasses "including" as well as "consisting" e.g. a composition "comprising" X may consist exclusively of X or may include something additional e.g. X+Y.

The word "substantially" does not exclude "completely" e.g. a composition which is "substantially free" from Y may be completely free from Y. Where necessary, the word "substantially" may be omitted from the definition of the invention.

"About" as used herein in reference to a measurable value such as an amount, a temporal duration, and the like, encompasses variations of ±20% or ±10%, more preferably ±5%, even more preferably ±1%, and still more preferably ±0.1% from the specified value, as such variations are appropriate to perform the disclosed methods. It also refers specifically to the exact value. Where necessary, the word "about" may be omitted from the definition of the invention.

Unless specifically stated, a process comprising a step of mixing two or more components does not require any specific order of mixing. Thus components can be mixed in any order. Where there are three components then two components can be combined with each other, and then the combination may be combined with the third component, etc.

Throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

It will be understood that the invention will be described by way of example only and modifications may be made while remaining within the scope and spirit of the invention.

Unless otherwise indicated, the following terms have the following meanings:

As used herein, "administration" or "administering" means providing a pharmaceutical agent or composition to an animal.

As used herein, "animal" means a human or non-human animal.

As used herein, "ameliorate" means a treatment that results in improvement in at least one symptom relative to the same symptom in the absence of the treatment. In some embodiments, amelioration is a reduction in the severity or frequency of a symptom or a delay in the onset or progression of the severity or frequency of a symptom.

"Amino acid," as used herein, refers to any molecule comprising at least one amine group (-NH₂) and at least one carboxylic acid group (-COOH), or functional equivalents thereof, which may be separated by one or more carbon atoms, and optionally bearing a side chain (R group) of variable structure and composition. This term includes, but is not limited to, the 20 standard L-α-amino acids encoded by the genetic code, their D-enantiomers, as well as naturally occurring non-standard amino acids (e.g., selenocysteine, hydroxyproline, β-alanine, or ornithine), and synthetic or non-naturally occurring amino acids. Synthetic or non-naturally occurring amino acids may include β-amino acids, γ-amino acids, N-methyl amino acids, peptidomimetics, and other structural analogues or derivatives designed to modulate or mimic the structural, chemical, or biological properties of the standard amino acids.

The term "antigen" as used herein is defined as a molecule that provokes an immune response, which may involve antibody production, activation of specific immunologically-competent cells, or both. Any macromolecule, including virtually all proteins or peptides, can serve as an antigen.

As used herein, the term "autologous" refers to any material derived from the same individual to which it is later to be re-introduced.

The term "cargo" refers to any moiety, molecule, or complex that is non-covalently or covalently coupled to a CPP for intracellular delivery. Cargo molecules include nucleic acids (e.g. DNA, RNA, CRISPR components or aptamers) and synthetic nucleic acid mimics (e.g., peptide nucleic acids (PNAs), or locked nucleic acids (LNAs)), peptides or proteins (e.g., enzymes, antibodies, antibody fragments, transcription factors or therapeutic peptides), small molecules (e.g., drugs, fluorescent probes, fluorescent dyes or inhibitors), nanoparticles (e.g., silica nanoparticles, liposomes, gold nanoparticles, polymeric nanoparticles or quantum dots), macromolecules, (e.g., polysaccharides, lipids or synthetic polymers), viral vectors or virus-like particles (e.g., lentiviral vectors or retroviral vectors or adenoviral vectors or adeno-associated viral vectors), or components for gene editing (e.g., zinc finger nucleases (ZFNs), transcription activator-like effector nucleases (TALENs), Site-specific recombinases (e.g. Cre recombinases (for Cre-Lox gene editing) or FLP), large serine recombinases (LSRs), components for CRISPR/cas9 gene editing, components for CRISPR/cas12 gene editing, components for base editing or components for prime editing). It is understood that, when the cargo molecule is a peptide, polypeptide or protein, it can comprise one or more peptides, polypeptides or proteins linked together. Also, when the cargo molecule is a nucleic acid, it can comprise one or more nucleic acids. Also, the cargo molecule can be a combination of a protein, a lipid, a nucleic acid, and/or a polysaccharide such as a lipoprotein, a ribonucleoprotein (RNP) complex, or a glycolipid.

As used herein, the term "Cas protein" encompasses all CRISPR associated proteins, including but not limited to Cas9, Cas12 and Cas13, and modified variants thereof. As used herein, the term "Cas9" encompasses all variants of Cas9, including but not limited to SpCas9, SaCas9, FnCas9, NmCas9 and TCas9. A modified SpCas9 may be a Cas9 nickase (nCas9), with only one functional nuclease domain, or a dCas9, with no nuclease activity. Preferably, a modified Cas protein includes one or more nuclear localisation sequence (NLS).

The term "cell-penetrating peptide" (CPP) refers to a peptide or peptidomimetic moiety capable of facilitating the transport of itself and/or an associated cargo across a cell membrane, particularly the plasma membrane of a eukaryotic cell. CPPs are typically short peptides comprising from about 8 to about 30 amino acid residues, and may be naturally occurring, derived from natural sequences, or synthetically designed. A CPP may be linear, cyclic, partially cyclic, inverted, retro-inverso, or otherwise conformationally constrained. At present, the theories of CPP translocation distinguish three main entry mechanisms: direct penetration in the membrane, endocytosis-mediated entry, and translocation through the formation of a transitory structure. "Modified CPP" or "synthetically modified CPP" refers to a CPP that has undergone chemical or structural modification to alter or improve its biological or physicochemical properties.

Reduction of "cell type variability" is a characteristic of a CPP whereby its ability to transport cargo into cells exhibits relatively consistent efficiency across multiple distinct cell types, regardless of differences in membrane composition, receptor expression, or physiological state. Reduced cell type variability indicates that the CPP functions with minimal dependence on specific cellular factors, enabling broader applicability across diverse tissues or species. Consistent performance across different cell types can be assessed by e.g. the CPP's ability to provide gene editing in different cell types when covalently or non-covalently coupled to cargo for CRISPR/Cas9 gene editing.

"Cell viability" refers to the proportion or number of living, healthy cells within a population following administration of a CPP. Cell viability may be assessed using various quantitative or qualitative methods that distinguish live cells from dead or dying cells, such as trypan blue exclusion, MTT assay, ATP quantification, flow cytometry using viability dyes, or other standard techniques. High cell viability indicates minimal cytotoxic effects and the preservation of normal cellular functions, morphology, and proliferative capacity.

As used herein, "coupling" refers to the association of a CPP with one or more cargo molecules by covalent or non-covalent interactions, such that the CPP is capable of facilitating the intracellular delivery of the cargo. Coupling may involve direct chemical linkage (e.g., via amide bonds such as peptide bonds, disulfide bonds, click chemistry, or other conjugation techniques), indirect linkage (e.g., via a linker or spacer), or non-covalent complexation (e.g., via electrostatic interactions, hydrogen bonding, hydrophobic interactions, or affinity-based binding). A CPP may be coupled to a cargo via expression as a CPP-cargo fusion protein, for example a CPP-Cas9 fusion protein (i.e., coupled by a peptide bond, such as when the CPP and cargo are encoded in the same open reading frame on the same plasmid). A CPP may also be coupled to a cargo by simple mixing of the CPP and cargo, i.e., without there being any direct interaction. Coupling may be reversible or irreversible, and may be accomplished in vitro or in vivo.

"Diagnostic use" refers to the use of a CPP or CPP-cargo complex to detect, localise, quantify, or monitor biological markers, disease states, or physiological conditions, whether in vivo, ex vivo or in vitro. This may include imaging-based methods (e.g., fluorescence, PET or MRI) or functional assays involving the intracellular delivery of reporter molecules.

A "disease" is a state of health of an animal wherein the animal cannot maintain homeostasis, and wherein if the disease is not ameliorated then the animal's health continues to deteriorate. In contrast, a "disorder" in an animal is a state of health in which the animal is able to maintain homeostasis, but in which the animal's state of health is less favourable than it would be in the absence of the disorder. Left untreated, a disorder does not necessarily cause a further decrease in the animal's state of health.

"Effective amount" or "therapeutically effective amount" are used interchangeably herein, and refer to an amount of a compound, formulation, material, or composition, as described herein effective to achieve a particular biological result or provide a therapeutic or prophylactic benefit. For example, a therapeutically effective amount improves a symptom of a disease or disorder.

"Encoding" refers to a nucleotide sequence's capacity to serve as a template in biological processes for the synthesis of defined nucleotide or amino acid sequences. A gene encodes a protein if its transcription and translation result in protein production.

As used herein "endogenous" refers to any material from or produced inside an organism, cell, tissue or system.

"Endosomal escape domain" refers to a peptide sequence, motif, or chemical moiety that facilitates the release of a CPP-cargo complex from endosomal compartments into the cytosol. These domains may function via pH-dependent membrane destabilisation, membrane fusion, or pore formation.

As used herein, the term "exogenous" refers to any material introduced from or produced outside an organism, cell, tissue or system.

"Ex vivo" refers to procedures carried out on cells, tissues, or organs outside the body, followed by optional reintroduction into the same or a different organism.

The term "expression" as used herein is defined as the transcription and/or translation of a particular nucleotide sequence driven by its promoter.

As used herein, the term "gene editing protein" encompasses proteins capable of editing DNA, proteins capable of editing RNA, and proteins capable of modifying expression of genes e.g. by activating or inhibiting the transcription of target genes.

"Gene editing cargo" refers to a class of cargoes that are capable of inducing targeted genomic alterations. Such cargo includes but are not limited to programmable nucleases such as Cas9, Cas12a (Cpf1), Cas13, zinc finger nucleases (ZFNs), transcription activator-like effector nucleases (TALENs), Cre recombinases (for Cre-Lox gene editing), large serine recombinases (LSRs), base editors, prime editors, and the associated guide RNAs (including single guide RNAs (sgRNAs) and dual annealed guide RNAs (i.e., comprising a crRNA annealed to a tracrRNA)) or template strands required for homology-directed repair.

"Identity" or "sequence identity" as used herein refers to the percentage of amino acid or nucleic acid residues that are identical when two sequences are aligned using a standard algorithm, such as BLAST, Clustal Omega, or EMBOSS Needle. Identity is typically expressed as a percentage of identical residues over a specified alignment length.

"In vitro" refers to a process or reaction occurring in an artificial environment outside a living organism, typically in a test tube, culture dish, or laboratory apparatus.

"In vivo" refers to procedures or applications carried out within a living organism.

"Isolated" means removed from its natural context. A naturally occurring nucleic acid or peptide is not "isolated," but becomes so when separated from other components or placed in a non-native environment.

A "lentivirus" as used herein refers to a genus of the Retroviridae family. Lentiviruses are unique among the retroviruses in being able to infect non-dividing cells. They can deliver a significant amount of genetic information into the DNA of the host cell, so they are one of the most efficient methods of a gene delivery vector. HIV, SIV, and FIV are all examples of lentiviruses.

As used herein, a "linker" refers to a molecular moiety or sequence that connects a CPP to a cargo molecule, thereby facilitating their coupling. Linkers may be cleavable or non-cleavable, flexible or rigid, and may be selected to optimise delivery efficiency, stability, or controlled release of the cargo. Cleavable linkers may be responsive to specific physiological conditions (e.g., pH, redox environment or enzymatic activity) to enable cargo release at a desired intracellular location. Linkers may include, for example, peptide sequences, polyethylene glycol (PEG) chains, disulfide bridges, ester or hydrazone bonds, or synthetic spacers.

As used herein, "nucleobase" means an unmodified nucleobase or a modified nucleobase. An "unmodified nucleobase" is adenine (A), thymine (T), cytosine (C), uracil (U), or guanine (G). A "modified nucleobase" is a nucleobase that has been chemically or structurally altered through the addition, deletion, or substitution of one or more atoms or functional groups, while retaining the ability to participate in base pairing or other nucleic acid-related interactions.

As used herein, "nucleoside" means a compound or fragment of a compound comprising a nucleobase and a sugar moiety. The nucleobase and sugar moiety are each, independently, unmodified or modified. A "modified nucleoside" means a nucleoside comprising a modified nucleobase and/or a modified sugar moiety.

Unless otherwise specified, a "nucleotide sequence encoding an amino acid sequence" includes all nucleotide sequences that are degenerate versions of each other and that encode the same amino acid sequence.

The term "oligonucleotide" refers to a short polymer composed of a sequence of nucleotides, typically ranging from about 2 to 100 nucleotides in length, which may be single-stranded or double-stranded and may include naturally occurring or modified nucleotides (e.g. naturally occurring modified nucleotides such as selenocysteine and pyrrolysine and/or synthetic modified nucleotides such as locked nucleic acids (LNAs)). The term oligonucleotide also encompasses synthetic oligonucleotide mimics, such as peptide nucleic acids (PNAs) or locked nucleic acids (LNAs). It will be understood that when a nucleotide sequence is represented by a DNA sequence (i.e., A, T, C, G), this also includes an RNA sequence (i.e., A, U, C, G) in which "U" replaces "T".

"Parenteral" administration of a composition includes, e.g., subcutaneous, intravenous, intramuscular, or intrastemal injection, or infusion techniques.

The term "polynucleotide" refers to a long polymer composed of a sequence of nucleotides, which may be of any length greater than an oligonucleotide, which may be single-stranded or double-stranded and may include naturally occurring or modified nucleotides (e.g. naturally occurring modified nucleotides such as 5-selenocysteine and pyrrolysine and/or synthetic modified nucleotides such as locked nucleic acids (LNAs)). The term polynucleotide also encompasses synthetic polynucleotide mimics, such as peptide nucleic acids (PNAs). It will be understood that when a nucleotide sequence is represented by a DNA sequence (i.e., A, T, C, G), this also includes an RNA sequence (i.e., A, U, C, G) in which "U" replaces "T".

As used herein, the terms "peptide," "polypeptide," and "protein" are used interchangeably, and refer to a compound comprised of amino acid residues covalently linked by peptide bonds.

The term "pharmaceutically acceptable" refers to substances that are suitable for use in contact with the tissues of humans and other animals without undue toxicity, irritation, allergic response or other complications, and commensurate with a reasonable benefit/risk ratio.

"Pharmaceutical composition" refers to a composition comprising a CPP and optionally a cargo, together with one or more pharmaceutically acceptable carriers, excipients, and/or diluents.

"Primary cell lines", and "primary cultures" are used interchangeably herein to refer to cells and cells cultures that have been derived from a subject and allowed to grow in vitro for a limited number of passages, i.e. splittings, of the culture. Typically, the primary cell lines are maintained for fewer than 10 passages in vitro.

The term "subject" or "patient" refers to an animal, including a human, that is the target of a treatment, observation, diagnosis, or experimental intervention. The term may refer to healthy individuals, patients, or experimental models. A "subject" or "patient" may be a human or non-human mammal. Non-human mammals include, for example, livestock and pets, such as ovine, bovine, porcine, canine, feline and murine mammals. Preferably, the subject is human.

As used herein, "symptom" or "hallmark" means any physical feature or test result that indicates the existence or extent of a disease or disorder. In some embodiments, a symptom is apparent to a subject or to a medical professional examining or testing said subject. In some embodiments, a hallmark is apparent upon invasive diagnostic testing, including, but not limited to, post-mortem tests.

The term "therapeutic" as used herein means a treatment and/or prophylaxis. A therapeutic effect is obtained by suppression, remission, or eradication of a disease state or disorder state.

"Transport", sometimes herein referred to as "transduction", refers to the uptake and internalisation of a CPP and its associated cargo into a living cell, and optionally further release of the cargo into the cytosol and/or nucleus. "Transfection" is a type of transport and refers to the delivery of nucleic acids (e.g. DNA, RNA, oligonucleotides) into a target cell e.g., a eukaryotic cell via non-viral methods.

"Transport/Transfection/Transduction efficiency" means the proportion or percentage of target cells that successfully receive cargo following a transport/transfection/transduction procedure.

As used herein, "treat", "treating" or "treatment" means administering a CPP or pharmaceutical composition comprising the same to a subject for the purpose of treating, preventing (i.e, a prophylactic treatment), or ameliorating a disease, disorder, or condition, or one or more symptoms thereof.

### DETAILED DESCRIPTION OF THE INVENTION

It is to be understood that both the foregoing general description and the following detailed description are exemplary and explanatory only and are not restrictive. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting.

The section headings used herein are for organisational purposes only and are not to be construed as limiting the subject matter described. All documents, or portions of documents, cited in this application, including, but not limited to, patents, patent applications, articles, books, and treatises, are hereby expressly incorporated-by-reference for the portions of the document discussed herein, as well as in their entirety.

It has previously been shown that CPP delivery efficiency can be dependent on the type of cell, the specific CPP used, and the CPP concentration, thereby requiring extensive optimisation across different cellular contexts (Nam et al., Arch. Pharm.Res., 2023, 46(1), 18-34; Pirhaghi et al., Mol. Pharm., 2024, 21(5), 2097-2117; Murugesan 2023 review; Dowaidar, Cell Signal., 2024, 117, 111116).

The present invention provides novel, improved CPPs based on fungal glycoside hydrolases, which overcome one or more of these drawbacks. Fungal glycoside hydrolases are enzymes produced by fungi that catalyse the hydrolysis of glycosidic bonds between carbohydrates or between carbohydrates and another molecules. These enzymes are essential for fungal nutrition and pathogenesis, facilitating access to nutrients and colonisation of host tissues.

Surprisingly, it has been discovered that a particular protein loop domain (as defined herein) within fungal glycoside hydrolases, and fragments thereof, can function as CPPs, i.e., are capable of transporting cargo across a cell membrane (see Figure 1 and Figure 2). This is the first time that such proteins have been shown to be effective as CPPs, i.e., proteins with sequences derived from a fungal enzyme, specifically a fungal glycoside hydrolase. These fungal glycoside hydrolase-derived CPPs exhibit efficient intracellular delivery of cargo and reduced cell type variability, whilst maintaining favourable cell viability. More surprisingly, these fungal glycoside hydrolase-derived CPPs facilitate efficient intracellular delivery of cargo into cell types that are traditionally difficult to transduce, such as pluripotent stem cells and T cells, particularly human induced pluripotent stem cells (hiPSCs) and primary T cells (Chen et al, Cell Stem Cell, 2014, 14, 13-26; Fajrial et al., Theranostics, 2020, 10, 5532-5549; Yu et al Cell Stem Cell, 2015, 16, 142-147). The novel, improved CPPs are particularly advantageous for transporting large proteins, such as Cas9, and complex gene editing systems, such as CRISPR/Cas9, across cell membranes into cells. Such complexes pose substantial delivery challenges, in part due to their size and low solubility.

The invention therefore provides CPPs based on fungal glycoside hydrolases for the transduction of cargo which is challenging to transduce, such as Cas9, into difficult-to-transduce cells, such as hiPSCs, thus representing a significant step forward in the field.

Methods for transporting cargo across a cell membrane into a cell using a CPP comprising the amino acid sequence of a fragment of a protein loop domain (as defined herein) of a fungal glycoside hydrolase are also provided, wherein the method comprises the step of contacting the cell with the CPP and the cargo, and wherein the CPP mediates the transport of the cargo across the cell membrane.

The surprising combination of improved transduction efficiency and good post-treatment cell viability across a variety of cell types afforded by the invention enables broad utility in cell-based therapeutic applications. The CPPs, pharmaceutical compositions, uses and methods of the invention are thus well-suited for innovative therapeutic strategies across a wide range of diseases.

### The Protein Loop Domain

The amino acid sequences of the CPPs of the invention are derived from the amino acid sequences of a particular protein loop domain found within fungal glycoside hydrolases. The particular protein loop domain of interest can easily be identified within the full-length sequence of a fungal glycoside hydrolase because it is positioned between a highly conserved N-terminal flagging sequence and a highly conserved C-terminal flagging sequence, i.e., highly conserved across fungal glycoside hydrolase enzymes.

In some embodiments, the N-terminal flagging sequence within the glycoside hydrolase is AWTXAGFPASQLVLGVPSYGYISRSXAXXLRXR (SEQ ID NO: 1) and the C-terminal flagging sequence within the glycoside hydrolase is XXXXNEDGGT (SEQ ID NO 2) (g) XXGQVQFRXLVXQG (SEQ ID NO: 3), wherein (g) is a gap of up to 6 amino acids.

In a preferred embodiment, the N-terminal flagging sequence is XWTXAGFPXXXXVLGVPSYGYXSXSXXXXLXXR (SEQ ID NO: 4) and the C-terminal flagging sequence is XXXXNXDGGX (SEQ ID NO: 5) (g) XXGQVQFRXLVXQG (SEQ ID NO: 6), wherein (g) is a gap of up to 6 amino acids.

In a particularly preferred embodiment, the N-terminal flagging sequence is XWX₁XX₂X₃X₄X₅XXXXX₆X₇GX₈PX₉YX₁₀YXX₁₁XX₁₂XXXXLXXX₁₃ (SEQ ID NO: 7),
wherein:
X₁ is T, S, V or A;
X₂ is A or S;
X₃ is G or H;
X₄ is F or V;
X₅ is P, A, D or S;
X₆ is V, M, I or A;
X₇ is L, I or M;
X₈ is V, L or I;
X₉ is S or A;
X₁₀ is G or A;
X₁₁ is S, A, N, Q or V;
X₁₂ is S or T; and
X₁₃ is R or K, and
wherein the C-terminal flagging sequence is XXXXX₁XX₂X₃X₄X(g)XXX₅X₆X₇QFX₈XX₉X₁₀XZX₁₂ , wherein:
X₁ is N, S, G, D or E;
X₂ is D, -, G, A or E;
X₃ is G, -, S or A;
X₄ is G, -, T, S, P, K or H;
(g) is a gap of up to 6 amino acids;
X₅ is G, - or N;
X₆ is Q, T or G;
X₇ is V or I;
X₈ is R, N or S;
X₉ is L, I or M;
X₁₀ is V, I, L, A or F;
X₁₂ is G, A or K, and wherein "-" represents a gap of one amino acid.

The protein loop domain within the fungal glycoside hydrolase begins towards the end of the N-terminal flagging sequence, ends close to the start of the C-terminal flagging sequence, and includes all of the amino acid sequence between the N-terminal and C-terminal flagging sequences. Across 77 of 79 exemplary fungal glycoside hydrolases for which sequences are provided in the present application, the protein loop domain begins at residue 26 of the N-terminal flagging sequence and ends at residue 8 of the C-terminal flagging sequence. These positions are shown below in the N-terminal and C-terminal flagging sequence consensus sequences marked by an *.

In some embodiments, the N-terminal flagging sequence within the glycoside hydrolase is AWTXAGFPASQLVLGVPSYGYISRSX*AXXLRXR (SEQ ID NO: 1) and the C-terminal flagging sequence within the glycoside hydrolase is XXXXNEDG*GT (SEQ ID NO 2) (g) XXGQVQFRXLVXQG (SEQ ID NO: 3), wherein (g) is a gap of up to 6 amino acids, and wherein X* marks the N-terminal amino acid of the protein loop domain and G* marks the C-terminal amino acid of the protein loop domain.

In a preferred embodiment, the N-terminal flagging sequence is

XWTXAGFPXXXXVLGVPSYGYXSXSX*XXXLXXR (SEQ ID NO: 4) and the C-terminal flagging sequence is XXXXNXDG*GX (SEQ ID NO: 5) (g) XXGQVQFRXLVXQG (SEQ ID NO: 6), wherein (g) is a gap of up to 6 amino acids, and wherein X* marks the N-terminal amino acid of the protein loop domain and G* marks the C-terminal amino acid of the protein loop domain.

In a particularly preferred embodiment, the N-terminal flagging sequence is XWX₁XX₂X₃X₄X₅XXXXX₆X₇GX₈PX₉YX₁₀YXX₁₁XX₁₂X*XXXLXXX₁₃ (SEQ ID NO: 7),
wherein:
X₁ is T, S, V or A;
X₂ is A or S;
X₃ is G or H;
X₄ is F or V;
X₅ is P, A, D or S;
X₆ is V, M, I or A;
X₇ is L, I or M;
X₈ is V, L or I;
X₉ is S or A;
X₁₀ is G or A;
X₁₁ is S, A, N, Q or V;
X₁₂ is S or T;
X₁₃ is R or K; and
X* marks the N-terminal amino acid of the protein loop domain, and
wherein the C-terminal flagging sequence is XXXXX₁XX₂X₃*X₄X(g)XXX₅X₆X₇QFX₈XX₉X₁₀XZX₁₂, wherein:
X₁ is N, S, G, D or E;
X₂ is D, -, G, A or E;
X₃ is G, -, S or A;
X₄ is G, -, T, S, P, K or H;
(g) is a gap of up to 6 amino acids;
X₅ is G, - or N;
X₆ is Q, T or G;
X₇ is V or I;
X₈ is R, N or S;
X₉ is L, I or M;
X₁₀ is V, I, L, A or F;
X₁₂ is G, A or K; and
X* marks the C-terminal amino acid of the protein loop domain, and wherein "-" represents a gap of one amino acid.

Therefore, for 77 of the 79 exemplary fungal glycoside hydrolases for which sequences are provided in the present application the protein loop domain of interest consists of the amino acid sequence within the glycoside hydrolase that begins at residue 26 of the N-terminal flagging sequence and ends at residue 8 of the C-terminal flagging sequence.

In one of the 79 exemplary fungal glycoside hydrolases for which sequences are provided in the present application (specifically the protein loop domain of SEQ ID NO: 176 *(Coprinopsis marcescibilis)* which is found between the N-terminal flagging sequence of SEQ ID NO: 28 and the C-terminal flagging sequence of SEQ ID NO: 29), the protein loop domain begins at residue 26 of the N-terminal flagging sequence and ends at residue 5 of the C-terminal flagging sequence.

In one of the 79 exemplary fungal glycoside hydrolases for which sequences are provided in the present application (specifically the protein loop domain of SEQ ID NO: 192 *(Ganoderma leucocontextum*) which is found between the N-terminal flagging sequence of SEQ ID NO: 60 and the C-terminal flagging sequence of SEQ ID NO: 61), the protein loop domain begins at residue 25 of the N-terminal flagging sequence and ends at residue 8 of the C-terminal flagging sequence.

Table 1 provides N-terminal flagging sequences and C-terminal flagging sequences from 79 exemplary fungal glycoside hydrolases. Within each N-terminal flagging sequence the first residue of the protein loop domain, i.e., the N-terminal residue of the protein loop domain, is marked with an * (i.e., position 26 for 78 of the 79 exemplary glycoside hydrolases, and position 25 for one of the exemplary glycoside hydrolases (specifically *Ganoderma leucocontextum,* SEQ ID NO 60). Within each C-terminal flagging sequence the final residue of the protein loop domain, i.e., the C-terminal residue of the protein loop domain, is marked with an * (i.e., position 8 for 78 of the 79 exemplary glycoside hydrolases, and position 5 for one of the exemplary glycoside hydrolases (specifically *Coprinopsis marcescibilis,* SEQ ID NO: 29). The consensus sequences for the N-terminal flagging sequence and C-terminal flagging sequences that are recited above are derived from these 79 exemplary glycoside hydrolase sequences, as shown in Figure 9.

Table 2 provides the full protein loop domain sequences from the same 79 exemplary fungal glycoside hydrolases, which are found between the pairs of N-terminal flagging sequences and C-terminal flagging sequences provided in Table 1. P1, P2, P3, and P4 regions are identified.

In some embodiments the protein loop domain of the fungal glycoside hydrolase consists of the amino acid sequence of any one of SEQ ID NOs: 166-244. In some embodiments the protein loop domain of the fungal glycoside hydrolase consists of the amino acid sequence of any one of SEQ ID NOs 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, and 244.

In some embodiments the protein loop domain of the fungal glycoside hydrolase consists of the amino acid sequence of SEQ ID NO: 166. In some embodiments the protein loop domain of the fungal glycoside hydrolase consists of the amino acid sequence of SEQ ID NO: 167. In some embodiments the protein loop domain of the fungal glycoside hydrolase consists of the amino acid sequence of SEQ ID NO: 168. In some embodiments the protein loop domain of the fungal glycoside hydrolase consists of the amino acid sequence of SEQ ID NO: 169. In some embodiments the protein loop domain of the fungal glycoside hydrolase consists of the amino acid sequence of SEQ ID NO: 170. In some embodiments the protein loop domain of the fungal glycoside hydrolase consists of the amino acid sequence of SEQ ID NO: 171. In some embodiments the protein loop domain of the fungal glycoside hydrolase consists of the amino acid sequence of SEQ ID NO: 172. In some embodiments the protein loop domain of the fungal glycoside hydrolase consists of the amino acid sequence of SEQ ID NO: 173. In some embodiments the protein loop domain of the fungal glycoside hydrolase consists of the amino acid sequence of SEQ ID NO: 174. In some embodiments the protein loop domain of the fungal glycoside hydrolase consists of the amino acid sequence of SEQ ID NO: 175. In some embodiments the protein loop domain of the fungal glycoside hydrolase consists of the amino acid sequence of SEQ ID NO: 176. In some embodiments the protein loop domain of the fungal glycoside hydrolase consists of the amino acid sequence of SEQ ID NO: 177. In some embodiments the protein loop domain of the fungal glycoside hydrolase consists of the amino acid sequence of SEQ ID NO: 178. In some embodiments the protein loop domain of the fungal glycoside hydrolase consists of the amino acid sequence of SEQ ID NO: 179. In some embodiments the protein loop domain of the fungal glycoside hydrolase consists of the amino acid sequence of SEQ ID NO: 180. In some embodiments the protein loop domain of the fungal glycoside hydrolase consists of the amino acid sequence of SEQ ID NO: 181. In some embodiments the protein loop domain of the fungal glycoside hydrolase consists of the amino acid sequence of SEQ ID NO: 182. In some embodiments the protein loop domain of the fungal glycoside hydrolase consists of the amino acid sequence of SEQ ID NO: 183. In some embodiments the protein loop domain of the fungal glycoside hydrolase consists of the amino acid sequence of SEQ ID NO: 184. In some embodiments the protein loop domain of the fungal glycoside hydrolase consists of the amino acid sequence of SEQ ID NO: 185. In some embodiments the protein loop domain of the fungal glycoside hydrolase consists of the amino acid sequence of SEQ ID NO: 186. In some embodiments the protein loop domain of the fungal glycoside hydrolase consists of the amino acid sequence of SEQ ID NO: 187. In some embodiments the protein loop domain of the fungal glycoside hydrolase consists of the amino acid sequence of SEQ ID NO: 188. In some embodiments the protein loop domain of the fungal glycoside hydrolase consists of the amino acid sequence of SEQ ID NO: 189. In some embodiments the protein loop domain of the fungal glycoside hydrolase consists of the amino acid sequence of SEQ ID NO: 190. In some embodiments the protein loop domain of the fungal glycoside hydrolase consists of the amino acid sequence of SEQ ID NO: 191. In some embodiments the protein loop domain of the fungal glycoside hydrolase consists of the amino acid sequence of SEQ ID NO: 192. In some embodiments the protein loop domain of the fungal glycoside hydrolase consists of the amino acid sequence of SEQ ID NO: 193. In some embodiments the protein loop domain of the fungal glycoside hydrolase consists of the amino acid sequence of SEQ ID NO: 194. In some embodiments the protein loop domain of the fungal glycoside hydrolase consists of the amino acid sequence of SEQ ID NO: 195. In some embodiments the protein loop domain of the fungal glycoside hydrolase consists of the amino acid sequence of SEQ ID NO: 196. In some embodiments the protein loop domain of the fungal glycoside hydrolase consists of the amino acid sequence of SEQ ID NO: 197. In some embodiments the protein loop domain of the fungal glycoside hydrolase consists of the amino acid sequence of SEQ ID NO: 198. In some embodiments the protein loop domain of the fungal glycoside hydrolase consists of the amino acid sequence of SEQ ID NO: 199. In some embodiments the protein loop domain of the fungal glycoside hydrolase consists of the amino acid sequence of SEQ ID NO: 200. In some embodiments the protein loop domain of the fungal glycoside hydrolase consists of the amino acid sequence of SEQ ID NO: 201. In some embodiments the protein loop domain of the fungal glycoside hydrolase consists of the amino acid sequence of SEQ ID NO: 202. In some embodiments the protein loop domain of the fungal glycoside hydrolase consists of the amino acid sequence of SEQ ID NO: 203. In some embodiments the protein loop domain of the fungal glycoside hydrolase consists of the amino acid sequence of SEQ ID NO: 204. In some embodiments the protein loop domain of the fungal glycoside hydrolase consists of the amino acid sequence of SEQ ID NO: 205. In some embodiments the protein loop domain of the fungal glycoside hydrolase consists of the amino acid sequence of SEQ ID NO: 206. In some embodiments the protein loop domain of the fungal glycoside hydrolase consists of the amino acid sequence of SEQ ID NO: 207. In some embodiments the protein loop domain of the fungal glycoside hydrolase consists of the amino acid sequence of SEQ ID NO: 208. In some embodiments the protein loop domain of the fungal glycoside hydrolase consists of the amino acid sequence of SEQ ID NO: 209. In some embodiments the protein loop domain of the fungal glycoside hydrolase consists of the amino acid sequence of SEQ ID NO: 210. In some embodiments the protein loop domain of the fungal glycoside hydrolase consists of the amino acid sequence of SEQ ID NO: 211. In some embodiments the protein loop domain of the fungal glycoside hydrolase consists of the amino acid sequence of SEQ ID NO: 212. In some embodiments the protein loop domain of the fungal glycoside hydrolase consists of the amino acid sequence of SEQ ID NO: 213. In some embodiments the protein loop domain of the fungal glycoside hydrolase consists of the amino acid sequence of SEQ ID NO: 214. In some embodiments the protein loop domain of the fungal glycoside hydrolase consists of the amino acid sequence of SEQ ID NO: 215. In some embodiments the protein loop domain of the fungal glycoside hydrolase consists of the amino acid sequence of SEQ ID NO: 216. In some embodiments the protein loop domain of the fungal glycoside hydrolase consists of the amino acid sequence of SEQ ID NO: 217. In some embodiments the protein loop domain of the fungal glycoside hydrolase consists of the amino acid sequence of SEQ ID NO: 218. In some embodiments the protein loop domain of the fungal glycoside hydrolase consists of the amino acid sequence of SEQ ID NO: 219. In some embodiments the protein loop domain of the fungal glycoside hydrolase consists of the amino acid sequence of SEQ ID NO: 220. In some embodiments the protein loop domain of the fungal glycoside hydrolase consists of the amino acid sequence of SEQ ID NO: 221. In some embodiments the protein loop domain of the fungal glycoside hydrolase consists of the amino acid sequence of SEQ ID NO: 222. In some embodiments the protein loop domain of the fungal glycoside hydrolase consists of the amino acid sequence of SEQ ID NO: 223. In some embodiments the protein loop domain of the fungal glycoside hydrolase consists of the amino acid sequence of SEQ ID NO: 224. In some embodiments the protein loop domain of the fungal glycoside hydrolase consists of the amino acid sequence of SEQ ID NO: 225. In some embodiments the protein loop domain of the fungal glycoside hydrolase consists of the amino acid sequence of SEQ ID NO: 226. In some embodiments the protein loop domain of the fungal glycoside hydrolase consists of the amino acid sequence of SEQ ID NO: 227. In some embodiments the protein loop domain of the fungal glycoside hydrolase consists of the amino acid sequence of SEQ ID NO: 228. In some embodiments the protein loop domain of the fungal glycoside hydrolase consists of the amino acid sequence of SEQ ID NO: 229. In some embodiments the protein loop domain of the fungal glycoside hydrolase consists of the amino acid sequence of SEQ ID NO: 230. In some embodiments the protein loop domain of the fungal glycoside hydrolase consists of the amino acid sequence of SEQ ID NO: 231. In some embodiments the protein loop domain of the fungal glycoside hydrolase consists of the amino acid sequence of SEQ ID NO: 232. In some embodiments the protein loop domain of the fungal glycoside hydrolase consists of the amino acid sequence of SEQ ID NO: 233. In some embodiments the protein loop domain of the fungal glycoside hydrolase consists of the amino acid sequence of SEQ ID NO: 234. In some embodiments the protein loop domain of the fungal glycoside hydrolase consists of the amino acid sequence of SEQ ID NO: 235. In some embodiments the protein loop domain of the fungal glycoside hydrolase consists of the amino acid sequence of SEQ ID NO: 236. In some embodiments the protein loop domain of the fungal glycoside hydrolase consists of the amino acid sequence of SEQ ID NO: 237. In some embodiments the protein loop domain of the fungal glycoside hydrolase consists of the amino acid sequence of SEQ ID NO: 238. In some embodiments the protein loop domain of the fungal glycoside hydrolase consists of the amino acid sequence of SEQ ID NO: 239. In some embodiments the protein loop domain of the fungal glycoside hydrolase consists of the amino acid sequence of SEQ ID NO: 240. In some embodiments the protein loop domain of the fungal glycoside hydrolase consists of the amino acid sequence of SEQ ID NO: 241. In some embodiments the protein loop domain of the fungal glycoside hydrolase consists of the amino acid sequence of SEQ ID NO: 242. In some embodiments the protein loop domain of the fungal glycoside hydrolase consists of the amino acid sequence of SEQ ID NO: 243. In some embodiments the protein loop domain of the fungal glycoside hydrolase consists of the amino acid sequence of SEQ ID NO: 244.

In a preferred embodiment, the protein loop domain of the fungal glycoside hydrolase consists of the amino acid sequence of any one of SEQ ID NOs: 184, 190, and 191.

In some embodiments, the protein loop domain is 38 to 142 amino acids in length. In a preferred embodiment, the protein loop domain is 38, 39, 43, 49, 50, 53, 54, 55, 56, 57, 59, 60, 63, 64, 65, 66, 67, 68, 69, 70, 71, 72, 73, 74, 75, 77, 79, 80, 81, 88, 89, 102 or 142 amino acids in length.

In a more preferred embodiment, the protein loop domain is 38 to 89 amino acids in length. More preferably, the protein loop domain is 38, 39, 43, 49, 50, 53, 54, 55, 56, 57, 59, 60, 63, 64, 65, 66, 67, 68, 69, 70, 71, 72, 73, 74, 75, 77, 79, 80, 81, 88, or 89 amino acids in length. In another preferred embodiment, the protein loop domain is 38 to 81 amino acids in length, more preferably 38, 39, 43, 49, 50, 53, 54, 55, 56, 57, 59, 60, 63, 64, 65, 66, 67, 68, 69, 70, 71, 72, 73, 74, 75, 77, 79, 80, or 81 amino acids in length. In another preferred embodiment, the protein loop domain is 38 to 75 amino acids in length, more preferably 38, 39, 43, 49, 50, 53, 54, 55, 56, 57, 59, 60, 63, 64, 65, 66, 67, 68, 69, 70, 71, 72, 73, 74, or 75 amino acids in length.

In some embodiments the total number of positively charged amino acids, specifically the total number of arginine, lysine, and histidine amino acids, found across the protein loop domain is between 4 and 30 and the total number of hydrophobic amino acids, specifically the total number of leucine, valine, isoleucine, methionine, and alanine amino acids, found across the protein loop domain is between 4 and 32. In a preferred embodiment, there are 3 to 21 arginine amino acids, 0 to 5 lysine amino acids, 0 to 11 histidine amino acids, 1 to 11 leucine amino acids, 0 to 7 valine amino acids, 0 to 7 isoleucine amino acids, 0 to 4 methionine amino acids, and 1 to 14 alanine amino acids found across the protein loop domain.

**Table 1 provides N-terminal flagging sequences and C-terminal flagging sequences from 79 exemplary fungal glycoside hydrolases. SEQ ID NO is abbreviated as SID. Within each N-terminal flagging sequence the first residue of the protein loop domain, i.e., the N-terminal residue of the protein loop domain, is marked with an *. Within each C-terminal flagging sequence the final residue of the protein loop domain, i.e., the C-terminal residue of the protein loop domain, is marked with an *.**

| **Scientific Name from NCBI (Species)** | **Scientific Name from NCBI (Protein)** | **Accession GenBank** | **N-terminal flagging sequence** | **SID** | **C-terminal Flagging sequence** | **SID** |
|---|---|---|---|---|---|---|
| Phellinidium pouzarii | Hypothetical protein EW145_g7877 | THG95934.1 | AWSAAHFPAGRLMLGVPAYGYLNP ST*ATSLMDK | 8 | ITLKSSDG*TTNSGQIQFNAIVAQG | 9 |
| Collybia nuda | glycoside hydrolase family 18 protein | KAF9464432.1 | AWTGAGFPASKLVLGLPSYGYVSN SE*ASGLRSR | 10 | VKIIGEDG*SSTVQFRELLRQG | 11 |
| Hohenbuehelia grisea | hypothetical protein HGRIS_009481 | KAL0949420.1 | AWTAAGFPASQLVLGLPSYGYISH ST*ATSLRTR | 12 | FRVTDEDG*GSDGQVQFRGLVAEG | 13 |
| Pleurotus ostreatus PC15 | glycoside hydrolase family 18 protein | KDQ29341.1 | AWTAAGFPASKIVLGVPSYGYISR SN*AQTLRQR | 14 | VHVASEDG*GDQVQFRDLVKQG | 15 |
| Pleurotus ostreatus | uncharacterized protein PC9H_005000 | XP_036633081.1 | AWTAAGFPASKIVLGVPSYGYISR SN*AQTLRQR | 16 | VHVASEDG*GDQVQFRDLVKQG | 17 |
| Abortiporus biennis | glycosyl hydrolase 18 family protein | KAL4242086.1 | TWSNAGFPVSQIVLGVPSYGYISR SS*ATTLRSR | 18 | VIATNEDG*GTDDGQVQFRQLVDQG | 19 |
| Pleurotus pulmonarius | glycosyl hydrolase 18 family protein | KAL4260222.1 | AWTAAGFPASKIVLGVPSYGYISR SN*AQTLRQR | 20 | VHVVSGDG*GDQVQFRDMVKQG | 21 |
| Pleurotus pulmonarius | hypothetical protein EYR36_006332 | KAF4574978.1 | AWTAAGFPASKIVLGVPSYGYISR SN*AQTLRQR | 22 | VHVVSGDG*GDQVQFRDMVKQG | 23 |
| Pleurotus cornucopiae | hypothetical protein CCMSSC00406_0001413 | KAG9219003.1 | AWTAAGFPASKIVLGVPSYGYISR SN*AQTLRQR | 24 | VHVVSEDG*GDQVQFRDLVKQG | 25 |
| Pleurotus eryngii | glycoside hydrolase | KAF9495424.1 | AWTKAGFPASQIVLGVPSYGYISR SN*AQTLRQR | 26 | VHVVSEDG*GDQVQFRDLVKQG | 27 |
| Coprinopsis marcescibilis | glycoside hydrolase | TFK29387.1 | AWTKSGFPPSQLVLGLPAYGYVQK SF*AQRLRNR | 28 | IQITE*DENQIQFRDLVKQG | 29 |
| Candolleomyces efflorescens | hypothetical protein MD484_g358, partial | KAJ2920166.1 | AWVKSGFPVSKLVLGLPSYGYVQR ST*SERLRTR | 30 | PFPANPGG*GNPVVVDDSDNQVQFR ELVKQG | 31 |
| Candolleomyces aberdarensis | hypothetical protein EST38_g1252 | RXW24650.1 | AWVKSGFPVSKLVLGLPSYGYVQR SS*SERLRTR | 32 | PLPANPGG*GTPVIVDDSDNQVQFR ELVEQG | 33 |
| Candolleomyces eurysporus | hypothetical protein H1R20_g6902, partial | KAJ2930238.1 | AWVESGFPLSKLVLGLPSYGYVQR SS*SERLITR | 34 | PLPANPGG*GTPVVVDDSDNQVQFR ELVEQG | 35 |
| Coprinellus micaceus | glycoside hydrolase family 18 protein | TEB35630.1 | AWAKAGFPVSKLVLGLPSYGYVSR SS*SERLRTR | 36 | SPPANPDS*TRPLTVVDSDGQVQFR DLVRQG | 37 |
| Tulosesus angulatus | hypothetical protein D9611_002412 | KAF5333128.1 | AWTKSGFPVSKLVLGLPSYGYVQR SS*YEQLRTR | 38 | STPENAGA*PHPITVTDSDNQVQFR DLVQQG | 39 |
| Tulosesus angulatus | glycoside hydrolase family 18 protein | KAF6762332.1 | AWTKSGFPVSKLVLGLPSYGYVQR SS*YEQLRTR | 40 | STPENAGA*PHPITVTDSDNQVQFR DLVQQG | 41 |
| Epithele typhae | glycoside hydrolase | XP_047882335.1 | AWTRAGVPADRLVLGVPSYAYVVR SS*ATRLRGR | 42 | AARPNDDG*GTDNGQVQFRALFAQA | 43 |
| Lentinus tigrinus ALCF2SS1-7 | glycoside hydrolase | XP_067541768.1 | AWTAAGFAAHQLVLGVPSYGYLSR ST*DTVLQTR | 44 | VRVKNEDG*GSDDGQVQFRELVKQG | 45 |
| Cerioporus squamosus | glycoside hydrolase superfamily | KAI0684816.1 | AWTDAGFPPHQLVLGVPSYGYISR ST*VSNLQTR | 46 | VRVQNEDG*GTNDGQVQFSDLVRQG | 47 |
| Cerioporus squamosus | glycoside hydrolase | KAI0719635.1 | AWTDAGFPPHQLVLGVPSYGYISR ST*VSNLQTR | 48 | VRVQNEDG*GTNDGQVQFSDLVRQG | 49 |
| Polyporus arcularius HHB13444 | glycoside hydrolase family 18 protein | TFK92187.1 | AWTGAGFPANRLVLGVPSYGYLSR ST*VSYLQTR | 50 | VRVENEDG*GMNDGQVQFSELVKQG | 51 |
| Lentinus brumalis | glycoside hydrolase | RDX55533.1 | AWTGAGFPANQLVLGVPSYGYLSR ST*VSYLQTR | 52 | VRVENEDG*GMNDGQVQFSELVKQG | 53 |
| Daedaleopsis nitida | glycoside hydrolase | KAI0750764.1 | LWTSAGFAPSQLVLGVPSYGYISR SS*ELSLQTR | 54 | VRVKNEDG*GTDDGQVQFRELVRQG | 55 |
| Fomes fomentarius | glycoside hydrolase | KAI0774197.1 | LWTAAGFAPRQLVLGVPSYGYISR SS*EKLLQSR | 56 | IRVKNDDG*GTDSGQVQFRELVSQG | 57 |
| Earliella scabrosa | glycoside hydrolase | KAI0723219.1 | VWTAAGFDPSQLVLGVPSYGYVSR SS*EMLLQTR | 58 | IRVKSEDG*GTDNGQVQFRELVKQG | 59 |
| Ganoderma leucocontextum | glycoside hydrolase | KAI1795158.1 | WTAAGFSPSQLVLGVPSYGYLSRS S*ATHLQTR | 60 | NVVVNEDG*GTDDGQVQFRELVRQG | 61 |
| Dichomitus squalens LYAD-421 SS1 | glycoside hydrolase | XP_007365995.1 | AWTAAGFPASQLVLGVPSYGYLSR SS*ATRLRTR | 62 | GLLVNEDG*GTDDGQVQFRELVRQG | 63 |
| Dichomitus squalens | glycoside hydrolase | TBU49421.1 | AWTAAGFPASQLVLGVPSYGYLSR SS*ATRLRTR | 64 | GLLVNEDG*GTDDGQVQFRELVRQG | 65 |
| Dichomitus squalens | glycoside hydrolase | TBU32852.1 | AWTAAGFPASQLVLGVPSYGYLSR SS*ATRLRTR | 66 | GLIVNEDG*GTDGGQVQFRELVRQG | 67 |
| Cubamyces sp. BRFM 1775 | glycoside hydrolase | KAI0327097.1 | AWTAAGFPASQLVLGVPSYGYISR SS*APLLRTR | 68 | TRVVNEDG*GMDDGQVQFRELVRQG | 69 |
| Cubamyces lactineus | glycoside hydrolase | KAH9899952.1 | AWTAAGFPASQLVLGVPSYGYISR SS*ASLLRTR | 70 | TRVVNEDG*GTDDGQVQFRELVRQG | 71 |
| Cubamyces menziesii | glycoside hydrolase | KAI0661066.1 | AWTAAGFPASQLVLGVPSYGYISR SS*ASLLRTR | 72 | TRVVNEDG*GTDDGQVQFRELVRQG | 73 |
| Trametes cubensis | hypothetical protein ONZ51_g6643 | KAJ8475288.1 | AWTAAGFPASQLVLGVPSYGYISR SS*ASLLRTR | 74 | TRVVNEDG*GTDDGQVQFRELVRQG | 75 |
| Trametes coccinea BRFM310 | glycoside hydrolase family 18 protein | OSD04163.1 | AWTAAGFPASQLVLGVPSYGYISR SS*ASLLRTR | 76 | TLVTNEDG*GTDDGQVQFRELVQQG | 77 |
| Trametes sanguinea | glycoside hydrolase family 18 protein | KAI9066735.1 | AWTAAGFPASQLVLGVPSYGYISR SS*ASLLRTR | 78 | TLVTNEDG*GTDDGQVQFRELVQQG | 79 |
| Trametes elegans | glycoside hydrolase superfamily | KAI0776101.1 | TWTAAGFPASQLVLGVPSYGYISR SS*ASLLRTR | 80 | TLVVNEDG*GTDGGQVQFRELVRQG | 81 |
| Trametes punicea | glycoside hydrolase | KAI9001092.1 | AWTAAGFPASKLVLGVPSYGYLSR SS*ASFLRTR | 82 | TLIVNEDG*GTDDGQVQFRELVRQG | 83 |
| Lenzites betulinus | glycoside hydrolase | KAH9853558.1 | AWTAAGFPAQQLVLGVPSYGYISR SD*AVALRAR | 84 | AQVHNEAG*GTDDGQIQFRDLVAQG | 85 |
| Trametes meyenii | glycoside hydrolase | KAI0650353.1 | TWTAAGFPASQLVLGVPSYGYLSR SS*ASFLRAR | 86 | TLVVNEDG*GTDNGQVQFRDLVRQG | 87 |
| Trametes maxima | glycoside hydrolase | KAI0677163.1 | TWTAAGFPASQLVLGVPSYGYISR SS*APFLRTR | 88 | TLVVNEDG*GTDNGQVQFRELVRQG | 89 |
| Trametes cingulata | glycoside hydrolase | KAI0353577.1 | AWTAAGFPASQLVLGVPSYGYISR SS*APLLRTR | 90 | RLVYNEDG*GTDNGQVQFRELVRQG | 91 |
| Pilatotrama Ijubarskyi | glycoside hydrolase | KAI0375555.1 | TWTAAGFPASKLVLGVPSYGYISR SS*ASLLRTR | 92 | RLAYNEDG*GTDNGQVQFRELVRQG | 93 |
| Trametes polyzona | glycoside hydrolase | KAI0638105.1 | AWTAAGFPASQLVLGVPSYGYISR SS*AAFLRTR | 94 | LLVYNEDG*GTDNGQVQFRALVEQG | 95 |
| Trametes pubescens | Chitinase A1 | OJT04115.1 | TWTAAGFPASQLVLGVPSYGYISR TS*AALLRTR | 96 | TLVHNEDG*GTDNGQVQFRDLVAQG | 97 |
| Trametes versicolor FP-101664 SS1 | glycoside hydrolase | XP_008035768.1 | TWTAAGFPASQLVLGVPSYGYISR TS*AALLRTR | 98 | TLVHNEDG*GTDNGQVQFRDLVAQG | 99 |
| Trametes versicolor | hypothetical protein VTO73DRAFT_14992 | KAL1946888.1 | TWTAAGFPASQLVLGVPSYGYISR TS*AALLRTR | 100 | TLVHNEDG*GTDNGQVQFRDLVAQG | 101 |
| Pleurotus pulmonarius | hypothetical protein EYR40_005288 | KAF4602086.1 | AWTAAGFPASKIVLGVPSYGYISR SN*AQTLRQR | 102 | VHVVSGDG*GDQVQFRDMVKQG | 103 |
| Abortiporus biennis | glycoside hydrolase superfamily | KAI0796747.1 | TWSNAGFPVSQIVLGVPSYGYISR SS*ATTLRSR | 104 | VIATNEDG*GTDDGQVQFRQLVDQG | 105 |
| Irpex lacteus | glycoside hydrolase superfamily | KAI0770846.1 | SWTAAGFPASKIVIGVPSYGYISR SG*ATNLMTR | 106 | FMVHNEDG*GTEDGQVQFRELINQK | 107 |
| Irpex rosettiformis | glycoside hydrolase superfamily | KAI0092710.1 | SWTSAGFPASKIVMGVPSYGYISR SG*ATNLMTR | 108 | FMVHNEDG*GTDDGQVQFRDLVKQK | 109 |
| Cytidiella melzeri | glycoside hydrolase | KAI0683604.1 | SWTSAGFPVSKIVMGVPSYGYISR SG*ASSLMTR | 110 | YMVQNEDG*GTDDGQVQFRELINQK | 111 |
| Trametopsis cervina | glycoside hydrolase | KAI0347133.1 | SWTSAGFPVNKIVMGLPSYGYISR SG*STSLMTR | 112 | FMVHNEDG*GTEDGQVQFRDLVDQK | 113 |
| Phanerochaete sordida | glycoside hydrolase family 18 protein | GJE84088.1 | AWTAAGFPASKIVLGVPSYGYIST SS*ASYLQTR | 114 | PMVMNEDG*KAADGGVQFRDLVQQG | 115 |
| Phlebiopsis gigantea 11061_1 CR5-6 | glycoside hydrolase family 18 protein | KIP02119.1 | TWTAAGFPASQIVLGVPSYGYIAR SA*SDALTTR | 116 | PAVTNEDG*GADAGQVQFRDLVGQG | 117 |
| Hermanssonia centrifuga | hypothetical protein PHLCEN_2v1610 | PSS35455.1 | AWTSAGFPPSKMVLGVPSYGYISR SS*ETVLQTR | 118 | VLVANDDG*GTDNGQVQFRDLIDQG | 119 |
| Hermanssonia centrifuga | hypothetical protein EW026_g4420 | THG97608.1 | AWTSAGFPPSKMVLGVPSYGYISR SS*ETVLQTR | 120 | MVANDDGG*TDNGQVQFRDLINQG | 121 |
| Cerrena zonata | hypothetical protein QCA50_000573 | KAK7695934.1 | AWTNAGFPASQIVLGVPSYGYISR SS*AQTLHTR | 122 | TIIQNEDG*GVDNGQVQFRDLLKQG | 123 |
| Somion occarium | unnamed protein product | CAL1700865.1 | SWTKAGFPASQIVLGVPSYGYISR SS*AQTLHSR | 124 | TLVTNEDG*GTDNGQVQFRDLVGQG | 125 |
| Grifola frondosa | Chitinase-3-like protein 2 | OBZ70066.1 | AWTAAGFPASKLVLGVPSYGYISR SS*ATLLRSR | 126 | VLATSEDG*GTDSGQVQFSELVQQG | 127 |
| Taiwanofungus camphoratus | hypothetical protein AcV5_000700 | KAI0939219.1 | AWTSAGFPASQLVLGVPSYGYLSR SN*ATTLRAR | 128 | VVATNEDG*GTDDGQVQFRELVGQG | 129 |
| Taiwanofungus camphoratus | hypothetical protein AcW2_006672 | KAI0921806.1 | AWTSAGFPASQLVLGVPSYGYLSR SN*ATTLRAR | 130 | VVATNEDG*GTDDGQVQFRELVGQG | 131 |
| Taiwanofungus camphoratus | hypothetical protein AcV7_008041 | KAI0952145.1 | AWTSAGFPASQLVLGVPSYGYLSR SN*ATALRAR | 132 | VVATNEDG*GTDDGQVQFRELVGQG | 133 |
| Taiwanofungus camphoratus | hypothetical protein AcW1_004319 | KAI0959515.1 | AWTSAGFPASQLVLGVPSYGYLSR SN*ATALRAR | 134 | VVATNEDG*GTDDGQVQFRELVSQG | 135 |
| Fomitopsis schrenkii | hypothetical protein FOMPIDRAFT_49353 | EPT03714.1 | AWTAAGFPADQLVLGVPAYGYISE SS*ATGLRQR | 136 | IMAANEDG*SPADGQVQFRGLVEQG | 137 |
| Fomitopsis betulina | glycoside hydrolase superfamily | KAI0733000.1 | AWTEAGFPADQLVLGVPAYGYLSQ SS*ATRLVQR | 138 | IMATNDDG*SPEDGQVQFRGLVEQG | 139 |
| Rhodofomes roseus | glycoside hydrolase superfamily | XP_047785035.1 | AWTSAGFPVNQLVLGVPSYGYISQ ST*ATHLRQR | 140 | IMATNDDG*SAADGQVQFRGLVLQG | 141 |
| Fomitopsis serialis | glycoside hydrolase superfamily | XP_047901050.1 | AWTSAGFPASQLVLGVPAYGYISQ TS*ATRLRQR | 142 | IMATNEDG*SAANGQVQFRALVAQG | 143 |
| Daedalea quercina L-15889 | glycoside hydrolase family 18 protein | KZT74309.1 | AWTAAGFPANQLVLGVPSYGYISQ SS*ATRLRQR | 144 | IMAANEDG*STADGQVQFRALVAQG | 145 |
| Laetiporus sulphureus 93-53 | glycoside hydrolase family 18 protein | XP_040770041.1 | AWTDAGFPASQLVLGVPSYGYISK SR*ATRLRER | 146 | IYAMNGDG*GTQNGQVQFRALVAQG | 147 |
| Sparassis crispa | glycoside hydrolase family 18 protein | XP_027610567.1 | AWTSAGFPASQLVLGVPSYGYISR SN*ATVLRTR | 148 | IQATNEDG*GTNNGQIQFRDLVSQG | 149 |
| Rhodonia placenta | hypothetical protein IEO21_02855 | KAF9818340.1 | AWTAAGFPASQLILGVPSYGYISQ SD*ATRLRAR | 150 | IQATNDDG*GTDDGQVQFRSLAAQG | 151 |
| Postia placenta Mad-698-R | hypothetical protein POSPLDRAFT_134913 | EED77690.1 | AWTAAGFPASQLILGVPSYGYISQ SD*ATRLRAR | 152 | IQATNDDG*GTDDGQVQFRSLVAQG | 153 |
| Postia placenta MAD-698-R-SB12 | glycoside hydrolase family 18 protein | XP_024337101.1 | AWTAAGFPASQLILGVPSYGYISQ SD*ATRLRAR | 154 | IQATNDDG*GTDDGQVQFRSLIAQG | 155 |
| Amylocystis lapponica | glycoside hydrolase superfamily | KAH9951662.1 | AWTSAGFPASQLVLGVPSYGYISR SS*ASSLRTR | 156 | VLAANDDG*GTDNGQIQFRALVQQG | 157 |
| Gelatoporia subvermispora B | glycoside hydrolase family 18 protein | EMD40796.1 | AWTSAGFPAAQLALGVPSYGYISK SS*VSSLRTR | 158 | VVVKDEDG*KTSGGQIQFRELVAQG | 159 |
| Obba rivulosa | glycoside hydrolase | OCH92911.1 | AWTSAGFPAAQLVLGVPSYGYISK SS*ATSLRTR | 160 | LVVKDEDG*HTTSGQIQFRELVQQG | 161 |
| Auricularia subglabra TFB-10046 SS5 | glycoside hydrolase | EJD53756.1 | SWTKSGFPAAQIVLGIPSYAYISQ ST*ATHLSGR | 162 | LKIVNEDG*GSNGGQVQFRGLIEQG | 163 |
| Auriculariales sp. MPI-PUGE-AT-0066 | glycoside hydrolase superfamily | KAH7105665.1 | SWTQSGFPASKLVLGIPAYGYISR SS*ATRLRNR | 164 | VTIENEEG*GIDGGQVQFRSIVEQG | 165 |

**Table 2 provides protein loop domain sequences from 79 exemplary fungal glycoside hydrolases, which are found between the pairs of N-terminal and C-terminal flagging sequences provided in Table 1. Each protein loop domain sequence starts within its corresponding N-terminal flagging sequence (marked in Table 1 with an *) and ends within its corresponding C terminal flagging sequence (marked in Table 1 with an *). P2 regions are underlined, P3 regions are in bold italics, P1 regions are the regions N-terminal to the P2 regions, and P4 regions are the regions C-terminal to the P3 regions. SEQ ID NO is abbreviated to SID.**

| **Scientific Name from NCBI (Species)** | **Scientific Name from NCBI (Protein)** | **Accession GenBank** | **Protein Loop Domain sequence** | **Length** | **SID** |
|---|---|---|---|---|---|
| Phellinidium pouzarii | hypothetical protein EW145_g7877 | THG95934.1 | TATSLMDKRKRDLTSRRFLMRRQSTGGDVDDDDGTAATADTVAAASA VGNGLITLKSSDG | 60 | 166 |
| Collybia nuda | glycoside hydrolase family 18 protein | KAF9464432.1 | EASGLRSRSTKTLKFRRLRS***LDDGSEEPG***TTVKIIGEDG | 39 | 167 |
| Hohenbuehelia grisea | hypothetical protein HGRIS_009481 | KAL0949420.1 | TATSLRTRAHGIHRKRQ***DAAAVSTA***STNSAFRVTDEDG | 38 | 168 |
| Pleurotus ostreatus PC15 | glycoside hydrolase family 18 protein | KDQ29341.1 | NAQTLRQRSQHGRRHHSPRRVQG***SRKVTRDDGLG***VVHVASEDG | 43 | 169 |
| Pleurotus ostreatus | uncharacterized protein PC9H_005000 | XP_036633081.1 | NAQTLRQRSQHGRRHHSPRRVQG***SRKVTRDDGLG***VVHVASEDG | 43 | 170 |
| Abortiporus biennis | glycosyl hydrolase 18 family protein | KAL4242086.1 | SATTLRSRSLSRRSPQYDTRPAYTFGHSLEGTSTVASTQSQDDSAVD ENTYKGGIVIATNEDG | 63 | 171 |
| Pleurotus pulmonarius | glycosyl hydrolase 18 family protein | KAL4260222.1 | NAQTLRQRSQHERKRHSSRRVQG***SREVARDDGLG***VVHVVSGDG | 43 | 172 |
| Pleurotus pulmonarius | hypothetical protein EYR36_006332 | KAF4574978.1 | NAQTLRQRSQHERKRHSSRRVQG***SREVARDDGLG***VVHVVSGDG | 43 | 173 |
| Pleurotus cornucopiae | hypothetical protein CCMSSC00406_0001413 | KAG9219003.1 | NAQTLRQRSQHERKRHSSRRMQA***SRKVTRDDGLG***VVHVVSEDG | 43 | 174 |
| Pleurotus eryngii | glycoside hydrolase | KAF9495424.1 | NAQTLRQRSQHERKRHSSRRVQG***SRKVTRDDGLG***VVHVVSEDG | 43 | 175 |
| Coprinopsis marcescibilis | glycoside hydrolase | TFK29387.1 | FAQRLRNRDYEHRGRHGYGGERHHSEHYGHAHSGSEHGLGDDPEDPD DDRGDGKGDMGGEPDIAPIQITE | 70 | 176 |
| Candolleomyces efflorescens | hypothetical protein MD484_g358, partial | KAJ2920166.1 | TSERLRTRWHRLRGNHGWHEHEDNEKHHDHQPGGSWWGDWGNGHASG GDDDDYDGGGSGSNGGDYDGHPDRPPFPANPGG | 80 | 177 |
| Candolleomyces aberdarensis | hypothetical protein EST38_g1252 | RXW24650.1 | SSERLRTRWHQLRRNHRWHEHEDSEKHHDHQPGGDWWGNWGHGHSGG DDDDNDDGSGSSGDDNDDEHSGPPPLPANPGG | 79 | 178 |
| Candolleomyces eurysporus | hypothetical protein H1R20_g6902, partial | KAJ2930238.1 | SSERLITRWHQLRGNHRWHEHEDSEKHHEHQPGGDFWGNWGHGHSGG DDDDNDDGSGGSGTSGDDGEHPGPSPLPANPGG | 80 | 179 |
| Coprinellus micaceus | glycoside hydrolase family 18 protein | TEB35630.1 | SSERLRTRSPWDHSKSHHWWHEHEDDEKHHVHEDDEGEGDADWHWWS GHGGWGGDNHNGDEDDDEDDDDYGREGGYRPPKSPPANPDS | 88 | 180 |
| Tulosesus angulatus | hypothetical protein D9611_002412 | KAF5333128.1 | SYEQLRTRSQGRGHWHDHEDNEKHHIPEDEHVESEDWHHWGSGGGNT KDGGKHGDGGGLDDDEAGHPSTPENAGA | 75 | 181 |
| Tulosesus angulatus | glycoside hydrolase family 18 protein | KAF6762332.1 | SYEQLRTRSQDRGHWHDHEDNEKHHIPEDEHVESEDWHHWGSGGGNT KNGGKHGDGGGLDDDEAGHPSTPENAGA | 75 | 182 |
| Epithele typhae | glycoside hydrolase | XP_047882335.1 | SATRLRGRATRSEDLAPGPAGSHLHSHSHPHPHSPRRRRGVHAPRRP HRAGRVPLGRWPAGVHPTGGRRDGLLFLRRRRRRAAARPNDDG | 89 | 183 |
| Lentinus tigrinus ALCF2SS1-7 | glycoside hydrolase | XP_067541768.1 | TDTVLQTRALRRRRQSSGASRDRHWLHRQRQRQRRRRSPVPAALEWF SSLADVLPGAVRVKNEDG | 65 | 184 |
| Cerioporus squamosus | glycoside hydrolase superfamily | KAI0684816.1 | TVSNLQTRALRRRQFSGASRGRRNRQRRQKQRRRQDHPGRSPVPTLE WFSSLADVFPGVVRVQNEDG | 67 | 185 |
| Cerioporus squamosus | glycoside hydrolase | KAI0719635.1 | TVSNLQTRALRRRQFSGASRGRRNRQRRQKQRRRQDHPGRSPVPTLE WFSSLADVFPGVVRVQNEDG | 67 | 186 |
| Polyporus arcularius HHB13444 | glycoside hydrolase family 18 protein | TFK92187.1 | TVSYLQTRALRRRQFSGASRGRRSRPQRQRRRRREEQGHGRRSPVPG LEWVSSLADVFPGGVRVENEDG | 69 | 187 |
| Lentinus brumalis | glycoside hydrolase | RDX55533.1 | TVSYLQTRALRRRQFSGASRGRRSRPQRPRRRRREEQGHGRRSPAPV PGLEWVSSLADVFPGGVRVENEDG | 71 | 188 |
| Daedaleopsis nitida | glycoside hydrolase | KAI0750764.1 | SELSLQTRSPRSRARARLRDGQRPGRRRRRRALLDVAAEGILPGVVR VKNEDG | 53 | 189 |
| Fomes fomentarius | glycoside hydrolase | KAI0774197.1 | SEKLLQSRASRSGRPRTRKRKSRNRRGRRAPLLDWAEAVTGFLPGAI RVKNDDG | 54 | 190 |
| Earliella scabrosa | glycoside hydrolase | KAI0723219.1 | SEMLLQTRGGPSRYGRGARGRRRQRRRRRLSGLGWASEAIAGFLPGA IRVKSEDG | 55 | 191 |
| Ganoderma leucocontextum | glycoside hydrolase | KAI1795158.1 | SATHLQTRALRSSPRILDSGPGSGLSALHRRREIRTRRRREGLLGML IGGSNVVVNEDG | 59 | 192 |
| Dichomitus squalens LYAD-421 SS1 | glycoside hydrolase | XP_007365995.1 | SATRLRTRAGGTRASPRVYRSGRLAENGRRRREQRQGQGQKRSMQRF VWDWIDSLLGPPVYDTDKSGLLVNEDG | 74 | 193 |
| Dichomitus squalens | glycoside hydrolase | TBU49421.1 | SATRLRTRAGGTRASPRVYRSGRLAENGRRRREQRQGQGQKRSMQRF VWDWIDSLLGPPVYDTDKSGLLVNEDG | 74 | 194 |
| Dichomitus squalens | glycoside hydrolase | TBU32852.1 | SATRLRTRAGGTRASPRVYRSGRLAENGRRRREQRQGQGQKRSMQRF VWDWIDSLLGPPVYDKSGLIVNEDG | 72 | 195 |
| Cubamyces sp. BRFM 1775 | glycoside hydrolase | KAI0327097.1 | SAPLLRTRSLETPRARFLWDARRRRRHRRRTQRHRAARSRVLGWVST LVDKTKEKLLSPGKTATRVVNEDG | 71 | 196 |
| Cubamyces lactineus | glycoside hydrolase | KAH9899952.1 | SASLLRTRALGTPRARFLWDARRGQGRRRRRTRRHRAARAPVLGWVS ALVDKAKEKLLPPGKTATATRVVNEDG | 74 | 197 |
| Cubamyces menziesii | glycoside hydrolase | KAI0661066.1 | SASLLRTRSLGTPRARFLWEARRRQRRHRRRTQRHRAARSQVLGWVS ALVDKTKEKLLPPGKTATRVVNEDG | 72 | 198 |
| Trametes cubensis | hypothetical protein ONZ51_g6643 | KAJ8475288.1 | SASLLRTRSLGTPRARFLWEARRRQRRHRRRTQRHRAARSQVLGWVS ALVDKTKEKLLPPGKTATRVVNEDG | 72 | 199 |
| Trametes coccinea BRFM310 | glycoside hydrolase family 18 protein | OSD04163.1 | SASLLRTRGMPPRRTGRLGRQRRAGRRKRDAGTPHHLLDLLGAVVDK AKNKLLPPGQTLVTNEDG | 65 | 200 |
| Trametes sanguinea | glycoside hydrolase family 18 protein | KAI9066735.1 | SASLLRTRGMPPRRRGRPGRKRRAARRKRDSGTPHHLLDLLEAVVDN AKSKLLPPGQTLVTNEDG | 65 | 201 |
| Trametes elegans | glycoside hydrolase superfamily | KAI0776101.1 | SASLLRTRAPAALPPAVFARRQKQRARRRSRVLNWVGALVDKAQPFL APGRTLVVNEDG | 59 | 202 |
| Trametes punicea | glycoside hydrolase | KAI9001092.1 | SASFLRTRAIFPRARSGRSRKQKGRRARDQRRAGHSRVLDWVGALVD KTKDKILPAGQTLIVNEDG | 66 | 203 |
| Lenzites betulinus | glycoside hydrolase | KAH9853558.1 | DAVALRARDY PRAPVAPSNTNMRRATQKPKGTRKANAARRVLGWVGA LVETAGAGVLGPDAQVHNEAG | 68 | 204 |
| Trametes meyenii | glycoside hydrolase | KAI0650353.1 | SASFLRARGPAPPPRFARTRPRPRRRLHTRVLDWVGALVDKTAALPI GQTLVVNEDG | 57 | 205 |
| Trametes maxima | glycoside hydrolase | KAI0677163.1 | SAPFLRTRELAPSRRFARTRKRSRRRLYTRVLDWVGALADKTGVLPL AKTLVVNEDG | 57 | 206 |
| Trametes cingulata | glycoside hydrolase | KAI0353577.1 | SAPLLRTRAAHRRTRPARTQVRTQSRKREGRSRRRLRSRVLEWVGAL VDKTNLLPSNSGARLVYNEDG | 68 | 207 |
| Pilatotrama Ijubarskyi | glycoside hydrolase | KAI0375555.1 | SASLLRTRASHRRGRPIRTRTRKRQRQRQRQRGRLGTRLRSRVLEWV GALVDKTKVNGLLPSGARLAYNEDG | 72 | 208 |
| Trametes polyzona | glycoside hydrolase | KAI0638105.1 | SAAFLRTRGTHAPHIRGAAWHHQMRKRRNRASLRSSRRRTKNGNRSR RLHGRVLEWVGAVVEKAKTKLLPPGGLLVYNEDG | 81 | 209 |
| Trametes pubescens | Chitinase A1 | OJT04115.1 | SAALLRTRGMPARPTPPKSSRFQKRKHNGVLKTRRHRVLDFVGALVD KTNILSAGTTLVHNEDG | 64 | 210 |
| Trametes versicolor FP-101664 SS1 | glycoside hydrolase | XP_008035768.1 | SAALLRTRGQSMRRTTLRPWRFHKRRHNGVRKTRRHRVLEWVGALVD KTNILSTGSTLVHNEDG | 64 | 211 |
| Trametes versicolor | hypothetical protein VTO73DRAFT_14992 | KAL1946888.1 | SAALLRTRGLPMRRTTLQPSRFHRRRHNGVRKTRRHRVLEWVGALVD KTNILSSGSTLVHNEDG | 64 | 212 |
| Pleurotus pulmonarius | hypothetical protein EYR40_005288 | KAF4602086.1 | NAQTLRQRSQHERKRHSSRRVQGSRK***IARDDGLGVVH***VVSGDG | 43 | 213 |
| Abortiporus biennis | glycoside hydrolase superfamily | KAI0796747.1 | SATTLRSRSLSRRSPQYDTRPAYTFGHSLEGTSTVASTQSQDDSAVD ENTYKGGIVIATNEDG | 63 | 214 |
| Irpex lacteus | glycoside hydrolase superfamily | KAI0770846.1 | GATNLMTRSLSRSKRAVPKHLPMHLDGASQAATADVSVSALGDDSLD DPSAQTEGTKPASPSAFMVHNEDG | 71 | 215 |
| Irpex rosettiformis | glycoside hydrolase superfamily | KAI0092710.1 | GATNLMTRSFSRSKRAVPKHLPMHVDGASQAASIADSEANVSAVGED SGDDPLAQMEGTKTGSSGSSSAFMVHNEDG | 77 | 216 |
| Cytidiella melzeri | glycoside hydrolase | KAI0683604.1 | GASSLMTRSVSEGLSRRRQRRTTTQHVPMHLDGASQEAEVAALGEEG PLAETEGAKPGSPNTPPAYMVQNEDG | 73 | 217 |
| Trametopsis cervina | glycoside hydrolase | KAI0347133.1 | GSTSLMTRSVVHASRRQRRAPHVPMHVDPASQEAQVTTMGEEEDPLA ETEGAKSGSSPPAAFMVHNEDG | 69 | 218 |
| Phanerochaete sordida | glycoside hydrolase family 18 protein | GJE84088.1 | SASYLQTRATLPVRRMPTRRHLPMHLNGIPADVLGDTGVPGTLLHSD GASADAVKAGVPMVMNEDG | 66 | 219 |
| Phlebiopsis gigantea 11061_1 CR5-6 | glycoside hydrolase family 18 protein | KIP02119.1 | ASDALTTRALVSRRPRHVPMHLDGPAGDALGTDALDDPLLHAAGAPG AAPAVTNEDG | 57 | 220 |
| Hermanssonia centrifuga | hypothetical protein PHLCEN_2v1610 | PSS35455.1 | SETVLQTRHIKHRRSKTHHTPIHDLTEKILPLPSDTDSPPLYADAMK PTPATAPGVLVANDDG | 63 | 221 |
| Hermanssonia centrifuga | hypothetical protein EW026_g4420 | THG97608.1 | SETVLQTRHIKHRRSKTHHTPIHDLTEKILPTSGTTDSPLYADAMKP TPVPGVMVANDDG | 60 | 222 |
| Cerrena zonata | hypothetical protein QCA50_000573 | KAK7695934.1 | SAQTLHTRRSLPEAPSHPLYASRNTVRTPRTPHPRGIRNRVHKRSHH HVRRSSHNHSIGRRRLLEYTELPPTQPSIPNFSLPAANPESETLQQP AVEHLIDPTEIDQSDDSTSDTAVTNVSNQQQDSAAVPPGTTIIQNED G | 142 | 223 |
| Somion occarium | unnamed protein product | CAL1700865.1 | SAQTLHSRSLLHTTTTRHYSRNRRPIQASPPTARRLLELTELGLGTR TRDSAPADSETVLQQDVHEHLVDPAEVDEQEISDAAVQADTTAGPGS TLVTNEDG | 102 | 224 |
| Grifola frondosa | Chitinase-3-like protein 2 | OBZ70066.1 | SATLLRSRSHAPPRTRRSRHARWRRLSLLQWTDALVPAPGPLLPPPV LATSEDG | 54 | 225 |
| Taiwanofungus camphoratus | hypothetical protein AcV5_000700 | KAI0939219.1 | NATTLRARSRHRRSRHSVRRHSLYRWPETLVSGIEADADPQSDTVPA AVVATNEDG | 56 | 226 |
| Taiwanofungus camphoratus | hypothetical protein AcW2_006672 | KAI0921806.1 | NATTLRARSRHRRSRHSVRRHSLYRWPETLVSGIEADADPQSDTVPA AVVATNEDG | 56 | 227 |
| Taiwanofungus camphoratus | hypothetical protein AcV7_008041 | KAI0952145.1 | NATALRARSRHRRSRHSVRRHSFYRWPETLVSGIEADADPQSDTVPA AVVATNEDG | 56 | 228 |
| Taiwanofungus camphoratus | hypothetical protein AcW1_004319 | KAI0959515.1 | NATALRARSRHRRSRHSVRRHSFYRWPETLVSGIEADADPQSDTVPA AVVATNEDG | 56 | 229 |
| Fomitopsis schrenkii | hypothetical protein FOMPIDRAFT_49353 | EPT03714.1 | SATGLRQRSAPSSPSVPRSHRGHLRNRHRARSSGASSEDVSDGASAN GSEGAPGISPIMAANEDG | 65 | 230 |
| Fomitopsis betulina | glycoside hydrolase superfamily | KAI0733000.1 | SATRLVQRSTLCSGLGSVPRSHRAHLRNRHRARSSLRLCANATEGAP GSSAIMATNDDG | 59 | 231 |
| Rhodofomes roseus | glycoside hydrolase superfamily | XP_047785035.1 | TATHLRQRSRPTLRRRSHGRRLRNRHRTRSSLVQWTEADSDSDTAGS PTIMATNDDG | 57 | 232 |
| Fomitopsis serialis | glycoside hydrolase superfamily | XP_047901050.1 | SATRLRQRSAPPRRRAHRRRLRNRHRARSSLLQWTDADTDASTSTPP IMATNEDG | 55 | 233 |
| Daedalea quercina L-15889 | glycoside hydrolase family 18 protein | KZT74309.1 | SATRLRQRSTLRPRSHRRRLRNRHRARSSLLHLQRTDGADSSGALSE DVSSGGAASQPENATSSSPIMAANEDG | 74 | 234 |
| Laetiporus sulphureus 93-53 | glycoside hydrolase family 18 protein | XP_040770041.1 | RATRLRERRSRHARSRHSHLPKRMQSLLSDEAQNSAPPGTPPIYAMN GDG | 50 | 235 |
| Sparassis crispa | glycoside hydrolase family 18 protein | XP_027610567.1 | NATVLRTRSQSWHSLHRNRLRRSLLGLTEAVVSGLETAASPAIQATN EDG | 50 | 236 |
| Rhodonia placenta | hypothetical protein IEO21_02855 | KAF9818340.1 | DATRLRARAQDFASEAKPLPATRRMSRSRDGTRHARRRASARDWPG AFRVLAEAGPAIQATNDDG | 66 | 237 |
| Postia placenta Mad-698-R | hypothetical protein POSPLDRAFT _134913 | EED77690.1 | DATRLRARAQDFASEAKPLPATRRKTRSRDRGTRHARRRASARDWPG AFRVLAEAGPAIQATNDDG | 66 | 238 |
| Postia placenta MAD-698-R-SB12 | glycoside hydrolase family 18 protein | XP_024337101.1 | DATRLRARAQDFASEAKPLPATRRKTRSRDRGTRHARRRASARDWPG AFRVLAEAGPAIQATNDDG | 66 | 239 |
| Amylocystis lapponica | glycoside hydrolase superfamily | KAH9951662.1 | SASSLRTRARRYKHLRRQNLLNWADSLVSGLAADVGDAPPAVLAAND DG | 49 | 240 |
| Gelatoporia subvermispora B | glycoside hydrolase family 18 protein | EMD40796.1 | SVSSLRTRSQHTDIRRGHRHRHRRRGEDRDPGLARERRQLFEMPGST LAEVAAPDSVRASGSPPYEGVVVKDEDG | 75 | 241 |
| Obba rivulosa | glycoside hydrolase | OCH92911.1 | SATSLRTRARDSHWHGYRTRYRHRRQHRLRPRHTLGAWAGSVLAATS LPDDSRIRTDNAPFAGLVVKDEDG | 71 | 242 |
| Auricularia subglabra TFB-10046 SS5 | glycoside hydrolase | EJD53756.1 | TATHLSGRRRRHEPSQAAFDAQPDVTRGMMELAAQNDAAAPSAEVAP LKIVNEDG | 55 | 243 |
| Auriculariales sp. MPI-PUGE-AT-0066 | glycoside hydrolase superfamily | KAH7105665.1 | SATRLRNRRSA***PDGTIALEAPNGINVVSTPDENG***MVTIENEEG | 43 | 244 |

### Cell Penetrating Peptides

### Cell Penetrating Peptide Lengths

CPPs of the invention comprise a 14 to 142 contiguous amino acid portion having at least 80% sequence identity to a same-length portion within a protein loop domain (as defined herein) of a fungal glycoside hydrolase. In some embodiments, the contiguous amino acid portion has at least 85% sequence identity to the same-length portion within the protein loop domain of the fungal glycoside hydrolase. In some embodiments, the contiguous amino acid portion has at least 90% sequence identity to the same-length portion within the protein loop domain of the fungal glycoside hydrolase. In some embodiments, the contiguous amino acid portion has at least 95% sequence identity to the same-length portion within the protein loop domain of the fungal glycoside hydrolase. In some embodiments, the contiguous amino acid portion has 100% sequence identity to the same-length portion within the protein loop domain of the fungal glycoside hydrolase.

The contiguous amino acid portion of the CPP is 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, or 142 amino acids in length.

In some embodiments, the contiguous amino acid portion of the CPP is 14 amino acids in length. In some embodiments, the contiguous amino acid portion of the CPP is 15 amino acids in length. In some embodiments, the contiguous amino acid portion of the CPP is 16 amino acids in length. In some embodiments, the contiguous amino acid portion of the CPP is 17 amino acids in length. In some embodiments, the contiguous amino acid portion of the CPP is 18 amino acids in length. In some embodiments, the contiguous amino acid portion of the CPP is 19 amino acids in length. In some embodiments, the contiguous amino acid portion of the CPP is 20 amino acids in length. In some embodiments, the contiguous amino acid portion of the CPP is 21 amino acids in length. In some embodiments, the contiguous amino acid portion of the CPP is 22 amino acids in length. In some embodiments, the contiguous amino acid portion of the CPP is 23 amino acids in length. In some embodiments, the contiguous amino acid portion of the CPP is 24 amino acids in length. In some embodiments, the contiguous amino acid portion of the CPP is 25 amino acids in length. In some embodiments, the contiguous amino acid portion of the CPP is 26 amino acids in length. In some embodiments, the contiguous amino acid portion of the CPP is 27 amino acids in length. In some embodiments, the contiguous amino acid portion of the CPP is 28 amino acids in length. In some embodiments, the contiguous amino acid portion of the CPP is 29 amino acids in length. In some embodiments, the contiguous amino acid portion of the CPP is 30 amino acids in length. In some embodiments, the contiguous amino acid portion of the CPP is 31 amino acids in length. In some embodiments, the contiguous amino acid portion of the CPP is 32 amino acids in length. In some embodiments, the contiguous amino acid portion of the CPP is 33 amino acids in length. In some embodiments, the contiguous amino acid portion of the CPP is 34 amino acids in length. In some embodiments, the contiguous amino acid portion of the CPP is 35 amino acids in length. In some embodiments, the contiguous amino acid portion of the CPP is 36 amino acids in length. In some embodiments, the contiguous amino acid portion of the CPP is 37 amino acids in length. In some embodiments, the contiguous amino acid portion of the CPP is 38 amino acids in length. In some embodiments, the contiguous amino acid portion of the CPP is 39 amino acids in length. In some embodiments, the contiguous amino acid portion of the CPP is 40 amino acids in length. In some embodiments, the contiguous amino acid portion of the CPP is 41 amino acids in length. In some embodiments, the contiguous amino acid portion of the CPP is 42 amino acids in length. In some embodiments, the contiguous amino acid portion of the CPP is 43 amino acids in length. In some embodiments, the contiguous amino acid portion of the CPP is 44 amino acids in length. In some embodiments, the contiguous amino acid portion of the CPP is 45 amino acids in length. In some embodiments, the contiguous amino acid portion of the CPP is 46 amino acids in length. In some embodiments, the contiguous amino acid portion of the CPP is 47 amino acids in length. In some embodiments, the contiguous amino acid portion of the CPP is 48 amino acids in length. In some embodiments, the contiguous amino acid portion of the CPP is 49 amino acids in length. In some embodiments, the contiguous amino acid portion of the CPP is 50 amino acids in length. In some embodiments, the contiguous amino acid portion of the CPP is 51 amino acids in length. In some embodiments, the contiguous amino acid portion of the CPP is 52 amino acids in length. In some embodiments, the contiguous amino acid portion of the CPP is 53 amino acids in length. In some embodiments, the contiguous amino acid portion of the CPP is 54 amino acids in length. In some embodiments, the contiguous amino acid portion of the CPP is 55 amino acids in length. In some embodiments, the contiguous amino acid portion of the CPP is 56 amino acids in length. In some embodiments, the contiguous amino acid portion of the CPP is 57 amino acids in length. In some embodiments, the contiguous amino acid portion of the CPP is 58 amino acids in length. In some embodiments, the contiguous amino acid portion of the CPP is 59 amino acids in length. In some embodiments, the contiguous amino acid portion of the CPP is 60 amino acids in length. In some embodiments, the contiguous amino acid portion of the CPP is 61 amino acids in length. In some embodiments, the contiguous amino acid portion of the CPP is 62 amino acids in length. In some embodiments, the contiguous amino acid portion of the CPP is 63 amino acids in length. In some embodiments, the contiguous amino acid portion of the CPP is 64 amino acids in length. In some embodiments, the contiguous amino acid portion of the CPP is 65 amino acids in length. In some embodiments, the contiguous amino acid portion of the CPP is 66 amino acids in length. In some embodiments, the contiguous amino acid portion of the CPP is 67 amino acids in length. In some embodiments, the contiguous amino acid portion of the CPP is 68 amino acids in length. In some embodiments, the contiguous amino acid portion of the CPP is 69 amino acids in length. In some embodiments, the contiguous amino acid portion of the CPP is 70 amino acids in length. In some embodiments, the contiguous amino acid portion of the CPP is 71 amino acids in length. In some embodiments, the contiguous amino acid portion of the CPP is 72 amino acids in length. In some embodiments, the contiguous amino acid portion of the CPP is 73 amino acids in length. In some embodiments, the contiguous amino acid portion of the CPP is 74 amino acids in length. In some embodiments, the contiguous amino acid portion of the CPP is 75 amino acids in length. In some embodiments, the contiguous amino acid portion of the CPP is 76 amino acids in length. In some embodiments, the contiguous amino acid portion of the CPP is 77 amino acids in length. In some embodiments, the contiguous amino acid portion of the CPP is 78 amino acids in length. In some embodiments, the contiguous amino acid portion of the CPP is 79 amino acids in length. In some embodiments, the contiguous amino acid portion of the CPP is 80 amino acids in length. In some embodiments, the contiguous amino acid portion of the CPP is 81 amino acids in length. In some embodiments, the contiguous amino acid portion of the CPP is 82 amino acids in length. In some embodiments, the contiguous amino acid portion of the CPP is 83 amino acids in length. In some embodiments, the contiguous amino acid portion of the CPP is 84 amino acids in length. In some embodiments, the contiguous amino acid portion of the CPP is 85 amino acids in length. In some embodiments, the contiguous amino acid portion of the CPP is 86 amino acids in length. In some embodiments, the contiguous amino acid portion of the CPP is 87 amino acids in length. In some embodiments, the contiguous amino acid portion of the CPP is 88 amino acids in length. In some embodiments, the contiguous amino acid portion of the CPP is 89 amino acids in length. In some embodiments, the contiguous amino acid portion of the CPP is 90 amino acids in length. In some embodiments, the contiguous amino acid portion of the CPP is 91 amino acids in length. In some embodiments, the contiguous amino acid portion of the CPP is 92 amino acids in length. In some embodiments, the contiguous amino acid portion of the CPP is 93 amino acids in length. In some embodiments, the contiguous amino acid portion of the CPP is 94 amino acids in length. In some embodiments, the contiguous amino acid portion of the CPP is 95 amino acids in length. In some embodiments, the contiguous amino acid portion of the CPP is 96 amino acids in length. In some embodiments, the contiguous amino acid portion of the CPP is 97 amino acids in length. In some embodiments, the contiguous amino acid portion of the CPP is 98 amino acids in length. In some embodiments, the contiguous amino acid portion of the CPP is 99 amino acids in length. In some embodiments, the contiguous amino acid portion of the CPP is 100 amino acids in length. In some embodiments, the contiguous amino acid portion of the CPP is 101 amino acids in length. In some embodiments, the contiguous amino acid portion of the CPP is 102 amino acids in length. In some embodiments, the contiguous amino acid portion of the CPP is 103 amino acids in length. In some embodiments, the contiguous amino acid portion of the CPP is 104 amino acids in length. In some embodiments, the contiguous amino acid portion of the CPP is 105 amino acids in length. In some embodiments, the contiguous amino acid portion of the CPP is 106 amino acids in length. In some embodiments, the contiguous amino acid portion of the CPP is 107 amino acids in length. In some embodiments, the contiguous amino acid portion of the CPP is 108 amino acids in length. In some embodiments, the contiguous amino acid portion of the CPP is 109 amino acids in length. In some embodiments, the contiguous amino acid portion of the CPP is 110 amino acids in length. In some embodiments, the contiguous amino acid portion of the CPP is 111 amino acids in length. In some embodiments, the contiguous amino acid portion of the CPP is 112 amino acids in length. In some embodiments, the contiguous amino acid portion of the CPP is 113 amino acids in length. In some embodiments, the contiguous amino acid portion of the CPP is 114 amino acids in length. In some embodiments, the contiguous amino acid portion of the CPP is 115 amino acids in length. In some embodiments, the contiguous amino acid portion of the CPP is 116 amino acids in length. In some embodiments, the contiguous amino acid portion of the CPP is 117 amino acids in length. In some embodiments, the contiguous amino acid portion of the CPP is 118 amino acids in length. In some embodiments, the contiguous amino acid portion of the CPP is 119 amino acids in length. In some embodiments, the contiguous amino acid portion of the CPP is 120 amino acids in length. In some embodiments, the contiguous amino acid portion of the CPP is 121 amino acids in length. In some embodiments, the contiguous amino acid portion of the CPP is 122 amino acids in length. In some embodiments, the contiguous amino acid portion of the CPP is 123 amino acids in length. In some embodiments, the contiguous amino acid portion of the CPP is 124 amino acids in length. In some embodiments, the contiguous amino acid portion of the CPP is 125 amino acids in length. In some embodiments, the contiguous amino acid portion of the CPP is 126 amino acids in length. In some embodiments, the contiguous amino acid portion of the CPP is 127 amino acids in length. In some embodiments, the contiguous amino acid portion of the CPP is 128 amino acids in length. In some embodiments, the contiguous amino acid portion of the CPP is 129 amino acids in length. In some embodiments, the contiguous amino acid portion of the CPP is 130 amino acids in length. In some embodiments, the contiguous amino acid portion of the CPP is 131 amino acids in length. In some embodiments, the contiguous amino acid portion of the CPP is 132 amino acids in length. In some embodiments, the contiguous amino acid portion of the CPP is 133 amino acids in length. In some embodiments, the contiguous amino acid portion of the CPP is 134 amino acids in length. In some embodiments, the contiguous amino acid portion of the CPP is 135 amino acids in length. In some embodiments, the contiguous amino acid portion of the CPP is 136 amino acids in length. In some embodiments, the contiguous amino acid portion of the CPP is 137 amino acids in length. In some embodiments, the contiguous amino acid portion of the CPP is 138 amino acids in length. In some embodiments, the contiguous amino acid portion of the CPP is 139 amino acids in length. In some embodiments, the contiguous amino acid portion of the CPP is 140 amino acids in length. In some embodiments, the contiguous amino acid portion of the CPP is 141 amino acids in length. In some embodiments, the contiguous amino acid portion of the CPP is 142 amino acids in length.

In some embodiments, the contiguous amino acid portion of the CPP is 24 to 142 amino acids in length. In a preferred embodiment, the contiguous amino acid portion of the CPP is 24 to 89 amino acids in length. In another preferred embodiment, the contiguous amino acid portion of the CPP is 24 to 81 amino acids in length. In another preferred embodiment, the contiguous amino acid portion of the CPP is 24 to 75 amino acids in length

In some embodiments, the contiguous amino acid portion of the CPP is 24 to 98 amino acids in length. In a preferred embodiment, the contiguous amino acid portion of the CPP is 24 to 59 amino acids in length. In another preferred embodiment, the contiguous amino acid portion of the CPP is 24 to 56 amino acids in length.

In some embodiments, the contiguous amino acid portion of the CPP is 33 to 107 amino acids in length. In a preferred embodiment, the contiguous amino acid portion of the CPP is 33 to 72 amino acids in length. In another preferred embodiment, the contiguous amino acid portion of the CPP is 33 to 71 amino acids in length. In another preferred embodiment, the contiguous amino acid portion of the CPP is 33 to 65 amino acids in length.

In some embodiments, the contiguous amino acid portion of the CPP is 20-90 amino acids in length, 20-80 amino acids in length, 20-70 amino acids in length, 20-60 amino acids in length, 20-50 amino acids in length, 20-40 amino acids in length, or 20-30 amino acids in length.

In some embodiments, the contiguous amino acid portion of the CPP is 30-90 amino acids in length, 30-80 amino acids in length, 30-70 amino acids in length, 30-60 amino acids in length, 30-50 amino acids in length, or 30-40 amino acids in length.

In some embodiments, the contiguous amino acid portion of the CPP is 40-90 amino acids in length, 40-80 amino acids in length, 40-70 amino acids in length, 40-60 amino acids in length, or 40-50 amino acids in length.

In a preferred embodiment, the contiguous amino acid portion of the CPP is 27 to 55 amino acids in length. In another preferred embodiment, the contiguous amino acid portion of the CPP is 33 to 54 amino acids in length. In a preferred embodiment, the contiguous amino acid portion of the CPP is 29 to 65 amino acids in length. In a particularly preferred embodiment, the contiguous amino acid portion of the CPP is 27 to 65 amino acids in length.

In some embodiments, the CPP consists of the contiguous amino acid portion. Thus, in some embodiments, the CPP is 14 to 142 amino acids in length. In some embodiments, the CPP is 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, or 142 amino acids in length.

In some embodiments, the CPP is 24 to 142 amino acids in length. In a preferred embodiment, the CPP is 24 to 89 amino acids in length. In another preferred embodiment, the CPP is 24 to 81 amino acids in length. In another preferred embodiment, the CPP is 24 to 75 amino acids in length

In some embodiments, the CPP is 24 to 98 amino acids in length. In a preferred embodiment, the CPP is 24 to 59 amino acids in length. In another preferred embodiment, the CPP is 24 to 56 amino acids in length.

In some embodiments, the CPP is 33 to 107 amino acids in length. In a preferred embodiment, the CPP is 33 to 72 amino acids in length. In another preferred embodiment, the CPP is 33 to 71 amino acids in length. In another preferred embodiment, the CPP is 33 to 65 amino acids in length.

In some embodiments, the CPP is 20-90 amino acids in length, 20-80 amino acids in length, 20-70 amino acids in length, 20-60 amino acids in length, 20-50 amino acids in length, 20-40 amino acids in length, or 20-30 amino acids in length.

In some embodiments, the CPP is 30-90 amino acids in length, 30-80 amino acids in length, 30-70 amino acids in length, 30-60 amino acids in length, 30-50 amino acids in length, or 30-40 amino acids in length.

In some embodiments, the CPP is 40-90 amino acids in length, 40-80 amino acids in length, 40-70 amino acids in length, 40-60 amino acids in length, or 40-50 amino acids in length.

### Cell Penetrating Peptide Regions

The fungal glycoside hydrolase protein loop domain from which the CPPs of the invention are derived can be divided into four peptide regions, designated P1, P2, P3, and P4. The P1 region is at the N-terminus of the protein loop domain, immediately followed by the P2 region, immediately followed by the P3 region, and immediately followed by the P4 region at the C-terminus of the protein loop domain (see Figure 2). Thus, the P1-P2-P3-P4 region sequence has the same amino acid sequence as the full-length protein loop domain.

Tables 3 and 4 provide P1, P2, P3, and P4 region sequences of 79 exemplary fungal glycoside hydrolases. Tables 5 and 6 provide P2-P3 region sequences and P2-P3-P4 region sequences of 79 exemplary fungal glycoside hydrolases.

The P4 region can be identified within the full-length protein loop domain sequence by a consensus sequence. In some embodiments, the P4 region has the following consensus sequence XXXXNXDGGX (SEQ ID NO: 716). In a preferred embodiment, the P4 region has the following consensus sequence is XXXXX₁X₂X₃X₄XX, wherein:
X₁ is N, S, G, D, or E
X₂ is -, E, D, G, S, A, or P
X₃ is D, -, G, A, or E, and
X₄ is G, -, S, or A (wherein "-" represents a gap of one amino acid).

**Table 3 provides P1 and P2 region sequences of 79 exemplary fungal glycoside hydrolases. SEQ ID NO is abbreviated to SID.**

| **Scientific Name from NCBI (Species)** | **Scientific Name from NCBI (Protein)** | **Accession GenBank** | **P1** | **Length** | **SID** | **P2** | **Length** | **SID** |
|---|---|---|---|---|---|---|---|---|
| Phellinidium pouzarii | hypothetical protein EW145_g7877 | THG95934.1 | TATSLMD | 7 | 245 | KRKRDLTSRRFLMRRQ STG | 19 | 321 |
| Collybia nuda | glycoside hydrolase family 18 protein | KAF9464432.1 | EASGL | 5 | 246 | RSRSTKTLKFRRLRS | 15 | 322 |
| Hohenbuehelia grisea | hypothetical protein HGRIS_009481 | KAL0949420.1 | TATSLMD | 7 | 247 | RTRAHGIHRKRQ | 12 | 323 |
| Pleurotus ostreatus PC15 | glycoside hydrolase family 18 protein | KDQ29341.1 | NAQTL | 5 | 248 | RQRSQHGRRHHSPRRV QG | 18 | 324 |
| Pleurotus ostreatus | uncharacterized protein PC9H_005000 | XP_036633081.1 | NAQTL | 5 | 249 | RQRSQHGRRHHSPRRV QG | 18 | 325 |
| Abortiporus biennis | glycosyl hydrolase 18 family protein | KAL4242086.1 | SATTL | 5 | 250 | RSRSLSRRSPQYDTRP AYTFGHS | 23 | 326 |
| Pleurotus pulmonarius | glycosyl hydrolase 18 family protein | KAL4260222.1 | NAQTL | 5 | 251 | RQRSQHERKRHSSRRV QG | 18 | 327 |
| Pleurotus pulmonarius | hypothetical protein EYR36_006332 | KAF4574978.1 | NAQTL | 5 | 252 | RQRSQHERKRHSSRRV QG | 18 | 328 |
| Pleurotus cornucopiae | hypothetical protein CCMSSC00406_00014 13 | KAG9219003.1 | NAQTL | 5 | 253 | RQRSQHERKRHSSRRM QA | 18 | 329 |
| Pleurotus eryngii | glycoside hydrolase | KAF9495424.1 | NAQTL | 5 | 254 | RQRSQHERKRHSSRRV QG | 18 | 330 |
| Coprinopsis marcescibilis | glycoside hydrolase | TFK29387.1 | FAQRLRNRDYEHR | 13 | 255 | GRHGYGGERHHSEHYG HAHSGSEHG | 25 | 331 |
| Candolleomyces efflorescens | hypothetical protein MD484_g358, partial | KAJ2920166.1 | TSERLRTRWHRLR | 13 | 256 | GNHGWHEHEDNEKHHD HQPG | 20 | 332 |
| Candolleomyces aberdarensis | hypothetical protein EST38_g1252 | RXW24650.1 | SSERLRTRWHQL | 12 | 257 | RRNHRWHEHEDSEKHH DHQPG | 21 | 333 |
| Candolleomyces eurysporus | hypothetical protein H1R20_g6902, partial | KAJ2930238.1 | SSERLITRWHQLR | 13 | 258 | GNHRWHEHEDSEKHHE HQPG | 20 | 334 |
| Coprinellus micaceus | glycoside hydrolase family 18 protein | TEB35630.1 | SSERLRTRSPWDHSK S | 16 | 259 | HHWWHEHEDDEKHHVH ED | 18 | 335 |
| Tulosesus angulatus | hypothetical protein D9611_002412 | KAF5333128.1 | SYEQLRTRSQGR | 12 | 260 | GHWHDHEDNEKHHIP | 15 | 336 |
| Tulosesus angulatus | glycoside hydrolase family 18 protein | KAF6762332.1 | SYEQLRTRSQDR | 12 | 261 | GHWHDHEDNEKHHIP | 15 | 337 |
| Epithele typhae | glycoside hydrolase | XP_047882335.1 | SATRLRGRATRSEDL APGPA | 20 | 262 | GSHLHSHSHPHPHSPR RRRG | 20 | 338 |
| Lentinus tigrinus ALCF2SS1-7 | glycoside hydrolase | XP_067541768.1 | TDTVLQTRALRRRRQ SSGASRDRHWLH | 27 | 263 | RQRQRQRRRRSP | 12 | 339 |
| Cerioporus squamosus | glycoside hydrolase superfamily | KAI0684816.1 | TVSNLQTRALRRRQF SGASR | 20 | 264 | GRRNRQRRQKQRRRQD HP | 18 | 340 |
| Cerioporus squamosus | glycoside hydrolase | KAI0719635.1 | TVSNLQTRALRRRQF SGASR | 20 | 265 | GRRNRQRRQKQRRRQD HP | 18 | 341 |
| Polyporus arcularius HHB13444 | glycoside hydrolase family 18 protein | TFK92187.1 | TVSYLQTRALRRRQF SGASR | 20 | 266 | GRRSRPQRQRRRRREE | 16 | 342 |
| Lentinus brumalis | glycoside hydrolase | RDX55533.1 | TVSYLQTRALRRRQF SGASR | 20 | 267 | GRRSRPQRPRRRRREE | 16 | 343 |
| Daedaleopsis nitida | glycoside hydrolase | KAI0750764.1 | SELSLQTRSPRSRAR ARLRD | 20 | 268 | GQRPGRRRRRRA | 12 | 344 |
| Fomes fomentarius | glycoside hydrolase | KAI0774197.1 | SEKLLQSRASRS | 12 | 269 | GRPRTRKRKSRNRRGR RA | 18 | 345 |
| Earliella scabrosa | glycoside hydrolase | KAI0723219.1 | SEMLLQTRGGPSRYG RGAR | 19 | 270 | GRRRQRRRRRLS | 12 | 346 |
| Ganoderma leucocontextum | glycoside hydrolase | KAI1795158.1 | SATHLQTRALRSSPR ILDSGPGS | 23 | 271 | GLSALHRRREIRTRRR | 16 | 347 |
| Dichomitus squalens LYAD-421 SS1 | glycoside hydrolase | XP_007365995.1 | SATRLRTRAGGTRAS PRVYRSGRLAEN | 27 | 272 | GRRRREQRQGQGQKR | 15 | 348 |
| Dichomitus squalens | glycoside hydrolase | TBU49421.1 | SATRLRTRAGGTRAS PRVYRSGRLAEN | 27 | 273 | GRRRREQRQGQGQKR | 15 | 349 |
| Dichomitus squalens | glycoside hydrolase | TBU32852.1 | SATRLRTRAGGTRAS PRVYRSGRLAEN | 27 | 274 | GRRRREQRQGQGQKR | 15 | 350 |
| Cubamyces sp. BRFM 1775 | glycoside hydrolase | KAI0327097.1 | SAPLLRTRSLETPRA RFLWDA | 21 | 275 | RRRRRHRRRTQRHRAA RSR | 19 | 351 |
| Cubamyces lactineus | glycoside hydrolase | KAH9899952.1 | SASLLRTRALGTPRA RFLWDARRGQ | 25 | 276 | GRRRRRTRRHRAARAP | 16 | 352 |
| Cubamyces menziesii | glycoside hydrolase | KAI0661066.1 | SASLLRTRSLGTPRA RFLWEA | 21 | 277 | RRRQRRHRRRTQRHRA ARSQ | 20 | 353 |
| Trametes cubensis | hypothetical protein ONZ51_g6643 | KAJ8475288.1 | SASLLRTRSLGTPRA RFLWEA | 21 | 278 | RRRQRRHRRRTQRHRA ARSQ | 20 | 354 |
| Trametes coccinea BRFM310 | glycoside hydrolase family 18 protein | OSD04163.1 | SASLLRTRGMPPRRT GRL | 18 | 279 | GRQRRAGRRKRDAGTP HH | 18 | 355 |
| Trametes sanguinea | glycoside hydrolase family 18 protein | KAI9066735.1 | SASLLRTRGMPPRRR GRP | 18 | 280 | GRKRRAARRKRDSGTP HH | 18 | 356 |
| Trametes elegans | glycoside hydrolase superfamily | KAI0776101.1 | SASLLRTRAPAALPP AVFA | 19 | 281 | RRQKQRARRRSR | 12 | 357 |
| Trametes punicea | glycoside hydrolase | KAI9001092.1 | SASFLRTRAIFPRAR S | 16 | 282 | GRSRKQKGRRARDQRR AGHSR | 21 | 358 |
| Lenzites betulinus | glycoside hydrolase | KAH9853558.1 | DAVALRARDYPRAPV APSNTN | 21 | 283 | MRRATQKPKGTRKANA ARR | 19 | 359 |
| Trametes meyenii | glycoside hydrolase | KAI0650353.1 | SASFLRARGPAPPPR F | 16 | 284 | ARTRPRPRRRLHTR | 14 | 360 |
| Trametes maxima | glycoside hydrolase | KAI0677163.1 | SAPFLRTRELAPSRR F | 16 | 285 | ARTRKRSRRRLYTR | 14 | 361 |
| Trametes cingulata | glycoside hydrolase | KAI0353577.1 | SAPLLRTRAAHRRTR P | 16 | 286 | ARTQVRTQSRKREGRS RRRLRSR | 23 | 362 |
| Pilatotrama Ijubarskyi | glycoside hydrolase | KAI0375555.1 | SASLLRTRASHRRGR PI | 17 | 287 | RTRTRKRQRQRQRQRG RLGTRLRSR | 25 | 363 |
| Trametes polyzona | glycoside hydrolase | KAI0638105.1 | SAAFLRTRGTHAPHI RGAAWHHQMRKRRNR A | 31 | 288 | SLRSSRRRTKNGNRSR RLHGR | 21 | 364 |
| Trametes pubescens | Chitinase A1 | OJT04115.1 | SAALLRTRGMPARPT PPKS | 19 | 289 | SRFQKRKHNGVLKTRR HR | 18 | 365 |
| Trametes versicolor FP-101664 SS1 | glycoside hydrolase | XP_008035768.1 | SAALLRTRGQSMRRT TLRPW | 20 | 290 | RFHKRRHNGVRKTRRH R | 17 | 366 |
| Trametes versicolor | hypothetical protein VTO73DRAFT_14992 | KAL1946888.1 | SAALLRTRGLPMRRT TLQPS | 20 | 291 | RFHRRRHNGVRKTRRH R | 17 | 367 |
| Pleurotus pulmonarius | hypothetical protein EYR40_005288 | KAF4602086.1 | NAQTL | 5 | 292 | RQRSQHERKRHSSRRV QGSRK | 21 | 368 |
| Abortiporus biennis | glycoside hydrolase superfamily | KAI0796747.1 | SATTL | 5 | 293 | RSRSLSRRSPQYDTRP AYTFGHS | 23 | 369 |
| Irpex lacteus | glycoside hydrolase superfamily | KAI0770846.1 | GATNLM | 6 | 294 | TRSLSRSKRAVPKHLP MH | 18 | 370 |
| Irpex rosettiformis | glycoside hydrolase superfamily | KAI0092710.1 | GATNLM | 6 | 295 | TRSFSRSKRAVPKHLP MH | 18 | 371 |
| Cytidiella melzeri | glycoside hydrolase | KAI0683604.1 | GASSLMTRSVSEGL | 14 | 296 | SRRRQRRTTTQHVPMH | 16 | 372 |
| Trametopsis cervina | glycoside hydrolase | KAI0347133.1 | GSTSLMTRSVV | 11 | 297 | HASRRQRRAPHVPMH | 15 | 373 |
| Phanerochaete sordida | glycoside hydrolase family 18 protein | GJE84088.1 | SASYLQ | 6 | 298 | TRATLPVRRMPTRRHL PMHLN | 21 | 374 |
| Phlebiopsis gigantea 11061_1 CR5-6 | glycoside hydrolase family 18 protein | KIP02119.1 | ASDALT | 6 | 299 | TRALVSRRPRHVPMHL D | 17 | 375 |
| Hermanssonia centrifuga | hypothetical protein PHLCEN_2v1610 | PSS35455.1 | SETVLQ | 6 | 300 | TRHIKHRRSKTHHTPI HDLTEK | 22 | 376 |
| Hermanssonia centrifuga | hypothetical protein EW026_g4420 | THG97608.1 | SETVLQ | 6 | 301 | TRHIKHRRSKTHHTPI HDLTEK | 22 | 377 |
| Cerrena zonata | hypothetical protein QCA50_000573 | KAK7695934.1 | SAQTLHTRRSLPEAP SHPLYASRNTVRTPR TPHPR | 35 | 302 | GIRNRVHKRSHHHVRR SSHNHSIGRRR | 27 | 378 |
| Somion occarium | unnamed protein product | CAL1700865.1 | SAQTL | 5 | 303 | HSRSLLHTTTTRHYSR NRRPIQASPPTARR | 30 | 379 |
| Grifola frondosa | Chitinase-3-like protein 2 | OBZ70066.1 | SATLL | 5 | 304 | RSRSHAPPRTRRSRHA RWRR | 20 | 380 |
| Taiwanofungus camphoratus | hypothetical protein AcV5_000700 | KAI0939219.1 | NATTL | 5 | 305 | RARSRHRRSRHSVRRH SLYR | 20 | 381 |
| Taiwanofungus camphoratus | hypothetical protein AcW2_006672 | KAI0921806.1 | NATTL | 5 | 306 | RARSRHRRSRHSVRRH SLYR | 20 | 382 |
| Taiwanofungus camphoratus | hypothetical protein AcV7_008041 | KAI0952145.1 | NATAL | 5 | 307 | RARSRHRRSRHSVRRH SFYR | 20 | 383 |
| Taiwanofungus camphoratus | hypothetical protein AcW1_004319 | KAI0959515.1 | NATAL | 5 | 308 | RARSRHRRSRHSVRRH SFYR | 20 | 384 |
| Fomitopsis schrenkii | hypothetical protein FOMPIDRAFT_49353 | EPT03714.1 | SATGLRQRSAPSSPS VP | 17 | 309 | RSHRGHLRNRHRARSS | 16 | 385 |
| Fomitopsis betulina | glycoside hydrolase superfamily | KAI0733000.1 | SATRLVQRSTLCSGL GSV | 18 | 310 | PRSHRAHLRNRHRARS S | 17 | 386 |
| Rhodofomes roseus | glycoside hydrolase superfamily | XP_047785035.1 | TATHLRQRSRPTL | 13 | 311 | RRRSHGRRLRNRHRTR SS | 18 | 387 |
| Fomitopsis serialis | glycoside hydrolase superfamily | XP_047901050.1 | SATRLRQRSAP | 11 | 312 | PRRRAHRRRLRNRHRA RSS | 19 | 388 |
| Daedalea quercina L-15889 | glycoside hydrolase family 18 protein | KZT74309.1 | SATRLRQRSTL | 11 | 313 | RPRSHRRRLRNRHRAR SS | 18 | 389 |
| Laetiporus sulphureus 93-53 | glycoside hydrolase family 18 protein | XP_040770041.1 | RAT | 3 | N/A | RLRERRSRHARSRHSH LPKRMQS | 23 | 390 |
| Sparassis crispa | glycoside hydrolase family 18 protein | XP_027610567.1 | NATVL | 5 | 314 | RTRSQSWHSLHRNRLR RS | 18 | 391 |
| Rhodonia placenta | hypothetical protein IEO21_02855 | KAF9818340.1 | DATRLRARAQDFASE AKPLPA | 21 | 315 | TRRMSRSRDRGTRHAR RRAS | 20 | 392 |
| Postia placenta Mad-698-R | hypothetical protein POSPLDRAFT_134913 | EED77690.1 | DATRLRARAQDFASE AKPLPA | 21 | 316 | TRRKTRSRDRGTRHAR RRAS | 20 | 393 |
| Postia placenta MAD-698-R-SB12 | glycoside hydrolase family 18 protein | XP_024337101.1 | DATRLRARAQDFASE AKPLPA | 21 | 317 | TRRKTRSRDRGTRHAR RRAS | 20 | 394 |
| Amylocystis lapponica | glycoside hydrolase superfamily | KAH9951662.1 | SASSL | 5 | 318 | RTRARRYKHLRRQN | 14 | 395 |
| Gelatoporia subvermispora B | glycoside hydrolase family 18 protein | EMD40796.1 | SVSSLRTRSQHTDIR R | 16 | 319 | GHRHRHRRRGEDRDPG LARERRQ | 23 | 396 |
| Obba rivulosa | glycoside hydrolase | OCH92911.1 | SATSLRTRARDSHWH GY | 17 | 320 | RTRYRHRRQHRLRPRH T | 17 | 397 |
| Auricularia subglabra TFB-10046 SS5 | glycoside hydrolase | EJD53756.1 | TAT | 3 | N/A | HLSGRRRRHEPSQ | 13 | 398 |
| Auriculariales sp. MPI-PUGE-AT-0066 | glycoside hydrolase superfamily | KAH7105665.1 | SAT | 3 | N/A | RLRNRRSA | 8 | 399 |

**Table 4 provides P3 and P4 region sequences of 79 exemplary fungal glycoside hydrolases. SEQ ID NO is abbreviated to SID.**

| **Scientific Name from NCBI (Species)** | **Scientific Name from NCBI (Protein)** | **Accession GenBank** | **P3** | **Length** | **SID** | **P4** | **Length** | **SID** |
|---|---|---|---|---|---|---|---|---|
| Phellinidium pouzarii | hypothetical protein EW145_g7877 | THG95934.1 | GDVDDDDGTAATADT VAAASAVGNG | 25 | 400 | LITLKSSDG | 9 | 479 |
| Collybia nuda | glycoside hydrolase family 18 protein | KAF9464432.1 | LDDGSEEPG | 9 | 401 | TTVKIIGEDG | 10 | 480 |
| Hohenbuehelia grisea | hypothetical protein HGRIS_009481 | KAL0949420.1 | DAAAVSTASTNSA | 13 | 402 | FRVTDEDG | 8 | 481 |
| Pleurotus ostreatus PC15 | glycoside hydrolase family 18 protein | KDQ29341.1 | SRKVTRDDGLG | 11 | 403 | VVHVASEDG | 9 | 482 |
| Pleurotus ostreatus | uncharacterized protein PC9H_005000 | XP_036633081.1 | SRKVTRDDGLG | 11 | 404 | VVHVASEDG | 9 | 483 |
| Abortiporus biennis | glycosyl hydrolase 18 family protein | KAL4242086.1 | LEGTSTVASTQSQDD SAVDENTYKG | 25 | 405 | IVIATNEDG | 9 | 484 |
| Pleurotus pulmonarius | glycosyl hydrolase 18 family protein | KAL4260222.1 | SREVARDDGLG | 11 | 406 | VVHVVSGDG | 9 | 485 |
| Pleurotus pulmonarius | hypothetical protein EYR36_006332 | KAF4574978.1 | SREVARDDGLG | 11 | 407 | VVHVVSGDG | 9 | 486 |
| Pleurotus cornucopiae | hypothetical protein CCMSSC00406_00014 13 | KAG9219003.1 | SRKVTRDDGLG | 11 | 408 | VVHVVSEDG | 9 | 487 |
| Pleurotus eryngii | glycoside hydrolase | KAF9495424.1 | SRKVTRDDGLG | 11 | 409 | VVHVVSEDG | 9 | 488 |
| Coprinopsis marcescibilis | glycoside hydrolase | TFK29387.1 | LGDDPEDPDDDRGDG KGDMGGEPD | 24 | 410 | IAPIQITE | 8 | 489 |
| Candolleomyces efflorescens | hypothetical protein MD484_g358, partial | KAJ2920166.1 | GSWWGDWGNGHASGG DDDDYDGGGSGSNGG | 30 | 411 | DYDGHPDRPPFPANPG G | 17 | 490 |
| Candolleomyces aberdarensis | hypothetical protein EST38_g1252 | RXW24650.1 | GDWWGNWGHGHSGGD DDDNDDGSGSSGDDN DDEHSG | 36 | 412 | PPPLPANPGG | 10 | 491 |
| Candolleomyces eurysporus | hypothetical protein H1R20_g6902, partial | KAJ2930238.1 | GDFWGNWGHGHSGGD DDDNDDGSGGSGTSG | 30 | 413 | DDGEHPGPSPLPANPG G | 17 | 492 |
| Coprinellus micaceus | glycoside hydrolase family 18 protein | TEB35630.1 | DEGEGDADWHWWSGH GGWGGDNHNGDEDDD EDDDDYG | 37 | 414 | REGGYRPPKSPPANPD S | 17 | 493 |
| Tulosesus angulatus | hypothetical protein D9611_002412 | KAF5333128.1 | EDEHVESEDWHHWGS GGGNTKDGGKHGDGG G | 31 | 415 | LDDDEAGHPSTPENAG A | 17 | 494 |
| Tulosesus angulatus | glycoside hydrolase family 18 protein | KAF6762332.1 | EDEHVESEDWHHWGS GGGNTKNGGKHGDGG G | 31 | 416 | LDDDEAGHPSTPENAG A | 17 | 495 |
| Epithele typhae | glycoside hydrolase | XP_047882335.1 | VHAPRRPHRAGRVPL GRWPAGVHPTGGRRD GLLFLR | 36 | 417 | RRRRAAARPNDDG | 13 | 496 |
| Lentinus tigrinus ALCF2SS1-7 | glycoside hydrolase | XP_067541768.1 | VPAALEWFSSLADVL PG | 17 | 418 | AVRVKNEDG | 9 | 497 |
| Cerioporus squamosus | glycoside hydrolase superfamily | KAI0684816.1 | GRSPVPTLEWFSSLA DVFPG | 20 | 419 | VVRVQNEDG | 9 | 498 |
| Cerioporus squamosus | glycoside hydrolase | KAI0719635.1 | GRSPVPTLEWFSSLA DVFPG | 20 | 420 | VVRVQNEDG | 9 | 499 |
| Polyporus arcularius HHB13444 | glycoside hydrolase family 18 protein | TFK92187.1 | QGHGRRSPVPGLEWV SSLADVFPG | 24 | 421 | GVRVENEDG | 9 | 500 |
| Lentinus brumalis | glycoside hydrolase | RDX55533.1 | QGHGRRSPAPVPGLE WVSSLADVFPG | 26 | 422 | GVRVENEDG | 9 | 501 |
| Daedaleopsis nitida | glycoside hydrolase | KAI0750764.1 | LLDVAAEGILPG | 12 | 423 | VVRVKNEDG | 9 | 502 |
| Fomes fomentarius | glycoside hydrolase | KAI0774197.1 | PLLDWAEAVTGFLPG | 15 | 424 | AIRVKNDDG | 9 | 503 |
| Earliella scabrosa | glycoside hydrolase | KAI0723219.1 | GLGWASEAIAGFLPG | 15 | 425 | AIRVKSEDG | 9 | 504 |
| Ganoderma leucocontextum | glycoside hydrolase | KAI1795158.1 | REGLLGMLIGG | 11 | 426 | SNVVVNEDG | 9 | 505 |
| Dichomitus squalens LYAD-421 SS1 | glycoside hydrolase | XP_007365995.1 | SMQRFVWDWIDSLLG | 15 | 427 | PPVYDTDKSGLLVNED G | 17 | 506 |
| Dichomitus squalens | glycoside hydrolase | TBU49421.1 | SMQRFVWDWIDSLLG | 15 | 428 | PPVYDTDKSGLLVNED G | 17 | 507 |
| Dichomitus squalens | glycoside hydrolase | TBU32852.1 | SMQRFVWDWIDSLLG | 15 | 429 | PPVYDKSGLIVNEDG | 15 | 508 |
| Cubamyces sp. BRFM 1775 | glycoside hydrolase | KAI0327097.1 | VLGWVSTLVDKTKEK LLSPG | 20 | 430 | KTATRVVNEDG | 11 | 509 |
| Cubamyces lactineus | glycoside hydrolase | KAH9899952.1 | VLGWVSALVDKAKEK LLPPG | 20 | 431 | KTATATRVVNEDG | 13 | 510 |
| Cubamyces menziesii | glycoside hydrolase | KAI0661066.1 | VLGWVSALVDKTKEK LLPPG | 20 | 432 | KTATRVVNEDG | 11 | 511 |
| Trametes cubensis | hypothetical protein ONZ51_g6643 | KAJ8475288.1 | VLGWVSALVDKTKEK LLPPG | 20 | 433 | KTATRVVNEDG | 11 | 512 |
| Trametes coccinea BRFM310 | glycoside hydrolase family 18 protein | OSD04163.1 | LLDLLGAVVDKAKNK LLPPG | 20 | 434 | QTLVTNEDG | 9 | 513 |
| Trametes sanguinea | glycoside hydrolase family 18 protein | KAI9066735.1 | LLDLLEAVVDNAKSK LLPPG | 20 | 435 | QTLVTNEDG | 9 | 514 |
| Trametes elegans | glycoside hydrolase superfamily | KAI0776101.1 | VLNWVGALVDKAQPF LAPG | 19 | 436 | RTLVVNEDG | 9 | 515 |
| Trametes punicea | glycoside hydrolase | KAI9001092.1 | VLDWVGALVDKTKDK ILPAG | 20 | 437 | QTLIVNEDG | 9 | 516 |
| Lenzites betulinus | glycoside hydrolase | KAH9853558.1 | VLGWVGALVETAGAG VLG | 18 | 438 | PDAQVHNEAG | 10 | 517 |
| Trametes meyenii | glycoside hydrolase | KAI0650353.1 | VLDWVGALVDKTAAL PIG | 18 | 439 | QTLVVNEDG | 9 | 518 |
| Trametes maxima | glycoside hydrolase | KAI0677163.1 | VLDWVGALADKTG | 13 | 440 | VLPLAKTLVVNEDG | 14 | 519 |
| Trametes cingulata | glycoside hydrolase | KAI0353577.1 | VLEWVGALVDKTNLL PSNSG | 20 | 441 | ARLVYNEDG | 9 | 520 |
| Pilatotrama Ijubarskyi | glycoside hydrolase | KAI0375555.1 | VLEWVGALVDKTKVN GLLPSG | 21 | 442 | ARLAYNEDG | 9 | 521 |
| Trametes polyzona | glycoside hydrolase | KAI0638105.1 | VLEWVGAVVEKAKTK LLPPG | 20 | 443 | GLLVYNEDG | 9 | 522 |
| Trametes pubescens | Chitinase A1 | OJT04115.1 | VLDFVGALVDKTNIL SAG | 18 | 444 | TTLVHNEDG | 9 | 523 |
| Trametes versicolor FP-101664 SS1 | glycoside hydrolase | XP_008035768.1 | VLEWVGALVDKTNIL STG | 18 | 445 | STLVHNEDG | 9 | 524 |
| Trametes versicolor | hypothetical protein VTO73DRAFT_14992 | KAL1946888.1 | VLEWVGALVDKTNIL SSG | 18 | 446 | STLVHNEDG | 9 | 525 |
| Pleurotus pulmonarius | hypothetical protein EYR40_005288 | KAF4602086.1 | IARDDGLGVVH | 11 | 447 | VVHVVSGDG | 9 | 526 |
| Abortiporus biennis | glycoside hydrolase superfamily | KAI0796747.1 | LEGTSTVASTQSQDD SAVDENTYKGG | 26 | 448 | IVIATNEDG | 9 | 527 |
| Irpex lacteus | glycoside hydrolase superfamily | KAI0770846.1 | LDGASQAATADVSVS ALGDDSLDDPSAQTE GTKPASPS | 38 | 449 | AFMVHNEDG | 9 | 528 |
| Irpex rosettiformis | glycoside hydrolase superfamily | KAI0092710.1 | VDGASQAASIADSEA NVSAVGEDSGDDPLA QMEGTKTGSSG | 41 | 450 | SSSAFMVHNEDG | 12 | 529 |
| Cytidiella melzeri | glycoside hydrolase | KAI0683604.1 | LDGASQEAEVAALGE EGPLAETEGAKPG | 28 | 451 | SPNTPPAYMVQNEDG | 15 | 530 |
| Trametopsis cervina | glycoside hydrolase | KAI0347133.1 | VDPASQEAQVTTMGE EEDPLAETEGAKSGS SPPA | 34 | 452 | AFMVHNEDG | 9 | 531 |
| Phanerochaete sordida | glycoside hydrolase family 18 protein | GJE84088.1 | GIPADVLGDTGVPGT LLHSDGASADAVKAG | 30 | 453 | VPMVMNEDG | 9 | 532 |
| Phlebiopsis gigantea 11061_1 CR5-6 | glycoside hydrolase family 18 protein | KIP02119.1 | GPAGDALGTDALDDP LLHAAGAPG | 24 | 454 | AAPAVTNEDG | 10 | 533 |
| Hermanssonia centrifuga | hypothetical protein PHLCEN_2v1610 | PSS35455.1 | ILPLPSDTDSPPLYA DAMKPTPATAPG | 27 | 455 | VLVANDDG | 8 | 534 |
| Hermanssonia centrifuga | hypothetical protein EW026_g4420 | THG97608.1 | ILPTSGTTDSPLYAD AMKPTPVPG | 24 | 456 | VMVANDDG | 8 | 535 |
| Cerrena zonata | hypothetical protein QCA50_000573 | KAK7695934.1 | LLEYTELPPTQPSIP NFSLPAANPESETLQ QPAVEHLIDPTEIDQ SDDSTSDTAVTNVSN QQQDSAAVPPG | 71 | 457 | TTIIQNEDG | 9 | 536 |
| Somion occarium | unnamed protein product | CAL1700865.1 | LLELTELGLGTRTRD SAPADSETVLQQDVH EHLVDPAEVDEQEIS DAAVQADTTAGPG | 58 | 458 | STLVTNEDG | 9 | 537 |
| Grifola frondosa | Chitinase-3-like protein 2 | OBZ70066.1 | LSLLQWTDALVPAPG | 15 | 459 | PLLPPPVLATSEDG | 14 | 538 |
| Taiwanofungus camphoratus | hypothetical protein AcV5_000700 | KAI0939219.1 | WPETLVSGIEADADP QSDTVPA | 22 | 460 | AVVATNEDG | 9 | 539 |
| Taiwanofungus camphoratus | hypothetical protein AcW2_006672 | KAI0921806.1 | WPETLVSGIEADADP QSDTVPA | 22 | 461 | AVVATNEDG | 9 | 540 |
| Taiwanofungus camphoratus | hypothetical protein AcV7_008041 | KAI0952145.1 | WPETLVSGIEADADP QSDTVPA | 22 | 462 | AVVATNEDG | 9 | 541 |
| Taiwanofungus camphoratus | hypothetical protein AcW1_004319 | KAI0959515.1 | WPETLVSGIEADADP QSDTVPA | 22 | 463 | AVVATNEDG | 9 | 542 |
| Fomitopsis schrenkii | hypothetical protein FOMPIDRAFT_49353 | EPT03714.1 | GASSEDVSDGASANG SEGAPG | 21 | 464 | ISPIMAANEDG | 11 | 543 |
| Fomitopsis betulina | glycoside hydrolase superfamily | KAI0733000.1 | LRLCANATEGAPG | 13 | 465 | SSAIMATNDDG | 11 | 544 |
| Rhodofomes roseus | glycoside hydrolase superfamily | XP_047785035.1 | LVQWTEADSDSDTAG | 15 | 466 | SPTIMATNDDG | 11 | 545 |
| Fomitopsis serialis | glycoside hydrolase superfamily | XP_047901050.1 | LLQWTDADTDASTS | 14 | 467 | TPPIMATNEDG | 11 | 546 |
| Daedalea quercina L-15889 | glycoside hydrolase family 18 protein | KZT74309.1 | LLHLQRTDGADSSGA LSEDVSSGGAASQ | 28 | 468 | PENATSSSPIMAANED G | 17 | 547 |
| Laetiporus sulphureus 93-53 | glycoside hydrolase family 18 protein | XP_040770041.1 | LLSDEAQNSAPPG | 13 | 469 | TPPIYAMNGDG | 11 | 548 |
| Sparassis crispa | glycoside hydrolase family 18 protein | XP_027610567.1 | LLGLTEAVVSGLETA | 15 | 470 | ASPAIQATNEDG | 12 | 549 |
| Rhodonia placenta | hypothetical protein IEO21_02855 | KAF9818340.1 | ARDWPGAFRVLAEAG | 15 | 471 | PAIQATNDDG | 10 | 550 |
| Postia placenta Mad-698-R | hypothetical protein POSPLDRAFT_134913 | EED77690.1 | ARDWPGAFRVLAEAG | 15 | 472 | PAIQATNDDG | 10 | 551 |
| Postia placenta MAD-698-R-SB12 | glycoside hydrolase family 18 protein | XP_024337101.1 | ARDWPGAFRVLAEAG | 15 | 473 | PAIQATNDDG | 10 | 552 |
| Amylocystis lapponica | glycoside hydrolase superfamily | KAH9951662.1 | LLNWADSLVSGLAAD VGDAPP | 21 | 474 | AVLAANDDG | 9 | 553 |
| Gelatoporia subvermispora B | glycoside hydrolase family 18 protein | EMD40796.1 | LFEMPGSTLAEVAAP DSVRASG | 22 | 475 | SPPYEGVVVKDEDG | 14 | 554 |
| Obba rivulosa | glycoside hydrolase | OCH92911.1 | LGAWAGSVLAATSLP DDSRIRTDN | 24 | 476 | APFAGLVVKDEDG | 13 | 555 |
| Auricularia subglabra TFB-10046 SS5 | glycoside hydrolase | EJD53756.1 | AAFDAQPDVTRGMME LAAQNDAAAPS | 26 | 477 | AEVAPLKIVNEDG | 13 | 556 |
| Auriculariales sp. MPI-PUGE-AT-0066 | glycoside hydrolase superfamily | KAH7105665.1 | PDGTIALEAPNGINV VSTPDENG | 23 | 478 | MVTIENEEG | 9 | 557 |

**Table 5 provides P2-P3 region sequences of 79 exemplary fungal glycoside hydrolases. P2 regions are underlined and P3 regions are in bold italics. SEQ ID NO is abbreviated to SID.**

| **Scientific Name from NCBI (Species)** | **Scientific Name from NCBI (Protein)** | **Accession GenBank** | **P2-P3** | **Length** | **SID** |
|---|---|---|---|---|---|
| Phellinidium pouzarii | hypothetical protein EW145_g7877 | THG95934.1 | KRKRDLTSRRFLMRRQSTGGDVDDDDGTAATADTVAAASAVG NG | 44 | 558 |
| Collybia nuda | glycoside hydrolase family 18 protein | KAF9464432.1 | RSRSTKTLKFRRLRS***LDDGSEEPG*** | 24 | 559 |
| Hohenbuehelia grisea | hypothetical protein HGRIS_009481 | KAL0949420.1 | RTRAHGIHRKRQ***DAAAVSTASTNSA*** | 25 | 560 |
| Pleurotus ostreatus PC15 | glycoside hydrolase family 18 protein | KDQ29341.1 | RQRSQHGRRHHSPRRVQG***SRKVTRDDGLG*** | 29 | 561 |
| Pleurotus ostreatus | uncharacterized protein PC9H_005000 | XP_036633081.1 | RQRSQHGRRHHSPRRVQG***SRKVTRDDGLG*** | 29 | 562 |
| Abortiporus biennis | glycosyl hydrolase 18 family protein | KAL4242086.1 | RSRSLSRRSPQYDTRPAYTFGHS LEGTSTVASTQSQDDSAVD ENTYKG | 48 | 563 |
| Pleurotus pulmonarius | glycosyl hydrolase 18 family protein | KAL4260222.1 | RQRSQHERKRHSSRRVQG***SREVARDDGLG*** | 29 | 564 |
| Pleurotus pulmonarius | hypothetical protein EYR36_006332 | KAF4574978.1 | RQRSQHERKRHSSRRVQG***SREVARDDGLG*** | 29 | 565 |
| Pleurotus cornucopiae | hypothetical protein CCMSSC00406_00014 13 | KAG9219003.1 | RQRSQHERKRHSSRRMQA***SRKVTRDDGLG*** | 29 | 566 |
| Pleurotus eryngii | glycoside hydrolase | KAF9495424.1 | RQRSQHERKRHSSRRVQG***SRKVTRDDGLG*** | 29 | 567 |
| Coprinopsis marcescibilis | glycoside hydrolase | TFK29387.1 | GRHGYGGERHHSEHYGHAHSGSEHGLGDDPEDPDDDRGDGKG DMGGEPD | 49 | 568 |
| Candolleomyces efflorescens | hypothetical protein MD484_g358, partial | KAJ2920166.1 | GNHGWHEHEDNEKHHDHQPGGSWWGDWGNGHASGGDDDDYDG GGSGSNGG | 50 | 569 |
| Candolleomyces aberdarensis | hypothetical protein EST38_g1252 | RXW24650.1 | RRNHRWHEHEDSEKHHDHQPGGDWWGNWGHGHSGGDDDDNDD GSGSSGDDNDDEHSG | 57 | 570 |
| Candolleomyces eurysporus | hypothetical protein H1R20_g6902, partial | KAJ2930238.1 | GNHRWHEHEDSEKHHEHQPGGDFWGNWGHGHSGGDDDDNDDG SGGSGTSG | 50 | 571 |
| Coprinellus micaceus | glycoside hydrolase family 18 protein | TEB35630.1 | HHWWHEHEDDEKHHVHEDDEGEGDADWHWWSGHGGWGGDNHN GDEDDDEDDDDYG | 55 | 572 |
| Tulosesus angulatus | hypothetical protein D9611_002412 | KAF5333128.1 | GHWHDHEDNEKHHIPEDEHVESEDWHHWGSGGGNTKDGGKHG DGGG | 46 | 573 |
| Tulosesus angulatus | glycoside hydrolase family 18 protein | KAF6762332.1 | GHWHDHEDNEKHHIPEDEHVESEDWHHWGSGGGNTKNGGKHG DGGG | 46 | 574 |
| Epithele typhae | glycoside hydrolase | XP_047882335.1 | GSHLHSHSHPHPHSPRRRRGVHAPRRPHRAGRVPLGRWPAGV HPTGGRRDGLLFLR | 56 | 575 |
| Lentinus tigrinus ALCF2SS1-7 | glycoside hydrolase | XP_067541768.1 | RQRQRQRRRRSP***VPAALEWFSSLADVLPG*** | 29 | 576 |
| Cerioporus squamosus | glycoside hydrolase superfamily | KAI0684816.1 | GRRNRQRRQKQRRRQDHP***GRSPVPTLEWFSSLADVFPG*** | 38 | 577 |
| Cerioporus squamosus | glycoside hydrolase | KAI0719635.1 | GRRNRQRRQKQRRRQDHP***GRSPVPTLEWFSSLADVFPG*** | 38 | 578 |
| Polyporus arcularius HHB13444 | glycoside hydrolase family 18 protein | TFK92187.1 | GRRSRPQRQRRRRREE***QGHGRRSPVPGLEWVSSLADVFPG*** | 40 | 579 |
| Lentinus brumalis | glycoside hydrolase | RDX55533.1 | GRRSRPQRPRRRRREE***QGHGRRSPAPVPGLEWVSSLADVFPG*** | 42 | 580 |
| Daedaleopsis nitida | glycoside hydrolase | KAI0750764.1 | GQRPGRRRRRRA***LLDVAAEGILPG*** | 24 | 581 |
| Fomes fomentarius | glycoside hydrolase | KAI0774197.1 | GRPRTRKRKSRNRRGRRA***PLLDWAEAVTGFLPG*** | 33 | 582 |
| Earliella scabrosa | glycoside hydrolase | KAI0723219.1 | GRRRQRRRRRLS***GLGWASEAIAGFLPG*** | 27 | 583 |
| Ganoderma leucocontextum | glycoside hydrolase | KAI1795158.1 | GLSALHRRREIRTRRR***REGLLGMLIGG*** | 27 | 584 |
| Dichomitus squalens LYAD-421 SS1 | glycoside hydrolase | XP_007365995.1 | GRRRREQRQGQGQKR***SMQRFVWDWIDSLLG*** | 30 | 585 |
| Dichomitus squalens | glycoside hydrolase | TBU49421.1 | GRRRREQRQGQGQKR***SMQRFVWDWIDSLLG*** | 30 | 586 |
| Dichomitus squalens | glycoside hydrolase | TBU32852.1 | GRRRREQRQGQGOKR***SMORFVWDWIDSLLG*** | 30 | 587 |
| Cubamyces sp. BRFM 1775 | glycoside hydrolase | KAI0327097.1 | RRRRRHRRRTQRHRAARSR***VLGWVSTLVDKTKEKLLSPG*** | 39 | 588 |
| Cubamyces lactineus | glycoside hydrolase | KAH9899952.1 | GRRRRRTRRHRAARAP***VLGWVSALVDKAKEKLLPPG*** | 36 | 589 |
| Cubamyces menziesii | glycoside hydrolase | KAI0661066.1 | RRRQRRHRRRTQRHRAARSQ***VLGWVSALVDKTKEKLLPPG*** | 40 | 590 |
| Trametes cubensis | hypothetical protein ONZ51_g6643 | KAJ8475288.1 | RRRQRRHRRRTQRHRAARSQ***VLGWVSALVDKTKEKLLPPG*** | 40 | 591 |
| Trametes coccinea BRFM310 | glycoside hydrolase family 18 protein | OSD04163.1 | GRQRRAGRRKRDAGTPHH***LLDLLGAVVDKAKNKLLPPG*** | 38 | 592 |
| Trametes sanguinea | glycoside hydrolase family 18 protein | KAI9066735.1 | GRKRRAARRKRDSGTPHH***LLDLLEAVVDNAKSKLLPPG*** | 38 | 593 |
| Trametes elegans | glycoside hydrolase superfamily | KAI0776101.1 | RRQKQRARRRSR***VLNWVGALVDKAQPFLAPG*** | 31 | 594 |
| Trametes punicea | glycoside hydrolase | KAI9001092.1 | GRSRKQKGRRARDQRRAGHSR***VLDWVGALVDKTKDKILPAG*** | 41 | 595 |
| Lenzites betulinus | glycoside hydrolase | KAH9853558.1 | MRRATQKPKGTRKANAARR***VLGWVGALVETAGAGVLG*** | 37 | 596 |
| Trametes meyenii | glycoside hydrolase | KAI0650353.1 | ARTRPRPRRRLHTR***VLDWVGALVDRKTAALPIG*** | 32 | 597 |
| Trametes maxima | glycoside hydrolase | KAI0677163.1 | ARTRKRSRRRLYTR***VLDWVGALADKTG*** | 27 | 598 |
| Trametes cingulata | glycoside hydrolase | KAI0353577.1 | ARTQVRTQSRKREGRSRRRLRSRVLEWVGALVDKTNLLPSNS G | 43 | 599 |
| Pilatotrama Ijubarskyi | glycoside hydrolase | KAI0375555.1 | RTRTRKRQRQRQRQRGRLGTRLRSRVLEWVGALVDKTKVNGL LPSG | 46 | 600 |
| Trametes polyzona | glycoside hydrolase | KAI0638105.1 | SLRSSRRRTKNGNRSRRLHGR***VLEWVGAVVEKAKTKLLPPG*** | 41 | 601 |
| Trametes pubescens | Chitinase A1 | OJT04115.1 | SRFQKRKHNGVLKTRRHR***VLDFVGALVDKTNILSAG*** | 36 | 602 |
| Trametes versicolor FP-101664 SS1 | glycoside hydrolase | XP_008035768.1 | RFHKRRHNGVRKTRRHR***VLEWVGALVDKTNILSTG*** | 35 | 603 |
| Trametes versicolor | hypothetical protein VTO73DRAFT_14992 | KAL1946888.1 | RFHRRRHNGVRKTRRHR***VLEWVGALVDKTNILSSG*** | 35 | 604 |
| Pleurotus pulmonarius | hypothetical protein EYR40_005288 | KAF4602086.1 | RQRSQHERKRHSSRRVQGSRK***IARDDGLGVVH*** | 32 | 605 |
| Abortiporus biennis | glycoside hydrolase superfamily | KAI0796747.1 | RSRSLSRRSPQYDTRPAYTFGHS**LEGTSTVASTQSQDDSAVD** **ENTYKGG** | 49 | 606 |
| Irpex lacteus | glycoside hydrolase superfamily | KAI0770846.1 | TRSLSRSKRAVPKHLPMHLDGASQAATADVSVSALGDDSLDD PSAQTEGTKPASPS | 56 | 607 |
| Irpex rosettiformis | glycoside hydrolase superfamily | KAI0092710.1 | TRSFSRSKRAVPKHLPMHVDGASQAASIADSEANVSAVGEDS GDDPLAQMEGTKTGSSG | 59 | 608 |
| Cytidiella melzeri | glycoside hydrolase | KAI0683604.1 | SRRRQRRTTTQHVPMHLDGASQEAEVAALGEEGPLAETEGAK PG | 44 | 609 |
| Trametopsis cervina | glycoside hydrolase | KAI0347133.1 | HASRRQRRAPHVPMH VDPASQEAQVTTMGEEEDPLAETEGAK SGSSPPA | 49 | 610 |
| Phanerochaete sordida | glycoside hydrolase family 18 protein | GJE84088.1 | TRATLPVRRMPTRRHLPMHLNGIPADVLGDTGVPGTLLHSDG ASADAVKAG | 51 | 611 |
| Phlebiopsis gigantea 11061_1 CR5-6 | glycoside hydrolase family 18 protein | KIP02119.1 | TRALVSRRPRHVPMHLD***GPAGDALGTDALDDPLLHAAGAPG*** | 41 | 612 |
| Hermanssonia centrifuga | hypothetical protein PHLCEN_2v1610 | PSS35455.1 | TRHIKHRRSKTHHTPIHDLTEKILPLPSDTDSPPLYADAMKP TPATAPG | 49 | 613 |
| Hermanssonia centrifuga | hypothetical protein EW026_g4420 | THG97608.1 | TRHIKHRRSKTHHTPIHDLTEKILPTSGTTDSPLYADAMKPT PVPG | 46 | 614 |
| Cerrena zonata | hypothetical protein QCA50_000573 | KAK7695934.1 | GIRNRVHKRSHHHVRRSSHNHSIGRRRLLEYTELPPTQPSIP NFSLPAANPESETLQQPAVEHLIDPTEIDQSDDSTSDTAVTN VSNQQQDSAAVPPG | 98 | 615 |
| Somion occarium | unnamed protein product | CAL1700865.1 | HSRSLLHTTTTRHYSRNRRPIQASPPTARRLLELTELGLGTR TRDSAPADSETVLQQDVHEHLVDPAEVDEQEISDAAVQADTT AGPG | 88 | 616 |
| Grifola frondosa | Chitinase-3-like protein 2 | OBZ70066.1 | RSRSHAPPRTRRSRHARWRR***LSLLQWTDALVPAPG*** | 35 | 617 |
| Taiwanofungus camphoratus | hypothetical protein AcV5_000700 | KAI0939219.1 | RARSRHRRSRHSVRRHSLYR***WPETLVSGIEADADPQSDTVPA*** | 42 | 618 |
| Taiwanofungus camphoratus | hypothetical protein AcW2_006672 | KAI0921806.1 | RARSRHRRSRHSVRRHSLYR***WPETLVSGIEADADPQSDTVPA*** | 42 | 619 |
| Taiwanofungus camphoratus | hypothetical protein AcV7_008041 | KAI0952145.1 | RARSRHRRSRHSVRRHSFYR***WPETLVSGIEADADPQSDTVPA*** | 42 | 620 |
| Taiwanofungus camphoratus | hypothetical protein AcW1_004319 | KAI0959515.1 | RARSRHRRSRHSVRRHSFYR***WPETLVSGIEADADPQSDTVPA*** | 42 | 621 |
| Fomitopsis schrenkii | hypothetical protein FOMPIDRAFT_49353 | EPT03714.1 | RSHRGHLRNRHRARSS***GASSEDVSDGASANGSEGAPG*** | 37 | 622 |
| Fomitopsis betulina | glycoside hydrolase superfamily | KAI0733000.1 | PRSHRAHLRNRHRARSS***LRLCANATEGAPG*** | 30 | 623 |
| Rhodofomes roseus | glycoside hydrolase superfamily | XP_047785035.1 | RRRSHGRRLRNRHRTRSS***LVQWTEADSDSDTAG*** | 33 | 624 |
| Fomitopsis serialis | glycoside hydrolase superfamily | XP_047901050.1 | PRRRAHRRRLRNRHRARSS***LLQWTDADTDASTS*** | 33 | 625 |
| Daedalea quercina L-15889 | glycoside hydrolase family 18 protein | KZT74309.1 | RPRSHRRRLRNRHRARSS*LLHLQRTDGADSSGALSEDVSSGG* *AASQ* | 46 | 626 |
| Laetiporus sulphureus 93-53 | glycoside hydrolase family 18 protein | XP_040770041.1 | RLRERRSRHARSRHSHLPKRMQS***LLSDEAQNSAPPG*** | 36 | 627 |
| Sparassis crispa | glycoside hydrolase family 18 protein | XP_027610567.1 | RTRSQSWHSLHRNRLRRS***LLGLTEAVVSGLETA*** | 33 | 628 |
| Rhodonia placenta | hypothetical protein IEO21_02855 | KAF9818340.1 | TRRMSRSRDRGTRHARRRAS***ARDWPGAFRVLAEAG*** | 35 | 629 |
| Postia placenta Mad-698-R | hypothetical protein POSPLDRAFT_134913 | EED77690.1 | TRRKTRSRDRGTRHARRRAS***ARDWPGAFRVLAEAG*** | 35 | 630 |
| Postia placenta MAD-698-R-SB12 | glycoside hydrolase family 18 protein | XP_024337101.1 | TRRKTRSRDRGTRHARRRAS***ARDWPGAFRVLAEAG*** | 35 | 631 |
| Amylocystis lapponica | glycoside hydrolase superfamily | KAH9951662.1 | RTRARRYKHLRRQN***LLNWADSLVSGLAADVGDAPP*** | 35 | 632 |
| Gelatoporia subvermispora B | glycoside hydrolase family 18 protein | EMD40796.1 | GHRHRHRRRGEDRDPGLARERRQLFEMPGSTLAEVAAPDSVR ASG | 45 | 633 |
| Obba rivulosa | glycoside hydrolase | OCH92911.1 | RTRYRHRRQHRLRPRHT***LGAWAGSVLAATSLPDDSRIRTDN*** | 41 | 634 |
| Auricularia subglabra TFB-10046 SS5 | glycoside hydrolase | EJD53756.1 | HLSGRRRRHEPSQ***AAFDAQPDVTRGMMELAAQNDAAAPS*** | 39 | 635 |
| Auriculariales sp. MPI-PUGE-AT-0066 | glycoside hydrolase superfamily | KAH7105665.1 | RLRNRRSA***PDGTIALEAPNGINVVSTPDENG*** | 31 | 636 |

**Table 6 provides P2-P3-P4 region sequences of 79 exemplary fungal glycoside hydrolases. P2 regions are underlined, P3 regions are in bold italics, and P4 regions are the regions C-terminal to the P3 regions. SEQ ID NO is abbreviated to SID.**

| **Scientific Name from NCBI (Species)** | **Scientific Name from NCBI (Protein)** | **Accession GenBank** | **P2-P3-P4** | **Length** | **SID** |
|---|---|---|---|---|---|
| Phellinidium pouzarii | hypothetical protein EW145_g7877 | THG95934.1 | KRKRDLTSRRFLMRRQSTGGDVDDDDGTAATADTVAAASAV GNGLITLKSSDG | 53 | 637 |
| Collybia nuda | glycoside hydrolase family 18 protein | KAF9464432.1 | RSRSTKTLKFRRLRS***LDDGSEEPG***TTVKIIGEDG | 34 | 638 |
| Hohenbuehelia grisea | hypothetical protein HGRIS_009481 | KAL0949420.1 | RTRAHGIHRKRQ***DAAAVSTASTNSA***FRVTDEDG | 33 | 639 |
| Pleurotus ostreatus PC15 | glycoside hydrolase family 18 protein | KDQ29341.1 | RQRSQHGRRHHSPRRVQG***SRKVTRDDGLG***VVHVASEDG | 38 | 640 |
| Pleurotus ostreatus | uncharacterized protein PC9H_005000 | XP_036633081.1 | RQRSQHGRRHHSPRRVQG***SRKVTRDDGLG***VVHVASEDG | 38 | 641 |
| Abortiporus biennis | glycosyl hydrolase 18 family protein | KAL4242086.1 | RSRSLSRRSPQYDTRPAYTFGHSLEGTSTVASTQSQDDSAV DENTYKGGIVIATNEDG | 58 | 642 |
| Pleurotus pulmonarius | glycosyl hydrolase 18 family protein | KAL4260222.1 | RQRSQHERKRHSSRRVQG***SREVARDDGLG***VVHVVSGDG | 38 | 643 |
| Pleurotus pulmonarius | hypothetical protein EYR36_006332 | KAF4574978.1 | RQRSQHERKRHSSRRVQG***SREVARDDGLG***VVHVVSGDG | 38 | 644 |
| Pleurotus cornucopiae | hypothetical protein CCMSSC00406_0001413 | KAG9219003.1 | RQRSQHERKRHSSRRMQA***SRKVTRDDGLG***VVHVVSEDG | 38 | 645 |
| Pleurotus eryngii | glycoside hydrolase | KAF9495424.1 | RQRSQHERKRHSSRRVQG***SRKVTRDDGLG***VVHVVSEDG | 38 | 646 |
| Coprinopsis marcescibilis | glycoside hydrolase | TFK29387.1 | GRHGYGGERHHSEHYGHAHSGSEHGLGDDPEDPDDDRGDGK GDMGGEPDIAPIQITE | 57 | 647 |
| Candolleomyces efflorescens | hypothetical protein MD484_g358, partial | KAJ2920166.1 | GNHGWHEHEDNEKHHDHQPGGSWWGDWGNGHASGGDDDDYD GGGSGSNGGDYDGHPDRPPFPANPGG | 67 | 648 |
| Candolleomyces aberdarensis | hypothetical protein EST38_g1252 | RXW24650.1 | RRNHRWHEHEDSEKHHDHQPGGDWWGNWGHGHSGGDDDDND DGSGSSGDDNDDEHSGPPPLPANPGG | 67 | 649 |
| Candolleomyces eurysporus | hypothetical protein H1R20_g6902, partial | KAJ2930238.1 | GNHRWHEHEDSEKHHEHQPGGDFWGNWGHGHSGGDDDDNDD GSGGSGTSGDDGEHPGPSPLPANPGG | 67 | 650 |
| Coprinellus micaceus | glycoside hydrolase family 18 protein | TEB35630.1 | HHWWHEHEDDEKHHVHEDDEGEGDADWHWWSGHGGWGGDNH NGDEDDDEDDDDYGREGGYRPPKSPPANPDS | 72 | 651 |
| Tulosesus angulatus | hypothetical protein D9611_002412 | KAF5333128.1 | GHWHDHEDNEKHHIPEDEHVESEDWHHWGSGGGNTKDGGKH GDGGGLDDDEAGHPSTPENAGA | 63 | 652 |
| Tulosesus angulatus | glycoside hydrolase family 18 protein | KAF6762332.1 | GHWHDHEDNEKHHIPEDEHVESEDWHHWGSGGGNTKNGGKH GDGGGLDDDEAGHPSTPENAGA | 63 | 653 |
| Epithele typhae | glycoside hydrolase | XP_047882335.1 | GSHLHSHSHPHPHSPRRRRGVHAPRRPHRAGRVPLGRWPAG VHPTGGRRDGLLFLRRRRRAAARPNDDG | 69 | 654 |
| Lentinus tigrinus ALCF2SS1-7 | glycoside hydrolase | XP_067541768.1 | RQRQRQRRRRSP***VPAALEWFSSLADVLPG***AVRVKNEDG | 38 | 655 |
| Cerioporus squamosus | glycoside hydrolase superfamily | KAI0684816.1 | GRRNRQRRQKQRRRQDHPGRSPVPTLEWFSSLADVFPGVVR VQNEDG | 47 | 656 |
| Cerioporus squamosus | glycoside hydrolase | KAI0719635.1 | GRRNRQRRQKQRRRQDHPGRSPVPTLEWFSSLADVFPGVVR VQNEDG | 47 | 657 |
| Polyporus arcularius HHB13444 | glycoside hydrolase family 18 protein | TFK92187.1 | GRRSRPQRQRRRRREEQGHGRRSPVPGLEWVSSLADVFPGG VRVENEDG | 49 | 658 |
| Lentinus brumalis | glycoside hydrolase | RDX55533.1 | GRRSRPQRPRRRRREEQGHGRRSPAPVPGLEWVSSLADVFP GGVRVENEDG | 51 | 659 |
| Daedaleopsis nitida | glycoside hydrolase | KAI0750764.1 | *GQRPGRRRRRA**LLDVAAEGILPG***VVRVKNEDG | 33 | 660 |
| Fomes fomentarius | glycoside hydrolase | KAI0774197.1 | GRPRTRKRKSRNRRGRRAPLLDWAEAVTGFLPGAIRVKNDD G | 42 | 661 |
| Earliella scabrosa | glycoside hydrolase | KAI0723219.1 | GRRRQRRRRRLS***GLGWASEAIAGFLPG***AIRVKSEDG | 36 | 662 |
| Ganoderma leucocontextum | glycoside hydrolase | KAI1795158.1 | GLSALHRRREIRTRRR***REGLLGMLIGG***SNVVVNEDG | 36 | 663 |
| Dichomitus squalens LYAD-421 SS1 | glycoside hydrolase | XP_007365995.1 | GRRRREQRQGQGQKRSMQRFVWDWIDSLLGPPVYDTDKSGL LVNEDG | 47 | 664 |
| Dichomitus squalens | glycoside hydrolase | TBU49421.1 | GRRRREQRQGQGQKRSMQRFVWDWIDSLLGPPVYDTDKSGL LVNEDG | 47 | 665 |
| Dichomitus squalens | glycoside hydrolase | TBU32852.1 | GRRRREQRQGQGQKRSMQRFVWDWIDSLLGPPVYDKSGLIV NEDG | 45 | 666 |
| Cubamyces sp. BRFM 1775 | glycoside hydrolase | KAI0327097.1 | RRRRRHRRRTQRHRAARSRVLGWVSTLVDKTKEKLLSPGKT ATRVVNEDG | 50 | 667 |
| Cubamyces lactineus | glycoside hydrolase | KAH9899952.1 | GRRRRRTRRHRAARAPVLGWVSALVDKAKEKLLPPGKTATA TRVVNEDG | 49 | 668 |
| Cubamyces menziesii | glycoside hydrolase | KAI0661066.1 | RRRQRRHRRRTQRHRAARSQVLGWVSALVDKTKEKLLPPGK TATRVVNEDG | 51 | 669 |
| Trametes cubensis | hypothetical protein ONZ51_g6643 | KAJ8475288.1 | RRRQRRHRRRTQRHRAARSQVLGWVSALVDKTKEKLLPPGK TATRVVNEDG | 51 | 670 |
| Trametes coccinea BRFM310 | glycoside hydrolase family 18 protein | OSD04163.1 | GRQRRAGRRKRDAGTPHHLLDLLGAVVDKAKNKLLPPGQTL VTNEDG | 47 | 671 |
| Trametes sanguinea | glycoside hydrolase family 18 protein | KAI9066735.1 | GRKRRAARRKRDSGTPHHLLDLLEAVVDNAKSKLLPPGQTL VTNEDG | 47 | 672 |
| Trametes elegans | glycoside hydrolase superfamily | KAI0776101.1 | RRQKQRARRRSR***VLNWVGALVDKAQPFLAPG***RTLVVNEDG | 40 | 673 |
| Trametes punicea | glycoside hydrolase | KAI9001092.1 | GRSRKQKGRRARDQRRAGHSRVLDWVGALVDKTKDKILPAG QTLIVNEDG | 50 | 674 |
| Lenzites betulinus | glycoside hydrolase | KAH9853558.1 | MRRATQKPKGTRKANAARRVLGWVGALVETAGAGVLGPDAQ VHNEAG | 47 | 675 |
| Trametes meyenii | glycoside hydrolase | KAI0650353.1 | ARTRPRPRRRLHTR***VLDWVGALVDKTAALPIG***QTLVVNEDG | 41 | 676 |
| Trametes maxima | glycoside hydrolase | KAI0677163.1 | ARTRKRSRRRLYTR***VLDWVGALADKTG***VLPLAKTLVVNEDG | 41 | 677 |
| Trametes cingulata | glycoside hydrolase | KAI0353577.1 | ARTQVRTQSRKREGRSRRRLRSRVLEWVGALVDKTNLLPSN SGARLVYNEDG | 52 | 678 |
| Pilatotrama Ijubarskyi | glycoside hydrolase | KAI0375555.1 | RTRTRKRQRQRQRQRGRLGTRLRSRVLEWVGALVDKTKVNG LLPSGARLAYNEDG | 55 | 679 |
| Trametes polyzona | glycoside hydrolase | KAI0638105.1 | SLRSSRRRTKNGNRSRRLHGRVLEWVGAVVEKAKTKLLPPG GLLVYNEDG | 50 | 680 |
| Trametes pubescens | Chitinase A1 | OJT04115.1 | SRFQKRKHNGVLKTRRHRVLDFVGALVDKTNILSAGTTLVH NEDG | 45 | 681 |
| Trametes versicolor FP-101664 SS1 | glycoside hydrolase | XP_008035768.1 | RFHKRRHNGVRKTRRHRVLEWVGALVDKTNILSTGSTLVHN EDG | 44 | 682 |
| Trametes versicolor | hypothetical protein VTO73DRAFT_14992 | KAL1946888.1 | RFHRRRHNGVRKTRRHRVLEWVGALVDKTNILSSGSTLVHN EDG | 44 | 683 |
| Pleurotus pulmonarius | hypothetical protein EYR40_005288 | KAF4602086.1 | RQRSQHERKRHSSRRVQGSRK***IARDDGLGVVH***VVSGDG | 38 | 684 |
| Abortiporus biennis | glycoside hydrolase superfamily | KAI0796747.1 | RSRSLSRRSPQYDTRPAYTFGHSLEGTSTVASTQSQDDSAV DENTYKGGIVIATNEDG | 58 | 685 |
| Irpex lacteus | glycoside hydrolase superfamily | KAI0770846.1 | TRSLSRSKRAVPKHLPMHLDGASQAATADVSVSALGDDSLD DPSAQTEGTKPASPSAFMVHNEDG | 65 | 686 |
| Irpex rosettiformis | glycoside hydrolase superfamily | KAI0092710.1 | TRSFSRSKRAVPKHLPMHVDGASQAASIADSEANVSAVGED SGDDPLAQMEGTKTGSSGSSSAFMVHNEDG | 71 | 687 |
| Cytidiella melzeri | glycoside hydrolase | KAI0683604.1 | SRRRQRRTTTQHVPMHLDGASQEAEVAALGEEGPLAETEGA KPGSPNTPPAYMVQNEDG | 59 | 688 |
| Trametopsis cervina | glycoside hydrolase | KAI0347133.1 | HASRRQRRAPHVPMHVDPASQEAQVTTMGEEEDPLAETEGA KSGSSPPAAFMVHNEDG | 58 | 689 |
| Phanerochaete sordida | glycoside hydrolase family 18 protein | GJE84088.1 | TRATLPVRRMPTRRHLPMHLNGIPADVLGDTGVPGTLLHSD GASADAVKAGVPMVMNEDG | 60 | 690 |
| Phlebiopsis gigantea 11061_1 CR5-6 | glycoside hydrolase family 18 protein | KIP02119.1 | TRALVSRRPRHVPMHLDGPAGDALGTDALDDPLLHAAGAPG AAPAVTNEDG | 51 | 691 |
| Hermanssonia centrifuga | hypothetical protein PHLCEN_2v1610 | PSS35455.1 | TRHIKHRRSKTHHTPIHDLTEKILPLPSDTDSPPLYADAMK PTPATAPGVLVANDDG | 57 | 692 |
| Hermanssonia centrifuga | hypothetical protein EW026_g4420 | THG97608.1 | TRHIKHRRSKTHHTPIHDLTEKILPTSGTTDSPLYADAMKP TPVPGVMVANDDG | 54 | 693 |
| Cerrena zonata | hypothetical protein QCA50_000573 | KAK7695934.1 | GIRNRVHKRSHHHVRRSSHNHSIGRRRLLEYTELPPTQPSI PNFSLPAANPESETLQQPAVEHLIDPTEIDQSDDSTSDTAV TNVSNQQQDSAAVPPGTTIIQNEDG | 107 | 694 |
| Somion occarium | unnamed protein product | CAL1700865.1 | HSRSLLHTTTTRHYSRNRRPIQASPPTARRLLELTELGLGT RTRDSAPADSETVLQQDVHEHLVDPAEVDEQEISDAAVQAD TTAGPGSTLVTNEDG | 97 | 695 |
| Grifola frondosa | Chitinase-3-like protein 2 | OBZ70066.1 | RSRSHAPPRTRRSRHARWRRLSLLQWTDALVPAPGPLLPPP VLATSEDG | 49 | 696 |
| Taiwanofungus camphoratus | hypothetical protein AcV5_000700 | KAI0939219.1 | RARSRHRRSRHSVRRHSLYRWPETLVSGIEADADPQSDTVP AAVVATNEDG | 51 | 697 |
| Taiwanofungus camphoratus | hypothetical protein AcW2_006672 | KAI0921806.1 | RARSRHRRSRHSVRRHSLYRWPETLVSGIEADADPQSDTVP AAVVATNEDG | 51 | 698 |
| Taiwanofungus camphoratus | hypothetical protein AcV7_008041 | KAI0952145.1 | RARSRHRRSRHSVRRHSFYRWPETLVSGIEADADPQSDTVP AAVVATNEDG | 51 | 699 |
| Taiwanofungus camphoratus | hypothetical protein AcW1_004319 | KAI0959515.1 | RARSRHRRSRHSVRRHSFYRWPETLVSGIEADADPQSDTVP AAVVATNEDG | 51 | 700 |
| Fomitopsis schrenkii | hypothetical protein FOMPIDRAFT_49353 | EPT03714.1 | RSHRGHLRNRHRARSSGASSEDVSDGASANGSEGAPGISPI MAANEDG | 48 | 701 |
| Fomitopsis betulina | glycoside hydrolase superfamily | KAI0733000.1 | PRSHRAHLRNRHRARSS***LRLCANATEGAPG***SSAIMATNDDG | 41 | 702 |
| Rhodofomes roseus | glycoside hydrolase superfamily | XP_047785035.1 | RRRSHGRRLRNRHRTRSSLVQWTEADSDSDTAGSPTIMATN DDG | 44 | 703 |
| Fomitopsis serialis | glycoside hydrolase superfamily | XP_047901050.1 | PRRRAHRRRLRNRHRARSSLLQWTDADTDASTSTPPIMATN EDG | 44 | 704 |
| Daedalea quercina L-15889 | glycoside hydrolase family 18 protein | KZT74309.1 | RPRSHRRRLRNRHRARSSLLHLQRTDGADSSGALSEDVSSG GAASQPENATSSSPIMAANEDG | 63 | 705 |
| Laetiporus sulphureus 93-53 | glycoside hydrolase family 18 protein | XP_040770041.1 | RLRERRSRHARSRHSHLPKRMQSLLSDEAQNSAPPGTPPIY AMNGDG | 47 | 706 |
| Sparassis crispa | glycoside hydrolase family 18 protein | XP_027610567.1 | RTRSQSWHSLHRNRLRRSLLGLTEAVVSGLETAASPAIQAT NEDG | 45 | 707 |
| Rhodonia placenta | hypothetical protein IEO21_02855 | KAF9818340.1 | TRRMSRSRDRGTRHARRRASARDWPGAFRVLAEAGPAIQAT NDDG | 45 | 708 |
| Postia placenta Mad-698-R | hypothetical protein POSPLDRAFT_134913 | EED77690.1 | TRRKTRSRDRGTRHARRRASARDWPGAFRVLAEAGPAIQAT NDDG | 45 | 709 |
| Postia placenta MAD-698-R-SB12 | glycoside hydrolase family 18 protein | XP_024337101.1 | TRRKTRSRDRGTRHARRRASARDWPGAFRVLAEAGPAIQAT NDDG | 45 | 710 |
| Amylocystis lapponica | glycoside hydrolase superfamily | KAH9951662.1 | RTRARRYKHLRRQNLLNWADSLVSGLAADVGDAPPAVLAAN DDG | 44 | 711 |
| Gelatoporia subvermispora B | glycoside hydrolase family 18 protein | EMD40796.1 | GHRHRHRRRGEDRDPGLARERRQLFEMPGSTLAEVAAPDSV RASGSPPYEGVVVKDEDG | 59 | 712 |
| Obba rivulosa | glycoside hydrolase | OCH92911.1 | RTRYRHRRQHRLRPRHTLGAWAGSVLAATSLPDDSRIRTDN APFAGLVVKDEDG | 54 | 713 |
| Auricularia subglabra TFB-10046 SS5 | glycoside hydrolase | EJD53756.1 | HLSGRRRRHEPSQAAFDAQPDVTRGMMELAAQNDAAAPSAE VAPLKIVNEDG | 52 | 714 |
| Auriculariales sp. MPI-PUGE-AT-0066 | glycoside hydrolase superfamily | KAH7105665.1 | RLRNRRSA***PDGTIALEAPNGINVVSTPDENG***MVTIENEEG | 40 | 715 |

### Cell Penetrating Peptide Region Lengths

In some embodiments, the P1 region is 3 to 35 amino acids in length. In some embodiments the P1 region is 3, 5, 6, 7, 11, 12, 13, 14, 16, 17, 18, 19, 20, 21, 23, 25, 27, 31, or 35 amino acids in length. In some embodiments the P1 region is 3 to 31 amino acids in length. In some embodiments the P1 region is 3 to 27 amino acids in length.

In some embodiments, the P2 region is 8 to 30 amino acids in length. In some embodiments the P2 region is 8, 12, 13, 14, 15,16, 17, 18, 19, 20, 21, 22, 23, 25, 27, or 30 amino acids in length. In some embodiments the P2 region is 8 to 25 amino acids in length.

In some embodiments, the P3 region is 9 to 71 amino acids in length. In some embodiments the P3 region is 9, 11, 12, 13, 14, 15, 17, 18, 19, 20, 21, 22, 23, 24, 25, 26, 27, 28, 30, 31, 34, 36, 37, 38, 41, 58, or 71 amino acids in length. In some embodiments the P3 region is 9 to 41 amino acids in length. In some embodiments the P3 region is 9 to 38 amino acids in length.

In some embodiments, the P4 region is 8 to 17 amino acids in length. In some embodiments the P4 region is 8, 9, 10, 11, 12, 13, 14, 15, or 17 amino acids in length.

In some embodiments, the P2-P3 region is 24 to 98 amino acids in length. In some embodiments the P2-P3 region is 24, 25, 27, 29, 30, 31, 32, 33, 35, 36, 37, 38, 39, 40, 41, 42, 43, 44, 45, 46, 48, 49, 50, 51, 55, 56, 57, 59, 88, or 98 amino acids in length. In some embodiments the P2-P3 region is 24 to 59 amino acids in length. In some embodiments the P2-P3 region is 24 to 56 amino acids in length. In a preferred embodiment, the P2-P3 region is 27 to 33 amino acids in length, such as 27, 29, or 33 amino acids in length.

In some embodiments, the P2-P3-P4 region is 33 to 107 amino acids in length. In some embodiments the P2-P3-P4 region is 33, 34, 35, 36, 38, 40, 41, 42, 44, 45, 47, 48, 49, 50, 51, 52, 53, 54, 55, 57, 58, 59, 60, 63, 65, 67, 69, 71, 72, 97, or 107 amino acids in length. In some embodiments the P2-P3-P4 region is 33 to 72 amino acids in length. In some embodiments the P2-P3-P4 region is 33 to 71 amino acids in length. In some embodiments the P2-P3-P4 region is 33 to 65 amino acids in length. In a preferred embodiment, the P2-P3-P4 region is 35 to 42 amino acids in length, such as 36, 38, or 42 amino acids in length.

In some embodiments, the P1-P2-P3-P4 region (i.e., the protein loop domain) is 38 to 142 amino acids in length. In some embodiments the P1-P2-P3-P4 region is 38, 39, 43, 49, 50, 53, 54, 55, 56, 57, 59, 60, 63, 64, 65, 66, 67, 68, 69, 70, 71, 72, 73, 74, 75, 77, 79, 80, 81, 88, 89, 102, or 142 amino acids in length. In some embodiments the P1-P2-P3-P4 region is 38 to 89 amino acids in length. In some embodiments the P1-P2-P3-P4 region is 38 to 81 amino acids in length. In some embodiments the P1-P2-P3-P4 region is 38 to 75 amino acids in length. In a preferred embodiment, the P1-P2-P3-P4 region is 54 to 65 amino acids in length, such as 54, 55, or 65 amino acids in length.

### Cell Penetrating Peptides Comprising a P2-P3 Region Sequence

In a preferred embodiment, the amino acid sequence of the CPP comprises a contiguous amino acid portion having at least 80% sequence identity to the amino acid sequence of a P2-P3 region of a fungal glycoside hydrolase, i.e., any one of SEQ ID NOs: 558-636. In a preferred embodiment, the amino acid sequence of the CPP comprises a contiguous amino acid portion having at least 80% sequence identity to the amino acid sequence of any one of SEQ ID NOs: 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 629, 630, 631, 632, 633, 634, 635, and 636. In further embodiments, the contiguous amino acid portion has at least 85% sequence identity to any one of SEQ ID NOs: 558-636. In further embodiments, the contiguous amino acid portion has at least 90% sequence identity to any one of SEQ ID NOs: 558-636. In further embodiments, the contiguous amino acid portion has at least 95% sequence identity to any one of SEQ ID NOs: 558-636. In further embodiments, the contiguous amino acid portion has 100% sequence identity to any one of SEQ ID NOs: 558-636. In further embodiments, the CPP consists of the contiguous amino acid portion.

In some embodiments, the contiguous amino acid portion has at least 80% sequence identity to the amino acid sequence of SEQ ID NOs: 558. In some embodiments, the contiguous amino acid portion has at least 80% sequence identity to the amino acid sequence of SEQ ID NOs: 559. In some embodiments, the contiguous amino acid portion has at least 80% sequence identity to the amino acid sequence of SEQ ID NOs: 560. In some embodiments, the contiguous amino acid portion has at least 80% sequence identity to the amino acid sequence of SEQ ID NOs: 561. In some embodiments, the contiguous amino acid portion has at least 80% sequence identity to the amino acid sequence of SEQ ID NOs: 562. In some embodiments, the contiguous amino acid portion has at least 80% sequence identity to the amino acid sequence of SEQ ID NOs: 563. In some embodiments, the contiguous amino acid portion has at least 80% sequence identity to the amino acid sequence of SEQ ID NOs: 564. In some embodiments, the contiguous amino acid portion has at least 80% sequence identity to the amino acid sequence of SEQ ID NOs: 565. In some embodiments, the contiguous amino acid portion has at least 80% sequence identity to the amino acid sequence of SEQ ID NOs: 566. In some embodiments, the contiguous amino acid portion has at least 80% sequence identity to the amino acid sequence of SEQ ID NOs: 567. In some embodiments, the contiguous amino acid portion has at least 80% sequence identity to the amino acid sequence of SEQ ID NOs: 568. In some embodiments, the contiguous amino acid portion has at least 80% sequence identity to the amino acid sequence of SEQ ID NOs: 569. In some embodiments, the contiguous amino acid portion has at least 80% sequence identity to the amino acid sequence of SEQ ID NOs: 570. In some embodiments, the contiguous amino acid portion has at least 80% sequence identity to the amino acid sequence of SEQ ID NOs: 571. In some embodiments, the contiguous amino acid portion has at least 80% sequence identity to the amino acid sequence of SEQ ID NOs: 572. In some embodiments, the contiguous amino acid portion has at least 80% sequence identity to the amino acid sequence of SEQ ID NOs: 573. In some embodiments, the contiguous amino acid portion has at least 80% sequence identity to the amino acid sequence of SEQ ID NOs: 574. In some embodiments, the contiguous amino acid portion has at least 80% sequence identity to the amino acid sequence of SEQ ID NOs: 575. In some embodiments, the contiguous amino acid portion has at least 80% sequence identity to the amino acid sequence of SEQ ID NOs: 576. In some embodiments, the contiguous amino acid portion has at least 80% sequence identity to the amino acid sequence of SEQ ID NOs: 577. In some embodiments, the contiguous amino acid portion has at least 80% sequence identity to the amino acid sequence of SEQ ID NOs: 578. In some embodiments, the contiguous amino acid portion has at least 80% sequence identity to the amino acid sequence of SEQ ID NOs: 579. In some embodiments, the contiguous amino acid portion has at least 80% sequence identity to the amino acid sequence of SEQ ID NOs: 580. In some embodiments, the contiguous amino acid portion has at least 80% sequence identity to the amino acid sequence of SEQ ID NOs: 581. In some embodiments, the contiguous amino acid portion has at least 80% sequence identity to the amino acid sequence of SEQ ID NOs: 582. In some embodiments, the contiguous amino acid portion has at least 80% sequence identity to the amino acid sequence of SEQ ID NOs: 583. In some embodiments, the contiguous amino acid portion has at least 80% sequence identity to the amino acid sequence of SEQ ID NOs: 584. In some embodiments, the contiguous amino acid portion has at least 80% sequence identity to the amino acid sequence of SEQ ID NOs: 585. In some embodiments, the contiguous amino acid portion has at least 80% sequence identity to the amino acid sequence of SEQ ID NOs: 586. In some embodiments, the contiguous amino acid portion has at least 80% sequence identity to the amino acid sequence of SEQ ID NOs: 587. In some embodiments, the contiguous amino acid portion has at least 80% sequence identity to the amino acid sequence of SEQ ID NOs: 588. In some embodiments, the contiguous amino acid portion has at least 80% sequence identity to the amino acid sequence of SEQ ID NOs: 589. In some embodiments, the contiguous amino acid portion has at least 80% sequence identity to the amino acid sequence of SEQ ID NOs: 590. In some embodiments, the contiguous amino acid portion has at least 80% sequence identity to the amino acid sequence of SEQ ID NOs: 591. In some embodiments, the contiguous amino acid portion has at least 80% sequence identity to the amino acid sequence of SEQ ID NOs: 592. In some embodiments, the contiguous amino acid portion has at least 80% sequence identity to the amino acid sequence of SEQ ID NOs: 593. In some embodiments, the contiguous amino acid portion has at least 80% sequence identity to the amino acid sequence of SEQ ID NOs: 594. In some embodiments, the contiguous amino acid portion has at least 80% sequence identity to the amino acid sequence of SEQ ID NOs: 595. In some embodiments, the contiguous amino acid portion has at least 80% sequence identity to the amino acid sequence of SEQ ID NOs: 596. In some embodiments, the contiguous amino acid portion has at least 80% sequence identity to the amino acid sequence of SEQ ID NOs: 597. In some embodiments, the contiguous amino acid portion has at least 80% sequence identity to the amino acid sequence of SEQ ID NOs: 598. In some embodiments, the contiguous amino acid portion has at least 80% sequence identity to the amino acid sequence of SEQ ID NOs: 599. In some embodiments, the contiguous amino acid portion has at least 80% sequence identity to the amino acid sequence of SEQ ID NOs: 600. In some embodiments, the contiguous amino acid portion has at least 80% sequence identity to the amino acid sequence of SEQ ID NOs: 601. In some embodiments, the contiguous amino acid portion has at least 80% sequence identity to the amino acid sequence of SEQ ID NOs: 602. In some embodiments, the contiguous amino acid portion has at least 80% sequence identity to the amino acid sequence of SEQ ID NOs: 603. In some embodiments, the contiguous amino acid portion has at least 80% sequence identity to the amino acid sequence of SEQ ID NOs: 604. In some embodiments, the contiguous amino acid portion has at least 80% sequence identity to the amino acid sequence of SEQ ID NOs: 605. In some embodiments, the contiguous amino acid portion has at least 80% sequence identity to the amino acid sequence of SEQ ID NOs: 606. In some embodiments, the contiguous amino acid portion has at least 80% sequence identity to the amino acid sequence of SEQ ID NOs: 607. In some embodiments, the contiguous amino acid portion has at least 80% sequence identity to the amino acid sequence of SEQ ID NOs: 608. In some embodiments, the contiguous amino acid portion has at least 80% sequence identity to the amino acid sequence of SEQ ID NOs: 609. In some embodiments, the contiguous amino acid portion has at least 80% sequence identity to the amino acid sequence of SEQ ID NOs: 610. In some embodiments, the contiguous amino acid portion has at least 80% sequence identity to the amino acid sequence of SEQ ID NOs: 611. In some embodiments, the contiguous amino acid portion has at least 80% sequence identity to the amino acid sequence of SEQ ID NOs: 612. In some embodiments, the contiguous amino acid portion has at least 80% sequence identity to the amino acid sequence of SEQ ID NOs: 613. In some embodiments, the contiguous amino acid portion has at least 80% sequence identity to the amino acid sequence of SEQ ID NOs: 614. In some embodiments, the contiguous amino acid portion has at least 80% sequence identity to the amino acid sequence of SEQ ID NOs: 615. In some embodiments, the contiguous amino acid portion has at least 80% sequence identity to the amino acid sequence of SEQ ID NOs: 616. In some embodiments, the contiguous amino acid portion has at least 80% sequence identity to the amino acid sequence of SEQ ID NOs: 617. In some embodiments, the contiguous amino acid portion has at least 80% sequence identity to the amino acid sequence of SEQ ID NOs: 618. In some embodiments, the contiguous amino acid portion has at least 80% sequence identity to the amino acid sequence of SEQ ID NOs: 619. In some embodiments, the contiguous amino acid portion has at least 80% sequence identity to the amino acid sequence of SEQ ID NOs: 620. In some embodiments, the contiguous amino acid portion has at least 80% sequence identity to the amino acid sequence of SEQ ID NOs: 621. In some embodiments, the contiguous amino acid portion has at least 80% sequence identity to the amino acid sequence of SEQ ID NOs: 622. In some embodiments, the contiguous amino acid portion has at least 80% sequence identity to the amino acid sequence of SEQ ID NOs: 623. In some embodiments, the contiguous amino acid portion has at least 80% sequence identity to the amino acid sequence of SEQ ID NOs: 624. In some embodiments, the contiguous amino acid portion has at least 80% sequence identity to the amino acid sequence of SEQ ID NOs: 625. In some embodiments, the contiguous amino acid portion has at least 80% sequence identity to the amino acid sequence of SEQ ID NOs: 626. In some embodiments, the contiguous amino acid portion has at least 80% sequence identity to the amino acid sequence of SEQ ID NOs: 627. In some embodiments, the contiguous amino acid portion has at least 80% sequence identity to the amino acid sequence of SEQ ID NOs: 628. In some embodiments, the contiguous amino acid portion has at least 80% sequence identity to the amino acid sequence of SEQ ID NOs: 629. In some embodiments, the contiguous amino acid portion has at least 80% sequence identity to the amino acid sequence of SEQ ID NOs: 630. In some embodiments, the contiguous amino acid portion has at least 80% sequence identity to the amino acid sequence of SEQ ID NOs: 631. In some embodiments, the contiguous amino acid portion has at least 80% sequence identity to the amino acid sequence of SEQ ID NOs: 632. In some embodiments, the contiguous amino acid portion has at least 80% sequence identity to the amino acid sequence of SEQ ID NOs: 633. In some embodiments, the contiguous amino acid portion has at least 80% sequence identity to the amino acid sequence of SEQ ID NOs: 634. In some embodiments, the contiguous amino acid portion has at least 80% sequence identity to the amino acid sequence of SEQ ID NOs: 635. In some embodiments, the contiguous amino acid portion has at least 80% sequence identity to the amino acid sequence of SEQ ID NOs: 636. In further embodiments, the contiguous amino acid portion has at least 85% sequence identity to the amino acid sequence of the P2-P3 region of the fungal glycoside hydrolase. In further embodiments, the contiguous amino acid portion has at least 90% sequence identity to the amino acid sequence of the P2-P3 region of the fungal glycoside hydrolase. In further embodiments, the contiguous amino acid portion has at least 95% sequence identity to the amino acid sequence of the P2-P3 region of the fungal glycoside hydrolase. In further embodiments, the contiguous amino acid portion has 100% sequence identity to the amino acid sequence of the P2-P3 region of the fungal glycoside hydrolase. In further embodiments, the CPP consists of the contiguous amino acid portion.

In another preferred embodiment, the amino acid sequence of the CPP comprises a contiguous amino acid portion having at least 80% sequence identity to the amino acid sequence of a P2-P3 region, wherein the P2 region has the sequence of one of SEQ ID NOs: 321-399, the P3 region has the sequence of one of SEQ ID NOs: 400-478, and wherein the P2 region and the P3 region are from different fungal glycoside hydrolases.

In some embodiments, the amino acid sequence of the CPP comprises a contiguous amino acid portion consisting of i) a P2-P3 region having at least 80% sequence identity to the amino acid sequence of any one of SEQ ID NOs: 558-636 and ii) part of a P4 region having the amino acid sequence of any one of SEQ ID NOs: 479-557, wherein the P4 region part is C-terminal to the P2-P3 region. The P4 region part can consist of any length portion of the P4 region sequence, including the full length P4 region sequence. In some embodiments, the P4 region part consists of the first, the first two, the first three, the first four, the first five, the first six, the first seven, the first eight, the first nine, or the first ten amino acids of the P4 region sequence. In a preferred embodiment, the P4 region part consists of the first amino acid of the P4 region sequence. The P2-P3 region and the P4 region part may be from the same fungal glycoside hydrolase, or from different fungal glycoside hydrolases. In further embodiments, the P2-P3 region of the CPP has at least 85% sequence identity to the amino acid sequence of any one of SEQ ID NOs: 558-636. In further embodiments, the P2-P3 region of the CPP has at least 90% sequence identity to the amino acid sequence of any one of SEQ ID NOs: 558-636. In further embodiments, the P2-P3 region of the CPP has at least 95% sequence identity to the amino acid sequence of any one of SEQ ID NOs: 558-636. In further embodiments, the P2-P3 region of the CPP has 100% sequence identity to the amino acid sequence of any one of SEQ ID NOs: 558-636. In further embodiments, the CPP consists of the contiguous amino acid portion.

In some embodiments, the amino acid sequence of the CPP comprises a contiguous amino acid portion consisting of i) a P2-P3 region having at least 80% sequence identity to the amino acid sequence of any one of SEQ ID NOs: 558-636 and ii) part of a P1 region having the amino acid sequence of any one of SEQ ID NOs: 245-320, the amino acid sequence RAT, the amino acid sequence TAT, or the amino acid sequence SAT, wherein the P1 region part is N-terminal to the P2-P3 region. The P1 region part can consist of any length portion of the P1 region sequence, including the full length P1 region sequence. In a preferred embodiment, the P1 region part consists of the last ten, the last nine, the last eight, the last seven, the last six, the last five, the last four, the last three, the last two, or the last amino acid of the P1 region sequence. More preferably, the P1 region part consists of the last ten amino acids of the P1 region sequence. The P2-P3 region and the P1 region part may be from the same fungal glycoside hydrolase, or from different fungal glycoside hydrolases. In further embodiments, the P2-P3 region of the CPP has at least 85% sequence identity to the amino acid sequence of any one of SEQ ID NOs: 558-636. In further embodiments, the P2-P3 region of the CPP has at least 90% sequence identity to the amino acid sequence of any one of SEQ ID NOs: 558-636. In further embodiments, the P2-P3 region of the CPP has at least 95% sequence identity to the amino acid sequence of any one of SEQ ID NOs: 558-636. In further embodiments, the P2-P3 region of the CPP has 100% sequence identity to the amino acid sequence of any one of SEQ ID NOs: 558-636. In further embodiments, the CPP consists of the contiguous amino acid portion.

In some embodiments, the amino acid sequence of the CPP comprises a contiguous amino acid portion consisting of i) a P2-P3 region having at least 80% sequence identity to the amino acid sequence of any one of SEQ ID NOs: 558-636, ii) part of a P4 region having the amino acid sequence of any one of SEQ ID NOs: 479-557, and iii) part of a P1 region having the amino acid sequence of any one of SEQ ID NOs: 245-320, the amino acid sequence RAT, the amino acid sequence TAT, or the amino acid sequence SAT, wherein the P4 region part is C-terminal to the P2-P3 region, and wherein the P1 region part is N-terminal to the P2-P3 region. The P4 region part can consist of any length portion of the P4 region sequence, including the full length P4 region sequence and the P1 region part can consist of any length portion of the P1 region sequence, including the full length P1 region sequence. In a preferred embodiment, the P4 region part consists of the first, the first two, the first three, the first four, the first five, the first six, the first seven, the first eight, the first nine, or the first ten amino acids of the P4 region sequence and the P1 region part consists of the last ten, the last nine, the last eight, the last seven, the last six, the last five, the last four, the last three, the last two, or the last amino acid of the P1 region sequence. More preferably, the P4 region part consists of the first amino acid of the P4 region sequence and the P1 region part consists of the last ten amino acids of the P1 region sequence. The P2-P3 region, the P4 region part, and/or the P1 region part may be from the same fungal glycoside hydrolase, or from different fungal glycoside hydrolases. In further embodiments, the P2-P3 region of the CPP has at least 85% sequence identity to the amino acid sequence of any one of SEQ ID NOs: 558-636. In further embodiments, the P2-P3 region of the CPP has at least 90% sequence identity to the amino acid sequence of any one of SEQ ID NOs: 558-636. In further embodiments, the P2-P3 region of the CPP has at least 95% sequence identity to the amino acid sequence of any one of SEQ ID NOs: 558-636. In further embodiments, the P2-P3 region of the CPP has 100% sequence identity to the amino acid sequence of any one of SEQ ID NOs: 558-636. In further embodiments, the CPP consists of the contiguous amino acid portion.

In some embodiments, the amino acid sequence of the CPP comprises a contiguous amino acid portion consisting of i) a P2-P3 region having at least 80% sequence identity to the amino acid sequence of any one of SEQ ID NOs: 558-636 and ii) a P4 region having at least 80% sequence identity to any one of SEQ ID NOs: 479-557, wherein the P4 region is C-terminal to the P2-P3 region. The P2-P3 region and the P4 region may be from the same fungal glycoside hydrolase, or from different fungal glycoside hydrolases. In further embodiments, the P2-P3 region of the CPP has at least 85% sequence identity to the amino acid sequence of any one of SEQ ID NOs: 558-636. In further embodiments, the P2-P3 region of the CPP has at least 90% sequence identity to the amino acid sequence of any one of SEQ ID NOs: 558-636. In further embodiments, the P2-P3 region of the CPP has at least 95% sequence identity to the amino acid sequence of any one of SEQ ID NOs: 558-636. In further embodiments, the P2-P3 region of the CPP has 100% sequence identity to the amino acid sequence of any one of SEQ ID NOs: 558-636. In further embodiments, the P4 region of the CPP has at least 85% sequence identity to the amino acid sequence of any one of SEQ ID NOs: 479-557. In further embodiments, the P4 region of the CPP has at least 90% sequence identity to the amino acid sequence of any one of SEQ ID NOs: 479-557. In further embodiments, the P4 region of the CPP has at least 95% sequence identity to the amino acid sequence of any one of SEQ ID NOs: 479-557. In further embodiments, the P4 region of the CPP has 100% sequence identity to the amino acid sequence of any one of SEQ ID NOs: 479-557. In further embodiments, the CPP consists of the contiguous amino acid portion.

In some embodiments, the amino acid sequence of the CPP comprises a contiguous amino acid portion consisting of i) a P2-P3 region having at least 80% sequence identity to the amino acid sequence of any one of SEQ ID NOs: 558-636 and ii) a P1 region having at least 80% sequence identity to any one of SEQ ID NOs: 245-320, the amino acid sequence RAT, the amino acid sequence TAT, or the amino acid sequence SAT, wherein the P1 region is N-terminal to the P2-P3 region. The P2-P3 region and the P1 region may be from the same fungal glycoside hydrolase, or from different fungal glycoside hydrolases. In further embodiments, the P2-P3 region of the CPP has at least 85% sequence identity to the amino acid sequence of any one of SEQ ID NOs: 558-636. In further embodiments, the P2-P3 region of the CPP has at least 90% sequence identity to the amino acid sequence of any one of SEQ ID NOs: 558-636. In further embodiments, the P2-P3 region of the CPP has at least 95% sequence identity to the amino acid sequence of any one of SEQ ID NOs: 558-636. In further embodiments, the P2-P3 region of the CPP has 100% sequence identity to the amino acid sequence of any one of SEQ ID NOs: 558-636. In further embodiments, the P1 region of the CPP has at least 85% sequence identity to the amino acid sequence of any one of SEQ ID NOs: 245-320, the amino acid sequence RAT, the amino acid sequence TAT, or the amino acid sequence SAT. In further embodiments, the P1 region of the CPP has at least 90% sequence identity to the amino acid sequence of any one of SEQ ID NOs: 245-320, the amino acid sequence RAT, the amino acid sequence TAT, or the amino acid sequence SAT. In further embodiments, the P1 region of the CPP has at least 95% sequence identity to the amino acid sequence of any one of SEQ ID NOs: 245-320, the amino acid sequence RAT, the amino acid sequence TAT, or the amino acid sequence SAT. In further embodiments, the P1 region of the CPP has 100% sequence identity to the amino acid sequence of any one of SEQ ID NOs: 245-320, the amino acid sequence RAT, the amino acid sequence TAT, or the amino acid sequence SAT. In further embodiments, the CPP consists of the contiguous amino acid portion.

In some embodiments, the amino acid sequence of the CPP comprises a contiguous amino acid portion consisting of i) a P2-P3 region having at least 80% sequence identity to the amino acid sequence of any one of SEQ ID NOs: 558-636, ii) a P4 region having at least 80% sequence identity to any one of SEQ ID NOs: 479-557, and iii) a P1 region having at least 80% sequence identity to any one of SEQ ID NOs: 245-320, the amino acid sequence RAT, the amino acid sequence TAT, or the amino acid sequence SAT, wherein the P4 region is C-terminal to the P3 region, and wherein the P1 region is N-terminal to the P2 region. The P2-P3 region, the P4 region, and/or the P1 region may be from the same fungal glycoside hydrolase, or from different fungal glycoside hydrolases. In further embodiments, the P2-P3 region of the CPP has at least 85% sequence identity to the amino acid sequence of any one of SEQ ID NOs: 558-636. In further embodiments, the P2-P3 region of the CPP has at least 90% sequence identity to the amino acid sequence of any one of SEQ ID NOs: 558-636. In further embodiments, the P2-P3 region of the CPP has at least 95% sequence identity to the amino acid sequence of any one of SEQ ID NOs: 558-636. In further embodiments, the P2-P3 region of the CPP has 100% sequence identity to the amino acid sequence of any one of SEQ ID NOs: 558-636. In further embodiments, the P4 region of the CPP has at least 85% sequence identity to the amino acid sequence of any one of SEQ ID NOs: 479-557. In further embodiments, the P4 region of the CPP has at least 90% sequence identity to the amino acid sequence of any one of SEQ ID NOs: 479-557. In further embodiments, the P4 region of the CPP has at least 95% sequence identity to the amino acid sequence of any one of SEQ ID NOs: 479-557. In further embodiments, the P4 region of the CPP has 100% sequence identity to the amino acid sequence of any one of SEQ ID NOs: 479-557. In further embodiments, the P1 region of the CPP has at least 85% sequence identity to the amino acid sequence of any one of SEQ ID NOs: 245-320, the amino acid sequence RAT, the amino acid sequence TAT, or the amino acid sequence SAT. In further embodiments, the P1 region of the CPP has at least 90% sequence identity to the amino acid sequence of any one of SEQ ID NOs: 245-320, the amino acid sequence RAT, the amino acid sequence TAT, or the amino acid sequence SAT. In further embodiments, the P1 region of the CPP has at least 95% sequence identity to the amino acid sequence of any one of SEQ ID NOs: 245-320, the amino acid sequence RAT, the amino acid sequence TAT, or the amino acid sequence SAT. In further embodiments, the P1 region of the CPP has 100% sequence identity to the amino acid sequence of any one of SEQ ID NOs: 245-320, the amino acid sequence RAT, the amino acid sequence TAT, or the amino acid sequence SAT. In further embodiments, the CPP consists of the contiguous amino acid portion.

### Cell Penetrating Peptides Comprising a P2-P3-P4 Region Sequence

In another preferred embodiment, the amino acid sequence of the CPP comprises a contiguous amino acid portion having at least 80% sequence identity to the amino acid sequence of a P2-P3-P4 region of a fungal glycoside hydrolase, i.e., any one of SEQ ID NOs: 637-715. In a preferred embodiment, the amino acid sequence of the CPP comprises a contiguous amino acid portion having at least 80% sequence identity to the amino acid sequence of any one of SEQ ID NOs: 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 677, 678, 679, 680, 681, 682, 683, 684, 685, 686, 687, 688, 689, 690, 691, 692, 693, 694, 695, 696, 697, 698, 699, 700, 701, 702, 703, 704, 705, 706, 707, 708, 709, 710, 711, 712, 713, 714, and 715. In further embodiments, the contiguous amino acid portion has at least 85% sequence identity to any one of SEQ ID NOs: 637-715. In further embodiments, the contiguous amino acid portion has at least 90% sequence identity to any one of SEQ ID NOs: 637-715. In further embodiments, the contiguous amino acid portion has at least 95% sequence identity to any one of SEQ ID NOs: 637-715. In further embodiments, the contiguous amino acid portion has 100% sequence identity to any one of SEQ ID NOs: 637-715. In further embodiments, the CPP consists of the contiguous amino acid portion.

In some embodiments, the contiguous amino acid portion has at least 80% sequence identity to the amino acid sequence of SEQ ID NOs: 637. In some embodiments, the contiguous amino acid portion has at least 80% sequence identity to the amino acid sequence of SEQ ID NOs: 638. In some embodiments, the contiguous amino acid portion has at least 80% sequence identity to the amino acid sequence of SEQ ID NOs: 639. In some embodiments, the contiguous amino acid portion has at least 80% sequence identity to the amino acid sequence of SEQ ID NOs: 640. In some embodiments, the contiguous amino acid portion has at least 80% sequence identity to the amino acid sequence of SEQ ID NOs: 641. In some embodiments, the contiguous amino acid portion has at least 80% sequence identity to the amino acid sequence of SEQ ID NOs: 642. In some embodiments, the contiguous amino acid portion has at least 80% sequence identity to the amino acid sequence of SEQ ID NOs: 643. In some embodiments, the contiguous amino acid portion has at least 80% sequence identity to the amino acid sequence of SEQ ID NOs: 644. In some embodiments, the contiguous amino acid portion has at least 80% sequence identity to the amino acid sequence of SEQ ID NOs: 645. In some embodiments, the contiguous amino acid portion has at least 80% sequence identity to the amino acid sequence of SEQ ID NOs: 646. In some embodiments, the contiguous amino acid portion has at least 80% sequence identity to the amino acid sequence of SEQ ID NOs: 647. In some embodiments, the contiguous amino acid portion has at least 80% sequence identity to the amino acid sequence of SEQ ID NOs: 648. In some embodiments, the contiguous amino acid portion has at least 80% sequence identity to the amino acid sequence of SEQ ID NOs: 649. In some embodiments, the contiguous amino acid portion has at least 80% sequence identity to the amino acid sequence of SEQ ID NOs: 650. In some embodiments, the contiguous amino acid portion has at least 80% sequence identity to the amino acid sequence of SEQ ID NOs: 651. In some embodiments, the contiguous amino acid portion has at least 80% sequence identity to the amino acid sequence of SEQ ID NOs: 652. In some embodiments, the contiguous amino acid portion has at least 80% sequence identity to the amino acid sequence of SEQ ID NOs: 653. In some embodiments, the contiguous amino acid portion has at least 80% sequence identity to the amino acid sequence of SEQ ID NOs: 654. In some embodiments, the contiguous amino acid portion has at least 80% sequence identity to the amino acid sequence of SEQ ID NOs: 655. In some embodiments, the contiguous amino acid portion has at least 80% sequence identity to the amino acid sequence of SEQ ID NOs: 656. In some embodiments, the contiguous amino acid portion has at least 80% sequence identity to the amino acid sequence of SEQ ID NOs: 657. In some embodiments, the contiguous amino acid portion has at least 80% sequence identity to the amino acid sequence of SEQ ID NOs: 658. In some embodiments, the contiguous amino acid portion has at least 80% sequence identity to the amino acid sequence of SEQ ID NOs: 659. In some embodiments, the contiguous amino acid portion has at least 80% sequence identity to the amino acid sequence of SEQ ID NOs: 660. In some embodiments, the contiguous amino acid portion has at least 80% sequence identity to the amino acid sequence of SEQ ID NOs: 661. In some embodiments, the contiguous amino acid portion has at least 80% sequence identity to the amino acid sequence of SEQ ID NOs: 662. In some embodiments, the contiguous amino acid portion has at least 80% sequence identity to the amino acid sequence of SEQ ID NOs: 663. In some embodiments, the contiguous amino acid portion has at least 80% sequence identity to the amino acid sequence of SEQ ID NOs: 664. In some embodiments, the contiguous amino acid portion has at least 80% sequence identity to the amino acid sequence of SEQ ID NOs: 665. In some embodiments, the contiguous amino acid portion has at least 80% sequence identity to the amino acid sequence of SEQ ID NOs: 666. In some embodiments, the contiguous amino acid portion has at least 80% sequence identity to the amino acid sequence of SEQ ID NOs: 667. In some embodiments, the contiguous amino acid portion has at least 80% sequence identity to the amino acid sequence of SEQ ID NOs: 668. In some embodiments, the contiguous amino acid portion has at least 80% sequence identity to the amino acid sequence of SEQ ID NOs: 669. In some embodiments, the contiguous amino acid portion has at least 80% sequence identity to the amino acid sequence of SEQ ID NOs: 670. In some embodiments, the contiguous amino acid portion has at least 80% sequence identity to the amino acid sequence of SEQ ID NOs: 671. In some embodiments, the contiguous amino acid portion has at least 80% sequence identity to the amino acid sequence of SEQ ID NOs: 672. In some embodiments, the contiguous amino acid portion has at least 80% sequence identity to the amino acid sequence of SEQ ID NOs: 673. In some embodiments, the contiguous amino acid portion has at least 80% sequence identity to the amino acid sequence of SEQ ID NOs: 674. In some embodiments, the contiguous amino acid portion has at least 80% sequence identity to the amino acid sequence of SEQ ID NOs: 675. In some embodiments, the contiguous amino acid portion has at least 80% sequence identity to the amino acid sequence of SEQ ID NOs: 676. In some embodiments, the contiguous amino acid portion has at least 80% sequence identity to the amino acid sequence of SEQ ID NOs: 677. In some embodiments, the contiguous amino acid portion has at least 80% sequence identity to the amino acid sequence of SEQ ID NOs: 678. In some embodiments, the contiguous amino acid portion has at least 80% sequence identity to the amino acid sequence of SEQ ID NOs: 679. In some embodiments, the contiguous amino acid portion has at least 80% sequence identity to the amino acid sequence of SEQ ID NOs: 680. In some embodiments, the contiguous amino acid portion has at least 80% sequence identity to the amino acid sequence of SEQ ID NOs: 681. In some embodiments, the contiguous amino acid portion has at least 80% sequence identity to the amino acid sequence of SEQ ID NOs: 682. In some embodiments, the contiguous amino acid portion has at least 80% sequence identity to the amino acid sequence of SEQ ID NOs: 683. In some embodiments, the contiguous amino acid portion has at least 80% sequence identity to the amino acid sequence of SEQ ID NOs: 684. In some embodiments, the contiguous amino acid portion has at least 80% sequence identity to the amino acid sequence of SEQ ID NOs: 685. In some embodiments, the contiguous amino acid portion has at least 80% sequence identity to the amino acid sequence of SEQ ID NOs: 686. In some embodiments, the contiguous amino acid portion has at least 80% sequence identity to the amino acid sequence of SEQ ID NOs: 687. In some embodiments, the contiguous amino acid portion has at least 80% sequence identity to the amino acid sequence of SEQ ID NOs: 688. In some embodiments, the contiguous amino acid portion has at least 80% sequence identity to the amino acid sequence of SEQ ID NOs: 689. In some embodiments, the contiguous amino acid portion has at least 80% sequence identity to the amino acid sequence of SEQ ID NOs: 690. In some embodiments, the contiguous amino acid portion has at least 80% sequence identity to the amino acid sequence of SEQ ID NOs: 691. In some embodiments, the contiguous amino acid portion has at least 80% sequence identity to the amino acid sequence of SEQ ID NOs: 692. In some embodiments, the contiguous amino acid portion has at least 80% sequence identity to the amino acid sequence of SEQ ID NOs: 693. In some embodiments, the contiguous amino acid portion has at least 80% sequence identity to the amino acid sequence of SEQ ID NOs: 694. In some embodiments, the contiguous amino acid portion has at least 80% sequence identity to the amino acid sequence of SEQ ID NOs: 695. In some embodiments, the contiguous amino acid portion has at least 80% sequence identity to the amino acid sequence of SEQ ID NOs: 696. In some embodiments, the contiguous amino acid portion has at least 80% sequence identity to the amino acid sequence of SEQ ID NOs: 697. In some embodiments, the contiguous amino acid portion has at least 80% sequence identity to the amino acid sequence of SEQ ID NOs: 698. In some embodiments, the contiguous amino acid portion has at least 80% sequence identity to the amino acid sequence of SEQ ID NOs: 699. In some embodiments, the contiguous amino acid portion has at least 80% sequence identity to the amino acid sequence of SEQ ID NOs: 700. In some embodiments, the contiguous amino acid portion has at least 80% sequence identity to the amino acid sequence of SEQ ID NOs: 701. In some embodiments, the contiguous amino acid portion has at least 80% sequence identity to the amino acid sequence of SEQ ID NOs: 702. In some embodiments, the contiguous amino acid portion has at least 80% sequence identity to the amino acid sequence of SEQ ID NOs: 703. In some embodiments, the contiguous amino acid portion has at least 80% sequence identity to the amino acid sequence of SEQ ID NOs: 704. In some embodiments, the contiguous amino acid portion has at least 80% sequence identity to the amino acid sequence of SEQ ID NOs: 705. In some embodiments, the contiguous amino acid portion has at least 80% sequence identity to the amino acid sequence of SEQ ID NOs: 706. In some embodiments, the contiguous amino acid portion has at least 80% sequence identity to the amino acid sequence of SEQ ID NOs: 707. In some embodiments, the contiguous amino acid portion has at least 80% sequence identity to the amino acid sequence of SEQ ID NOs: 708. In some embodiments, the contiguous amino acid portion has at least 80% sequence identity to the amino acid sequence of SEQ ID NOs: 709. In some embodiments, the contiguous amino acid portion has at least 80% sequence identity to the amino acid sequence of SEQ ID NOs: 710. In some embodiments, the contiguous amino acid portion has at least 80% sequence identity to the amino acid sequence of SEQ ID NOs: 711. In some embodiments, the contiguous amino acid portion has at least 80% sequence identity to the amino acid sequence of SEQ ID NOs: 712. In some embodiments, the contiguous amino acid portion has at least 80% sequence identity to the amino acid sequence of SEQ ID NOs: 713. In some embodiments, the contiguous amino acid portion has at least 80% sequence identity to the amino acid sequence of SEQ ID NOs: 714. In some embodiments, the contiguous amino acid portion has at least 80% sequence identity to the amino acid sequence of SEQ ID NOs: 715.. In further embodiments, the contiguous amino acid portion has at least 85% sequence identity to the amino acid sequence of the P2-P3-P4 region of the fungal glycoside hydrolase. In further embodiments, the contiguous amino acid portion has at least 90% sequence identity to the amino acid sequence of the P2-P3-P4 region of the fungal glycoside hydrolase. In further embodiments, the contiguous amino acid portion has at least 95% sequence identity to the amino acid sequence of the P2-P3-P4 region of the fungal glycoside hydrolase. In further embodiments, the contiguous amino acid portion has 100% sequence identity to the amino acid sequence of the P2-P3-P4 region of the fungal glycoside hydrolase. In further embodiments, the CPP consists of the contiguous amino acid portion.

In some embodiments, the amino acid sequence of the CPP comprises a contiguous amino acid portion consisting of i) a P2-P3-P4 region having at least 80% sequence identity to the amino acid sequence of any one of SEQ ID NOs: 637-715 and ii) part of a P1 region having the amino acid sequence of any one of SEQ ID NOs: 245-320, the amino acid sequence RAT, the amino acid sequence TAT, or the amino acid sequence SAT, wherein the P1 region part is N-terminal to the P2-P3-P4 region. The P1 region part can consist of any length portion of the P1 region sequence, including the full length P1 region sequence. In a preferred embodiment, the P1 region part consists of the last ten, the last nine, the last eight, the last seven, the last six, the last five, the last four, the last three, the last two, or the last amino acid of the P1 region sequence. More preferably, the P1 region part consists of the last ten amino acids of the P1 region sequence. The P2-P3-P4 region and the P1 region part may be from the same fungal glycoside hydrolase, or from different fungal glycoside hydrolases. In further embodiments, the P2-P3-P4 region has at least 85% sequence identity to the amino acid sequence of any one of SEQ ID NOs: 637-715. In further embodiments, the P2-P3-P4 region has at least 90% sequence identity to the amino acid sequence of any one of SEQ ID NOs: 637-715. In further embodiments, the P2-P3-P4 region has at least 95% sequence identity to the amino acid sequence of any one of SEQ ID NOs: 637-715. In further embodiments, the P2-P3-P4 region has 100% sequence identity to the amino acid sequence of any one of SEQ ID NOs: 637-715. In further embodiments, the CPP consists of the contiguous amino acid portion.

In some embodiments, the amino acid sequence of the CPP comprises a contiguous amino acid portion consisting of i) a P2-P3-P4 region having at least 80% sequence identity to the amino acid sequence of any one of SEQ ID NOs: 637-715 and ii) a P1 region having at least 80% sequence identity to any one of SEQ ID NOs: 245-320, the amino acid sequence RAT, the amino acid sequence TAT, or the amino acid sequence SAT, wherein the P1 region is N-terminal to the P2-P3-P4 region. The P2-P3-P4 region and the P1 region may be from the same fungal glycoside hydrolase, or from different fungal glycoside hydrolases. In some embodiments, the P2 region is from *Fomes fomentarius* (i.e., SEQ ID NO 345), the P3 region is from *Lentinus tigrinus* (i.e., SEQ ID NO 418), and the P4 region is from *Earliella scabrosa* (i.e., SEQ ID NO 504). In further embodiments, the P2-P3-P4 region has at least 85% sequence identity to the amino acid sequence of any one of SEQ ID NOs: 637-715. In further embodiments, the P2-P3-P4 region has at least 90% sequence identity to the amino acid sequence of any one of SEQ ID NOs: 637-715. In further embodiments, the P2-P3-P4 region has at least 95% sequence identity to the amino acid sequence of any one of SEQ ID NOs: 637-715. In further embodiments, the P2-P3-P4 region has 100% sequence identity to the amino acid sequence of any one of SEQ ID NOs: 637-715. In further embodiments, the P1 region has at least 85% sequence identity to the amino acid sequence of any one of SEQ ID NOs: 245-320, the amino acid sequence RAT, the amino acid sequence TAT, or the amino acid sequence SAT. In further embodiments, the P1 region has at least 90% sequence identity to the amino acid sequence of any one of SEQ ID NOs: 245-320, the amino acid sequence RAT, the amino acid sequence TAT, or the amino acid sequence SAT. In further embodiments, the P1 region has at least 95% sequence identity to the amino acid sequence of any one of SEQ ID NOs: 245-320, the amino acid sequence RAT, the amino acid sequence TAT, or the amino acid sequence SAT. In further embodiments, the P1 region has 100% sequence identity to the amino acid sequence of any one of SEQ ID NOs: 245-320, the amino acid sequence RAT, the amino acid sequence TAT, or the amino acid sequence SAT. In further embodiments, the CPP consists of the contiguous amino acid portion.

### Cell Penetrating Peptides Comprising a P1-P2-P3-P4 Region Sequence

CPPs of the invention comprise a contiguous amino acid portion having at least 80% sequence identity to a same-length portion of a P1-P2-P3-P4 region sequence, e.g., any one of SEQ ID NOs: 166-244, which are found between the pairs of N-terminal flagging sequences and C-terminal flagging sequences in Table 1.

In a preferred embodiment, the amino acid sequence of the CPP comprises a contiguous amino acid portion having at least 80% sequence identity to the amino acid sequence of a P1-P2-P3-P4 region of a fungal glycoside hydrolase, i.e., the full length protein loop domain which is any one of SEQ ID NOs: 166-244. In a preferred embodiment, the amino acid sequence of the CPP comprises a contiguous amino acid portion having at least 80% sequence identity to the amino acid sequence of any one of SEQ ID NOs: 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, and 244. In further embodiments, the contiguous amino acid portion has at least 85% sequence identity to the amino acid sequence of any one of SEQ ID NOs: 166-244. In further embodiments, the contiguous amino acid portion has at least 90% sequence identity to the amino acid sequence of any one of SEQ ID NOs: 166-244. In further embodiments, the contiguous amino acid portion has at least 95% sequence identity to the amino acid sequence of any one of SEQ ID NOs: 166-244. In further embodiments, the contiguous amino acid portion has 100% sequence identity to the amino acid sequence of any one of SEQ ID NOs: 166-244. In further embodiments, the CPP consists of the contiguous amino acid portion.

In some embodiments, the CPP includes the N-terminal flagging I sequence and/or the C-terminal flagging sequence, wherein the N-terminal flagging sequence is XWX₁XX₂X₃X₄X₅XXXXX₆X₇GX₈PX₉YX₁₀YXX₁₁XX₁₂XXXXLXXX₁₃ (SEQ ID NO: 7),
wherein:
X₁ is T, S, V or A;
X₂ is A or S;
X₃ is G or H;
X₄ is F or V;
X₅ is P, A, D or S;
X₆ is V, M, I or A;
X₇ is L, I or M;
X₈ is V, L or I;
X₉ is S or A;
X₁₀ is G or A;
X₁₁ is S, A, N, Q or V;
X₁₂ is S or T; and
X₁₃ is R or K, and
wherein the C-terminal flagging sequence is XXXXX₁XX₂X₃X₄X(g)XXX₅X₆X₇QFX₈XX₉X₁₀XZX₁₂, wherein:
X₁ is N, S, G, D or E;
X₂ is D, -, G, A or E;
X₃ is G, -, S or A;
X₄ is G, -, T, S, P, K or H;
(g) is a gap of up to 6 amino acids;
X₅ is G, - or N;
X₆ is Q, T or G;
X₇ is V or I;
X₈ is R, N or S;
X₉ is L, I or M;
X₁₀ is V, I, L, A or F;
X₁₂ is G, A or K, and
wherein "-" represents a gap of one amino acid.

### Cell Penetrating Peptides from the Glycoside Hydrolase of Earliella Scabrosa (GHES)

CPPs of the invention can be derived from the glycoside hydrolase of *Earliella scabrosa* (GHES). In GHES, the protein loop domain is 55 amino acids in length (SEQ ID NO 191), the P1 region is 19 amino acids in length (SEQ ID NO 270), the P2 region is 12 amino acids in length (SEQ ID NO 346), the P3 region is 15 amino acids in length (SEQ ID NO 425), and the P4 region is 9 amino acids in length (SEQ ID NO 504).

In some embodiments, the amino acid sequence of the CPP comprises a P2-P3 region of 27 contiguous amino acids that is at least 60% identical to the amino acid sequence of SEQ ID NO 583. In a further embodiment the P2-P3 region is at least 65%, at least 70%, at least 75%, at least 80%, at least 85%, at least 90%, at least 95%, or 100% identical to the amino acid sequence of SEQ ID NO 583. In a preferred embodiment the CPP comprises the P2-P3 region of GHES (SEQ ID NO 583).

In some embodiments, the amino acid sequence of the CPP comprises a P2-P3 region of 27 contiguous amino acids that is at least 60% identical to the amino acid sequence of SEQ ID NO 583 and a P4 region C-terminal to the P2-P3 region that has amino acid 1, amino acids 1-2, amino acids 1-3, amino acids 1-4, amino acids 1-5, amino acids 1-6, amino acids 1-7, amino acids 1-8, or amino acids 1-9 of SEQ ID NO 504. In a preferred embodiment, the amino acid sequence of the CPP comprises a P2-P3 region of 27 contiguous amino acids that is at least 60% identical to the amino acid sequence of SEQ ID NO 583 and a P4 region C-terminal to the P2-P3 region that has amino acid 1 of SEQ ID NO 504. In further embodiments, the P2-P3 region is at least 65%, at least 70%, at least 75%, at least 80%, at least 85%, at least 90%, at least 95%, or 100% identical to the amino acid sequence of SEQ ID NO 583.

In some embodiments, the amino acid sequence of the CPP comprises a P2-P3 region of 27 contiguous amino acids that is at least 60% identical to the amino acid sequence of SEQ ID NO 583 and a P1 region N-terminal to the P2-P3 region that has amino acid 19, amino acids 18-19, amino acids 17-19, amino acids 16-19, amino acids 15-19, amino acids 14-19, amino acids 13-19, amino acids 12-19, amino acids 11-19, amino acids 10-19, amino acids 9-19, amino acids 8-19, amino acids 7-19, amino acids 6-19, amino acids 5-19, amino acids 4-19, amino acids 3-19, amino acids 2-19, or amino acids 1-19 of SEQ ID NO: 270. In a preferred embodiment, the amino acid sequence of the CPP comprises a P2-P3 region of 27 contiguous amino acids that is at least 60% identical to the amino acid sequence of SEQ ID NO 583 and a P1 region N-terminal to the P2-P3 region that has amino acids 10-19 of SEQ ID NO 270. In further embodiments the P2-P3 region is at least 65%, at least 70%, at least 75%, at least 80%, at least 85%, at least 90%, at least 95%, or 100% identical to the amino acid sequence of SEQ ID NO 583.

In some embodiments, the amino acid sequence of the CPP comprises a P2-P3 region of 27 contiguous amino acids that is at least 60% identical to the amino acid sequence of SEQ ID NO 583, a P4 region C-terminal to the P2-P3 region that has amino acid 1, amino acids 1-2, amino acids 1-3, amino acids 1-4, amino acids 1-5, amino acids 1-6, amino acids 1-7, amino acids 1-8, or amino acids 1-9 of SEQ ID NO 504, and a P1 region N-terminal to the P2-P3 region that has amino acid 19, amino acids 18-19, amino acids 17-19, amino acids 16-19, amino acids 15-19, amino acids 14-19, amino acids 13-19, amino acids 12-19, amino acids 11-19, amino acids 10-19, amino acids 9-19, amino acids 8-19, amino acids 7-19, amino acids 6-19, amino acids 5-19, amino acids 4-19, amino acids 3-19, amino acids 2-19, or amino acids 1-19 of SEQ ID NO: 270. In a preferred embodiment the amino acid sequence of the CPP comprises a P2-P3 region of 27 contiguous amino acids that is at least 60% identical to the amino acid sequence of SEQ ID NO 583, a P4 region adjacent to the C-terminus of the P2-P3 region that has amino acids 1-9 of SEQ ID NO: 504, and a P1 region adjacent to the N-terminus of the P2-P3 region that has amino acids 10-19 of SEQ ID NO: 270. In further embodiments the P2-P3 region is at least 65%, at least 70%, at least 75%, at least 80%, at least 85%, at least 90%, at least 95%, or 100% identical to the amino acid sequence of SEQ ID NO 583.

In some embodiments, the amino acid sequence of the CPP comprises a P2-P3 region of 27 contiguous amino acids that is at least 60% identical to the amino acid sequence of SEQ ID NO 583 and a P4 region of 9 contiguous amino acids that is at least 60% identical to the amino acid sequence of SEQ ID NO 504. In a further embodiment the P2-P3 region is at least 65%, at least 70%, at least 75%, at least 80%, at least 85%, at least 90%, at least 95%, or 100% identical to the amino acid sequence of SEQ ID NO 583. In a further embodiment the P4 region is at least 65%, at least 70%, at least 75%, at least 80%, at least 85%, at least 90%, at least 95%, or 100% identical to the amino acid sequence of SEQ ID NO 504.

In some embodiments, the amino acid sequence of the CPP comprises a P2-P3 region of 27 contiguous amino acids that is at least 60% identical to the amino acid sequence of SEQ ID NO 583 and a P1 region of 19 contiguous amino acids that is at least 60% identical to the amino acid sequence of SEQ ID NO 270. In a further embodiment the P2-P3 region is at least 65%, at least 70%, at least 75%, at least 80%, at least 85%, at least 90%, at least 95%, or 100% identical to the amino acid sequence of SEQ ID NO 583. In a further embodiment the P1 region is at least 65%, at least 70%, at least 75%, at least 80%, at least 85%, at least 90%, at least 95%, or 100% identical to the amino acid sequence of SEQ ID NO 270.

In some embodiments, the amino acid sequence of the CPP comprises a P2-P3 region of 27 contiguous amino acids that is at least 60% identical to the amino acid sequence of SEQ ID NO 583, a P4 region of 9 contiguous amino acids that is at least 60% identical to the amino acid sequence of SEQ ID NO 504, and a P1 region of 19 contiguous amino acids that is at least 60% identical to the amino acid sequence of SEQ ID NO 270. In a further embodiment the P2-P3 region is at least 65%, at least 70%, at least 75%, at least 80%, at least 85%, at least 90%, at least 95%, or 100% identical to the amino acid sequence of SEQ ID NO 583. In a further embodiment the P4 region is at least 65%, at least 70%, at least 75%, at least 80%, at least 85%, at least 90%, at least 95%, or 100% identical to the amino acid sequence of SEQ ID NO 504. In a further embodiment the P1 region is at least 65%, at least 70%, at least 75%, at least 80%, at least 85%, at least 90%, at least 95%, or 100% identical to the amino acid sequence of SEQ ID NO 270.

In some embodiments the amino acid sequence of the CPP comprises a P2-P3-P4 region of 36 contiguous amino acids that is at least 50% identical to the amino acid sequence of SEQ ID NO 662. In a further embodiment, the P2-P3-P4 region is at least at least 55%, at least 60%, at least 65%, at least 70%, at least 75%, at least 80%, at least 85%, at least 90%, at least 95%, or 100% identical to the amino acid sequence of SEQ ID NO 662.

In some embodiments, the amino acid sequence of the CPP comprises a P2-P3-P4 region of 36 contiguous amino acids that is at least 50% identical to the amino acid sequence of SEQ ID NO 662 and a P1 region N-terminal to the P2-P3-P4 region that has amino acid 19, amino acids 18-19, amino acids 17-19, amino acids 16-19, amino acids 15-19, amino acids 14-19, amino acids 13-19, amino acids 12-19, amino acids 11-19, amino acids 10-19, amino acids 9-19, amino acids 8-19, amino acids 7-19, amino acids 6-19, amino acids 5-19, amino acids 4-19, amino acids 3-19, amino acids 2-19, or amino acids 1-19 of SEQ ID NO: 270. In a preferred embodiment, the amino acid sequence of the CPP comprises a P2-P3-P4 region of 36 contiguous amino acids that is at least 50% identical to the amino acid sequence of SEQ ID NO 663 and a P1 region N-terminal to the P2-P3-P4 region that has amino acids 10-19 of SEQ ID NO 270. In further embodiments the P2-P3-P4 region is at least at least 55%, at least 60%, at least 65%, at least 70%, at least 75%, at least 80%, at least 85%, at least 90%, at least 95%, or 100% identical to the amino acid sequence of SEQ ID NO 662.

In some embodiments, the amino acid sequence of the CPP comprises a P2-P3-P4 region of 36 contiguous amino acids that is at least 50% identical to the amino acid sequence of SEQ ID NO 662 and a P1 region of 19 contiguous amino acids that is at least 60% identical to the amino acid sequence of SEQ ID NO 270. In a further embodiment the P2-P3-P4 region is at least 55%, at least 60%, at least 65%, at least 70%, at least 75%, at least 80%, at least 85%, at least 90%, at least 95%, or 100% identical to the amino acid sequence of SEQ ID NO 662. In a further embodiment the P1 region is at least 65%, at least 70%, at least 75%, at least 80%, at least 85%, at least 90%, at least 95%, or 100% identical to the amino acid sequence of SEQ ID NO 270.

In some embodiments the amino acid sequence of the CPP comprises a P1-P2-P3-P4 region of 55 contiguous amino acids that is at least 35% identical to the amino acid sequence of SEQ ID NO 191. In a further embodiment, the P1-P2-P3-P4 region is at least 40%, at least 45%, at least 50%, at least 55%, at least 60%, at least 65%, at least 70%, at least 75%, at least 80%, at least 85%, at least 90%, at least 95%, or 100% identical to the amino acid sequence of SEQ ID NO 191.

In some embodiments, the amino acid sequence of the CPP consists of the above described P2-P3 region, P2-P3-P4 region, P1-P2-P3 region, or P1-P2-P3-P4s region.

In some embodiments, the amino acid sequence of the CPP comprises the sequence of the GHES-P1-P2-P3 peptide of Table 7, having the sequence of SEQ ID NO: 718.

In some embodiments, the amino acid sequence of the CPP comprises the sequence of the GHES-P2-P3 peptide of Table 7 having the sequence of SEQ ID NO: 583.

In some embodiments, the amino acid sequence of the CPP comprises the sequence of the GHES-P2-P3-P4 peptide of Table 7, having the sequence of SEQ ID NO: 662.

In some embodiments, the amino acid sequence of the CPP comprises the sequence of the GHES-P1-P2-P3-P4 peptide of Table 7, having the sequence of SEQ ID NO: 191.

In some embodiments, the amino acid sequence of the CPP comprises the sequence of the GHES-(P2-P3) + P4-8 (19) peptide of Table 7, having the sequence of SEQ ID NO: 719.

In some embodiments, the amino acid sequence of the CPP comprises the sequence of the GHES-Disulfide cyclic C1-C30 (19) peptide of Table 7, having the sequence of SEQ ID NO: 720.

In some embodiments, the amino acid sequence of the CPP comprises the sequence of the GHES-Disulfide cyclic C1-C29 (19) peptide of Table 7, having the sequence of SEQ ID NO: 721.

In some embodiments, the amino acid sequence of the CPP comprises the sequence of the RI GHES-P2-P3-P4 peptide of Table 7, having the sequence of SEQ ID NO: 722.

In some embodiments, the amino acid sequence of the CPP comprises the sequence of the GHES-P2-P3-P4 inverted peptide of Table 7, having the sequence of SEQ ID NO: 723.

In some embodiments, the amino acid sequence of the CPP comprises the sequence of the GHFF-P2-P3-A peptide of Table 7, having the sequence of SEQ ID NO: 725.

In some embodiments, the amino acid sequence of the CPP comprises the sequence of the GHFF-P2-P3-P4 peptide of Table 7, having the sequence of SEQ ID NO: 661.

In some embodiments, the amino acid sequence of the CPP comprises the sequence of the GHLT-P2-P3-A peptide of Table 7, having the sequence of SEQ ID NO: 726.

In some embodiments, the amino acid sequence of the CPP comprises the sequence of the GHLT-P2-P3-P4 peptide of Table 7, having the sequence of SEQ ID NO: 655.

In some embodiments, the amino acid sequence of the CPP comprises the sequence of the GHFF-P2/GHLT-P3/GHES-P4 peptide of Table 7, having the sequence of SEQ ID NO: 727.

In some embodiments, the amino acid sequence of the CPP comprises the sequence of any one of the peptides of Figure 4a or Figure 4b, i.e., a peptide having the sequence of any one of SEQ ID NOs 191, 583, 662, 719, and 771-798.

In some embodiments, the CPP is a GHES-P1-P2-P3 peptide (SEQ ID NO:718) and is used to transport gene editing cargo comprising a Cas9 protein and a dual annealed gRNA targeting the human B2M gene into a target cell. In some embodiments, the CPP is GHES-P2-P3 peptide (SEQ ID NO:583) and is used to transport gene editing cargo comprising a Cas9 protein and a dual annealed gRNA targeting the human B2M gene into a target cell. In some embodiments, the CPP is a GHES-P2-P3-P4 peptide (SEQ ID NO:662) and is used to transport gene editing cargo comprising a Cas9 protein and a dual annealed gRNA targeting the human B2M gene into a target cell. In some embodiments, the CPP is a GHES-P1-P2-P3-P4 peptide (SEQ ID NO:191) and is used to transport gene editing cargo comprising a Cas9 protein and a dual annealed gRNA targeting the human B2M gene into a target cell.

In some embodiments, the amino acid sequence of the CPP comprises the sequence of any one of the peptides of Figure 4a or Figure 4b, i.e., a peptide having the sequence of any one of SEQ ID NOs 191, 583, 662, 719, and 771-798, and is used to transport gene editing cargo comprising a Cas9 protein and a dual annealed gRNA targeting the human B2M gene into a target cell e.g., a CD8+ T cell.

In some embodiments, the CPP is a GHES-(P2-P3) + P4-8 (19) peptide (SEQ ID NO:719) and is used to transport gene editing cargo comprising a Cas9 protein and a dual annealed gRNA targeting the human B2M gene into a target cell. In some embodiments, the CPP is a GHES-disulfide cyclic C1-C30 (19) peptide (SEQ ID NO:720) and is used to transport gene editing cargo comprising a Cas9 protein and a dual annealed gRNA targeting the human B2M gene into a target cell. In some embodiments, the CPP is a GHES-disulfide cyclic C1-C29 (19) peptide (SEQ ID NO:721) and is used to transport gene editing cargo comprising a Cas9 protein and a dual annealed gRNA targeting the human B2M gene into a target cell. In some embodiments, the CPP is a RI GHES-P2-P3-P4 peptide (SEQ ID NO:722) and is used to transport gene editing cargo comprising a Cas9 protein and a dual annealed gRNA targeting the human B2M gene into a target cell. In some embodiments, the CPP is a GHES-P2-P3-P4 inverted peptide (SEQ ID NO:723) and is used to transport gene editing cargo comprising a Cas9 protein and a dual annealed gRNA targeting the human B2M gene into a target cell. In some embodiments, the CPP is a GHFF-P2-P3-A peptide (SEQ ID NO:725) and is used to transport gene editing cargo comprising a Cas9 protein and a dual annealed gRNA targeting the human B2M gene into a target cell. In some embodiments, the CPP is a GHFF-P2-P3-P4 peptide (SEQ ID NO:661) and is used to transport gene editing cargo comprising a Cas9 protein and a dual annealed gRNA targeting the human B2M gene into a target cell. In some embodiments, the CPP is a GHLT-P2-P3-A peptide (SEQ ID NO:726) and is used to transport gene editing cargo comprising a Cas9 protein and a dual annealed gRNA targeting the human B2M gene into a target cell. In some embodiments, the CPP is a GHLT-P2-P3-P4 peptide (SEQ ID NO:655) and is used to transport gene editing cargo comprising a Cas9 protein and a dual annealed gRNA targeting the human B2M gene into a target cell. In some embodiments, the CPP is a GHFF-P2/GHLT-P3/GHES-P4 peptide (SEQ ID NO:727) and is used to transport gene editing cargo comprising a Cas9 protein and a dual annealed gRNA targeting the human B2M gene into a target cell.

In some embodiments, the CPP is a GHES-(P2-P3) + P4-8 (19) peptide (SEQ ID NO: 719) and is used to transport gene editing cargo comprising a Cas9 protein and dual annealed gRNA targeting either human B2M, CTLA-4 or PD-1 genes into a target cell. In some embodiments, the CPP is a GHES-P2-P3-P4 peptide (SEQ ID NO:662) and is used to transport gene editing cargo comprising a base editor protein (ABE) and a dual annealed gRNA targeting either human B2M, CTLA-4, PD-1 or CD33 genes into a target cell. In some embodiments, the CPP is a GHES-P2-P3-P4 peptide (SEQ ID NO: 662) and is used to transport gene editing cargo comprising a Cas12a protein and a single gRNA targeting the human B2M gene into a target cell. In some embodiments, the CPP is a GHES-P2-P3-P4 peptide (SEQ ID NO: 662) and is used to transport gene editing proteins comprising prime editing proteins and a single gRNA (e.g., a pegRNA) targeting the human B2M gene into a target cell.

In some embodiments, the CPP is a peptide selected from GHES-P2-P3 (SEQ ID NO: 583), GHES-P2-P3-P4 (SEQ ID NO: 662), GHES (P2-P3) +P4-8 (19) (SEQ ID NO: 719), GHFF-P2-P3-A (SEQ ID NO: 725), and GHLT-P2-P3-A (SEQ ID NO: 726) and is used to transport gene editing cargo comprising a Cas9 protein and a dual annealed gRNA targeting the human B2M gene into a Jurkat cell. In some embodiments, the CPP is a peptide selected from GHES-P2-P3 (SEQ ID NO: 583), GHES-P2-P3-P4 (SEQ ID NO: 662), GHES (P2-P3) +P4-8 (19) (SEQ ID NO: 719), GHFF-P2-P3-A (SEQ ID NO: 725), and GHLT-P2-P3-A (SEQ ID NO: 726) and is used to transport gene editing cargo comprising a Cas9 protein and a dual annealed gRNA targeting the human B2M gene into a CD4+ T cell. In some embodiments, the CPP is a peptide selected from GHES-P2-P3 (SEQ ID NO: 583), GHES-P2-P3-P4 (SEQ ID NO: 662), GHES (P2-P3) +P4-8 (19) (SEQ ID NO: 719), GHFF-P2-P3-A (SEQ ID NO: 725), and GHLT-P2-P3-A (SEQ ID NO: 726) and is used to transport gene editing cargo comprising a Cas9 protein and a dual annealed gRNA targeting the human B2M gene into a NK cell. In some embodiments, the CPP is a peptide selected from GHES-P2-P3 (SEQ ID NO: 583), GHES-P2-P3-P4 (SEQ ID NO: 662), GHES (P2-P3) +P4-8 (19) (SEQ ID NO: 719), GHFF-P2-P3-A (SEQ ID NO: 725), and GHLT-P2-P3-A (SEQ ID NO: 726) and is used to transport gene editing cargo comprising a Cas9 protein and a dual annealed gRNA targeting the human B2M gene into a CD8+ T cell. In some embodiments, the CPP is a peptide selected from GHES-P2-P3 (SEQ ID NO: 583), GHES-P2-P3-P4 (SEQ ID NO: 662), GHES (P2-P3) +P4-8 (19) (SEQ ID NO: 719), GHFF-P2-P3-A (SEQ ID NO: 725), and GHLT-P2-P3-A (SEQ ID NO: 726) and is used to transport gene editing cargo comprising a Cas9 protein and a dual annealed gRNA targeting the human B2M gene into a hIPSC cell. In some embodiments, the CPP is a peptide selected from GHES-P2-P3 (SEQ ID NO: 583), GHES-P2-P3-P4 (SEQ ID NO: 662), GHES (P2-P3) +P4-8 (19) (SEQ ID NO: 719), GHFF-P2-P3-A (SEQ ID NO: 725), and GHLT-P2-P3-A (SEQ ID NO: 726) and is used to transport gene editing cargo comprising a Cas9 protein and a dual annealed gRNA targeting the human B2M gene into a CD34+ hSC cell.

In some embodiments. the CPP is a GHES-P2-P3-P4 peptide (SEQ ID NO: 662) and is used to transport lentivirus cargo into a HEK293T target cell.

### Modifications to Cell Penetrating Peptides

CPPs of the invention may be chemically or structurally modified to enhance one or more desirable properties, including but not limited to stability, resistance to proteolytic degradation, bioavailability, cellular uptake, favourable pharmacokinetics, solubility, localisation within the cell, and increased affinity for specific cargoes.

In some embodiments, the CPP comprises one or more non-standard amino acids, including but not limited to D-amino acids, naturally occurring non-standard amino acids, and/or synthetic amino acids.

In some embodiments, the CPP comprises one or more D-amino acids in place of their corresponding L amino acids. D-amino acid-containing peptides exhibit increased stability in vitro and in vivo relative to their L-amino acid-containing counterparts. In particular, incorporation of D-amino acids confers resistance to endogenous peptidases and proteases, thereby prolonging the half-life of the CPP and enhancing systemic bioavailability. Furthermore, because D-amino acid-containing peptides are inefficiently processed for MHC class II presentation, they are less likely to elicit humoral immune responses, making them advantageous for therapeutic applications.

In some embodiments, the CPP comprises one or more naturally occurring non-standard amino acids in place of their corresponding natural amino acids. Naturally occurring non-standard amino acids may confer enhanced protease resistance, greater structural rigidity, modulation of physicochemical properties (e.g., charge, polarity, hydrophobicity), and/or reduced immunogenicity. Examples of naturally occurring non-standard amino acids are Ornithine, Citrulline, β-Alanine, γ-Aminobutyric acid (GABA), Taurine, Selenocysteine, Pyrrolysine, L-DOPA, Homocysteine, Hydroxyproline, Hydroxylysine, Canavanine, Lanthionine, Allothreonine, Theanine, and Hypusine.

In some embodiments, the CPP comprises one or more synthetic amino acids in place of their corresponding natural amino acids. Synthetic amino acids may be incorporated to improve peptide stability, functionality, or biological compatibility. Examples of synthetic amino acids are N-Methylated amino acids (e.g., N-methylglycine/sarcosine), α,α-Disubstituted amino acids (e.g., Aib: α-aminoisobutyric acid), β-Amino acids (e.g., β-alanine analogs), γ-Amino acids, Norleucine (Nle), Homophenylalanine, Cyclohexylalanine (Cha), Homoarginine, Biphenylalanine (Bip), Norvaline (Nva), 4-Fluorophenylalanine, Azidohomoalanine (AHA), Propargylglycine, Phenylglycine, Thioproline, α-Aminoadipic acid, Diaminobutyric acid (Dab), Diaminopropionic acid (Dap), Aminocyclopropane carboxylic acid (ACPC), α,β-Dehydroamino acids (e.g., dehydroalanine), and locked nucleic acids (LNAs).

In some embodiments, the CPP comprises a synthetic nucleic acid mimetic, such as peptide nucleic acids (PNAs) or locked nucleic acids (LNAs).

In some embodiments, the CPP is modified at the termini to prevent degradation or improve membrane interaction. For example, in some embodiments the CPP is modified by N-terminal acetylation. In some embodiments, the CPP is modified by C-terminal amidation.

In some embodiments, the CPP comprises one or more post-translational modifications. Examples of post-translational modifications include, but are not limited to, glycosylation, acetylation, phosphorylation, lipidation (e.g., palmitoylation or myristoylation), PEGylation, glycolipid conjugation, or acylation. Such modifications may enhance membrane affinity, solubility, circulatory half-life, or targeted delivery.

In some embodiments, the CPP is conjugated to a lipid moiety. Suitable lipid moieties include, but are not limited to, palmitic acid, myristic acid, stearic acid, cholesterol, lipoic acid, docosahexaenoic acid (DHA), and diacylglycerol analogs. Lipid conjugation can promote membrane binding, facilitate endosomal escape, increase plasma stability, and enhance cellular uptake. In some embodiments, lipidated CPPs may be incorporated into lipid nanoparticles, liposomes, or micellar structures for co-delivery with cargo.

In some embodiments, the CPP is a retro-inverso peptide, wherein the peptide comprises D-amino acids arranged in the reverse sequence of the corresponding L-form CPP. Retro-inverso peptides preserve the side-chain topology of the original sequence, allowing them to mimic biological activity while enhancing resistance to proteolysis.

In some embodiments, the CPP is an inverted peptide, wherein the peptide comprises L-amino acids arranged in the reverse sequence of the corresponding CPP.

In some embodiments, the CPP is modified to increase its solubility, for example via glycosylation or PEGylation.

In some embodiments, the CPP is modified to control its localisation within the cell, for example via addition of an organelle-specific targeting sequence and/or tag. Examples of targeting sequences include, but are not limited to, a nuclear localisation signal (NLS) for localisation to the nucleus, a mitochondrial targeting sequence (MTS) for localisation to the mitochondria, a signal peptide and/or a KDEL motif for localisation to the endoplasmic reticulum (ER), a mannose-6-phosphate (M6P) tag for localisation to the lysosome, and/or a peroxisomal targeting signal (PTS) for localisation to the peroxisome.

In some embodiments, the CPP is modified to increase its affinity for specific cargoes. For example, the CPP may be modified to introduce a specific cargo-binding domain, including and not limited to a nucleic acid binding domain such as a zinc-finger like domain, or a protein-binding domain such as an SH3 domain. In some embodiments, the CPP and cargo may be modified with specific binding pairs to increase their binding affinity, for example via use of biotin-avidin/streptavidin binding pairs and derivatives thereof.

### Cyclic Cell Penetrating Peptides

CPPs of the invention can be linear or cyclic. Cyclic CPPs can be partially cyclic or fully cyclic. In a partially cyclic CPP only a portion of the peptide sequence is cyclised, while the rest remains linear. In a fully cyclic CPP, the full peptide sequence is cyclised, with no linear portion remaining. Cyclic CPPs can offer several advantages over linear CPPs. For example, they can exhibit a longer half-life due to increased proteolytic stability. This can result from the absence of free N- or C-termini which protect against degradation by exopeptidases, and/or from conformational constraints that limit access by endopeptidases. Cyclic CPPs may also demonstrate enhanced membrane permeability, as cyclisation can present cationic and hydrophobic residues in a spatial arrangement that facilitates interaction with lipid bilayers. Additionally, structural rigidity conferred by cyclisation can lead to more selective and efficient membrane interactions. Furthermore, the cyclic scaffold allows for precise positioning of functional groups, which can support tuneable cell selectivity and more targeted delivery, thus reducing off-target effects.

Cyclic CPPs can be formed via various types of cyclisation. One example is head-to-tail cyclisation, in which a peptide bond is formed between the N-terminal amine and the C-terminal carboxyl group, resulting in a backbone-cyclised peptide. This type of cyclisation enhances structural rigidity, protects against exopeptidase degradation, and can preserve or improve bioactivity. Another example is side chain-to-side chain cyclisation, in which a covalent bond is formed between the side chains of two amino acids. Examples include a disulfide bridge between two cysteine residues, a thioether bond (e.g. between a cysteine and a bromoacetylated lysine), an amide bond (e.g., between the amine of lysine and the carboxyl of glutamic acid or aspartic acid, forming a lactam bridge). This approach allows control over loop size and maintains free N- and C-termini, enabling further functionalisation. Another example is head-to-side chain cyclisation, where the N-terminal amine or C-terminal carboxyl forms a covalent bond with a side chain functional group (e.g., the amine of lysine or the carboxyl of aspartic/glutamic acid). This offers intermediate ring size flexibility. Another example is Hydrocarbon stapling, which involves installing a covalent hydrocarbon bridge between alkenylated amino acid side chains on the same face of an α-helix. This method stabilises the helical conformation and can improve membrane translocation and protease resistance.

In some embodiments, the CPPs are partially cyclic or cyclic. In some embodiments, the CPP is cyclised via a head-to-tail peptide bond. In some embodiments, the CPP is cyclised via a side chain-to-side chain bond. In some embodiments, the CPP is cyclised via a head-to-side chain bond. In another embodiment, the CPP is cyclic via hydrocarbon stapling. In preferred embodiments, the CPP is cyclised via a disulfide bond, an amide bond, or lactam bridge formation. In a particularly preferred embodiment, the CPP is cyclised via a disulfide bond.

In some embodiments, the CPPs are bicyclic or multicyclic, containing two or more interconnected cyclic structures.

### Sequence Identity

In preferred embodiments, the contiguous amino acid portion has at least 80% sequence identity to a same-length portion within a protein loop domain of a fungal glycoside hydrolase. In further embodiments, the contiguous amino acid portion has at least 85% sequence identity to a same-length portion within the protein loop domain of the fungal glycoside hydrolase. In further embodiments, the contiguous amino acid portion has at least 90% sequence identity to a same-length portion within the protein loop domain of the fungal glycoside hydrolase. In further embodiments, the contiguous amino acid portion has at least 95% sequence identity to a same-length portion within the protein loop domain of the fungal glycoside hydrolase. In further embodiments, the contiguous amino acid portion has 100% sequence identity to a same-length portion within the protein loop domain of the fungal glycoside hydrolase.

Likewise, in preferred embodiments, the CPP P2-P3 region, P1-P2-P3 region, P2-P3-P4 region, or P1-P2-P3-P4 region has at least 80% sequence identity to the corresponding P2-P3 region, P1-P2-P3 region, P2-P3-P4 region, or P1-P2-P3-P4 region sequence within a protein loop domain of a fungal glycoside hydrolase. In further embodiments, the CPP P2-P3 region, P1-P2-P3 region, P2-P3-P4 region, or P1-P2-P3-P4 region has at least 85% sequence identity to the corresponding P2-P3 region, P1-P2-P3 region, P2-P3-P4 region, or P1-P2-P3-P4 region sequence within the protein loop domain of the fungal glycoside hydrolase. In further embodiments, the CPP P2-P3 region, P1-P2-P3 region, P2-P3-P4 region, or P1-P2-P3-P4 region has at least 90% sequence identity to the corresponding P2-P3 region, P1-P2-P3 region, P2-P3-P4 region, or P1-P2-P3-P4 region sequence within the protein loop domain of the fungal glycoside hydrolase. In further embodiments, the CPP P2-P3 region, P1-P2-P3 region, P2-P3-P4 region, or P1-P2-P3-P4 region has at least 95% sequence identity to the corresponding P2-P3 region, P1-P2-P3 region, P2-P3-P4 region, or P1-P2-P3-P4 region sequence within the protein loop domain of the fungal glycoside hydrolase. In further embodiments, the CPP P2-P3 region, P1-P2-P3 region, P2-P3-P4 region, or P1-P2-P3-P4 region has 100% sequence identity to the corresponding P2-P3 region, P1-P2-P3 region, P2-P3-P4 region, or P1-P2-P3-P4 region sequence within the protein loop domain of the fungal glycoside hydrolase.

Differences in sequence between the contiguous amino acid portion and the same-length portion within the protein loop domain, or between the CPP P2-P3 region, P1-P2-P3 region, P2-P3-P4 region, or P1-P2-P3-P4 region and the corresponding P2-P3 region, P1-P2-P3 region, P2-P3-P4 region, or P1-P2-P3-P4 region sequence within the protein loop domain can be additions, deletions, and/or substitutions. In a more preferred embodiment, any differences in sequence are substitutions. In a particularly preferred embodiment, any substitutions share the same physiochemical properties, i.e., are conservative substitutions. Conservative substitution tables providing functionally similar amino acids are well-known in the art. The following eight groups contain exemplary amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)).

### Measuring Transport of Cargo

The CPPs, pharmaceutical compositions, methods, and uses of the invention facilitate efficient intracellular delivery of cargo. Efficient transport of cargo across a cell membrane, i.e., CPP efficacy, can be measured by a variety of techniques. In the present application, intracellular delivery of cargo by the CPPs of the invention is measured by their ability to provide gene editing in a cell, for example when covalently or noncovalently coupled to cargo for CRISPR/Cas9 gene editing. The presence of absence of gene editing in a cell can be easily determined by the skilled person by assays known in the art. In some embodiments, the efficiency of gene editing, such as CRSIPR/Cas9 gene editing, is measured using flow cytometry. For example, following transduction the knock-out efficiency of a gene targeted for CRISPR-Cas9 gene editing may be determined via antibody staining and cell sorting via flow cytometry to quantify the loss of expression of the target gene.

In some embodiments, a gene targeted for CRISPR-Cas9 gene editing can be amplified by PCR using the relevant primers and sent for Sanger sequencing to quantify the efficiency of gene editing. For example, the frequency of Insertion-deletions (Indels) in the target gene may be evaluated using software such as TIDE version 3.3.0 (Brinkman et al., 2014).

Efficient intracellular delivery of cargo can also be detected qualitatively or quantitatively using reporter constructs known in the art and available commercially, e.g. a luciferase or a GFP reporter construct, wherein levels of fluorescence correspond to levels of expression.

Efficient intracellular delivery of cargo can also be assessed by measuring the level of knockout achieved in a target gene, for example when the cargo is a siRNA. Methods to measure the level of knockout of a target gene are known in the art, for example using flow cytometry (FACS), or quantitative polymerase chain reaction (qPCR).

The Examples section provides further details of particularly preferred methods for measuring the transport of cargo across a cell membrane.

### Cell Viability

Known methods for transporting cargo across cell membranes, such as electroporation, can have a negative impact on cell viability. CPPs of the invention facilitate efficient intracellular delivery of cargo whilst maintaining good levels of cell viability. In a preferred embodiment, the CPPs, pharmaceutical compositions, methods, and uses of the invention have minimal impact on the viability of the cells.

Assays to measure proliferation, viability and cytotoxicity are known in the art and available commercially. Such assays can be used to monitor the response and health of cells in culture after treatment with various stimuli. The proper choice of an assay method depends on the number and type of cells used as well as the expected outcome.

In some embodiments, cell viability is assessed by quantifying the amount of ATP present to determine the number of metabolically active cells. For example, the amount of ATP can be quantified via use of a luciferin-luciferase luminescence assay, in which a luciferase enzyme catalyses a reaction involving luciferin and ATP to produce a measurable luminescent signal that is proportional to the amount of ATP present. Assays to determine the number of metabolically active cells via measurement of cellular ATP levels are available commercially (e.g., the CellTiter-Glo^{®} 2.0 Cell Viability Assay from Promega).

In preferred embodiments, more than 50%, more than 60%, more than 70%, more than 80%, more than 90%, more than 99% or all cells are viable after transduction.

There may be a recovery period prior to assessing cell viability. During the recovery period, the cells are typically cultured in cell culture medium suitable for the particular cell type. In some embodiments, the recovery period is between 12 hours and 3 days.

### Transport Temperature

The CPPs of the invention may be used to transport one or more cargo molecules into a target cell at any suitable temperature. For example, a suitable temperature may be from about 4°C to about 65°C, from about 10°C to about 50°C, from about 20°C to about 40°C, or from about 30°C to about 40°C, preferably 37°C.

In some embodiments, the temperature is at least 20°C. In some embodiments, the temperature is at least 30°C.

In some embodiments, the temperature is about 20°C. In some embodiments, the temperature is about 25°C. In some embodiments, the temperature is about 30°C. In some embodiments, the temperature is about 35°C. In some embodiments, the temperature is about 37°C. In some embodiments. the temperature is about 40°C.

### CPP Concentration

The CPPs of the invention may be used at a suitable concentration to facilitate the transport of cargo into target cells. For example, a suitable concentration may be from about 0.1µM to about 1000µM, from about 10µM to about 900µM, or from about 20µM to about 800µM. In some embodiments, the CPP may have a concentration from about 25µM to about 150µM.

In some embodiments, the CPP may have a concentration of about 5µM. In some embodiments, the CPP may have a concentration of about 10µM. In some embodiments, the CPP may have a concentration of about 20µM. In some embodiments, the CPP may have a concentration of about 25µM. In some embodiments, the CPP may have a concentration of about 40µM. In some embodiments, the CPP may have a concentration of about 50µM. In some embodiments, the CPP may have a concentration of about 75µM. In some embodiments, the CPP may have a concentration of about 100µM. In some embodiments, the CPP may have a concentration of about 125µM. In some embodiments, the CPP may have a concentration of about 150µM. In some embodiments, the CPP may have a concentration of about 200µM. In some embodiments, the CPP may have a concentration of about 400µM. In some embodiments, the CPP may have a concentration of about 800µM. In some embodiments, the CPP may have a concentration of about 900µM. In some embodiments, the CPP may have a concentration of about 1000µM.

In some embodiments, the CPP may have a concentration of less than 1000µM. In some embodiments, the CPP may have a concentration of less than 900µM. In some embodiments, the CPP may have a concentration of less than 800µM. In some embodiments, the CPP may have a concentration of less than 700µM.

In some embodiments, the CPP may have a concentration of less than 600µM. In some embodiments, the CPP may have a concentration of less than 500µM. In some embodiments, the CPP may have a concentration of less than 400µM. In some embodiments, the CPP may have a concentration of less than 300µM. In some embodiments, the CPP may have a concentration of less than 200µM. In some embodiments, the CPP may have a concentration of less than 100µM. In some embodiments, the CPP may have a concentration of less than 75µM. In some embodiments, the CPP may have a concentration of less than 50µM. In some embodiments, the CPP may have a concentration of less than 30µM.

### Pharmaceutical Compositions

The invention provides a pharmaceutical composition comprising a CPP of the invention, together with one or more pharmaceutically acceptable carriers, excipients, and/or diluents.

The pharmaceutical composition will typically include a pharmaceutically acceptable carrier, which may be any substance that facilitates administration and does not induce the production of harmful antibodies or elicit undue toxicity. Suitable carriers are well known in the art and include, but are not limited to, aqueous solvents such as sterile water, saline, glycerol and ethanol. Auxiliary substances such as wetting agents, emulsifying agents, and pH buffering substances may also be included. Suitable carriers can be easily determined by the skilled person using routine techniques.

The pharmaceutical composition may further comprise one or more pharmaceutically acceptable diluents, which function as inert vehicles for delivering the components of the composition. Suitable diluents are well known in the art and include, but are not limited to, sterile water for injection (WFI), water formulated with DMSO (e.g., 10% DMSO), normal saline (0.9% sodium chloride), phosphate-buffered saline (PBS), and dextrose solutions. The skilled person can easily determine the choice of diluent, which will depend on the route of administration and can be selected to optimise peptide solubility, stability, and delivery efficiency.

The pharmaceutical composition may additionally comprise one or more pharmaceutically acceptable excipients to support peptide formulation, manufacturing, and stability. Suitable excipients are well known in the art and include, but are not limited to, stabilising agents such as mannitol, trehalose, sucrose, histidine, arginine, lysine, aspartic acid, glutamic acid, proline, methionine or glycine, which preserve peptide integrity during processing, lyophilisation, and reconstitution. Buffering agents, including phosphate, citrate, or acetate buffers, may also be included to maintain a physiologically acceptable pH. Solubilising agents, such as polyethylene glycol (PEG), polysorbate 80 (Tween 80), or propylene glycol, may also be included to improve aqueous solubility. The skilled person can easily determine the choice of excipients, which will depend on the route of administration, release kinetics, and physicochemical properties of the components of the composition.

In some embodiments, the pharmaceutical composition may include an antimicrobial preservative, such as thiomersal or 2-phenoxyethanol. Preferably, the preservative is mercury-free or omitted altogether.

In some embodiments, the pharmaceutical composition may further comprise adjuvants, antioxidants, chelating agents, or targeting ligands to enhance therapeutic performance or site-specific delivery.

In some embodiments, the pharmaceutical composition may include an additional reagent, such as polyethyleneimine (PEl).

The pharmaceutical composition may be sterile and/or pyrogen-free.

### Formulations

In some embodiments, the CPPs are formulated for parenteral administration, including intravenous, subcutaneous, or intramuscular administration. Injection for local sub-cutaneous or intramuscular administration may be via a needle (e.g. a hypodermic needle), but needle-free injection may alternatively be used. Injectable formulation may comprise i) aqueous carriers, such as sterile WFI, normal saline, PBS, or dextrose solutions; ii) buffering agents such as phosphate, acetate, or citrate buffers; iii) stabilisers such as mannitol, trehalose, sucrose, or glycine; iv) solubilising agents, such as PEG, polysorbate 80 (Tween 80), or propylene glycol; and/or v) preservatives, such as benzyl alcohol or phenol, particularly where multi-dose formulations are employed. Such formulations may be provided in solution or lyophilised form, to be reconstituted prior to use.

In other embodiments the CPPs are formulated for oral, buccal, ophthalmic or sublingual administration in the form of tablets, capsules, powders, granules, solutions, gels or suspensions. Oral formulation may comprise i) diluents such as lactose, mannitol, sorbitol, or microcrystalline cellulose; ii) binders such as hydroxypropyl methylcellulose (HPMC), povidone, gelatin, or starch; iii) disintegrants such as croscarmellose sodium or sodium starch glycolate; iv) enteric or protective coatings such as cellulose acetate phthalate, Eudragit^{®}, or shellac; v) enzyme inhibitors such as aprotinin, leupeptin, or EDTA; and/or vi) absorption enhancers, such as sodium caprate, bile salts, or chitosan derivatives.

In other embodiments the CPPs may be delivered via the pulmonary or intranasal route, optionally in the form of sprays, aerosols, or dry powders. The formulation may comprise i) mucoadhesive agents, such as chitosan or carbomers; ii) permeation enhancers, such as bile salts, surfactants, or fatty acids; iii) isotonic carriers, such as saline or buffered solutions suitable for mucosal delivery; and/or iv) propellants, such as HFA-134a in pressurised metered-dose inhalers.

In some embodiments the CPPs are formulated for topical or transdermal application, using creams, gels, ointments, patches, or lipid-based carriers such as liposomes, ethosomes, or nanoemulsions.

### Combinations

In some embodiments, the invention provides a pharmaceutical composition comprising one or more CPPs and one or more cargo molecules. The pharmaceutical compositions may contain the CPP and the cargo in a non-covalent complex. The pharmaceutical compositions may contain the CPP covalently linked to the cargo. The CPP and the cargo may simply be mixed in the pharmaceutical composition.

In some embodiments, the CPP is maintained in a solution comprising DMSO in a concentration of from about 9% DMSO to about 15% DMSO, e.g., about 10% DMSO, for a period of time before being contacted with the cargo. When a CPP is prepared in a solution of less than 10% DMSO, the peptides may bind to each other and in some cases may not productively associate with the cargo. Thus, in some embodiments, the CPP is maintained in a solution comprising DMSO in a concentration of from about 10% DMSO to about 15% DMSO, e.g., about 10% DMSO, for a period of time before being contacted with the cargo that is present in a solution (e.g., a buffered aqueous solution) without DMSO.

The CPP and cargo may be administered simultaneously, sequentially, or separately depending on the therapeutic context.

### Dosage Forms

The appropriate dose may vary depending upon the health and physical condition of the individual to be treated, age, taxonomic group (e.g. human, non human primate, primate, etc.), the degree of transduction desired, the formulation of the pharmaceutical composition, the treating doctor's assessment of the medical situation, and other relevant factors. Administration may be in single or multiple doses. Multiple doses may be administered via the same or different routes and to the same or different location. Alternatively, doses can be via a sustained release formulation, in which case less frequent administration is required.

Dosage and frequency may vary depending on the half-life of the pharmaceutical composition in the patient and the duration of treatment desired.

Dosage regimens may be adjusted to provide the optimum desired response, for example a therapeutic response. For example, a single dose may be administered, several divided doses maybe administered over time or the dose may be proportionally reduced or increased as indicated by the exigencies of the therapeutic situation.

Effective dosage volumes can be routinely established, depending on the purpose of the composition. Typical human dose of the composition might be, for example about 0.5ml e.g. for intramuscular injection (e.g. local injection into the muscle or tissue of interest).

### Methods for Transporting Cargo Across a Cell Membrane

The invention provides a method for transporting cargo across a cell membrane into a cell using a CPP of the invention or a pharmaceutical composition of the invention, wherein the method comprises the step of contacting the cell with the CPP and the cargo, and wherein the CPP mediates the transport of the cargo across the cell membrane.

In some embodiments, the method comprises the step of contacting the cell with a CPP and a first cargo, followed by the step of contacting the cell with a CPP and a second cargo. In some embodiments, the method comprises the step of contacting the cell with a CPP and a first cargo, followed by the step of contacting the cell with a CPP and a second cargo, followed by the step of contacting the cell with a CPP and a third cargo. In some embodiments, the second cargo is different from the first cargo. In some embodiments, the third cargo is different from the second cargo and different from the first cargo.

The CPP and the cargo can be contacted with the cell in combination, either simultaneously, sequentially, or separately in any order. In a preferred embodiment, they are administered simultaneously (e.g. from a container containing the combination). In some embodiments, the method involves the first step of mixing the CPP and the cargo.

In some embodiments, the method comprises the step of obtaining and/or maintaining the cells in culture medium prior to transduction. In some embodiments, the cell is plated in a culture medium, suitable for the particular cell, prior to transduction. In some embodiments, the method further comprises contacting the cell with a culture medium during transduction, for at least the necessary time period for transduction to occur. In some embodiments, the method comprises the step of obtaining the cells and/or maintaining the cells in culture medium after transduction.

The method for transporting cargo across a cell membrane may be performed in vivo, ex vivo, or in vitro. Preferably, the method for transporting cargo across a cell membrane is performed in vivo or ex vivo.

In some embodiments, the method comprises one round of transduction. However, in other embodiments, multiple rounds of transduction may be desirable. For example, in some embodiments 2, 3, 4, 5, 6, 7, 8, 9, 10 or more rounds of transduction are carried out on the same cells. Each round of transduction may involve transduction of the same cargo or of different cargoes.

Methods for gene editing are also provided, wherein cargo is transported across a cell membrane into a cell using a CPP or a pharmaceutical composition of the invention, wherein the method comprises the step of contacting the cell with the CPP and the cargo, wherein the CPP mediates the transport of the cargo across the cell membrane, and wherein the cargo comprises gene editing components or a gene editing system. In some embodiments the cargo comprises components for CRISPR/cas9 gene editing or a CRISPR/cas9 gene editing system.

For the avoidance of any doubt, it is to be understood that these methods and protocols are compatible and combinable with any cargo or cargoes, including gene editing proteins that are challenging to transduce into cells, and any cell type, including cell types that are hard to transduce.

### Uses in Therapy

The invention provides CPPs and pharmaceutical compositions comprising the same for use in therapy. CPPs and pharmaceutical compositions of the invention are for use in transporting cargo across a cell membrane into the cells of a subject, wherein the cargo has a therapeutic effect on the cells thereby treating, preventing, or ameliorating a disease or disorder in the subject.

The CPPs and pharmaceutical compositions of the invention can be used to transport a wide range of diverse cargo into a variety of cell types. Therefore, the CPPs and pharmaceutical compositions of the invention can be used to treat a wide range of diverse diseases and disorders in a subject. Types of diseases or disorders that may be treated with the CPPs and pharmaceutical compositions of the invention include, but are not limited to, cancers, genetic disorders, infectious diseases, autoimmune diseases, inflammatory diseases, metabolic disorders, neurodegenerative diseases, cardiovascular diseases, retinal diseases and eye disorders, respiratory diseases, musculoskeletal and connective tissue disorders, endocrine disorders, dermatological disorders, and age-related disorders.

A subject can be any animal, preferably a human. Non-human animals include, for example, livestock and pets, such as ovine, bovine, porcine, canine, feline and murine mammals.

Therapy using CPPs and pharmaceutical compositions of the invention can be in vivo.

Therapy using CPPs and pharmaceutical compositions of the invention can be ex vivo. For example, the CPPs and pharmaceutical compositions of the invention can be used to transport cargo (e.g., proteins, nucleic acids, gene editing tools) into isolated cells outside the body of a subject. Cells modified outside the body may then be transplanted back into the subject to treat a wide range of diseases and disorders. Sources of cells for ex vivo therapy may be autologous (the transduced cell is transplanted back into the same subject it was taken from), allogeneic (the transduced cell is transplanted back into a different subject) or heterologous (the transduced cell is from a different species than the subject). Preferably, the subject is a human. Cells of any cell type e.g., stem cells, immune cells, may be isolated from a subject for ex vivo manipulation. The cells typically are primary cells, such as those isolated directly from a subject and/or isolated from a subject and frozen.

In some embodiments, the cells isolated for ex vivo modification are immune cells. For example, sources of cells for ex vivo manipulation may include, but are not limited to autologous, allogenic or heterologous donor blood, cord blood, or bone marrow. A blood-derived sample for ex vivo manipulation may be derived from an apheresis or leukapheresis product. Exemplary samples include whole blood, peripheral blood mononuclear cells (PBMCs), leukocytes, bone marrow, thymus, tissue biopsy, tumour, leukaemia, lymphoma, lymph node, gut associated lymphoid tissue, mucosa associated lymphoid tissue, spleen, other lymphoid tissues, liver, lung, stomach, intestine, colon, kidney, pancreas, breast, bone, prostate, cervix, testes, ovaries, tonsil, or other organ, and/or cells derived therefrom.

In some embodiments, the CPP or pharmaceutical compositions of the invention can be used to introduce a chimeric antigen receptor (CAR) into T cells, for use in CAR-T cell therapy. The CPPs and pharmaceutical compositions of the invention may be particularly suitable for this application, due to their ability to efficiently transport cargo into primary T cells.

In some embodiments, the CPP or pharmaceutical composition thereof is administered to a subject who does not exhibit signs of the disease and/or disorder. In some embodiments, treatment may be administered to a subject who only exhibits early signs of a disease and/or disorder and may for example decrease risk of developing pathology associated with the disease and/or disorder. In some embodiments, treatment may be of a subject who exhibits one or more signs of the relevant disease and/or disorder. In some embodiments, treatment may be of a subject who has been diagnosed as suffering from the relevant disease and/or disorder. In some embodiments, treatment may be of a subject known to have one or more susceptibility factors, e.g., that are statistically correlated with increased risk of development of the relevant disease and/or disorder. In some embodiments the treatment results in improvement in at least one symptom relative to the same symptom in the absence of the treatment. In some embodiments, the treatment results in a reduction in the severity or frequency of a symptom or a delay in the onset or progression of the severity or frequency of a symptom.

### Genetic Disorders

The CPPs and pharmaceutical compositions of the invention can transport DNA, RNA (e.g., mRNA, siRNA, or antisense oligonucleotides) or gene editing components (e.g., CRISPR/Cas components, base editing components, or prime editing components) into cells to correct or modulate disease-associated genetic mutations. For example, delivery of CRISPR/Cas components enables in situ gene correction, while delivery of mRNA may transiently restore expression of a missing or non-functional protein. Therefore, in some embodiments, the CPPs and pharmaceutical compositions of the invention can be used in gene therapy, e.g. for the treatment of genetic disorders, including inherited disorders in a subject.

Exemplary genetic disorders include, but are not limited to, 22q11.2 deletion syndrome, Angelman syndrome, Canavan disease, Charcot-Marie-Tooth disease, Colour blindness, Cri du chat, Cystic fibrosis, Down syndrome, Haemochromatosis, Haemophilia, Klinefelter syndrome, Type 1 neurofibromatosis, Type 2 neurofibromatosis Phenylketonuria, Polycystic kidney disease, Prader-Willi syndrome, Sickle-cell disease, Tay-Sachs disease, Turner syndrome, Marfan syndrome, familial hypercholesterolemia, achondroplasia, hereditary spherocytosis, spinal muscular atrophy, thalassemia, glycogen storage diseases, Wilson's disease, Duchenne muscular dystrophy, haemophilia, G6PD deficiency, Fragile X syndrome, X-linked agammaglobulinemia, Rett syndrome, Alport syndrome, DiGeorge syndrome, Williams syndrome, Angelman syndrome, Huntington's disease, Myotonic dystrophy type 1, Spinocerebellar ataxias, and Friedreich's ataxia.

### Infectious Diseases

The CPPs and pharmaceutical compositions of the invention can transport agents into infected cells that act directly on pathogens (e.g. by inhibiting pathogen replication or blocking pathogen-host interactions) or modulate the host immune responses. For example, CPPs and pharmaceutical compositions of the invention can transport antiviral, antibacterial (e.g., antibiotics), antifungal or antiparasitic agents into cells, or immunomodulators or vaccine antigens into cells. Therefore, in some embodiments, the CPPs and pharmaceutical compositions of the invention can be used in the treatment of infectious diseases, for example viral, bacterial, fungal and/or parasitic infectious diseases in a subject. In some embodiments, the CPPs and pharmaceutical compositions of the invention can be used for vaccination against an infectious disease.

The infectious disease to be treated can be a bacterial disease. In some embodiments, the infection is caused by a gram-positive bacterium. In other embodiments, the infection is caused by a gram-negative bacterium. The CPPs and pharmaceutical compositions of the invention may also transport antibacterial agents directly into bacterial cells, enhancing therapeutic efficacy against hard-to-treat infections (e.g., those caused by multidrug-resistant bacteria). Exemplary bacterial infections include, but are not limited to, pneumonia, cholera, meningitis, strep throat, MRSA infection, tuberculosis, typhoid and shigellosis.

The infectious disease to be treated can be a viral disease. Exemplary viral diseases include, but are not limited to HIV, dengue, hepatitis A/B/C, influenza, Japanese encephalitis, rotavirus, measles, meningitis, coronaviruses e.g., SARS-CoV-2, herpesviruses (e.g., herpes simplex virus or cytomegalovirus) and pneumonia.

The infectious disease to be treated can be a parasitic disease. In some embodiments, the parasitic disease is a protozoan infection. In some embodiments, the parasitic disease is a helminth infection. Exemplary parasitic diseases include, but are not limited to, malaria, African trypanosomiasis, cryptosporidiosis, leishmaniasis, Onchocerciasis ("river blindness"), and schistosomiasis.

The infectious disease to be treated can be a fungal infection. Exemplary fungal infections include, but are not limited to, candidiasis, aspergillosis and cryptococcosis.

### Cancer

The CPPs and pharmaceutical compositions of the invention can transport therapeutic agents, such as small molecule chemotherapeutics, RNA therapeutics (e.g., siRNA or miRNA), proteins and peptides (e.g., cytokines or tumour suppressor proteins), immunotherapies (e.g., chimeric antigen receptors (CARs), such as in CAR-T, CAR-NK, CAR-M (Macrophages), CAR-γδ T cells, CAR-iNKT (Invariant Natural Killer T cells), CAR-B (CAR-engineered B cells), or gene editing components (e.g., CRISPR/Cas components, base editing components, or prime editing components) into cancer cells. Therefore, in some embodiments, the CPPs and pharmaceutical compositions of the invention can be used in the treatment of cancer in a subject.

The CPPs and pharmaceutical compositions of the invention can be used to treat any type of cancer in a subject. In some embodiments, the cancer is a solid tumour. In some embodiments the cancer is a hematologic malignancy. In some embodiments, the cancer is a sarcoma. In some embodiments the cancer is a malignancy of the central nervous system.

Exemplary solid tumours include, but are not limited to, breast cancer, lung cancer (including non-small cell and small cell), colorectal cancer, prostate cancer, pancreatic cancer, liver cancer, stomach cancer, esophageal cancer, bladder cancer, kidney cancer, cervical cancer, ovarian cancer, endometrial cancer, head and neck cancers (including oral, pharyngeal, and laryngeal cancers), and skin cancers such as melanoma. Exemplary hematologic malignancies include, but are not limited to, leukemia (e.g., acute lymphoblastic leukemia and acute myeloid leukemia), lymphoma (e.g., Hodgkin lymphoma and non-Hodgkin lymphoma), and multiple myeloma. Exemplary sarcomas include, but are not limited to, osteosarcoma, liposarcoma, and rhabdomyosarcoma. Exemplary central nervous system malignancies include, but are not limited to, glioblastoma and medulloblastoma.

### Autoimmune and Inflammatory Diseases

The CPPs and pharmaceutical compositions of the invention can transport therapeutics such as anti-inflammatory and immunomodulatory drugs to modulate pathological immune responses. Such drugs may modulate immune cell function, suppress pro-inflammatory cytokine expression, or restore regulatory immune pathways. Therefore, in some embodiments, the CPPs and pharmaceutical compositions of the invention can be used in the treatment of an autoimmune or inflammatory disease or disorder in a subject.

Exemplary autoimmune or inflammatory diseases include, but are not limited to, rheumatoid arthritis, systemic lupus erythematosus (SLE), multiple sclerosis, inflammatory bowel disease (IBD) including Crohn's disease and ulcerative colitis, psoriasis, ankylosing spondylitis, systemic sclerosis (scleroderma), polymyositis, dermatomyositis, vasculitis, Sjögren's syndrome, juvenile idiopathic arthritis, chronic obstructive pulmonary disease (COPD), asthma, or acute or chronic inflammatory conditions such as osteoarthritis and tendinitis.

### Metabolic Disorders

The CPPs and pharmaceutical compositions of the invention can transport gene editing components (e.g., CRISPR/Cas components, base editing components, or prime editing components) for the correction of inherited genetic metabolic disorders, or therapeutic enzymes for enzyme replacement therapy (ERT). Therefore, in some embodiments, the CPPs and pharmaceutical compositions of the invention can be used in the treatment of a metabolic disorder in a subject. In some embodiments, the metabolic disorder is a lysosomal storage disease, a carbohydrate metabolism disorder, a lipid metabolism disorder, a mitochondrial disorder, or an amino acid metabolism disorder.

Exemplary metabolic diseases and disorders include, but are not limited to diabetes mellitus (type 1 and type 2), familial hypercholesterolemia, phenylketonuria (PKU), maple syrup urine disease, homocystinuria, galactosemia, glycogen storage diseases (such as Von Gierke disease and Pompe disease), Leigh syndrome, disorders of fatty acid oxidation such as medium-chain acyl-CoA dehydrogenase deficiency (MCADD), Gaucher disease, Fabry disease, and Niemann-Pick disease.

### Neurodegenerative Diseases

The CPPs and pharmaceutical compositions of the invention can transport therapeutic proteins, nucleic acids, small molecules and/or genome editing machinery into neuronal cells. For example, the CPPs and pharmaceutical compositions of the invention can transport neuroprotective proteins (e.g., BDNF or NGF), RNA-based therapies (e.g., siRNAs), gene editing components (e.g., CRISPR/Cas components, base editing components, or prime editing components), or peptides that inhibit aggregation of pathological proteins (e.g., tau or Aβ) into cells. Therefore, in some embodiments, the CPPs and pharmaceutical compositions of the invention can be used in the treatment of a neurodegenerative disease or disorder in a subject.

Exemplary neurodegenerative diseases or disorders include, but are not limited to, Alzheimer's disease, Parkinson's disease, Huntington's disease, amyotrophic lateral sclerosis (ALS), frontotemporal dementia (FTD), multiple system atrophy (MSA), progressive supranuclear palsy (PSP), spinocerebellar ataxias, spinal muscular atrophy (SMA), prion diseases (such as Creutzfeldt-Jakob disease), Lewy body dementia, corticobasal degeneration, Friedreich's ataxia, and Charcot-Marie-Tooth disease.

### Cardiovascular Diseases

The CPPs and pharmaceutical compositions of the invention can transport agents into cardiomyocytes, vascular endothelial cells, or smooth muscle cells. Therefore, in some embodiments, the CPPs and pharmaceutical compositions of the invention can be used in the treatment of a cardiovascular disease or disorder in a subject. For example, the CPPs and pharmaceutical compositions of the invention can transport siRNA or antisense oligonucleotides to modulate gene expression (e.g., targeting PCSK9 to lower LDL cholesterol), gene editing components to correct mutations in cardiomyopathy genes (e.g., CRISPR/Cas components, base editing components, or prime editing components), proteins or peptides that restore contractile function or modulate hypertrophy, mRNA encoding vasodilatory or anti-fibrotic factors (e.g., VEGF, ANP, NO synthase), or enzymes to reduce oxidative stress or metabolic burden.

Exemplary cardiovascular diseases or disorders include, but are not limited to, familial hypercholesterolemia, atherosclerosis, ischemic heart disease, congestive heart failure, dilated or hypertrophic cardiomyopathy, arrhythmias (e.g., long QT syndrome), and hypertension.

### Respiratory Diseases

The CPPs and pharmaceutical compositions of the invention can be used to transport agents into airway epithelial cells, alveolar macrophages, or smooth muscle cells in the respiratory tract. For example, the CPPs and pharmaceutical compositions of the invention can transport antisense oligonucleotides or siRNA to reduce expression of inflammatory or fibrotic genes, mRNA or plasmid DNA encoding surfactant proteins, ion channels, or anti-inflammatory cytokines, proteins or peptides to restore mucus clearance or inhibit bronchoconstriction, or genome editing components (e.g., CRISPR/Cas components, base editing components, or prime editing components) to repair mutations causing disorders like cystic fibrosis.

Exemplary respiratory diseases or disorders include, but are not limited to asthma, chronic obstructive pulmonary disease (COPD), idiopathic pulmonary fibrosis, cystic fibrosis, bronchiectasis, and acute respiratory distress syndrome (ARDS).

### Therapy Using Gene Editing Cargo

The CPPs and pharmaceutical compositions of the invention are particularly useful for transporting gene editing cargo across cell membranes, e.g., CRISPR/Cas cargo, base editing cargo, or prime editing cargo. In a preferred embodiment, the CPP or pharmaceutical composition of the invention is for use in therapy, wherein the CPP transports gene editing cargo across a cell membrane into the cells of a subject, wherein the gene editing cargo has a therapeutic effect on the cells thereby treating, preventing, or ameliorating a disease or disorder in the subject. In some embodiments, the gene editing cargo comprises components for CRISPR/cas gene editing, preferably CRISPR/cas9 gene editing. In some embodiments, the gene editing cargo comprises components for CRISPR/cas12 gene editing. In some embodiments, the gene editing cargo comprises components for CRISPR/cas13 gene editing. In some embodiments, the gene editing cargo comprises components for base editing. In some embodiments, the gene editing cargo comprises components for prime editing. In some embodiments the gene editing cargo comprises a protein selected from zinc finger nucleases, TALENS, a Cascade complex, Cre recombinases, a TtAgo protein, and an Argonaute protein.

Such cargo may be useful for treating a disease or disorder selected from HIV, Hemophilia B, Mucopolysaccharidosis I, Mucopolysaccharidosis II, Sickle Cell Disease, Thalassemia (such as β-Thalassemia, Thalassemia Major, β-thalassemia Major, and Transfusion Dependent Beta-thalassemia), Leber Congenital Amaurosis 10 (LAC10), Human Papillomavirus-Related Malignant Neoplasm, Acute Myeloid Leukemia, Multiple Myeloma, B-cell Acute Lymphoblastic Leukemia, Metastatic Non-small Cell Lung Cancer, B Cell Leukemia/B Cell Lymphoma, EBV positive advanced stage malignancies, Esophageal Cancer, T cell malignancy, Solid Tumour, Melanoma, Synovial Sarcoma, Liposarcoma, B-cell malignancies, D19+ leukemia or lymphoma, Gastro-Intestinal (GI) Cancer, Renal Cell Carcinoma, and Advanced Hepatocellular Carcinoma. These disorders are all the subject of clinical trials of gene therapy using genome-editing technology (see Xu and Li, CRISPR-Cas systems: Overview, innovations and applications in human disease research and gene therapy. Comput Struct Biotechnol J., 2020, 18: 2401-2415).

### Uses in Diagnosis

The CPPs and pharmaceutical compositions of the invention are for use in methods of diagnosis, wherein the CPP transports diagnostic cargo across a cell membrane into the cells of a subject, wherein the diagnostic cargo is for the purpose of imaging, cell labelling, and/or cell tracking.

Exemplary diagnostic cargo transported by the CPPs and pharmaceutical compositions of the invention include, but are not limited to, fluorescent probes, fluorescent dyes, radiolabels or contrast agents, bioluminescent proteins or peptides, and reporter genes or reporter enzymes.

Fluorescent probes or dyes may be used for diagnostic techniques such as real-time imaging, fluorescence microscopy, flow cytometry, and deep tissue imaging. Suitable fluorescent probes or dyes include, but are not limited to, small molecule fluorophores such as fluorescein, rhodamine, cyanine dyes, Alexa Fluor^{®} dyes, BODIPY dyes, near-infrared dyes e.g., indocyanine green for deep tissue imaging, or other fluorescent molecules capable of emitting light upon excitation.

Radiolabels may be used for techniques such as single photon emission computerized tomography (SPECT) and positron emission tomography (PET). Suitable radiolabels include and are not limited to technetium-99m (Tc-99m), rubidium-82 (Rb-82), molybdenum-99 (Mo-99), fluorine 18 (F-18), thallium-201 chloride (TI-201), and/or Indium-111 (In-111). Contrast agents may include gadolinium-based contrast agents for magnetic resonance imaging (MRI), microbubble contrast agents for ultrasound imaging, and/or iodine-based contrast agents for X-ray or computed tomography (CT) imaging.

Bioluminescent proteins or peptides can allow for the real-time monitoring of cellular and molecular processes. Suitable bioluminescent proteins or peptides include, but are not limited to, luciferases (e.g., firefly luciferase, Renilla luciferase, or NanoLuc^{®} luciferase, and their corresponding substrates), and/or aequorin.

Reporter genes may be used to monitor gene expression, cellular uptake, identify signalling pathways or monitor other biological processes. Reporter enzymes can generate detectable products following delivery into cells, allowing indirect measurement of cell viability, proliferation, or metabolic activity. Suitable reporter genes include, but are not limited to, genes encoding fluorescent proteins (e.g., GFP, RFP), luminescent enzymes (e.g., luciferase), or enzymes such as β-galactosidase, β-lactamase, chloramphenicol acetyltransferase (CAT), glycosidases, and/or alkaline phosphatase. Suitable reporter enzymes include, but are not limited to luciferases, β-galactosidase, alkaline phosphatase, horseradish peroxidase, and β-glucuronidase.

### Uses in Agriculture

The CPPs and pharmaceutical compositions of the invention can be used in agricultural biotechnology, such as in the field of plant genetic engineering. The CPPs of the invention can facilitate the efficient transport of cargo such as proteins, nucleic acids and gene editing components into plant cells, including protoplasts and whole plant tissues. The CPPs of the invention may enable the modification of plant genomes without reliance on traditional methods such as Agrobacterium-mediated transformation or biolistic gene delivery.

For example, the CPPs of the invention may be used to deliver CRISPR/Cas gene editing components into cells, facilitating the engineering of crops with advantageous traits such as disease resistance, drought or cold tolerance, improved crop growth, and improved nutritional profiles.

### Uses in Research

The CPPs and pharmaceutical compositions of the invention may also be used in research for a variety of applications. The CPPs of the invention may be used to deliver nucleic acids, proteins, gene editing components and/or other cargo into various cell types. For example, the CPPs of the invention may be used to transport plasmids to enable the transformation of bacterial cells.

### Uses as a Health Supplement

The CPPs and pharmaceutical compositions of the invention may also have applications as health supplements by enhancing the delivery of bioactive compounds into target cells. For example, the CPPs of the invention may be used to transport cargo such as weight-loss agents, antioxidants, vitamins, or collagen-stimulating peptides directly into target cells, which may have benefits such as weight-loss, improved skin condition and/or improved hair follicle health.

### Cargo

The CPPs, pharmaceutical compositions, methods, and uses of the invention can be used with any type of cargo. Exemplary types of cargo for use with all CPPs, pharmaceutical compositions, methods, and uses of the invention are discussed below. Cargo molecules include nucleic acids (e.g. DNA, RNA, CRISPR components or aptamers) and synthetic nucleic acid mimics (e.g., peptide nucleic acids (PNAs)), peptides or proteins (e.g., enzymes, antibodies, antibody fragments, therapeutic peptides or transcription factors), small molecules (e.g., drugs, fluorescent probes, fluorescent dyes or inhibitors), nanoparticles (e.g., silica nanoparticles, liposomes, gold nanoparticles, polymeric nanoparticles or quantum dots), macromolecules, (e.g., polysaccharides, lipids or synthetic polymers), viral vectors or virus-like particles (e.g., lentiviral vectors or retroviral vectors), or components for gene editing (e.g., zinc finger nucleases (ZFNs), transcription activator-like effector nucleases (TALENs), Cre recombinases (for Cre-Lox gene editing), components for CRISPR/cas9 gene editing, components for CRISPR/cas12 gene editing, components for base editing or components for prime editing).

The cargo may be coupled to the CPP non-covalently and/or covalently. The cargo may also be simply mixed with the CPP. Examples of cargo include, but are not limited to, nucleic acids, proteins or peptides, small molecules, nanoparticles, macromolecules, viral vectors or virus-like particles, and components for gene editing.

In some embodiments, the CPP-cargo complex comprises one cargo molecule for transport. In other embodiments, the CPP comprises more than one cargo molecule for transport, for example two, three, four, five or more different cargo molecules.

Any combination of one or more cargo molecules may be transported across a cell membrane by a CPP of the invention. The one or more cargo molecules may be of the same type, e.g. proteins, or of different types, e.g., a protein and a nucleic acid. In some embodiments 1, 2, 3, 4, 5, 6, 7, 8, 9, 10 or more different types of cargo are transported into a cell. The one or more cargo molecules may be transported across a membrane simultaneously, sequentially or separately.

### Nucleic Acids

In some embodiments, the CPPs and pharmaceutical compositions of the invention are used to transport one or more nucleic acids across a cell membrane into a cell.

In some embodiments, the nucleic acid is a natural nucleic acid, such as DNA or RNA. In other embodiments, the nucleic acid is a non-natural nucleic acid, such as xeno nucleic acids (XNAs). In some embodiments, the nucleic acid is a synthetic nucleic acid mimetic, such as peptide nucleic acids (PNAs).

In some embodiments, the nucleic acid comprises DNA, such as gDNA or cDNA. Exemplary DNA constructs for transport include, but are not limited to, plasmid DNA, minicircle DNA, and linear DNA constructs. DNA constructs may encode any suitable product, such as therapeutic proteins, transcription factors or genome-editing machinery.

In some embodiments, the nucleic acid comprises RNA, such as small interfering RNAs (siRNAs) for targeted gene silencing, short hairpin RNAs (shRNAs) for stable RNA interference, messenger RNAs (mRNAs) encoding e.g., therapeutic proteins, enzymes, antigens, or gene-editing nucleases, antisense oligonucleotides for modulation of pre-mRNA splicing or inhibition of gene expression, and microRNAs (miRNAs) for the regulation of endogenous gene networks in diseases such as cancer.

In some embodiments, the nucleic acid is for use in gene editing. In a preferred embodiment, the nucleic acid is a guide nucleic acid, including but not limited to an sgRNA, a gDNA, a dual annealed gRNA or a pegRNA. In some embodiments, the nucleic acid is an sgRNA or a dual annealed gRNA.

In some embodiments, the nucleic acid is an aptamer, which may bind to a variety of targets, including but not limited to small molecules, heavy metal ions, proteins, viruses, bacteria, and whole cells (e.g., cancer cells).

In some embodiments, the nucleic acid comprises modified nucleotides, such as naturally occurring modified nucleotides (e.g., 5-selenocysteine and pyrrolysine) or synthetic modified nucleotides such as locked nucleic acids (LNAs).

### Proteins and Peptides

In some embodiments, the CPPs and pharmaceutical compositions of the invention are used to transport one or more proteins or peptides across a cell membrane into a cell.

In some embodiments, the protein is an enzyme. For example, a metabolic enzyme (such as galactosidases, glucosidases, decarboxylases e.g. glutamate decarboxylase, dehydrogenases e.g. lactate dehydrogenase, aminotransferases e.g. aspartate transaminase, synthases e.g. citrate synthase, reductases e.g. dihydrofolate reductase or hydrolases e.g. arginase), a DNA modifying enzyme (e.g., a DNA polymerase, nuclease or ligase), a Cre recombinase, or an enzyme for use in gene editing (e.g., a Cas protein, particularly a Cas9 protein).

In some embodiments, the protein is an antibody or antibody fragment, including but not limited to full-length antibodies, single-chain variable fragments (scFvs), Fab fragments, diabodies, or nanobodies (VHH domains). Antibodies or antibody fragments thereof may be derived from any antibody isotype (e.g., IgA, IgD, IgE, IgG, and IgM). Antibodies and antibody fragments may be monoclonal or polyclonal. Antibodies may be monospecific or bispecific. Antibodies may be therapeutic antibodies, for example checkpoint inhibitors (e.g., an anti-PD-1 or anti-PD-L1/2 antibody).

In some embodiments, the protein is a therapeutic peptide. The therapeutic peptide may be a pro-apoptotic peptide, for example BH3-mimetics, DIF-1, or DP1, or a signalling peptide.

In some embodiments, the protein is a cytokine. The cytokine may be an interleukin, for example IL-1, IL-2, IL-12 or IL-10, an interferon, for example IFN-α, IFN-β or IFN-γ, a member of the tumour necrosis factor (TNF) superfamily, for example TNF-α or TNF-β, a colony-stimulating factor (CSF), for example Granulocyte-colony stimulating factor (G-CSF) or granulocyte-macrophage colony-stimulating factor (GM-CSF), or a chemokine, for example CCL2, CCL3, CXCL1, CXCL2 or CXCL8.

In some embodiments, the protein is a transcription factor. Exemplary transcription factor include, but are not limited to, general transcription factors (GTF), such as TIIFB or TIIFD, steroid hormone receptors, such as oestrogen receptor, androgen receptor, or glucocorticoid receptor, members of the NF-κB family, such as NF-κB1, NF-κB2 or RelA, a basic Helix-Loop-Helix (bHLH) transcription factor, a Zinc finger transcription factor, such as Sp1, a forkhead box (FOX) transcription factor, such as FOXO1 or FOXP3, a member of the signal transducers and activators of transcription (STAT) family, or p53.

### Small Molecules

In some embodiments, the CPPs and pharmaceutical compositions of the invention are used to transport one or more small molecules across a cell membrane into a cell.

In some embodiments, the small molecule is a drug. Exemplary drugs include, but are not limited to, chemotherapeutic agents such as doxorubicin, paclitaxel or cisplatin, anti-inflammatory drugs such as Nonsteroidal anti-inflammatory drugs (e.g., aspirin), corticosteroids (e.g., prednisolone or dexamethasone) or methotrexate, antibiotics such as beta-lactam antibiotics (e.g., penicillin), macrolide antibiotics (e.g., erythromycin) or aminoglycoside antibiotics (e.g., gentamicin), or antiviral drugs, such as acyclovir, oseltamivir, or remdesivir.

In some embodiments, the small molecule is a radioactive tracer, including and not limited to technetium-99m (Tc-99m), rubidium-82 (Rb-82), molybdenum-99 (Mo-99), fluorine 18 (F-18), thallium-201 chloride (TI-201), or Indium-111 (In-111).

In some embodiments, the small molecule is a metal chelator, such as an iron-chelating drug, a gadolinium-based chelator, or a chelator derived from indium, gallium, or zirconium.

In some embodiments, the small molecule comprises a fluorescent probe or dye, including but not limited to fluorescein, rhodamine, cyanine dyes, Alexa Fluor^{®} dyes, BODIPY dyes, or other fluorescent molecules capable of emitting light upon excitation.

In some embodiments, the small molecule is an inhibitor molecule, including but not limited to kinase inhibitors (e.g., imatinib, vemurafenib, or sorafenib), or protease inhibitors (e.g., HIV protease inhibitors or MMP inhibitors).

### Nanoparticles

In some embodiments, the CPPs and pharmaceutical compositions of the invention are used to transport one or more nanoparticles across a cell membrane into a cell.

In some embodiments, the nanoparticle is a liposome, a gold nanoparticle, a polymeric nanoparticle such as those made from poly (lactic-co-glycolic acid) (PLGA), polylactic acid (PLA), or polycaprolactone (PCL), or a quantum dot semiconductor particle.

In some embodiments, the nanoparticle is a silica nanoparticle, for example a mesoporous silica nanoparticle (MSN). MSNs have easily tunable pore sizes, shapes and surface properties which makes them well suited for drug delivery.

### Macromolecules

In some embodiments, the CPPs and pharmaceutical compositions of the invention are used to transport one or more macromolecules across a cell membrane into a cell.

In some embodiments the macromolecules is a polysaccharide such as a glycosaminoglycan (e.g., heparin or hyaluronic acid), dextran, or starch, a lipid molecule such as a phospholipid, sphingolipid, glycolipid, or other lipid derivative, a larger lipid molecule used as part of a drug delivery system such as a liposome, micelle, or lipid nanoparticle, or a synthetic polymer such as a PEGylated molecule or a polyglycerol (PG) polymer.

### Viral Vectors and Virus-like Particles

In some embodiments, the CPPs and pharmaceutical compositions of the invention are used to transport one or more viral vectors or virus-like particles (VLPs) across a cell membrane into a cell. A virus-like particle may include, for example, a viral capsid and/or viral envelope.

In some embodiments, the viral vector is a retroviral vector, including but not limited to Murine Leukaemia Virus (MLV)-based vectors and Murine Stem Cell Virus (MSCV)-based vectors.

In a preferred embodiment, the retroviral vector is a lentiviral vector, for example HIV-1-based vectors, simian immunodeficiency virus (SIV)-based vectors, equine infectious anaemia virus (EIAV)-derived vectors, or FIV feline Immunodeficiency Virus (FIV)-based vectors. The CPPs and pharmaceutical compositions of the invention can increase the permeability of the cell membrane to large molecules, such as lentiviral vectors, and are therefore particularly suited to transporting lentiviral vectors. The CPPs and pharmaceutical compositions of the invention may also assist with lentiviral vector endocytosis and endosomal escape.

In some embodiments, the viral vector is an adenovirus vector.

In some embodiments, the viral vector is an adeno-associated virus (AAV) vector for example AAV2, AAV5, or AAV9.

### Components for Gene Editing

The CPPs and pharmaceutical compositions of the invention are particularly useful for transporting gene editing cargo. Therefore, in preferred embodiments, the CPPs and pharmaceutical compositions of the invention are for transporting gene editing components and/or gene editing systems across a cell membrane into a cell.

Gene editing systems typically comprise proteins and nucleic acids. Therefore, CPPs for transporting gene editing components transport both proteins and nucleic acids into cells. Proteins and nucleic acids used for DNA-editing and RNA-editing are well known in the art (East-Seletsky et al, Mol. Cell, 2017, 66(3) 373-383.e3; Makarova et al, Nat. Rev. Microbiol., 2015, 13(11) 722-736; O'Connell, 2019, J. Mol. Biol., 431(1), 66-87; Xu and Li, Comput Struct Biotechnol J., 2020, 18: 2401-2415).

In some embodiments, the gene editing system comprises or consists of gene editing proteins. Gene editing proteins can target a specific sequence, such as zinc finger nucleases (ZFNs), Cre recombinases or TALENs or large serine recombinases (LSRs), or can be guided to a target sequences by a (separate) guide molecule, such as Cas proteins, proteins from the Cascade system, TtAgo and other Argonaute proteins, and other FOKI-nuclease associated proteins. Therefore, in some embodiments, CPPs and pharmaceutical compositions of the invention are used for transporting gene editing systems comprising zinc finger nucleases, TALENS, Cas proteins, a Cascade complex, a TtAgo protein or an Argonaute protein into cells.

In some embodiments, the gene editing system comprises or consists of gene editing proteins and guide molecules. Examples of guide molecules include, but are not limited to, sgRNA, gDNA, dual annealed gRNA, or pegRNA. In some embodiments, the guide molecule is a dual annealed gRNA. Such guide nucleic acids can be designed by methods known in the art to target a specific sequence in the target nucleic acid (see for example, Mali, P., et al., RNA-guided human genome engineering via Cas9. Science, 2013. 339(6121): p. 823-6 for sgRNA; and Swarts, D. et al, DNA-guided DNA interference by a prokaryotic Argonaute. Nature, 2014. 507, 258-261 for gDNA). Thus, in some embodiments, CPPs and pharmaceutical compositions of the invention are used for transporting gene editing systems comprising Cas proteins, a Cascade complex, a TtAgo protein or an Argonaute protein, and a sgRNA, a gDNA, a dual annealed gRNA or a pegRNA. A preferred example of a protein and nucleic acid combination which may be transported using the CPPs and pharmaceutical compositions of the invention is the combination of sgRNA and a Cas9 protein. Further specific preferred examples of protein and nucleic acid combinations include sgRNA and SpCas9; sgRNA and SpCas9-1xNLS; sgRNA and 4xNLS-SpCas9-2xNLS; dual annealed gRNA and SpCas9; dual annealed gRNA and SpCas9-1xNLS; and dual annealed gRNA and 4xNLS-SpCas9-2xNLS. In some embodiments, the nucleic acid and protein are present as nucleic acid-protein complexes. In some embodiments, the gene editing protein and the guide molecule are mixed prior to the addition of any of the CPPs of the invention.

### Cas Proteins

In a preferred embodiment, a gene editing protein is a Cas protein, including but not limited to Cas9, Cas12 and Cas13. Exemplary Cas9, Cas12 and Cas13 proteins include, but are not limited to, SpCas9, SaCas9, FnCas9, NmCas9, TCas9, dCas9, nCas9, Cas12a (Cpf1), Cas12b1 (C2c1), Cas12b2, Cas12c (C2c3), Cas12d (CasY), Cas12e (CasX), Cas13a (C2c2), Cas13b (C2c4), Cas13c (C2c7), Cas13d. Most preferably, the protein is a Cas9 protein. Cas9 proteins include variants such as SpCas9 and modified versions of these variants such as SpCas9-1xNLS. The same definitions apply to Cas12 and Cas13. Preferred examples of Cas9 proteins are SpCas9, most preferably SpCas9-1xNLS or 4xNLS-SpCas9-2xNLS.

In a particular embodiment, the Cas protein is a Cas12 protein, preferably Cas12a.

Generally, Cas proteins are part of CRISPR/Cas gene editing systems. Any of these Cas proteins may be transported in the form of a CRISPR-Cas system, with a suitable guide molecule, such as a single or dual annealed gRNA. In some embodiments, the protein is a part of a CRISPR-Cas system selected from CRISPR-Cas9, CRISPR-Cas prime editing, CRISPR-Cas base editing, CRISPR-dCas9, CRISPR-Cas12, and CRISPR-Cas13. In a particular embodiment, the CRISPR/Cas gene editing system comprises Cas12a and a single gRNA.

In a preferred embodiment, the CRISPR-Cas system is CRISPR-Cas9. For example, the CRISPR-Cas9 gene editing system may comprise a Cas9 protein and an sgRNA or a dual annealed gRNA. In another preferred embodiment, the CPP and Cas protein cargo are expressed as a fusion protein, for example a CPP-Cas9 fusion protein.

### Base Editing

In some embodiments, the gene editing system comprises a CRISPR-Cas base editing system. The CRISPR-Cas base editing system can comprise protein(s), polypeptide(s), or fragment(s) thereof capable of catalysing the conversion of a target nucleobase in a nucleic acid to another (e.g., A to G, A to C, A to T, C to T, C to G, C to A, G to A, G to C, G to T, T to A, T to C, T to G). In some embodiments, the base editing system comprises a programmable DNA binding domain (such as a Cas9 protein or variant, e.g., dCas9 or nCas9) fused to a nucleobase modifying enzyme (referred to herein as a "base editor") and a guide RNA (gRNA) comprising a guide sequence that is complementary to the target nucleic acid sequence to be edited. In some embodiments, the guide RNA may be a dual annealed gRNA. In a particular embodiment, a base editing system may comprise an adenine base editor (ABE) and a dual annealed gRNA.

In some embodiments, a nucleobase modifying enzyme may target cytosine (C) bases in a nucleic acid sequence and convert the C to thymine (T) base. In a preferred embodiment, the C to T editing is carried out by a deaminase, e.g., a cytosine deaminase. Exemplary cytosine deaminases include, and are not limited to, an apolipoprotein B mRNA-editing complex (APOBEC) family deaminase or an activation-induced deaminase (AID).

In other embodiments, the nucleobase modifying enzyme may target adenosine (A) bases in a nucleic acid sequence and convert the A to a guanine (G) base. The A to G editing may be carried out by a deaminase, e.g., an adenosine deaminase. The adenosine deaminase may be from any organism (such as a bacterium), or it may be artificial. In some embodiments, the adenosine deaminase is from a bacterium, such as, E.coli. For example, the adenosine deaminase may be a TadA deaminase.

### Prime Editing

In a preferred embodiment, the gene editing system comprises a CRISPR-Cas prime editing system. The CRISPR-Cas prime editing system can comprise protein(s), polypeptide(s), or fragment(s) thereof capable of catalysing the modification (insertion, deletion, or base-to-base conversion) of a target nucleotide or nucleotide sequence in a nucleic acid. In some embodiments, the CRISPR-Cas prime editing system comprises a DNA binding domain (e.g., a programmable DNA binding domain such as a Cas protein or variant thereof) and a polymerase. In various embodiments, the prime editing system also comprises a prime editing guide RNA (pegRNA). In a particular embodiment, the pegRNA may be a single guide RNA. In some embodiments, the programmable DNA binding domain and the polymerase may be provided as a fusion protein, or provided in trans i.e., not as a fusion protein.

Any suitable Cas protein may be used in the prime editors described herein, including and not limited to a Cas9 or variant thereof (e.g., SpCas9, SaCas9, FnCas9, NmCas9, TCas9, nCas9 or dCas9), a Cas12 or variant thereof (e.g., Cas12a (Cpf1), Cas12b1 (C2c1), Cas12b2, Cas12c (C2c3), Cas12d (CasY), or Cas12e (CasX)), or a Cas13 or variant thereof (e.g., Cas13a (C2c2), Cas13b (C2c4), Cas13c (C2c7), or Cas13d). In a preferred embodiment, the Cas protein is a Cas9 or variant thereof, for example dCas9 or nCas9.

In some embodiments, the CRISPR-Cas prime editing system comprises a polymerase. Any polymerase may be used, including wild type or modified polymerases. In some embodiments, the polymerase is a DNA-dependent DNA polymerase. In some embodiments, the polymerase is a RNA-dependent DNA polymerase. In some embodiments, the polymerase is a reverse transcriptase.

### Coupling Methods

The CPPs of the invention are coupled to one or more cargo molecules. In some embodiments, the cargo molecule is coupled at the C-terminal part or the N-terminal part of the CPP.

In some embodiments, the CPP and cargo are coupled non-covalently. For example, non-covalent complexes between a CPP and one or more cargo molecules may be achieved via electrostatic, polar, π-electron-cation, hydrophobic interactions, hydrogen bonding, van der Waals forces, or affinity-based binding. Specific binding pairs, such as biotin-avidin/streptavidin complexes and their derivatives, antibody-antigen interactions, or aptamer-ligand interactions, may also be used to non-covalently couple a CPP to one or more cargo molecules.

In some embodiments, the CPP and cargo are coupled covalently, without a linker or spacer. Suitable covalent bonds for covalent coupling include, but are not limited to, amide bonds such as peptide bonds, ester bonds, thioether bonds, disulfide bonds, hydrazone bonds, and click chemistry linkages (such as triazole linkages formed via azide-alkyne cycloaddition). In some embodiments, the CPP and cargo are coupled via expression as a CPP-cargo fusion protein, for example a CPP-Cas9 fusion protein (i.e., coupled by a peptide bond, such as when the CPP and cargo are encoded in the same open reading frame on the same plasmid). In some embodiments, the CPP and cargo are coupled covalently using a linker or spacer. A linker or a spacer is a non-immunologic chemical or biochemical moiety linking the CPP to the cargo molecule, and/or linking successive cargo molecules. A linker or a spacer may be cleavable, or non-cleavable. Cleavable linkers and spacers can respond to intracellular conditions such as pH, redox environment, or specific enzymes (e.g., disulfide bonds or protease-sensitive peptides) to allow controlled cargo release inside a cell. Alternatively, non-cleavable linkers or spacers provide stable, permanent attachment for applications where sustained conjugation is desirable.

Linkers and spacers can be peptidic or non-peptidic. A peptidic linker or spacer can consist of about 1, 2, 3, 4, 5, 6, 7, 8, 9, or 10 amino acids, a non-peptidic linker or spacer can include esters, thioesters or di-sulfides. In some embodiments, the CPP and cargo are coupled using a peptidic spacer placed between the CPP sequence and the peptidic, polypeptidic, or proteic cargo. Linkers and spacers can also comprises a polyethylene glycol (PEG) moiety to improve solubility, reduce immunogenicity, or enhance pharmacokinetics.

Spacers and linkers can be chosen by one skilled in the art, depending on the desired stability, bioavailability, targeting specificity, and functional release of the CPP-cargo construct.

A CPP may also be coupled to a cargo by simple mixing of the CPP and cargo, i.e., without there being any direct interaction.

Coupling may be reversible or irreversible, and may be accomplished in vitro or in vivo.

### Cell Types

In a preferred embodiment, the CPPs, pharmaceutical compositions, methods, and uses of the invention have reduced cell type variability. In a particularly preferred embodiment, the CPPs, pharmaceutical compositions, methods, and uses of the invention have efficient intracellular delivery of cargo and reduced cell type variability, whilst maintaining favourable cell viability.

The CPPs, pharmaceutical compositions, methods, and uses of the invention may be used to transport cargo into any cell type. The choice of target cell type may vary depending on the application, including but not limited to therapy, diagnosis, gene editing, and imaging.

In some embodiments the cell is in vivo, optionally in situ. For example, when treating or diagnosing a medical condition, the cargo could be administered directly and locally in combination with the CPP to an organism or tissue in need thereof.

In some embodiments, the cell is ex vivo. For example, cells may be isolated from a subject, exposed to the CPP and cargo outside the body under controlled conditions (e.g., for gene editing or labelling), and then reintroduced into the same or a different subject. This approach is applicable in therapies such as ex vivo gene-modified cell therapy, immunotherapy (e.g., T-cell or B-cell or NK-cell or macrophage cell engineering), or autologous stem cell transplantation.

In some embodiments, the cell is in vitro. For example, the cell may be maintained in a culture vessel such as a petri dish, well plate, or bioreactor, under laboratory conditions. The CPP and cargo may be added directly to the culture medium to facilitate uptake into the cells. Applications include high-throughput screening of gene function, delivery of reporter constructs for imaging assays, investigation of intracellular signalling pathways in immortalized cell lines, primary cells, or stem cells, or methods for drug development and screening, for example via the use of in vitro disease models such as 2D cell cultures, 3D spheroids, organoids, iPSC-derived disease models, or lab-on-a-chip (e.g. organ-on-a-chip) derived disease models.

In all embodiments, where the disclosure refers to a "cell", it refers to a single cell and also applies to a "cell population", for example of 2 or more, 10 or more, 100 or more, 1000 or more, 10⁴ or more, 10⁵ or more, 10⁶ or more, 10⁷ or more, 10⁸ or more, 10⁹ or more, 10¹⁰ or more cells.

### Mammalian Cells

In a preferred embodiment, the cell is a mammalian cell, such as a human, primate, rodent (e.g. mouse or rat), rabbit, dog, cat, horse, cow or pig cell. These mammals are useful for research purposes and/or may benefit from treatment or diagnosis using CPPs of the invention. Among these cell types, human cells are most preferred for use with the CPPs of the invention.

In some embodiments, the cell is an immune cell. In some embodiments, the immune cell is a T cell such as a CD8+ T cell (e.g., a primary CD8+ T cell, a CD8+ naive T cell, central memory T cell, or effector memory T cell), a CD4+ T cell, a natural killer T cell (NKT cells), a regulatory T cell (Treg), a stem cell memory T cell, a helper T cell, or a cytotoxic T cell. In some embodiments the immune cell is a B cell or leukocyte. In some embodiments the immune cell is a phagocyte (macrophage, neutrophil, or dendritic cell), mast cell, eosinophil, basophil, or natural killer cell. In preferred embodiments, the cell is a T cell, more preferably a primary T cell, such as a CD4+ cell. In a particularly preferred embodiment the immune cell is a T cell, such as a CD4+ T cell, a CD8+ T cell, or a Jurkat cell, or a natural killer cell.

In some embodiments, the cell is a stem cell or a cell derived by differentiation of a stem cell. In some embodiments the stem cell is a pluripotent stem cell, such as an embryonic stem cell, optionally a human embryonic stem cell. In some embodiments, the cell is not a human embryonic stem cell. In some embodiments, the stem cell is not obtained by methods that involve the use of human embryos for commercial or industrial purposes. In some embodiments, the stem cell is not obtained by methods that necessarily involve the destruction of a human embryo. In some embodiments the stem cell is a murine embryonic stem cell. In some embodiments, the cell is a murine or human neural stem cell, neuron cell or glia cell. In some embodiments, the cell is a somatic cell (e.g. a fibroblast) or a germ cell (e.g., an oocyte, a sperm, an oogonia, a spermatogonia, etc). In some preferred embodiments, the stem cell is an induced pluripotent stem cell. In particularly preferred embodiments, the cell is a human induced pluripotent stem cell (hiPSC). In another preferred embodiment, the cell is a hematopoietic stem cell such as a CD34+ hematopoietic stem cell.

Adult stem cells or somatic stem cells are particularly preferred and are resident in differentiated tissue, but retain the properties of self-renewal and ability to give rise to multiple cell types, usually cell types typical of the tissue in which the stem cells are found. Numerous examples of somatic stem cells are known to those of skill in the art, including muscle stem cells; hematopoietic stem cells; epithelial stem cells; neural stem cells; mesenchymal stem cells; mammary stem cells; intestinal stem cells; mesodermal stem cells; endothelial stem cells; olfactory stem cells; and neural crest stem cells.

In other embodiments the cell line is a primary cell line. A primary cell or cell line is derived from a cell taken directly from a living organism.

In some embodiments, the cell is derived from an established cell line, such as an established human cell line. In some embodiments, the established cell line is an immortalised cell line. Examples of established human cell lines suitable for use in the context of the invention include but are not limited to ARPE-19 (human retinal epithelial cell line), Jurkat (human T lymphocyte cell line), HEK293 (human embryonic kidney cell line), HeLa, ESTDAB database, DU145 (prostate cancer), Lncap (prostate cancer), MCF-7 (breast cancer), MDA-MB-438 (breast cancer), PC3 (prostate cancer), T47D (breast cancer), THP-1 (acute myeloid leukemia), COS7 (immortalised CV-1 cells from kidney tissue), U87 (glioblastoma), SHSY5Y human neuroblastoma cells, cloned from a myeloma, and Saos-2 cells (bone cancer). In some embodiments, the established cell line is a primate cell line, such as Vero (African green monkey Chlorocebus kidney epithelial cell line initiated in 1962). In some embodiments, the established cell line is a rodent cell line, such as GH3 (pituitary tumor), PC12 (pheochromocytoma) or MC3T3 (embryonic calvarium).

The CPPs of the invention have been shown to be particularly effective for transporting cargo into Jurkat cells, T cells, hematopoietic stem cell, natural killer cells, and hiPSCs. Therefore, in a preferred embodiment, the cell is a Jurkat cell, T cell, hematopoietic stem cell, natural killer cell, or hiPSC.

It is known in the art that transport can be difficult in in some cell types, such as hiPSCs and primary T cells. It has surprisingly been found that the CPPs of the invention can be used to transduce cargo into difficult-to-transduce cell lines, such as hiPSCs and primary T cells. In some embodiments, the cell is a difficult-to-transduce cell. Preferably, the cell is an induced pluripotent stem cell or a T cell. In particularly preferred embodiments, the cell is a hiPSC or a primary T cell.

A particular advantage of the CPPs and methods of the invention is the ability to transport proteins that are challenging to transport into cells, such as Cas9 proteins, into difficult-to-transduce cells. Thus, in one preferred embodiment, the invention provides a method for transporting a Cas9 protein into an induced pluripotent stem cell or a T cell, preferably a hiPSC or a primary T cell, using a CPP of the invention, wherein the method comprises the step of contacting the cell with the CPP and the Cas9 protein, and wherein the CPP mediates the transport of the cargo across the cell membrane.

### Plant Cells

In some embodiments, the CPP is for transporting cargo into plant cells. The structural composition of plant cells, including the presence of a rigid cell wall composed primarily of cellulose, hemicellulose, and pectin, presents additional barriers to cellular entry. Nevertheless, CPPs have been demonstrated to facilitate intracellular delivery in plant systems, particularly when used in combination with permeabilization agents, nanoparticle carriers, or protoplast-based systems.

In some embodiments, the CPPs are used to deliver cargo into plant protoplasts, which are plant cells with the cell wall enzymatically removed. Suitable plant species for CPP-based delivery include, but are not limited to, Arabidopsis thaliana, Nicotiana tabacum, Oryza sativa (rice), Zea mays (maize), Solanum lycopersicum (tomato), and Medicago truncatula.

In some embodiments, the CPPs are employed in intact plant cells using delivery systems that facilitate penetration of the cell wall, including but not limited to electroporation, sonoporation, or co-application with nanocarriers or cell wall-disrupting agents. In some embodiments, CPPs are applied to leaves, roots, or seedlings via infiltration, topical application, or hydroponic medium to achieve localized or systemic uptake.

Transport of cargo into plant cells via CPPs can be useful for transient expression of transgenes or genome editing components (e.g., CRISPR/Cas systems), delivery of fluorescent or luminescent markers for imaging, plant protection via intracellular delivery of pest- or pathogen-responsive molecules, induction of stress responses or metabolic reprogramming, and delivery of regulatory RNAs for post-transcriptional gene silencing.

### Other Cell Types

In some embodiments, CPPs are used for delivery into fungal cells. Fungal cell walls are composed primarily of chitin and β-glucans, posing a barrier to intracellular delivery. However, certain CPPs, particularly when used in conjunction with permeabilizing agents or nanoparticle formulations, have demonstrated efficacy in fungi such as Saccharomyces cerevisiae (yeast), Candida albicans, and Aspergillus spp. Transport of cargo into fungi cells via CPPs can be useful for antifungal testing, metabolic engineering, or delivery of gene editing systems.

Although less common due to the highly charged and protective bacterial cell envelope, CPPs have been used in some contexts to deliver nucleotides, peptides or small molecules into bacteria. In some embodiments, the CPPs are employed in Gram-negative or Gram-positive bacteria, including Escherichia coli, Bacillus subtilis, and Pseudomonas aeruginosa. Applications include delivery of antimicrobial peptides, biosensor components, or regulatory RNAs.

In some embodiments, CPPs are used for intracellular delivery in protozoan parasites such as Trypanosoma brucei, Leishmania donovani, Plasmodium falciparum (malaria), and Toxoplasma gondii. These applications may be relevant for antiparasitic drug delivery, genetic manipulation, or intracellular labelling for imaging and screening.

CPPs may also be applied to unicellular or multicellular algae, such as Chlamydomonas reinhardtii or Spirulina spp., for delivery of cargo. These systems are of interest in biofuel research, synthetic biology, and environmental monitoring.

In some embodiments, CPPs are used in insect cell lines (e.g., Spodoptera frugiperda Sf9/Sf21, Drosophila melanogaster S2 cells) or directly in live insects. Applications include delivery of transgenes, insecticidal agents, or RNA interference (RNAi) components. Such approaches may be useful in pest control, vector management, and transgenic insect studies.

CPPs have been used in aquatic organisms such as zebrafish (Danio rerio), frog embryos (Xenopus laevis), and marine invertebrates for delivery of biomolecules during development or toxicology studies. In some embodiments, CPPs are applied to embryos, larvae, or cell cultures derived from aquatic species for delivery of fluorescent tracers, CRISPR-Cas components, or small molecule modulators.

In some embodiments, CPPs may be adapted for use in archaeal cells, which possess unique membrane lipids and environmental tolerances. Though limited data exist, CPPs may be modified for delivery into extremophiles, for example thermophiles, halophiles, methanogens or psychrophiles, for bioengineering or synthetic biology.

### Preparing Cell Penetrating Peptides of the Invention

The invention also provides nucleic acids encoding the CPPs of the invention, vectors comprising the nucleic acids of the invention, and host cells comprising the vectors of the invention.

Numerous expression systems can be used, including without limitation chromosomes, episomes, and derived viruses. More particularly, the vector can be a recombinant vector. The recombinant vectors used can be derived from bacterial plasmids, transposons, yeast episomes, insertion elements, yeast chromosome elements, viruses such as baculovirus, papilloma viruses such as SV40, vaccinia viruses, adenoviruses, fox pox viruses, pseudorabies viruses, retroviruses. These recombinant vectors can equally be cosmid or phagemid derivatives. The nucleotide sequence can be inserted in the recombinant expression vector by methods well known to a person skilled in the art.

The recombinant vector can include nucleotide sequences that control the regulation of the polynucleotide expression as well as nucleotide sequences permitting the expression and the transcription of a nucleic acid of the invention and the translation of a CPP of the invention, these sequences being selected according to the host cells that are used. Thus, for example, an appropriate secretion signal can be integrated in the recombinant vector so that the CPP, encoded by the nucleic acid of the invention, will be directed towards the lumen of the endoplasmic reticulum, towards the periplasmic space, on the membrane or towards the extracellular environment. The choice of an appropriate secretion signal may facilitate subsequent protein purification.

The introduction of the recombinant vector in a host cell can be carried out according to methods that are well known to a person skilled in the art, such as transfection by calcium phosphate, transfection by DEAE dextran, transfection, microinjection, transfection by cationic lipids, electroporation, transduction or infection. The host cell can be, for example, bacterial cells such as E. coli, cells of fungi such as yeast cells and cells of Aspergillus, Streptomyces, insect cells, Chinese Hamster Ovary cells (CHO), C127 mouse cell line, BHK cell line of Syrian hamster cells, or Human Embryonic Kidney 293 (HEK 293) cells.

It is a further aspect of the invention to provide a method for preparing a CPP of the invention, comprising cultivating a host cell as mentioned above in a culture medium and separating the CPP from the culture medium or from the host cell lysate after host cell lysis.

In another embodiment, the CPP can be prepared by synthetic chemistry methods, such as solid-phase peptide synthesis. Purification of those peptides may be carried out by means of any technique known in the art for protein/peptide purification. Exemplary techniques include ion-exchange chromatography, hydrophobic interaction chromatography, and immunoaffinity methods.

### Kits

The invention also provides a kit comprising the CPP of the invention and cargo, wherein the CPP and the cargo are for simultaneous, sequential or separate administration. The kit may additionally comprise cells for transduction. The kit may also comprise instructions for use. The kit may include the various components packaged in one or more containers, e.g. 1, 2, 3, 4, 5, 6 or more separate containers. For example, the kit may comprise a container comprising a salt solution, a container comprising the CPP, a container comprising the cargo, and/or a container comprising a diluent or media. In addition the kit may comprise any one or more of the additional other components as described herein, wherein they are suitable for simultaneous, sequential or separate administration with the CPP.

The above components may be provided in a lyophilised or dry form or dissolved in a suitable buffer. The kit may also comprise additional reagents including, for instance, preservatives, growth media, and/or buffers for storage and/or reconstitution of the above-referenced components, washing solutions, and the like.

### EXAMPLES

### Example 1: Identification of CPPs Derived from the GHES Protein Loop Domain Sequence

### Cell Culture

Jurkat (Clone E6-1 TIB-152) cells were cultured at 1×10⁵ cells/mL in RPMI medium 1640 containing L-glutamine and sodium bicarbonate **(R8758 Sigma-Aldrich)** supplemented with 10% FBS **(F7524 Sigma-Aldrich),** 1% Penicillin-Streptomycin **(15140-122 Gibco),** 1% MEM Non-essential amino acids **(11140050 Gibco)** and 1% Sodium Pyruvate **(11360070 Gibco).** Cells were cultured at 37°C with 5% CO₂ and tested for mycoplasma using the MycoAlert Mycoplasma detection kit **(LT07-418 Lonza)** according to the manufacturer's instructions. Prior to the transduction procedure, Jurkat cells were seeded to 50.000 cells per well in a V-bottom 96-well plate **(651201 Greiner BIO-ONE)** in a final volume of 50 µL of RPMI medium per well without supplements and then kept in an incubator at 37°C with 5% CO₂ while preparing the transduction formulation.

### Peptides, qRNAs, Primers and Recombinant Cas Protein Preparation

All peptides were purchased and custom synthetised at Genscript with purity greater than 98%. They were dissolved to a working concentration of 2mM in nuclease-free water **(Invitrogen, AM9937)** containing 10% DMSO **(D8418 Sigma-Aldrich).** The peptide sequences are specified in **Table 7.**

crRNA and tracrRNA were purchased from Integrated DNA Technologies as lyophilized Alt-R^{®} CRISPR/Cas9 products which were then resuspended in nuclease-free water **(Invitrogen, AM9937).** crRNA and tracrRNA were mixed at equimolar concentrations to a final gRNA concentration of 100µM, heated at 95°C for 10 min, slowly cooled down to room temperature for 30 min and stored at -20°C. Guide RNAs were designed by using CHOPCHOP version 3 (Labun et al., 2019). Guide RNA sequence is specified in **Table 8.**

His-tagged recombinant Cas9 protein was produced as described in by D'Astolfo et.,al, Cell, 2015, 161(3), 674-690, (see recombinant protein purification section of "Examples: Additional Information", for a detailed description),. Recombinant Cas9 protein was stored in buffer containing 250 mM GABA, 75 mM Glycine, 25 mM phosphate buffer pH 8.0, 500 mM NaCl, 1.25 mM MgCl2, 150 mM glycerol and 2 mM 2-ME at a final working concentration of 75 µM.

### Formulation and Transduction Procedure

Recombinant Cas9 protein and dual annealed gRNA targeting human B2M (hB2M #7 in **Table 8)** were mixed to a final concentration of 5 µM each. The resulting ribonucleoprotein (RNP) was then incubated for 10 mins at room temperature. All tested peptides (TAT-HA2, GHES-P1-P2-P3, GHES-P2-P3, GHES-P2-P3-P4, and GHES-P1-P2-P3-P4 as shown in **Table 7)** were added to the RNP mixture to final concentrations of 0, 25, 50, 75, 100, 125 and 150 µM. Dulbecco's phosphate-buffered saline **(14190-144 Gibco)** was then added to each formulation to reach a transduction mixture volume of 15 µL. Cas9, gRNA and peptide concentrations were calculated in a final reaction volume of 65 µL which accounts for the transduction mixture volume (15 µL) and 50 µL of cell culture per well. The transduction mixture was incubated for an additional 10 min at room temperature, added to cells in V-bottom 96-well plate **(651201 Greiner BIO-ONE)** and mixed by pipetting up and down. The plates were incubated at 37°C with 5% CO₂ for 45 mins. The reaction was stopped by adding 135 µL of medium without supplements, plates were centrifuged at 400xg for 3 minutes and supernatant was discarded. Cells were then resuspended in 200 µL of medium with supplements. Plate was incubated for 3 days at 37°C with 5% CO₂ until using fluorescence activated cell sorting (FACS) for analysis.

### Flow Cytometry

To assess transduction efficiencies and genome editing by flow cytometry, cells were stained with antibody staining solution; 1% FITC anti-human HLA-A, B, C clone W6/32 **(311404 Biolegend)** in FACS buffer (5% FBS in PBS) and SYTOX red staining solution; 0.25% SYTOX red **(S34859 Invitrogen)** in FACS buffer. Plates containing transduced Jurkat cells were centrifuged for 3 minutes at 400 xg, supernatant was discarded, and cells were resuspended in 50 µL of staining solution, except for controls unstained and SYTOX red staining only controls. Samples were incubated for 10 mins in the dark at 4°C. Cells were then centrifuged for 3 minutes at 400 xg, supernatant was discarded, cells were washed with 150 µL of FACS buffer and centrifuged again for 3 minutes at 400 xg. Subsequently, cells were resuspended in 150 µL SYTOX red staining solution, except for unstained and only FACS buffer controls. Samples were incubated in the dark on ice for 15 mins before analysis in the flow cytometer **(Guava easyCyte).** Data analysis was performed using Guava easyCyte software according to the manufacturer's instructions.

### Results

Figure 3 shows the MHCI knock-out percentage efficiency in Jurkat cells following transduction with the tested peptides. Peptides containing the P2-P3 region sequence showed increasing editing efficiencies with increasing concentration of peptide up to a maximum knock-out percentage of 50% (Figure 3b to e). All P2-P3 region containing peptides led to higher efficiencies when compared to the TAT-HA2 peptide control. The addition of the P4 region sequence to the P2-P3 region sequence increased editing efficiency at a lower peptide concentration of 50 µM (Figure 3d). Conversely, the addition of the P1 region sequence to the P2-P3 region sequence did not increase editing efficiencies significantly (Figure 3b). The addition of both the P1 region and the P4 region sequences to the P2-P3 region sequence increased efficiencies to 50% at the lowest concentration tested of 25 µM (Figure 3e). Thus, Figure 3 shows how the tested peptides can effectively function as CPPs.

### Example 2: Effect of Shortening GHES P1 and P4 Regions on Transduction Efficacy

### Cell Culture

CD8+ T-cells were cultured at 0.5-1×10⁶ cells/mL in IMDM with GlutaMAX **(31980022 Gibco)** supplemented with 5% human serum **(H3667 Sigma-Aldrich),** 5% FBS **(F7524 Sigma-Aldrich),** 1% Penicillin-Streptomycin **(15140-122 Gibco),** 1.5% L-glutamine **(15410314 Gibco)** and 100 IU/mL recombinant human IL-2 **(202-IL-050 R&D systems).** Cells were stimulated when thawing and after 2 weeks of culturing using 10µL/1×10⁶ cells or 2µL/1×10⁶ cells of T Cell TransAct, Human **(130-111-160 Miltenyi Biotech),** respectively. 8 days after thawing and prior to the transduction procedure, CD8+ cells were seeded to 2.5×10⁴ cells per well in a V-bottom 96-well plate **(651201 Greiner BIO-ONE)** in a final volume of 50 µL of RPMI medium per well without supplements and kept in an incubator at 37°C with 5% CO₂ while preparing the transduction formulation.

### Peptides, qRNAs, Primers and Recombinant Cas Protein Preparation

All peptides were purchased and custom synthetised at Genscript with purity greater than 98%. They were dissolved to a working concentration of 2mM in nuclease-free water **(Invitrogen, AM9937)** containing 10% DMSO **(D8418 Sigma-Aldrich).** The peptide sequences are specified in **tables in** **Figure 4a** **and** **4b****.**

crRNAs and tracrRNAs were purchased from Integrated DNA Technologies as lyophilized Alt-R^{®} CRISPR/Cas9 products which were then resuspended in nuclease-free water **(Invitrogen, AM9937).** crRNA and tracrRNA were mixed at equimolar concentrations to a final gRNA concentration of 100µM, heated at 95°C for 10 min, slowly cooled down to room temperature for 30 min and stored at -20°C. Guide RNAs were designed by using CHOPCHOP version 3 (Labun et al., 2019). Guide RNA sequence is specified in **Table 8.**

All forward, reverse and sequencing primers were purchased from Integrated DNA Technologies as desalted custom synthesis and were then resuspended in nuclease-free water **(Invitrogen, AM9937)** to a final working concentration of 100 µM. The primer sequences are specified in **Table 9.**

His-tagged recombinant Cas9 protein was produced as described in by D'Astolfo et.,al, Cell, 2015, 161(3), 674-690, (see recombinant protein purification section of "Examples: Additional Information", for a detailed description). Recombinant Cas9 protein was stored in buffer containing 250 mM GABA, 75 mM Glycine, 25 mM phosphate buffer pH 8.0, 500 mM NaCl, 1.25 mM MgCl2, 150 mM glycerol and 2 mM 2-ME at a final working concentration of 75 µM.

### Formulation and Transduction Procedure

Recombinant Cas9 protein and dual annealed gRNA targeting human B2M (hB2M #7 in **Table 8)** were mixed to a final concentration of 5 µM each. The resulting ribonucleoprotein (RNP) was then incubated for 10 mins at room temperature. All tested peptides (see **tables in** **Figure 4a** **and** **4b** for sequences) were added to the RNP mixture to final concentrations of 40 µM. Dulbecco's phosphate-buffered saline **(14190-144 Gibco)** was then added to each formulation to reach a transduction mixture volume of 15 µL. Cas9, gRNA and peptide concentrations were calculated in a final reaction volume of 65 µL which accounts for the transduction mixture volume (15 µL) and 50 µL of cell culture per well. The transduction mixture was incubated for an additional 10 min at room temperature, added to cells in V-bottom 96-well plate **(651201 Greiner BIO-ONE)** and mixed by pipetting up and down. The plates were incubated at 37°C with 5% CO₂ for 45 mins. The reaction was stopped by adding 135 µL of medium without supplements, plates were centrifuged at 400xg for 3 minutes and supernatant was discarded. Cells were then resuspended in 200 µL of medium with supplements and incubated at 37°C with 5% CO₂ until viability assay and editing analysis was performed.

### Viability Assay

Viability assay was assessed 24 hrs after the transduction procedure. 25 µL of transduced cells were analysed using the CellTiter-Glo^{®} 2.0 Cell Viability Assay **(G9242 Promega)** in a GloMax Discover microplate reader **(Promega)** according to the manufacturer's instructions.

### DNA Extraction and PCR

3 days after transduction, genomic DNA was extracted using the QuickExtract DNA Solution **(QE09050 Lucigen).** Plates were centrifuged for 3 minutes at 400 xg, medium was discarded, and pellet resuspended in 50 µL QuickExtract per well. The plate was then incubated according to the manufacturer's instructions. Q5^{®} High-Fidelity 2X Master Mix **(M0515 NEB)** was used to amplify the edited region in the B2M gene using primers 258_HUM_B2M-5_Fw and 261_HUM_B2M-10_Rv (see **Table 9).** The PCR products were purified using QIAquick 96 PCR Purification Kit **(28181 QIAGEN)** following manufacturer's protocol.

### Editing Analysis

Purified DNA fragments (15 ng/100 bp) were mixed with 25 pmol of sequencing primer 22_HUM-B2M-PCR-Fw (see **Table 9)** and were sent for sanger sequencing **(BaseClear).** Insertion-deletion (Indel) frequency results were evaluated by TIDE version 3.3.0 (Brinkman et al., 2014) with an alignment window of 200 bp, decomposition window of 270-580 bp, indel size range of 15 bp and P-value threshold of 0.001.

### Results

Figure 4 shows the efficacy of shorter peptide variants derived from the sequence of the full-length GHES protein loop domain. The transduction efficacy on CD8+ T-cells was analysed by indel frequency percentage when targeting the human B2M gene and also its effect on cell viability.

Figure 4a tests peptides containing the P2-P3-P4 region sequence and a portion of the P1 region sequence (peptides 1-11), and peptides containing the P2-P3 region sequence and a portion of the P4 region sequence (peptides 12-20). Figure 4a shows that higher editing efficiencies were achieved for these peptide variants, up to 91.7% for peptides containing the P2-P3-P4 region sequence (peptides 1-11), and up to 86.8% for peptides containing the P2-P3 region sequence (peptides 12-20).

In Figure 4b, the peptide containing the P2-P3 region sequence and the first amino acid from the P4 region sequence (peptide 19 from Figure 4a), was further extended on the N-terminal to include a portion of the P1 region sequence (peptides 21-32). All tested peptides were effective and functional as CPPs, though addition of either the entire length of or a portion of the P1 region sequence to peptide 19 resulted in lower editing efficiencies than those of Figure 4a, i.e., comparable to that of peptide 20 at around 67.6%, which contains only the P2-P3 region sequence.

Thus, Figure 4 shows that shorter peptide variants derived from the sequence of the full-length GHES protein loop domain all function effectively as CPPs, and that variation in length of the P1 and P4 region sequences can allow for fine tuning of CPP efficacy.

### Example 3: Transduction Efficiency of Further CPP Variants

### Cell Culture

CD8+ T-cells were cultured at 0.5-1×10⁶ cells/mL in IMDM with GlutaMAX **(31980022 Gibco)** supplemented with 5% human serum **(H3667 Sigma-Aldrich),** 5% FBS **(F7524 Sigma-Aldrich),** 1% Penicillin-Streptomycin **(15140-122 Gibco),** 1.5% L-glutamine **(15410314 Gibco)** and 100 IU/mL recombinant human IL-2 **(202-IL-050 R&D systems).** Cells were stimulated when thawing and after 2 weeks of culturing using 10µL/1×10⁶ cells or 2µL/1×10⁶ cells of T Cell TransAct, Human **(130-111-160 Miltenyi Biotech),** respectively. 8 days after thawing and prior to the transduction procedure, CD8+ cells were seeded to 2.5×10⁴ cells per well in a V-bottom 96-well plate **(651201 Greiner BIO-ONE)** in a final volume of 50 µL of RPMI medium per well without supplements and kept in an incubator at 37°C with 5% CO₂ while preparing the transduction formulation.

### Peptides, qRNAs, Primers and Recombinant Cas Protein Preparation

All peptides were purchased and custom synthetised at Genscript with purity greater than 98%. They were dissolved to a working concentration of 2mM in nuclease-free water **(Invitrogen, AM9937)** containing 10% DMSO **(D8418 Sigma-Aldrich).** The peptide sequences are specified in **Table 7.**

crRNAs and tracrRNAs were purchased from Integrated DNA Technologies as lyophilized Alt-R^{®} CRISPR/Cas9 products which were then resuspended in nuclease-free water **(Invitrogen, AM9937).** crRNA and tracrRNA were mixed at equimolar concentrations to a final gRNA concentration of 100µM, heated at 95°C for 10 min, slowly cooled down to room temperature for 30 min and stored at -20°C. Guide RNAs were designed by using CHOPCHOP version 3 (Labun et al., 2019). Guide RNA sequence is specified in **Table 8.**

All forward, reverse and sequencing primers were purchased from Integrated DNA Technologies as desalted custom synthesis and were then resuspended in nuclease-free water **(Invitrogen, AM9937)** to a final working concentration of 100 µM. The primer sequences are specified in **Table 9.**

His-tagged recombinant Cas9 protein was produced as described in by D'Astolfo et.,al, Cell, 2015, 161(3), 674-690, (see recombinant protein purification section of "Examples: Additional Information", for a detailed description). Recombinant Cas9 protein was stored in buffer containing 250 mM GABA, 75 mM Glycine, 25 mM phosphate buffer pH 8.0, 500 mM NaCl, 1.25 mM MgCl2, 150 mM glycerol and 2 mM 2-ME at a final working concentration of 75 µM.

### Formulation and Transduction Procedure

Recombinant Cas9 protein and dual annealed gRNA targeting human B2M (hB2M #7 in **Table 8)** were mixed to a final concentration of 5 µM each. The resulting ribonucleoprotein (RNP) was then incubated for 10 mins at room temperature. The following peptides were tested: TAT-HA2, GHES-P2-P3, GHES-P2-P3-P4, GHES-(P2-P3) + P4-8 (19) (peptide 19 from Figure 4), GHES-disulfide cyclic C1-C30 (19), GHES-disulfide cyclic C1-C29 (19), RI GHES-P2-P3-P4, GHES-P2-P3-P4 inverted, G-TAT-HA2-A, GHFF-P2-P3-A, GHFF-P2-P3-P4, GHLT-P2-P3-A, GHLT-P2-P3-P4, and GHFF-P2/GHLT-P3/GHES-P4 (see **Table 7** for sequences). All peptides were added to their respective RNP mixtures to a final concentration of 40 µM. Dulbecco's phosphate-buffered saline **(14190-144 Gibco)** was then added to each formulation to reach a transduction mixture volume of 15 µL. Cas9, gRNA and peptide concentrations were calculated in a final reaction volume of 65 µL which accounts for the transduction mixture volume (15 µL) and 50 µL of cell culture per well. The transduction mixture was incubated for an additional 10 min at room temperature, added to cells in V-bottom 96-well plate **(651201 Greiner BIO-ONE)** and mixed by pipetting up and down. The plates were incubated at 37°C with 5% CO₂ for 45 mins. The reaction was stopped by adding 135 µL of medium without supplements, plates were centrifuged at 400xg for 3 minutes and supernatant was discarded. Cells were then resuspended in 200 µL of medium with supplements and incubated at 37°C with 5% CO₂ until editing analysis was performed.

### DNA Extraction and PCR

3 days after transduction, genomic DNA was extracted using the QuickExtract DNA Solution **(QE09050 Lucigen).** Plates were centrifuged for 3 minutes at 400 xg, medium was discarded, and pellet resuspended in 50 µL QuickExtract per well. The plate was then incubated according to the manufacturer's instructions. Q5^{®} High-Fidelity 2X Master Mix **(M0515 NEB)** was used to amplify the edited region in the B2M gene using primers 258_HUM_B2M-5_Fw and 261_HUM_B2M-10_Rv (see **Table 9).** The PCR products were purified using QIAquick 96 PCR Purification Kit **(28181 QIAGEN)** following manufacturer's protocol.

### Editing Analysis

Purified DNA fragments (15 ng/100 bp) were mixed with 25 pmol of sequencing primer 22_HUM-B2M-PCR-Fw (see **Table 9)** and were sent for sanger sequencing **(BaseClear).** Insertion-deletion (Indel) frequency results were evaluated by TIDE version 3.3.0 (Brinkman et al., 2014) with an alignment window of 200 bp, decomposition window of 270-580 bp, indel size range of 15 bp and P-value threshold of 0.001.

### Results

Figure 5 shows editing efficiency for the tested peptides.

The addition of a glycine (G) and alanine (A) to the N-terminus and C-terminus of the TAT-HA2 peptide resulted in improved editing efficiencies from 42 to 63%.

The GHES-(P2-P3) + P4-8 (19) peptide and its cyclic versions (GHES-disulfide cyclic C1-30 (19) and GHES-disulfide cyclic C1-C29 (19)) were all efficient as CPPs and led to indel frequencies ranging from 50 to 69%.

GHES-P2-P3-P4 inverted and retro-inverted peptides showed editing efficiencies of 57 and 67%, slightly lower than the naturally occurring GHES-P2-P3-P4 peptide.

Peptides derived from the protein loop domain sequences of other fungi (GHFF is from *Fomes fomentarius* and GHLT is from *Lentinus tigrinus)* were also shown to be effective CPPs. Peptides containing either P2-P3-A or P2-P3-P4 sequences from GHFF and GHLT achieved a maximum editing efficiency of 86.5%. Additionally, the results from this study demonstrate that regions from different glycoside hydrolases may be combined in a single CPP. For example, peptide GHFF-P2/GHLT-P3/GHES-P4 combines regions from *Fomes fomentarius, Lentinus tigrinus,* and *Earliella scabrosa,* and shows effective transduction.

In this experiment a final peptide concentration of 40 µM was used, however, more effective peptides may require lower concentrations to improve editing efficiencies while reducing peptide toxicity.

### Example 4: Further Experimentation when Targeting Select Genes

### Cell Culture

CD8+ T-cells were cultured at 0.5-1×10⁶ cells/mL in IMDM with GlutaMAX **(31980022 Gibco)** supplemented with 5% human serum **(H3667 Sigma-Aldrich),** 5% FBS **(F7524 Sigma-Aldrich),** 1% Penicillin-Streptomycin **(15140-122 Gibco),** 1.5% L-glutamine **(15410314 Gibco)** and 100 IU/mL recombinant human IL-2 **(202-IL-050 R&D systems).** Cells were stimulated when thawing and after 2 weeks of culturing using 10µL/1×10⁶ cells or 2µL/1×10⁶ cells of T Cell TransAct, Human **(130-111-160 Miltenyi Biotech),** respectively. 8 days after thawing and prior to the transduction procedure, CD8+ cells were seeded to 2.5×10⁴ cells per well in a V-bottom 96-well plate **(651201 Greiner BIO-ONE)** in a final volume of 50 µL of RPMI medium per well without supplements and kept in an incubator at 37°C with 5% CO₂ while preparing the transduction formulation.

### Peptides, qRNAs, Primers and Recombinant Cas Protein Preparation

All peptides were purchased and custom synthetised at Genscript with purity greater than 98%. They were dissolved to a working concentration of 2mM in nuclease-free water **(Invitrogen, AM9937)** containing 10% DMSO **(D8418 Sigma-Aldrich).** The peptide sequences are specified in **Table 7.**

All crRNAs and tracrRNAs were purchased from Integrated DNA Technologies as lyophilized Alt-R^{®} CRISPR/Cas9 products which were then resuspended in nuclease-free water **(Invitrogen, AM9937).** crRNA and tracrRNA were mixed at equimolar concentrations to a final gRNA concentration of 100µM, heated at 95°C for 10 min, slowly cooled down to room temperature for 30 min and stored at -20°C. Guide RNAs were designed by using CHOPCHOP version 3 (Labun et al., 2019), Cas-Designer (Zetsche et al., 2015), BE-Designer (Hwang et al., 2018) and PE-Designer (Hwang et al., 2021). Guide RNA sequences are specified in **Table 8.**

All forward, reverse and sequencing primers were purchased from Integrated DNA Technologies as desalted custom synthesis and were then resuspended in nuclease-free water **(Invitrogen, AM9937)** to a final working concentration of 100 µM. The primer sequences are specified in **Table 9.**

His-tagged recombinant proteins were produced as described in by D'Astolfo et.,al, Cell, 2015, 161(3), 674-690, (see recombinant protein purification section of "Examples: Additional Information", for a detailed description). Recombinant proteins were stored in buffer containing 250 mM GABA, 75 mM Glycine, 25 mM phosphate buffer pH 8.0, 500 mM NaCl, 1.25 mM MgCl2, 150 mM glycerol and 2 mM 2-ME at a final working concentration of 75 µM.

### Formulation and Transduction Procedure

In Figure 6a, recombinant Cas9 protein and dual annealed gRNA targeting either human B2M, CTLA-4, or PD-1 (hB2M #7, Hs.Cas9.CTLA4.1.AA and Hs.Cas9.PDCD1.1.AN see **Table 8)** were mixed to a final concentration of 3.4 µM each, as single, dual or triple guides per targeted gene. The resulting ribonucleoprotein (RNP) was then incubated for 10 mins at room temperature and the peptide GHES-(P2-P3) + P4-8 (19) (see **Table 7** for sequences) was added to a final concentration of 40 µM. Dulbecco's phosphate-buffered saline **(14190-144 Gibco)** was then added to each formulation to reach a transduction mixture volume of 15 µL. Cas9, gRNA and peptide concentrations were calculated in a final reaction volume of 65 µL which accounts for the transduction mixture volume (15 µL) and 50 µL of cell culture per well. The transduction mixture was incubated for an additional 10 min at room temperature.

In Figure 6b, recombinant base editor protein (ABE) and dual annealed gRNA targeting either human B2M, CTLA-4, PD-1 or CD33 (hB2M gRNA 1 ABE, hB2M gRNA 2 ABE, CTLA-4 gRNA 1 ABE, CTLA-4 gRNA 2 ABE, PD-1 gRNA 1 ABE, PD-1 gRNA 2 ABE, hCD33_gRNA_ABE2, see **Table 8)** were mixed to a final concentration of 2 µM each. The resulting ribonucleoproteins (RNP) were then incubated for 10 mins at room temperature and the peptide GHES-P2-P3-P4 (see **Table 7)** was added to a final concentration of 50 µM. Dulbecco's phosphate-buffered saline **(14190-144 Gibco)** was then added to each formulation to reach a transduction mixture volume of 15 µL. Cas9, gRNA and peptide concentrations were calculated in a final reaction volume of 65 µL which accounts for the transduction mixture volume (15 µL) and 50 µL of cell culture per well. The transduction mixture was incubated for an additional 10 min at room temperature.

In Figure 6c, either recombinant Cas12a or prime editor proteins and single gRNA targeting human B2M (hB2M pegRNA 1, hB2M pegRNA 2, hB2M pegRNA 3, hB2M gRNA 1 Cas12a, hB2M gRNA 2 Cas12a, hB2M gRNA 3 Cas12a, hB2M gRNA 4 Cas12a, see **Table 8)** were mixed to a final concentration of 4.5 µM each. The resulting ribonucleoproteins (RNP) were then incubated for 10 mins at room temperature and the peptide GHES-P2-P3-P4 (see **Table 7)** was added to a final concentration of 40 µM. Dulbecco's phosphate-buffered saline **(14190-144 Gibco)** was then added to each formulation to reach a transduction mixture volume of 15 µL. Cas9, gRNA and peptide concentrations were calculated in a final reaction volume of 65 µL which accounts for the transduction mixture volume (15 µL) and 50 µL of cell culture per well. The transduction mixture was incubated for an additional 10 min at room temperature.

The 15 µL of all transduction mixtures were added to cells in V-bottom 96-well plate **(651201 Greiner BIO-ONE)** and mixed by pipetting up and down. The plates were incubated at 37°C with 5% CO₂ for 45 mins. The reaction was stopped by adding 135 µL of medium without supplements, plates were centrifuged at 400xg for 3 minutes and supernatant was discarded. Cells were then resuspended in 200 µL of medium with supplements and incubated at 37°C with 5% CO₂ until editing analysis was performed.

### DNA Extraction and PCR

3 days after transduction, genomic DNA was extracted using the QuickExtract DNA Solution **(QE09050 Lucigen).** Plates were centrifuged for 3 minutes at 400 xg, medium was discarded, and pellet resuspended in 50 µL QuickExtract per well. The plate was then incubated according to the manufacturer's instructions. Q5^{®} High-Fidelity 2X Master Mix **(M0515 NEB)** was used to amplify the edited region in either the B2M, CTLA-4, PD-1 or CD33 genes using primers; 258_HUM_B2M-5_Fw, 261_HUM_B2M-10_Rv, hB2M Fw1 ABE, hB2M Rv1 ABE, hCTLA4_FW1 (AC), hCTLA4_RV1 (AC), CTLA-4 Fw 1 ABE, CTLA -4 Rv 1 ABE, hPD-1_FW2 (AC), hPD-1_RV2 (AC), hCD33_Fw1.3, hCD33_Rv1.3 (see **Table 9).** The PCR products were purified using QIAquick 96 PCR Purification Kit **(28181 QIAGEN)** following manufacturer's protocol.

### Editing Analysis

Purified DNA fragments (15 ng/100 bp) were mixed with 25 pmol of sequencing primer either for B2M (22_HUM-B2M-PCR-Fw or hB2M Fw3 ABE, see **Table 9),** CTLA-4 (hCTLA4_FW3 (AC) or CTLA-4 Fw2 ABE, see **Table 9),** PD-1 (hPD-1_RV3 (AC), see **Table 9)** or CD33 (hCD33_Fw1.3, see **Table 9),** and were sent for sanger sequencing (BaseClear). Insertion-deletion (Indel) frequency results were evaluated by TIDE version 3.3.0 (Brinkman et al., 2014) with an alignment window of 150-200 bp, decomposition window of 200-580 bp, indel size range of 10-15 bp and P-value threshold of 0.001. Base editing was analysed using EditR version 1.0.10.

### Results

Figure 6a shows indel frequencies on CD8+ T-cells when targeting the genes B2M, CTLA-4, and PD-1. For this experiment, the peptide GHES-P2-P3 + P4-8 (19) (see **Table 7)** was used for the transduction of single, dual or triple guide RNAs per target gene. The human B2M gene reached the highest indel frequency of 63.8% when using a single guide RNA targeting the B2M gene. When combining 2 guides for B2M and PD-1 or B2M and CTLA-4, editing efficiency decreased to 59.4% or 52.2% respectively. The lowest editing efficiency was observed when combining all 3 guides for B2M, CTLA-4 and PD-1, achieving a 41.7% indel frequency. Indel frequencies in the CTLA-4 gene showed a different pattern, where the combination of guides for B2M and CTLA-4 reached the highest editing efficiency of 43.6% compared to the single guide CTLA-4, with 38% indel frequency. Combining guides for CTLA-4 with PD-1 or all 3 guides resulted in lower editing efficiencies as observed in the other targeted genes. On the other hand, the PD-1 gene showed no significant differences in indel frequency when comparing results from single or dual guides targeting PD-1 (47.9%) or PD-1 and B2M (48.4%), respectively.

Figure 6b shows base editing efficiencies when targeting the start codon and/or splice site in genes B2M, CTLA-4, PD-1 and CD33. A to G base editing was successfully achieved in splice sites when using guide RNAs B2M 2, CTLA-4 2, PD-1 2 and CD33 1 (hB2M gRNA 2 ABE, CTLA-4 gRNA 2 ABE, PD-1 gRNA 2 ABE and hCD33_gRNA_ABE2, see **Table 8),** achieving base editing frequencies of 36.7%, 17.3% and 23.7%, respectively. Base editing targeting the start codon was only achieved when targeting the PD-1 gene with guide PD-1 1 (PD-1 gRNA 1 ABE see **Table 8),** resulting in a 26% base editing frequency.

Figure 6c shows that the GHES-P2-P3-P4 peptide was effective at delivering a Prime editor and Cas12a to cells, as demonstrated by the editing efficiencies achieved when using the following guide RNAs: hB2M pegRNA3, hB2M gRNA2 Cas12a, hB2M gRNA3 Cas12a, and hB2M gRNA4 Cas12a.

Collectively, Figures 6a-6c show that the tested CPPs are effective in multiplex editing and across various editing systems, including base editing, Prime editing and Cas12 editing systems. Efficient delivery of RNPs via the CPPs of the invention resulted in different editing efficiencies which is determined by the efficacy of the chosen guide RNA.

### Example 5: Transduction Efficacy of CPPs Derived from GHES in Different Cell Types

### Cell Culture

Jurkat (Clone E6-1 TIB-152) cells were cultured at 1×10⁵ cells/mL in RPMI medium 1640 containing L-glutamine and sodium bicarbonate **(R8758 Sigma-Aldrich)** supplemented with 10% FBS **(F7524 Sigma-Aldrich),** 1% Penicillin-Streptomycin **(15140-122 Gibco),** 1% MEM Non-essential amino acids **(11140050 Gibco)** and 1% Sodium Pyruvate **(11360070 Gibco).** Prior to the transduction procedure, Jurkat cells were seeded to 50.000 cells per well in a V-bottom 96-well plate **(651201 Greiner BIO-ONE)** in a final volume of 50 µL of RPMI medium per well without supplements and kept in an incubator at 37°C with 5% CO₂ while preparing the transduction formulation.

CD8+ and CD4+ T-cells were cultured at 0.5-1×10⁶ cells/mL in IMDM with GlutaMAX **(31980022 Gibco)** supplemented with 5% human serum **(H3667 Sigma-Aldrich),** 5% FBS **(F7524 Sigma-Aldrich),** 1% Penicillin-Streptomycin **(15140-122 Gibco),** 1.5% L-glutamine **(15410314 Gibco),** and 100 IU/mL recombinant human IL-2 **(202-IL-050 R&D systems).** Cells were stimulated when thawing and after 2 weeks of culturing using 10µL/1×10⁶ cells or 2µL/1×10⁶ cells of T Cell TransAct **(130-111-160** Miltenyi Biotech), respectively. 8 days after thawing and prior to the transduction procedure, CD8+ and CD4+ cells were seeded to 2.5×10⁴ cells per well in a V-bottom 96-well plate **(651201 Greiner BIO-ONE)** in a final volume of 50 µL of IMDM medium per well without supplements and kept in an incubator at 37°C with 5% CO₂ while preparing the transduction formulation.

NK cells were cultured at 1×10⁶ cells/mL in NK MACS medium **(130-114-429 Miltenyi)** supplemented with 5 % human serum **(H3667 Sigma-Aldrich)** and 500 IU/mL recombinant human IL-2 **(202-IL-050 R&D systems).** Prior to the transduction procedure, NK cells were seeded to 2.5×10⁴ cells per well in a V-bottom 96-well plate **(651201 Greiner BIO-ONE)** in a final volume of 50 µL of NK MACS medium per well without supplements and kept in an incubator at 37°C with 5% CO₂ while preparing the transduction formulation.

CD34+ hematopoietic stem cells from cord blood and bone marrow were cultured in StemSpan^{™} SFEM II medium **(09605 STEMCELL Technologies)** supplemented with 10% StemSpan^{™} CD34+ Expansion Supplement (10X) **(02691 STEMCELL technologies).** CD34+ cells were seeded to 1x10⁴ cells in 6-well plate **(657185 Greiner BIO-ONE)** with 2 mL total volume per well and keep in culture until transduction. Prior to the transduction procedure, CD34+ cells were seeded to 2.5x10⁴ cells per well in a V-bottom 96-well plate **(651201 Greiner BIO-ONE)** in a final volume of 50 µL of StemSpan^{™} SFEM II medium per well without supplements and kept in an incubator at 37°C with 5% CO2 while preparing the transduction formulation.

hIPSCs cells were cultured in Essential 8^{™} medium **(15190617 Gibco)** supplemented with RevitaCell Supplement **(A2644501 Gibco).** Prior to the transduction procedure, cells were seeded using Geltrex **(A1413301 Gibco)** for adherence and culture was kept at 70-80% confluency.

All cells were cultured at 37°C with 5% CO₂ and tested for mycoplasma using the MycoAlert Mycoplasma detection kit **(LT07-418 Lonza)** according to the manufacturer's instructions.

### Peptides, qRNAs, Primers and Recombinant Cas Protein Preparation

All peptides were purchased and custom synthetised at Genscript with purity greater than 98%. They were dissolved to a working concentration of 2mM in nuclease-free water (**Invitrogen, AM9937**) containing 10% DMSO (**D8418 Sigma-Aldrich**). The peptide sequences are specified in **Table 7.**

All crRNAs and tracrRNAs were purchased from Integrated DNA Technologies as lyophilized Alt-R^{®} CRISPR/Cas9 products which were then resuspended in nuclease-free water (**Invitrogen, AM9937**)**.** crRNA and tracrRNA were mixed at equimolar concentrations to a final gRNA concentration of 100µM, heated at 95°C for 10 min, slowly cooled down to room temperature for 30 min and stored at -20°C. Guide RNAs were designed by using CHOPCHOP version 3. Guide RNA sequences are specified in **Table 8.**

All forward, reverse and sequencing primers were purchased from Integrated DNA Technologies as desalted custom synthesis and were then resuspended in nuclease-free water **(Invitrogen, AM9937)** to a final working concentration of 100 µM. The primer sequences are specified in **Table 9.**

His-tagged recombinant Cas9 protein was produced as described in by D'Astolfo et.,al, Cell, 2015, 161(3), 674-690, (see recombinant protein purification section of "Examples: Additional Information", for a detailed description). Recombinant Cas9 protein was stored in buffer containing 250 mM GABA, 75 mM Glycine, 25 mM phosphate buffer pH 8.0, 500 mM NaCl, 1.25 mM MgCl2, 150 mM glycerol and 2 mM 2-ME at a final working concentration of 75 µM.

### Formulation and Transduction Procedure

Recombinant Cas9 protein and dual annealed gRNA targeting human B2M (hB2M #7 in **Table 8**) were mixed to a final concentration of 5 µM each. The resulting ribonucleoprotein (RNP) was then incubated for 10 mins at room temperature. Peptide was added to RNP mixture to final concentration of 40 µM in experiments using Jurkat, CD8+, CD4+ and NK cells, 100 µM peptide when using hIPSCs cells, and 50 or 100 µM to experiments using CD34+ hSC. Peptides used were TAT-HA2, GHES-P2-P3, GHES-P2-P3-P4, GHES-(P2-P3) + P4-8 (19), G-TAT-HA2-A, GHFF-P2-P3-A and GHLT-P2-P3-A (see **Table 7** for sequences).

Dulbecco's phosphate-buffered saline (**14190-144 Gibco**) was then added to each formulation to reach a transduction mixture volume of 15 µL. Cas9, gRNA and peptide concentrations were calculated in a final reaction volume of 65 µL which accounts for the transduction mixture volume (15 µL) and 50 µL of cell culture per well. The transduction mixture was incubated for an additional 10 min at room temperature, added to cells in V-bottom 96-well plate (**651201 Greiner BIO-ONE**) and mixed by pipetting up and down. The plates were incubated at 37°C with 5% CO₂ for 45 mins. The reaction was stopped by adding 135 µL of medium without supplements, plates were centrifuged at 400xg for 3 minutes and supernatant was discarded. Cells were then resuspended in 200 µL of medium with supplements and incubated at 37°C with 5% CO₂ until editing analysis was performed.

### DNA Extraction and PCR

3 days after transduction, genomic DNA was extracted using the QuickExtract DNA Solution (**QE09050 Lucigen**). Plates were centrifuged for 3 minutes at 400 xg, medium was discarded, and pellet resuspended in 50 µL QuickExtract per well. The plate was then incubated according to the manufacturer's instructions. Q5^{®} High-Fidelity 2X Master Mix (**M0515 NEB**) was used to amplify the edited region in the B2M gene using primers 258_HUM_B2M-5_Fw and 261_HUM_B2M-10_Rv (see

**Table 9**). The PCR products were purified using QIAquick 96 PCR Purification Kit (**28181 QIAGEN**) following manufacturer's protocol.

### Editing Analysis

Purified DNA fragments (15 ng/100 bp) were mixed with 25 pmol of sequencing primer 22_HUM-B2M-PCR-Fw (see **Table 9**) and were sent for sanger sequencing (**BaseClear**). Insertion-deletion (Indel) frequency results were evaluated by TIDE version 3.3.0 (Brinkman et al., 2014) with an alignment window of 200 bp, decomposition window of 270-580 bp, indel size range of 15 bp and P-value threshold of 0.001.

### Results

Figures 7a-7f shows the Indel frequency in the human B2M gene when using TAT-HA2, improved TAT-HA2 (G-TAT-HA2-A), GHES domains (P2-P3, P2-P3-P4, (P2-P3) + P4-8 (19)) and GHES CPP variants (GHFF-P2-P3-A and GHLT-P2-P3-A) in different cell types (see Table 7 for peptide sequences).

In Figures 7a-7e, peptide G-TAT-HA2-A improved editing efficiencies compared to TAT-HA2 control from 16 to 51%, 66 to 73%, 37 to 55%, 41 to 68% and 18 to 32% in Jurkat, CD4+, NK, CD8+ and hIPSCs cells, respectively. In figures 7a, 7b, 7c, 7e and 7f, peptide P2-P3-P4 resulted in the highest editing efficiencies of 92, 87, 59, 46 and 96% compared to all the tested peptides per cell type. In Figures 7a-7e, peptide variants GHFF-P2-P3-A and GHLT-P2-P3-A were functional and effective in all cell types tested. GHFF-P2-P3-A and GHLT-P2-P3-A peptides resulted in indel frequencies ranging from 20 to 85% and 33 to 85%, respectively, depending on the cell type tested.

Figure 7f shows indel frequencies of CD34+ hSC (Cord blood and Bone marrow) when transduced with different concentrations of TAT-HA2, GHES-P2-P3, and GHES-P2-P3-P4 peptides. Peptide GHES-P2-P3-P4 at 50 µM and 100 µM peptide achieved 82% and 96% indel frequencies in CD34+ hSC cells, respectively. Figure 7 thus shows that the tested CPPs are effective across various cell types.

### Example 6: In Vivo Transduction Efficacy of TAT-HA2 and GHES Peptides

### Mouse Model

In this study, 2-3 months old Ai9 mouse model (Mus musculus) with Specific Pathogen Free (SPF) health status were bred, housed and treatment was applied at InnoSer België NV. The study was performed according to the standard operating procedures and methods described by ISO 9001:2015 and the Association for Assessment and Accreditation of Laboratory Animal Care (AAALAC). Animals were maintained at a range temperature between 20-24°C with 12/12 light/dark cycle and air renewal of 8-20 times per hour. Animals were anesthetized using isoflurane gas anesthesia, eye gel was applied to eyes, and they were placed on a heating pad. The hair at the hind legs was removed using a clipper and skin at the tibialis anterior (TA) muscle was cleaned and disinfected using chlorhexidine and ethanol. Animals were dosed once by single intramuscular injection in both legs (TA muscle) with 50 µL of respective formulations (2 animals or 4 legs per formulation) or PBS (2 animals or 4 legs) using a 29G needle and an insulin syringe. The animals were placed in a thermacage to recover and into a home cage after recovery. 2 weeks after transduction, animals were euthanized by cervical dislocation. After sacrifice, the left and right TA muscle were snap-frozen as a whole in separate cryopreservation tubes in liquid nitrogen and tissues were stored at -80 °C.

### Peptides, gRNAs, Primers and Recombinant Cas Protein Preparation

All peptides were purchased and custom synthetised at Genscript with purity greater than 98%. They were dissolved to a working concentration of 2mM in nuclease-free water (**Invitrogen, AM9937**) containing 10% DMSO (**D8418 Sigma-Aldrich**). The peptide sequences are specified in **Table 7.**

sgRNA was purchased from Integrated DNA Technologies as lyophilized Alt-R^{®} CRISPR/Cas9 products which were then resuspended in nuclease-free water (**Invitrogen, AM9937**). Guide RNAs were designed by using CHOPCHOP version 3. Guide RNA sequence is specified in **Table 8.**

All forward, reverse and sequencing primers were purchased from Integrated DNA Technologies as desalted custom synthesis and were then resuspended in nuclease-free water (**Invitrogen, AM9937**) to a final working concentration of 100 µM. The primer sequences are specified in **Table 9.**

His-tagged recombinant Cas9 protein was produced as described in by D'Astolfo et.,al, Cell, 2015, 161(3), 674-690, (see recombinant protein purification section of "Examples: Additional Information", for a detailed description). Recombinant Cas9 protein was stored in buffer containing 250 mM GABA, 75 mM Glycine, 25 mM phosphate buffer pH 8.0, 500 mM NaCl, 1.25 mM MgCl2, 150 mM glycerol and 2 mM 2-ME at a final working concentration of 75 µM.

### Formulation

Recombinant Cas9 protein and single gRNA targeting mouse B2M (Mus_B2M_sgRNA see **Table 9**) were mixed to a final concentration of 20 µM each in a final reaction volume of 50 µL. The resulting ribonucleoprotein (RNP) was then incubated for 10 mins at room temperature. Peptides (TAT-HA2 and GHES-P2-P3-P4 in **Table 7**) were added to their respective RNP mixtures to final concentrations of 0, 100, 200, 400 and 800 µM in a final reaction volume of 50 µL. Dulbecco's phosphate-buffered saline (**14190-144 Gibco**) was then added to each formulation to reach a final reaction volume of 50 µL and incubated for an additional 10 min at room temperature. Only PBS control was prepared with 50 µL of PBS solution. All formulations were snap-frozen in liquid nitrogen and shipped to InnoSer België NV. Prior to the injection procedure, the formulations were thawed and injected within 30 mins.

### DNA Extraction and PCR

The DNeasy blood and tissue kit (**69582 QIAGEN**) was used for this procedure according to the manufacturers protocol. Q5^{®} High-Fidelity 2X Master Mix (**M0515 NEB**) was used to amplify the edited region in the mouse B2M gene using primers 209_Mus_B2m_1_Fw and 216_Mus_B2m_4_Rv (see **Table 9**). The PCR products were purified using QIAquick 96 PCR Purification Kit (**28181 QIAGEN**) following manufacturer's protocol.

### Editing Analysis

Purified DNA fragments (15 ng/100 bp) were mixed with 25 pmol of sequencing primer 215_Mus_B2m_4_Fw (see **Table 9**) and were sent for sanger sequencing (**BaseClear**). Insertion-deletion (Indel) frequency results were evaluated by TIDE version 3.3.0 (Brinkman et al., 2014) with an alignment window of 200 bp, decomposition window of 270-580 bp, indel size range of 15 bp and P-value threshold of 0.001.

### Results

Figure 8 shows the indel frequencies in the mouse B2M gene. The mouse was injected in the tibialis anterior muscle with RNP and PBS only, as well as with increasing concentrations of GHES-P2-P3-P4 and TAT-HA2. Cas9 fused to 4 and 2 nuclear localization signals at the N or C-terminus of the protein shows that the RNP can be delivered without additional components in the formulation. At the lowest concentration tested of 100 µM, peptides GHES-P2-P3-P4 and TAT-HA2 achieved average indel frequencies of 17 and 13%, respectively. Peptide concentrations between 100 and 400 µM resulted in higher average indel frequencies compared to the control containing only RNP. Contrarily, the highest concentration tested of 800 µM peptide achieved the same indel frequency as obtained with the formulation containing only RNP.

### Example 7: Lentivirus Delivery

### Cell Culture

HEK293T cells were cultured in DMEM **(41965039 Gibco)** medium supplemented with 10% FBS **(F7524 Sigma-Aldrich)** and 1% Penicillin-Streptomycin **(15140-122 Gibco).** Prior transduction procedure, cells were seeded using Geltrex **(A1413301 Gibco)** for adherence and culture was kept at 70-80% confluency. Cells were cultured at 37°C with 5% CO₂ and tested for mycoplasma using the MycoAlert Mycoplasma detection kit **(LT07-418 Lonza)** according to manufacturer's instructions. Prior to transduction procedure, medium was aspirated and 50 µL of pre-warmed medium with supplement was added into each well.

### Peptides Preparation

The peptide GHES-P2-P3-P4 was purchased and custom synthetised at Genscript with purity greater than 98%. It was dissolved to a working concentration of 2mM in nuclease-free water (**Invitrogen, AM9937**) containing 10% DMSO (**D8418 Sigma-Aldrich**). The peptide sequence can be found in **Table 7.**

### Lentivirus Packaging Protocol

HEK 293T cells were transfected with FuGENE^{®} HD transfection reagent **(E2311 Promega)** with the packaging plasmids pHDM-Hgpm2 (Addgene Plasmid #164441), pHDM-Tat1b (Addgene Plasmid #164442), pRC-CMV-Rev1b (Addgene Plasmid #164443), pHDM.VSV.G (VSVG) (Addgene Plasmid #164440) (Garcia-Beltran et al., 2021) and a donor plasmid containing an eGFP reporter gene (Yang et al., 1996), according to the manufacturer's instructions. On days 3 and 4 after transfection, cultures were centrifuged at 500 x g for 5 min, supernatant was filtrated through 0.45 µm filters and stored at 4°C.

### Formulation and Transduction Procedure

Lentivirus was added to transduction mixtures to reach a final dilution of 1:10, 1:100 and 1:1000 lentivirus:cell culture (v/v). The peptide GHES-P2-P3-P4 (see **Table 7**) was added to the transduction mixture to final concentrations of 0, 50, 100 and 200 µM. Dulbecco's phosphate-buffered saline (**14190-144 Gibco**) was then added to each formulation to reach a transduction mixture volume of 15 µL. Lentivirus dilutions and peptide concentrations were calculated in a final reaction volume of 65 µL which accounts for the transduction mixture volume (15 µL) and 50 µL of cell culture per well. The transduction mixture was incubated for an additional 10 min at room temperature, added to 50 µL culture in 96-well plate **(655161 Greiner BIO-ONE)** and mixed by pipetting up and down. The plates were incubated at 37°C with 5% CO₂ for 60 mins. The reaction was stopped by adding 135 µL of medium with supplements and plate was incubated for 3 days at 37°C with 5% CO₂.

### Flow Cytometry

On day 3 cells were fixated with 0.8-1 % paraformaldehyde and green fluorescence was analysed using a flow cytometer. Samples were incubated in the dark on ice for 15 mins before analysis in the flow cytometer **(Guava easyCyte).** Data analysis was performed using Guava easyCyte software according to the manufacturer's instructions.

### Results

Figure 9 shows lentivirus transduction efficiencies in HEK293T cells as a percentage of positive green fluorescent cells. Lentivirus was added in ratios of 1:10, 1:100 and 1:1000 (v:v), with GHES-P2-P3-P4 peptide added at concentrations of 0, 50, 100 and 200 µM. Using a lentivirus:cell culture ratio of 1:1000, 1:100 and 1:10 without additional peptide resulted in 2%, 7%, and 16% of cells exhibiting positive green fluorescence, respectively. At the highest peptide concentration tested of 200µM, transduction efficiencies reached 24%, 39% and 32%, respectively. This experiment demonstrates that peptides such as GHES-P2-P3-P4 derived from a glycoside hydrolase protein loop domain can be used as an enhancer in lentivirus transduction procedures.

### Examples: Additional Information

**Table 7 provides the sequences for the peptides that are used in the Examples.**

| All peptides were purchased and custom synthetised at Genscript with purity greater than 98%. They were dissolved to a working concentration of 2mM in nuclease-free water (**Invitrogen, AM9937**) containing 10% DMSO (**D8418 Sigma-Aldrich**). | | |
|---|---|---|
| **Name** | **Sequence** | **SEQ ID NO** |
| TAT-HA2 | RRRQRRKKRGGDIMGEWGNEIFGAIAGFLG | 717 |
| GHES-P1-P2-P3 | SEMLLQTRGGPSRYGRGARGRRRQRRRRRLSGLGWASEAI AGFLPG | 718 |
| GHES-P2-P3 | GRRRQRRRRRLSGLGWASEAIAGFLPG | 583 |
| GHES-P2-P3-P4 | GRRRQRRRRRLSGLGWASEAIAGFLPGAIRVKSEDG | 662 |
| GHES-P1-P2-P3-P4 | SEMLLQTRGGPSRYGRGARGRRRQRRRRRLSGLGWASEAI AGFLPGAIRVKSEDG | 191 |
| GHES-(P2-P3) + P4-8 (19) | GRRRQRRRRRLSGLGWASEAIAGFLPGA | 719 |
| GHES-Disulfide cyclic C1-C30 (19) | CGRRRQRRRRRLSGLGWASEAIAGFLPGAC | 720 |
| GHES-Disulfide cyclic C1-C29 (19) | CGRRRQRRRRRLSGLGWASEAIAGFLPGCA | 721 |
| RI GHES-P2-P3-P4 | d-(GDESKVRIAGPLFGAIAESAWGLGSLRRRRRQRRRG) | 722 |
| GHES-P2-P3-P4 inverted | GDESKVRIAGPLFGAIAESAWGLGSLRRRRRQRRRG | 723 |
| G-TAT-HA2-A | GRRRQRRKKRGGDIMGEWGNEIFGAIAGFLGA | 724 |
| GHFF-P2-P3-A | GRPRTRKRKSRNRRGRRAPLLDWAEAVTGFLPGA | 725 |
| GHFF-P2-P3-P4 | GRPRTRKRKSRNRRGRRAPLLDWAEAVTGFLPGAIRVKND DG | 661 |
| GHLT-P2-P3-A | RQRQRQRRRRSPVPAALEWFSSLADVLPGA | 726 |
| GHLT-P2-P3-P4 | RQRQRQRRRRSPVPAALEWFSSLADVLPGAVRVKNEDG | 655 |
| GHFF-P2/GHLT-P3/GHES-P4 | GRPRTRKRKSRNRRGRRAPALEWFSSLADVLPGAIRVKSE DG | 727 |

**Table 8 provides the sequences for the crRNAs and tracrRNAs that are used in the Examples.**

| Although dual annealed guide RNAs are used in several of the above-described Examples, these experiments may also be performed using single guide RNAs. | | | |
|---|---|---|---|
| All crRNAs and tracrRNAs were purchased from Integrated DNA Technologies as lyophilized Alt-R^{®} CRISPR/Cas9 products which were then resuspended in nuclease-free water (**Invitrogen, AM9937**). crRNA and tracrRNA were mixed at equimolar concentrations to a final gRNA concentration of 100µM, heated at 95°C for 10 min, slowly cooled down to room temperature for 30 min and stored at -20°C. Guide RNAs were designed by using CHOPCHOP version 3 (Labun et al., 2019), Cas-Designer (Zetsche et al., 2015), BE-Designer (Hwang et al., 2018) and PE-Designer (Hwang et al., 2021). | | | |
| **Name** | **Sequence** | **Target** | **SEQ ID NO** |
| hB2M #7 | GAAGUUGACUUACUGAAGAA | Human B2M | 728 |
| hB2M gRNA 1 ABE | GGCCGAGAUGUCUCGCUCCG | Human B2M | 729 |
| hB2M gRNA 2 ABE | ACUCACGCUGGAUAGCCUCC | Human B2M | 730 |
| hB2M pegRNA 1 | GGCCGAGAUGUCUCGCUCCGGUUUUAGAGC UAGAAAUAGCAAGUUAAAAUAAGGCUAGUC CGUUAUCAACUUGAAAAAGUGGCACCGAGU CGGUGCGAGCACAGCUAAGGCGGUUAGCGA GACAUCUC | Human B2M | 731 |
| hB2M pegRNA 2 | GGCCGAGAUGUCUCGCUCCGGUUUUAGAGC UAGAAAUAGCAAGUUAAAAUAAGGCUAGUC CGUUAUCAACUUGAAAAAGUGGCACCGAGU CGGUGCACAGCUAAGGCGGUUAGCGAGACA UCUC | Human B2M | 732 |
| hB2M pegRNA 3 | GAGUAGCGCGAGCACAGCUAGUUUUAGAGC UAGAAAUAGCAAGUUAAAAUAAGGCUAGUC CGUUAUCAACUUGAAAAAG UGGCACCGAGUCGGUGCUCCGUGGCCUGAG CUGUGCUCGCGCUUUU | Human B2M | 733 |
| hB2M gRNA 1 Cas12a | UUUCUGGCCUGGAGGCUAUCCAGCGUGA | Human B2M | 734 |
| hB2M gRNA 2 Cas12a | UUUCCCGAUAUUCCUCAGGUACUCCAAA | Human B2M | 735 |
| hB2M gRNA 3 Cas12a | UUUCCAUUCUCUGCUGGAUGACGU | Human B2M | 736 |
| hB2M gRNA 4 Cas12a | UUUCAAUUCUCUCUCCAUUCUUCA | Human B2M | 737 |
| Hs.Cas9.CTLA4.1.AA | GUGCGGCAACCUACAUGAUG | Human CTLA-4 | 738 |
| CTLA-4 gRNA 1 ABE | AAGCCAUGGCUUUAUGGGAG | Human CTLA-4 | 739 |
| CTLA-4 gRNA 2 ABE | CACUCACCUUUGCAGAAGAC | Human CTLA-4 | 740 |
| Hs.Cas9.PDCD1.1.AN | GUCUGGGCGGUGCUACAACU | Human PD-1 | 741 |
| PD-1 gRNA 1 ABE | UCCAGGCAUGCAGAUCCCAC | Human PD-1 | 742 |
| PD-1 gRNA 2 ABE | CACCUACCUAAGAACCAUCC | Human PD-1 | 743 |
| hCD33_gRNA_ABE2 | CACUCACCUGCCCACAGCAG | Human CD33 | 744 |
| Mus_B2M_sgRNA | CUGGUGCUUGUCUCACUGAC | Mouse B2M | 745 |

**Table 9 provides the sequences for the primers that are used in the Examples.**

| All forward, reverse and sequencing primers were purchased from Integrated DNA Technologies as desalted custom synthesis and were then resuspended in nuclease-free water (**Invitrogen, AM9937**) to a final working concentration of 100 µM. | | | |
|---|---|---|---|
| **Name** | **Primer sequence (5' to 3')** | **Use** | **SEQ ID NO** |
| 258_HUM_B2M-5_Fw | AGGCTCTTAAAAATGCAGCG | Forward Primer | 746 |
| 261_HUM_B2M-10_Rv | GGTTCAGAAACCATGCTGTG | Reverse primer | 747 |
| 22_HUM-B2M-PCR-Fw | GAGGTGGCTTGTTGGGAAGG | Sequencing | 748 |
| hB2M Fw1 ABE | CAGCCTGAAGTCCTAGAATGAG | Forward Primer | 749 |
| hB2M Rv1 ABE | CACCAAGGAGAACTTGGAGAA | Reverse primer | 750 |
| hB2M Fw3 ABE | CCGATGTACAGACAGCAAACTCACC | Sequencing | 751 |
| hCTLA4_FW1 (AC) | GGTAAGTGATAGATTCGTTGAAGG | Forward Primer | 752 |
| hCTLA4_RV1 (AC) | GCCACCCACAATAAGCAAGG | Reverse primer | 753 |
| hCTLA4_FW3 (AC) | GAAAAGGCCGTGGGGATGAA | Sequencing | 754 |
| CTLA-4 Fw 1 ABE | AGGATGCCCAGAAGATTGAATAA | Forward Primer | 755 |
| CTLA -4 Rv 1 ABE | AGACTGGTGGCTTTCTTTCTT | Reverse primer | 756 |
| CTLA-4 Fw2 ABE | GGCTTGTTTTGTTCAGTTGAGTG | Sequencing | 757 |
| hPD-1_FW2 (AC) | CACTGCCTCTGTCACTCTCG | Forward Primer | 758 |
| hPD-1_RV2 (AC) | CGTAAAGCCAAGGTTAGTCCCAC | Reverse primer | 759 |
| hPD-1_RV3 (AC) | GTGAGGACCAAGGATGCCTG | Sequencing | 760 |
| PD-1 Fw2 ABE | CACTGCCTCTGTCACTCTCG | Forward and sequencing primer | 761 |
| PD-1 Rv2 ABE | CGTAAAGCCAAGGTTAGTCCCAC | Reverse primer | 762 |
| PD-1 Fw 4 ABE | TTTCAGGAGGTCCTTGTCTTG | Forward Primer | 763 |
| PD-1 Rv 4 ABE | TTCCAGAGCTAGAGGACAGAG | Reverse primer | 764 |
| PD-1 Fw 5 ABE | TTCCTCACCTCTCTCCATCTC | Forward and sequencing primer | 765 |
| hCD33_Fw1.3 | GACTCTTCTTTGTTTCTCTGCCC | Forward and sequencing primer | 766 |
| hCD33_Rv1.3 | CATTATCCCTCCTCCTGGCG | Reverse primer | 767 |
| 209_Mus_B2m_1_Fw | CCTGCTAGAAGCAAGGTCAG | Forward primer | 768 |
| 216_Mus_B2m_4_Rv | TTTCTCTCGACTTCGGTTGG | Reverse primer | 769 |
| 215_Mus_B2m_4_Fw | GCTGAACGACCAGATACACC | Sequencing | 770 |

### Recombinant Protein Purification

Although recombinant proteins are used in the above-described examples, commercially available gene editing proteins may also be used, in the Examples or in any of the methods of the invention (e.g., Cas9 from Aldevron).

His-tagged recombinant proteins (Cas9, Cas12a, Prime editor and base editor ABE) were produced as described in by D'Astolfo et.,al, Cell, 2015, 161(3), 674-690. Cell lysate containing the recombinant protein was loaded onto a 5 mL HisTrap HP column (**17-5248-01 Cytiva**) by the AKTA pure protein purification system (**Cytiva**). The protein was eluted from the column using buffer A (300 mM Trehalose dihydrate, 50 mM Tris-HCl pH 8.0, 1 M NaCl, 10% glycerol and 5 mM 2-ME) and buffer B (300 mM Trehalose dihydrate, 50 mM Tris-HCl pH 8.0, 1 M NaCl, 500 mM imidazole pH 8.0, 10% glycerol and 5 mM 2-ME). Elution gradient was performed by increasing composition of buffer B from 0% to 100% in 45 mins with a system flow of 3 mL/min and fractionation of 1 mL/tube. Fractions containing the recombinant protein were collected, pooled and buffer exchanged in a 30 mL dialysis cassette 20K MWCO (**A52978 ThermoFisher Scientific**) with dialysis buffer (300 mM Trehalose dihydrate, 20 mM HEPES-KOH pH 8.0, 1 mM EDTA, 250 mM KCI, 10% glycerol and 2 mM 2-ME). Dialysis was performed overnight at 4°C in 4 liters of dialysis buffer with slow stirring. After dialysis, sample was mixed with cold sample dilution buffer (300 mM Trehalose dihydrate, 20 mM HEPES-KOH pH 8.0, 50 mM KCI, 10% glycerol and 2 mM 2-ME) and loaded onto a 5 mL HiTrap Heparin HP column (**17-0407-01 Cytiva**) by the AKTA pure protein purification system (**Cytiva**). The protein was eluted from the column using IEX buffer A (300 mM Trehalose dihydrate, 20 mM HEPES-KOH pH 8.0, 150 mM KCI, 10% glycerol and 2 mM 2-ME) and IEX buffer B (300 mM Trehalose dihydrate, 20 mM HEPES-KOH pH 8.0, 2 mM KCI, 10% glycerol and 2 mM 2-ME). Elution gradient was performed by increasing composition of buffer B from 0% to 50% in 30 mins with a system flow of 2 mL/min and fractionation of 1 mL/tube. Fractions containing the recombinant protein were collected, pooled and loaded onto a GE HiLoad 26/200 Superdex 200 pg gel filtration column (**28-9893-36 Cytiva**) and eluted in 4 ml fractions by the AKTA pure protein purification system (**Cytiva**). Samples were eluted in destination buffer (250 mM GABA, 75 mM Glycine, 25 mM phosphate buffer pH 8.0, 500 mM NaCl, 1.25 mM MgCl2, 150 mM glycerol and 2 mM 2-ME) and concentrated to a final working concentration of 75 µM using Amicon Ultra-15 Centrifugal Filter Units **(UFC910024 Millipore)** according to the manufacturer's instructions.

## Claims

1. A cell penetrating peptide (CPP) for transporting cargo across a cell membrane,
wherein the amino acid sequence of the CPP comprises a 14 to 142 contiguous amino acid portion having at least 80% sequence identity to a same-length portion within a protein loop domain of a fungal glycoside hydrolase,
wherein the protein loop domain of the fungal glycoside hydrolase is found between an N-terminal flagging sequence in the glycoside hydrolase and a C-terminal flagging sequence in the glycoside hydrolase,
wherein the N-terminal flagging sequence is XWX₁XX₂X₃X₄X₅XXXXX₆X₇GX₈PX₉YX₁₀YXX₁₁XX₁₂XXXXLXXX₁₃ (SEQ ID NO: 7),
wherein:
X₁ is T, S, V or A;
X₂ is A or S;
X₃ is G or H;
X₄ is F or V;
X₅ is P, A, D or S;
X₆ is V, M, I or A;
X₇ is L, I or M;
X₈ is V, L or I;
X₉ is S or A;
X₁₀ is G or A;
X₁₁ is S, A, N, Q or V;
X₁₂ is S or T; and
X₁₃ is R or K,
wherein the C-terminal flagging sequence is XXXXX₁XX₂X₃X₄X(g)XXX₅X₆X₇QFX₈XX₉X₁₀XZX₁₂,
wherein:
X₁ is N, S, G, D or E;
X₂ is D, -, G, A or E;
X₃ is G, -, S or A;
X₄ is G, -, T, S, P, K or H;
(g) is a gap of up to 6 amino acids;
X₅ is G, - or N;
X₆ is Q, T or G;
X₇ is V or I;
X₈ is R, N or S;
X₉ is L, I or M;
X₁₀ is V, I, L, A or F; and
X₁₂ is G, A or K, and
wherein the CPP is capable of providing gene editing in a cell when covalently or non-covalently coupled to cargo for CRISPR/Cas9 gene editing.

2. The CPP of claim 1, wherein:
a) the N-terminal flagging sequence is XWTXAGFPXXXXVLGVPSYGYXSXSXXXXLXXR (SEQ ID NO: 4) and the C-terminal flagging sequence is XXXXNXDGGX (SEQ ID NO: 5) (g) XXGQVQFRXLVXQG (SEQ ID NO: 6), wherein (g) is a gap of up to 6 amino acids; or
b) the N-terminal flagging sequence is AWTXAGFPASQLVLGVPSYGYISRSXAXXLRXR (SEQ ID NO: 1) and the C-terminal flagging sequence is XXXXNEDGGT (SEQ ID NO 2) (g) XXGQVQFRXLVXQG (SEQ ID NO: 3), wherein (g) is a gap of up to 6 amino acids.

3. The CPP of claim 1 or claim 2, wherein:
a) the contiguous amino acid portion of the CPP is 33 to 72 amino acids in length or 24 to 59 amino acids in length; and/or
b) the protein loop domain of the fungal glycoside hydrolase consists of the amino acid sequence of any one of SEQ ID NOs: 166-244 or consists of the amino acid sequence of any one of SEQ ID NOs 184, 190, and 191.

4. A CPP for transporting cargo across a cell membrane, wherein the amino acid sequence of the CPP comprises a contiguous amino acid portion having at least 80% sequence identity to the amino acid sequence of any one of SEQ ID NOs: 558-636, and wherein the CPP is capable of providing gene editing in a cell when covalently or non-covalently coupled to cargo for CRISPR/Cas9 gene editing.

5. The CPP of claim 4, wherein the amino acid sequence of the CPP comprises a contiguous amino acid portion having at least 80% sequence identity to the amino acid sequence of any one of SEQ ID NOs: 637-715.

6. The CPP of any one of claims 1-5, wherein:
a) the contiguous amino acid portion has at least 90% or 100% sequence identity; and/or
b) the CPP consists of the contiguous amino acid portion.

7. A CPP for transporting cargo across a cell membrane, wherein the amino acid sequence of the CPP comprises a P2-P3 region of 27 contiguous amino acids that is at least 60% identical to the amino acid sequence of SEQ ID NO: 583, and wherein the CPP is capable of providing gene editing in a cell when covalently or non-covalently coupled to cargo for CRISPR/Cas9 gene editing.

8. The CPP of claim 7, wherein:
a) the P2-P3 region is at least 65%, at least 70%, at least 75%, at least 80%, at least 85%, at least 90%, at least 95%, or 100% identical to the amino acid sequence of SEQ ID NO: 583; and/or
b) the amino acid sequence of the CPP further comprises a P4 region C-terminal to the P2-P3 region that has amino acid 1, amino acids 1 and 2, amino acids 1-3, amino acids 1-4, amino acids 1-5, amino acids 1-6, amino acids 1-7, amino acids 1-8, or amino acids 1-9 of SEQ ID NO: 504, optionally wherein the P4 region has amino acid 1 of SEQ ID NO: 504; and/or
c) the amino acid sequence of the CPP further comprises a P1 region N-terminal to the P2-P3 region that has amino acid 19, amino acids 18 and 19, amino acids 17-19, amino acids 16-19, amino acids 15-19, amino acids 14-19, amino acids 13-19, amino acids 12-19, amino acids 11-19, amino acids 10-19, amino acids 9-19, amino acids 8-19, amino acids 7-19, amino acids 6-19, amino acids 5-19, amino acids 4-19, amino acids 3-19, amino acids 2-19, or amino acids 1-19 of SEQ ID NO: 270; and/or
d) the amino acid sequence of the CPP further comprises a P4 region C-terminal to the P2-P3 region that has the amino acid sequence of SEQ ID NO: 504 and a P1 region N-terminal to the P2-P3 region that has amino acids 10-19 of SEQ ID NO: 270.

9. The CPP of any one of claims 1-8, wherein:
a) the CPP is modified to enhance one or more of stability, protease resistance, bioavailability, and pharmacokinetics, optionally wherein the modification comprises one or more of:
i. incorporation of one or more D-amino acids,
ii. N-terminal acetylation,
iii. C-terminal amidation,
iv. incorporation of one or more non-natural or synthetic amino acids, and/or
v. conjugation to a lipid moiety; and/or
b) the CPP is a retro-inverso peptide; and/or
c) the CPP is cyclic or partially cyclic, optionally wherein the CPP is cyclised via a head-to-tail peptide bond, a side chain-to-side chain bond, a head-to-side chain bond, or hydrocarbon stapling, further optionally wherein the CPP is cyclised through a disulfide bond, an amide bond, and/or lactam bridge formation.

10. A pharmaceutical composition comprising a CPP of any one of claims 1-9 and one or more pharmaceutically acceptable carriers, diluents, and/or excipients.

11. A method for transporting cargo across a cell membrane into a cell using a CPP of any of claims 1-9 or a pharmaceutical composition of claim 10, wherein the method comprises the step of contacting the cell with the CPP and the cargo, and wherein the CPP mediates the transport of the cargo across the cell membrane.

12. A CPP of any one of claims 1-9 or a pharmaceutical composition of claim 10 for use in therapy, wherein the CPP transports cargo across a cell membrane into the cells of a subject, wherein the cargo has a therapeutic effect on the cells thereby treating, preventing, or ameliorating a disease or disorder in the subject.

13. A CPP of any one of claims 1-9 or a pharmaceutical composition of claim 10 for use in a method of diagnosis, wherein the CPP transports diagnostic cargo across a cell membrane into the cells of a subject, wherein the diagnostic cargo is for the purpose of imaging, cell labelling, and/or cell tracking.

14. The method of claim 11, or the CPP or pharmaceutical composition for use according to claim 12 or claim 13, wherein the cargo comprises one or more nucleic acids, proteins or peptides, small molecules, nanoparticles or viral vectors, optionally wherein the cargo comprises gene editing components or a gene editing system, further optionally wherein the cargo comprises components for CRISPR/cas9 gene editing or a CRISPR/cas9 gene editing system.

15. The method of claim 11 or claim 14, or the CPP or pharmaceutical composition for use according to any one of claims 12-14, wherein the CPP is used to transport cargo across a cell membrane into one or more cell types selected from the group consisting of:
a) T cells, optionally CD8+ T cells or CD4+ T cells;
b) Natural killer cells;
c) human induced pluripotent stem cells; and
d) Hematopoietic Stem Cells.
